# EUROPEAN PATENT SPECIFICATION

(11) **EP 2 450 376 B1**
(45) Date of publication and mention of the grant of the patent: **28.02.2018**
(21) Application number: 12153009.1
(22) Date of filing: 06.09.2007
(51) Int. Cl.: C07K 16/10, A61K 39/145, C12N 5/10, C12N 15/09, A61K 39/00

(54) **Human binding molecules capable of neutralizing influenza virus H5N1 and uses thereof**
Zur Neutralisierung des Influenzavirus H5N1 fähige humane Bindungsmoleküle sowie deren Verwendungen
Molécules de liaison humaines capables de neutraliser le virus de la grippe H5N1 et utilisations associées

(30) Priority: 07.09.2006 US 842930 P; 07.09.2006 EP 06120316; 14.09.2006 EP 06120644; 30.11.2006 EP 06125107; 28.06.2007 EP 07111235
(43) Date of publication of application: 09.05.2012
(62) Divisional of application: 07803310.7
(73) Proprietor: Janssen Vaccines & Prevention B.V., 2333 CN Leiden (NL)
(72) Inventor: Van Den Brink, Edward Norbert, 2333 CN Leiden (NL); De Kruif, Cornelis Adriaan, 2333 CN Leiden (NL); Throsby, Mark, 2333 CN Leiden (NL)
(74) Representative: Manten, Annemieke

(56) References cited:
- US-A- 4 625 015
- US-A- 5 631 350
- OKUNO Y ET AL: "A COMMON NEUTRALIZING EPITOPE CONSERVED BETWEEN THE HEMAGGLUTIININS OF INFLUENZA A VIRUS H1 AND H2 STRAINS", JOURNAL OF VIROLOGY, NEW YORK, US, US, vol. 67, no. 5, May 1993 (1993-05), pages 2552-2558, XP001005475, ISSN: 0022-538X
- SMIRNOV Y A ET AL: "An epitope shared by the hemagglutinins of H1, H2, H5, and H6 subtypes of influenza A virus", ACTA VIROLOGICA, vol. 43, no. 4, August 1999 (1999-08), pages 237-244, XP009095964, ISSN: 0001-723X
- HORIMOTO TAISUKE ET AL: "Antigenic differences between H5N1 human influenza viruses isolated in 1997 and 2003", JOURNAL OF VETERINARY MEDICAL SCIENCE, vol. 66, no. 3, March 2004 (2004-03), pages 303-305, XP002469048, ISSN: 0916-7250
- THROSBY MARK ET AL: "Heterosubtypic neutralizing monoclonal antibodies cross-protective against H5N1 and H1N1 recovered from human IgM+ memory B cells", PLOS ONE, PUBLIC LIBRARY OF SCIENCE, vol. 3, no. 12, 1 January 2008 (2008-01-01), pages E3942.1-E3942.15, XP002615561, ISSN: 1932-6203, DOI: 10.1371/JOURNAL.PONE.0003942
- EKIERT DAMIAN C ET AL: "Antibody Recognition of a Highly Conserved Influenza Virus Epitope", SCIENCE, AMERICAN ASSOCIATION FOR THE ADVANCEMENT OF SCIENCE, WASHINGTON, DC; US, vol. 324, no. 5924, 1 April 2009 (2009-04-01), pages 246-251, XP009144786, ISSN: 0036-8075
- RUDIKOFF S ET AL: "Single amino acid substitution altering antigen-binding specificity", PROCEEDINGS OF THE NATIONAL ACADEMY OF SCIENCES OF USA, NATIONAL ACADEMY OF SCIENCE, WASHINGTON, DC; US, vol. 79, 1 March 1982 (1982-03-01), pages 1979-1983, XP007901436, ISSN: 0027-8424, DOI: 10.1073/PNAS.79.6.1979
- LERNER RICHARD A: "Rare antibodies from combinatorial libraries suggests an SOS component of the human immunological repertoire", MOLECULAR BIOSYSTEMS, ROYAL SOCIETY OF CHEMISTRY, GB, vol. 7, no. 4, 1 January 2011 (2011-01-01) , pages 1004-1012, XP009154610, ISSN: 1742-206X, DOI: 10.1039/C0MB00310G [retrieved on 2011-02-04]
- N. OHSHIMA ET AL: "Naturally Occurring Antibodies in Humans Can Neutralize a Variety of Influenza Virus Strains, Including H3, H1, H2, and H5", JOURNAL OF VIROLOGY, vol. 85, no. 21, 1 November 2011 (2011-11-01), pages 11048-11057, XP055014225, ISSN: 0022-538X, DOI: 10.1128/JVI.05397-11
- W. POS ET AL: "VH1-69 germline encoded antibodies directed towards ADAMTS13 in patients with acquired thrombotic thrombocytopenic purpura", JOURNAL OF THROMBOSIS AND HAEMOSTASIS, vol. 7, no. 3, 1 March 2009 (2009-03-01), pages 421-428, XP055014098, ISSN: 1538-7933, DOI: 10.1111/j.1538-7836.2008.03250.x
- SAKABE S ET AL: "A cross-reactive neutralizing monoclonal antibody protects mice from H5N1 and pandemic (H1N1) 2009 virus infection", ANTIVIRAL RESEARCH, ELSEVIER BV, NL, vol. 88, no. 3, 1 December 2010 (2010-12-01), pages 249-255, XP027509935, ISSN: 0166-3542, DOI: 10.1016/J.ANTIVIRAL.2010.09.007 [retrieved on 2010-11-20]
- G. S. TAN ET AL: "A Pan-H1 Anti-Hemagglutinin Monoclonal Antibody with Potent Broad-Spectrum Efficacy In Vivo", JOURNAL OF VIROLOGY, vol. 86, no. 11, 1 June 2012 (2012-06-01), pages 6179-6188, XP055067954, ISSN: 0022-538X, DOI: 10.1128/JVI.00469-12
- DREYFUS CYRILLE ET AL: "Structure of a classical broadly neutralizing stem antibody in complex with a pandemic h2 influenza virus hemagglutinin", JOURNAL OF VIROLOGY, AMERICAN SOCIETY FOR MICROBIOLOGY, US, vol. 87, no. 12, 1 June 2013 (2013-06-01), pages 7149-7154, XP009170571, ISSN: 1098-5514, DOI: 10.1128/JVI.02975-12

## Description

### FIELD OF THE INVENTION

The invention relates to medicine. In particular, the invention relates to the diagnosis, prophylaxis and/or treatment of an infection by influenza virus H5N1.

### BACKGROUND OF THE INVENTION

Influenza viruses consist of three types, A, B and C. Influenza A viruses infect a wide variety of birds and mammals, including humans, horses, marine mammals, pigs, ferrets, and chickens. In animals most influenza A viruses cause mild localized infections of the respiratory and intestinal tract. However, highly pathogenic influenza A strains such as H5N1 exist that cause systemic infections in poultry in which mortality may reach 100%. Animals infected with influenza A often act as a reservoir for the influenza viruses and certain subtypes have been shown to cross the species barrier to humans.

Influenza A viruses can be classified into subtypes based on allelic variations in antigenic regions of two genes that encode surface glycoproteins, namely, hemagglutinin (HA) and neuraminidase (NA) which are required for viral attachment and cellular release. Other major viral proteins include the nucleoprotein, the nucleocapsid structural protein, membrane proteins (M1 and M2), polymerases (PA, PB and PB2) and non-structural proteins (NS1 and NS2).

Currently, sixteen subtypes of HA (H1-H16) and nine NA (N1-N9) antigenic variants are known in influenza A virus. Previously, only three subtypes have been known to circulate in humans (H1N1, H1N2, and H3N2). However, in recent years, the pathogenic H5N1 subtype of avian influenza A has been reported to cross the species barrier and infect humans as documented in Hong Kong in 1997 and 2003, leading to the death of several patients.

In humans, the avian influenza virus infects cells of the respiratory tract as well as the intestinal tract, liver, spleen, kidneys and other organs. Symptoms of avian influenza infection include fever, respiratory difficulties including shortness of breath and cough, lymphopenia, diarrhea and difficulties regulating blood sugar levels. In contrast to seasonal influenza the group most at risk are healthy adults which make up the bulk of the population.Due to the high pathogenicity of certain avian influenza A subtypes, particularly H5N1, and their demonstrated ability to cross over to infect humans, there is a significant economic and public health risk associated with these viral strains, including a real epidemic and pandemic threat. The scale of the threat is illustrated by the 1918 influenza pandemic which killed over 50 million people.

Currently, no effective vaccines for H5N1 infection are available, so passive immunotherapy with immunoglobulins may be an alternative strategy. Use of passive immunization during the 1918 pandemic reportedly halved the death rate. In view of their therapeutic benefit in humans, there is thus a need for binding molecules, preferably human binding molecules, capable of neutralizing H5N1. The present invention provides these binding molecules and shows that they can be used in medicine, in particular for diagnosis, prevention and/or treatment of H5N1 infections.

### DESCRIPTION OF THE FIGURES

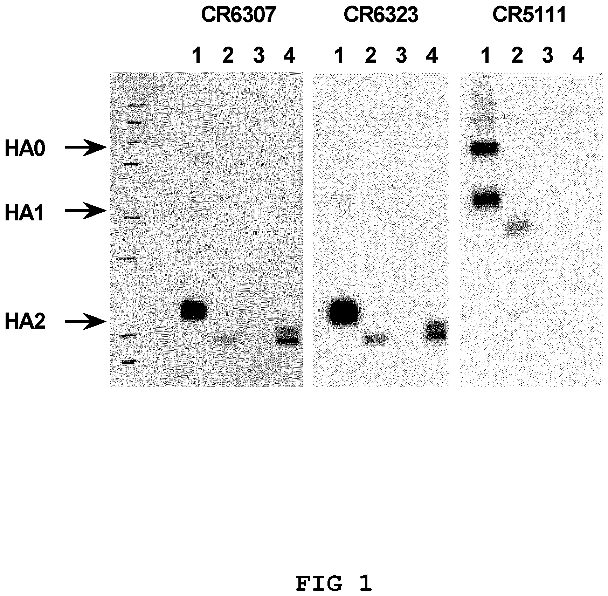

In Fig 1 immunoblot analysis of different hemagglutinins (HAs) using antibodies CR6307 (left part), CR6323 (middle part) and CR5111 (right part) is presented. Recombinant HAs were subjected to reducing SDS-PAGE analysis and immunoblot analysis. In lanes 1 sHA of H5N1TV is shown; in lanes 2 recombinant HA, subtype H5 (A/Vietnam/1203/2004 (H5N1)) is shown; in lanes 3 recombinant HA, subtype H3 (A/Wyoming/3/2003(H3N2)) is shown; and in lanes 4 recombinant HA, subtype H1 (A/New Caledonia/20/99 (H1N1)) is shown. The position where HA0, HA1 and HA2 can be found is also indicated.

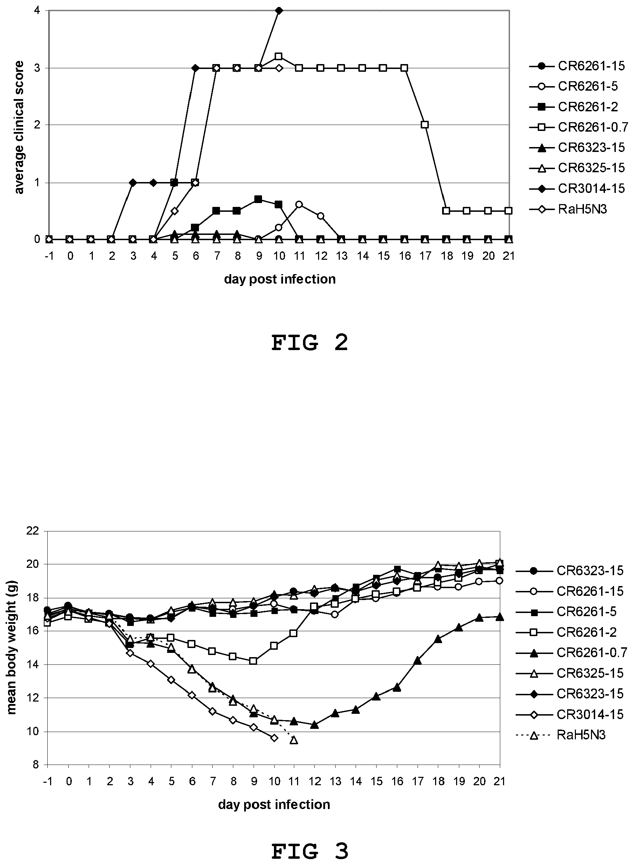

Fig 2 shows the average clinical score per group of mice in a study (example 12) wherein, one day before infection with influenza H5N1 virus, mice were prophylactically treated with three human H5N1 monoclonal antibodies CR6261, CR6323, and CR6325, in different doses.
Fig 3 shows the change in body weight during the prophylactic treatment of mice with anti-H5N1 antibodies during 21 days post-infection (example 12).

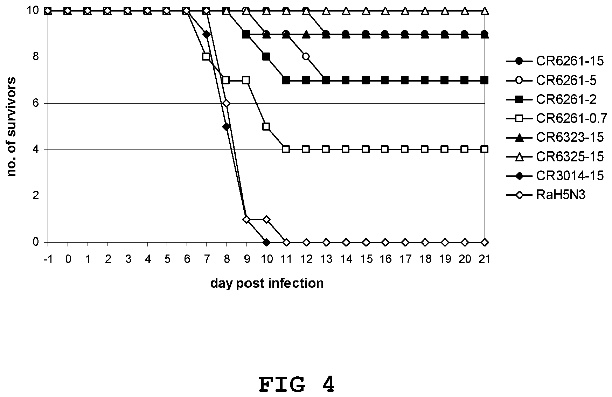

Fig 4 shows the number of surviving mice in the different groups in the study of Fig 2 and 3 (example 12).

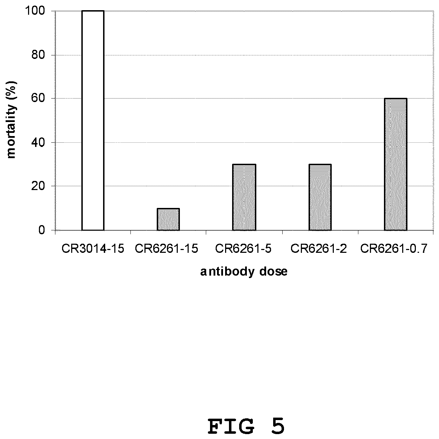

Fig 5 shows the mortality rate in relation to the dose given of the anti-H5N1 antibodies in the study of Figs 2-4 (example 12).

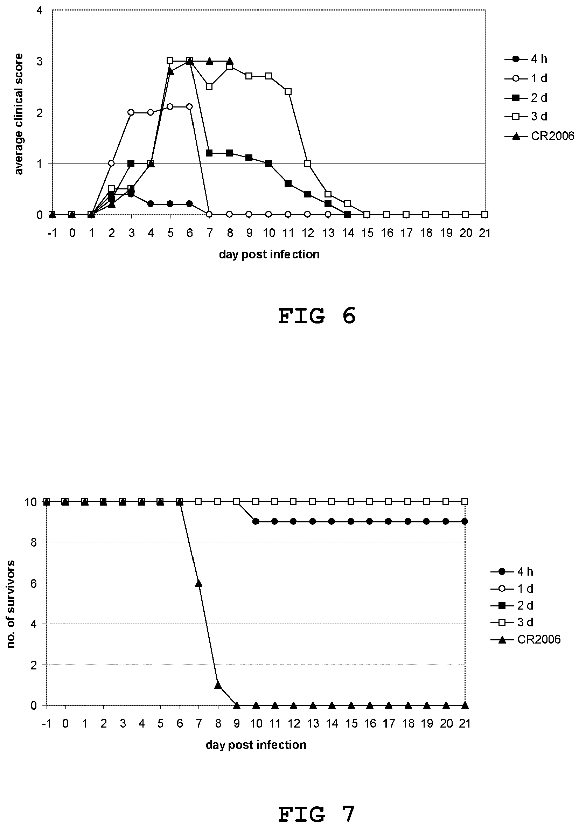

Fig 6 shows the average clinical score per group of mice in a study (example 13) wherein mice were infected with a lethal dose of H5N1 influenza virus and treated at different time points after infection (4 hr, 1, 2 and 3 days) with CR6261 anti-H5N1 monoclonal antibody, or a non-related antibody CR2006 (administered at day 1 post-infection).
Fig 7 shows the number of surviving animals in each group of the study described in fig 6. All animals of group 1-4, except for one animal in group 1 survived the entire study up to day 21 post-infection. All animals of group 5 had died at day 9.

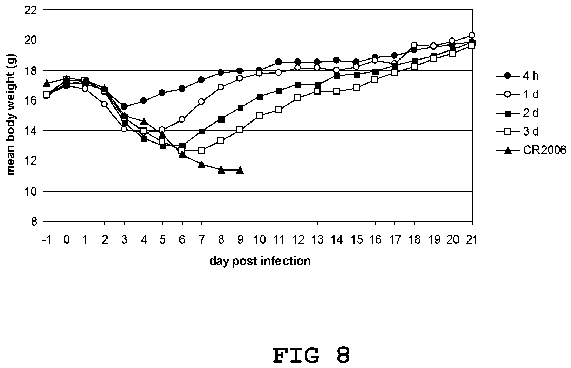

Fig 8 shows the average body weight of the mice in each group during the study as described for fig 6. Measuring the body weight of the mice in group 5 stopped at day 9 as all mice in that group had died by that time. All remaining mice in groups 1-4 reached normal levels of body weight at day 21 post-infection, although it depended on the time of treatment how fast each group recovered.

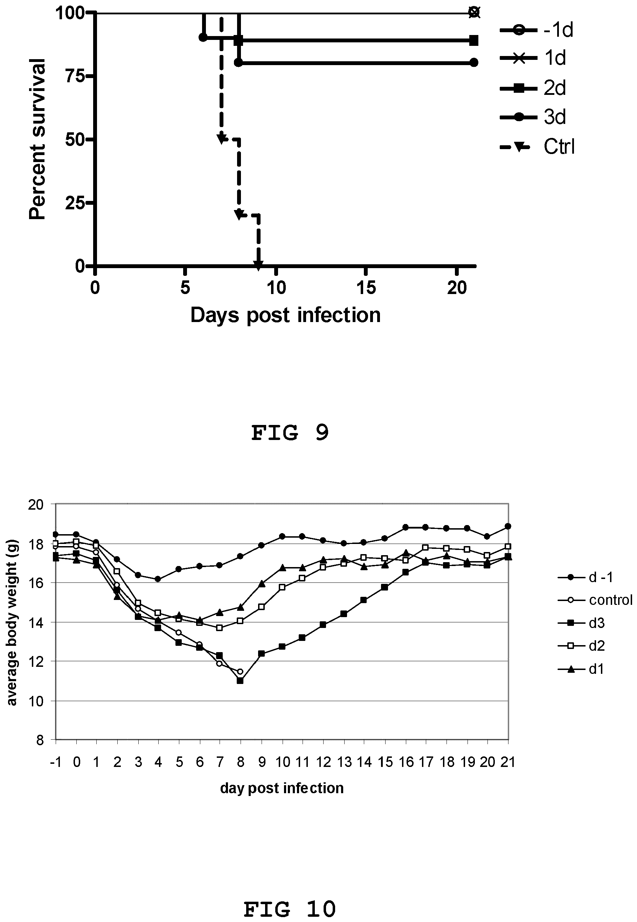

Fig 9 shows the percentage of surviving animals in each group of a study wherein mice were infected with a lethal dose of H1N1 influenza virus and treated at different time points (1 day prior-, 1, 2 and 3 days post-infection) with CR6261 anti-H5N1 monoclonal antibody, or a non-related antibody CR57 (administered at day 1 post-infection).
Fig 10 shows the average body weight of the mice in each group during the study as described for fig 9. Measuring the body weight of the mice in group 5 stopped at day 9 as all mice in that group had died by that time. All remaining mice in groups 1-4 reached normal levels of body weight at day 21 post-infection, although it depended on the time of treatment how fast each group recovered.

### DESCRIPTION OF THE INVENTION

Here below follow definitions of terms as used in the invention.

As used herein the term "binding molecule" refers to an intact immunoglobulin including monoclonal antibodies, such as chimeric, humanized or human monoclonal antibodies, or to an antigen-binding and/or variable domain comprising fragment of an immunoglobulin that competes with the intact immunoglobulin for specific binding to the binding partner of the immunoglobulin, *e.g.* H5N1. Regardless of structure, the antigen-binding fragment binds with the same antigen that is recognized by the intact immunoglobulin. An antigen-binding fragment can comprise a peptide or polypeptide comprising an amino acid sequence of at least 2, 5, 10, 15, 20, 25, 30, 35, 40, 50, 60, 70, 80, 90, 100, 125, 150, 175, 200, or 250 contiguous amino acid residues of the amino acid sequence of the binding molecule.

The term "binding molecule", as used herein includes all immunoglobulin classes and subclasses known in the art. Depending on the amino acid sequence of the constant domain of their heavy chains, binding molecules can be divided into the five major classes of intact antibodies: IgA, IgD, IgE, IgG, and IgM, and several of these may be further divided into subclasses (isotypes), *e.g.,* IgA1, IgA2, IgG1, IgG2, IgG3 and IgG4.

Antigen-binding fragments include, *inter alia,* Fab, F(ab'), F(ab')2, Fv, dAb, Fd, complementarity determining region (CDR) fragments, single-chain antibodies (scFv), bivalent single-chain antibodies, single-chain phage antibodies, diabodies, triabodies, tetrabodies, (poly)peptides that contain at least a fragment of an immunoglobulin that is sufficient to confer specific antigen binding to the (poly)peptide, etc. The above fragments may be produced synthetically or by enzymatic or chemical cleavage of intact immunoglobulins or they may be genetically engineered by recombinant DNA techniques. The methods of production are well known in the art and are described, for example, in Antibodies: A Laboratory Manual, Edited by: E. Harlow and D, Lane (1988), Cold Spring Harbor Laboratory, Cold Spring Harbor, New York. A binding molecule or antigen-binding fragment thereof may have one or more binding sites. If there is more than one binding site, the binding sites may be identical to one another or they may be different.

The binding molecule can be a naked or unconjugated binding molecule but can also be part of an immunoconjugate. A naked or unconjugated binding molecule is intended to refer to a binding molecule that is not conjugated, operatively linked or otherwise physically or functionally associated with an effector moiety or tag, such as *inter alia* a toxic substance, a radioactive substance, a liposome, an enzyme. It will be understood that naked or unconjugated binding molecules do not exclude binding molecules that have been stabilized, multimerized, humanized or in any other way manipulated, other than by the attachment of an effector moiety or tag. Accordingly, all post-translationally modified naked and unconjugated binding molecules are included herewith, including where the modifications are made in the natural binding molecule-producing cell environment, by a recombinant binding molecule-producing cell, and are introduced by the hand of man after initial binding molecule preparation. Of course, the term naked or unconjugated binding molecule does not exclude the ability of the binding molecule to form functional associations with effector cells and/or molecules after administration to the body, as some of such interactions are necessary in order to exert a biological effect. The lack of associated effector group or tag is therefore applied in definition to the naked or unconjugated binding molecule *in vitro,* not *in vivo.*

As used herein, the term "biological sample" encompasses a variety of sample types, including blood and other liquid samples of biological origin, solid tissue samples such as a biopsy specimen or tissue cultures, or cells derived there from and the progeny thereof. The term also includes samples that have been manipulated in any way after their procurement, such as by treatment with reagents, solubilization, or enrichment for certain components, such as proteins or polynucleotides. The term encompasses various kinds of clinical samples obtained from any species, and also includes cells in culture, cell supernatants and cell lysates.

The term "complementarity determining regions" (CDR) as used herein means sequences within the variable regions of binding molecules, such as immunoglobulins, that usually contribute to a large extent to the antigen binding site which is complementary in shape and charge distribution to the epitope recognized on the antigen. The CDR regions can be specific for linear epitopes, discontinuous epitopes, or conformational epitopes of proteins or protein fragments, either as present on the protein in its native conformation or, in some cases, as present on the proteins as denatured, *e.g.,* by solubilization in SDS. Epitopes may also consist of posttranslational modifications of proteins.

The term "deletion", as used herein, denotes a change in either amino acid or nucleotide sequence in which one or more amino acid or nucleotide residues, respectively, are absent as compared to the parent, often the naturally occurring, molecule.

The term "expression-regulating nucleic acid sequence" as used herein refers to polynucleotide sequences necessary for and/or affecting the expression of an operably linked coding sequence in a particular host organism. The expression-regulating nucleic acid sequences, such as *inter alia* appropriate transcription initiation, termination, promoter, enhancer sequences; repressor or activator sequences; efficient RNA processing signals such as splicing and polyadenylation signals; sequences that stabilize cytoplasmic mRNA; sequences that enhance translation efficiency (*e.g.,* ribosome binding sites); sequences that enhance protein stability; and when desired, sequences that enhance protein secretion, can be any nucleic acid sequence showing activity in the host organism of choice and can be derived from genes encoding proteins, which are either homologous or heterologous to the host organism. The identification and employment of expression-regulating sequences is routine to the person skilled in the art.

The term "functional variant", as used herein, refers to a binding molecule that comprises a nucleotide and/or amino acid sequence that is altered by one or more nucleotides and/or amino acids compared to the nucleotide and/or amino acid sequences of the parental binding molecule and that is still capable of competing for binding to the binding partner, *e.g.* H5N1, with the parental binding molecule. In other words, the modifications in the amino acid and/or nucleotide sequence of the parental binding molecule do not significantly affect or alter the binding characteristics of the binding molecule encoded by the nucleotide sequence or containing the amino acid sequence, *i.e.* the binding molecule is still able to recognize and bind its target. The functional variant may have conservative sequence modifications including nucleotide and amino acid substitutions, additions and deletions. These modifications can be introduced by standard techniques known in the art, such as site-directed mutagenesis and random PCR-mediated mutagenesis, and may comprise natural as well as non-natural nucleotides and amino acids.

Conservative amino acid substitutions include the ones in which the amino acid residue is replaced with an amino acid residue having similar structural or chemical properties. Families of amino acid residues having similar side chains have been defined in the art. These families include amino acids with basic side chains (*e.g.,* lysine, arginine, histidine), acidic side chains (*e.g.,* aspartic acid, glutamic acid), uncharged polar side chains (*e.g.,* asparagine, glutamine, serine, threonine, tyrosine, cysteine, tryptophan), non-polar side chains (*e.g.,* glycine, alanine, valine, leucine, isoleucine, proline, phenylalanine, methionine), beta-branched side chains (*e.g.,* threonine, valine, isoleucine) and aromatic side chains (*e.g.,* tyrosine, phenylalanine, tryptophan). It will be clear to the skilled artisan that other classifications of amino acid residue families than the one used above can also be employed. Furthermore, a variant may have non-conservative amino acid substitutions, *e.g.,* replacement of an amino acid with an amino acid residue having different structural or chemical properties. Similar minor variations may also include amino acid deletions or insertions, or both. Guidance in determining which amino acid residues may be substituted, inserted, or deleted without abolishing immunological activity may be found using computer programs well known in the art.

A mutation in a nucleotide sequence can be a single alteration made at a locus (a point mutation), such as transition or transversion mutations, or alternatively, multiple nucleotides may be inserted, deleted or changed at a single locus. In addition, one or more alterations may be made at any number of loci within a nucleotide sequence. The mutations may be performed by any suitable method known in the art.

The term "host", as used herein, is intended to refer to an organism or a cell into which a vector such as a cloning vector or an expression vector has been introduced. The organism or cell can be prokaryotic or eukaryotic. It should be understood that this term is intended to refer not only to the particular subject organism or cell but to the progeny of such an organism or cell as well. Because certain modifications may occur in succeeding generations due to either mutation or environmental influences, such progeny may not, in fact, be identical to the parent organism or cell, but are still included within the scope of the term "host" as used herein.

The term "human", when applied to binding molecules as defined herein, refers to molecules that are either directly derived from a human or based upon a human sequence. When a binding molecule is derived from or based on a human sequence and subsequently modified, it is still to be considered human as used throughout the specification. In other words, the term human, when applied to binding molecules is intended to include binding molecules having variable and constant regions derived from human germline immunoglobulin sequences or based on variable or constant regions occurring in a human or human lymphocyte and modified in some form. Thus, the human binding molecules may include amino acid residues not encoded by human germline immunoglobulin sequences, comprise substitutions and/or deletions (*e.g.,* mutations introduced by for instance random or site-specific mutagenesis *in vitro* or by somatic mutation *in vivo*). "Based on" as used herein refers to the situation that a nucleic acid sequence may be exactly copied from a template, or with minor mutations, such as by error-prone PCR methods, or synthetically made matching the template exactly or with minor modifications. Semi-synthetic molecules based on human sequences are also considered to be human as used herein.

The term "insertion", also known as the term "addition", denotes a change in an amino acid or nucleotide sequence resulting in the addition of one or more amino acid or nucleotide residues, respectively, as compared to the parent sequence.

The term "isolated", when applied to binding molecules as defined herein, refers to binding molecules that are substantially free of other proteins or polypeptides, particularly free of other binding molecules having different antigenic specificities, and are also substantially free of other cellular material and/or chemicals. For example, when the binding molecules are recombinantly produced, they are preferably substantially free of culture medium, and when the binding molecules are produced by chemical synthesis, they are preferably substantially free of chemical precursors or other chemicals, *i.e.,* they are separated from chemical precursors or other chemicals which are involved in the synthesis of the protein. The term "isolated" when applied to nucleic acid molecules encoding binding molecules as defined herein, is intended to refer to nucleic acid molecules in which the nucleotide sequences encoding the binding molecules are free of other nucleotide sequences, particularly nucleotide sequences encoding binding molecules that bind binding partners other than H5N1. Furthermore, the term "isolated" refers to nucleic acid molecules that are substantially separated from other cellular components that naturally accompany the native nucleic acid molecule in its natural host, *e.g.,* ribosomes, polymerases, or genomic sequences with which it is naturally associated. Moreover, "isolated" nucleic acid molecules, such as cDNA molecules, can be substantially free of other cellular material, or culture medium when produced by recombinant techniques, or substantially free of chemical precursors or other chemicals when chemically synthesized.

The term "monoclonal antibody" as used herein refers to a preparation of antibody molecules of single molecular composition. A monoclonal antibody displays a single binding specificity and affinity for a particular epitope. Accordingly, the term "human monoclonal antibody" refers to an antibody displaying a single binding specificity which has variable and constant regions derived from or based on human germline immunoglobulin sequences or derived from completely synthetic sequences. The method of preparing the monoclonal antibody is not relevant.

The term "naturally occurring" as used herein as applied to an object refers to the fact that an object can be found in nature. For example, a polypeptide or polynucleotide sequence that is present in an organism that can be isolated from a source in nature and which has not been intentionally modified by man in the laboratory is naturally occurring.

The term "nucleic acid molecule" as used in the present invention refers to a polymeric form of nucleotides and includes both sense and anti-sense strands of RNA, cDNA, genomic DNA, and synthetic forms and mixed polymers of the above. A nucleotide refers to a ribonucleotide, deoxynucleotide or a modified form of either type of nucleotide. The term also includes single- and double-stranded forms of DNA. In addition, a polynucleotide may include either or both naturally occurring and modified nucleotides linked together by naturally occurring and/or non-naturally occurring nucleotide linkages. The nucleic acid molecules may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (*e.g.,* methyl phosphonates, phosphotriesters, phosphoramidates, carbamates, etc.), charged linkages (*e.g.,* phosphorothioates, phosphorodithioates, etc.), pendent moieties (*e.g.,* polypeptides), intercalators (*e.g.,* acridine, psoralen, etc.), chelators, alkylators, and modified linkages (*e.g.,* alpha anomeric nucleic acids, etc.). The above term is also intended to include any topological conformation, including single-stranded, double-stranded, partially duplexed, triplex, hairpinned, circular and padlocked conformations. Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. A reference to a nucleic acid sequence encompasses its complement unless otherwise specified. Thus, a reference to a nucleic acid molecule having a particular sequence should be understood to encompass its complementary strand, with its complementary sequence. The complementary strand is also useful, *e.g.,* for anti-sense therapy, hybridisation probes and PCR primers.

The term "operably linked" refers to two or more nucleic acid sequence elements that are usually physically linked and are in a functional relationship with each other. For instance, a promoter is operably linked to a coding sequence, if the promoter is able to initiate or regulate the transcription or expression of a coding sequence, in which case the coding sequence should be understood as being "under the control of" the promoter.

By "pharmaceutically acceptable excipient" is meant any inert substance that is combined with an active molecule such as a drug, agent, or binding molecule for preparing an agreeable or convenient dosage form. The "pharmaceutically acceptable excipient" is an excipient that is non-toxic to recipients at the dosages and concentrations employed, and is compatible with other ingredients of the formulation comprising the drug, agent or binding molecule. Pharmaceutically acceptable excipients are widely applied in the art.

The term "specifically binding", as used herein, in reference to the interaction of a binding molecule, *e.g.* an antibody, and its binding partner, *e.g.* an antigen, means that the interaction is dependent upon the presence of a particular structure, *e.g.* an antigenic determinant or epitope, on the binding partner. In other words, the antibody preferentially binds or recognizes the binding partner even when the binding partner is present in a mixture of other molecules or organisms. The binding may be mediated by covalent or non-covalent interactions or a combination of both. In yet other words, the term "specifically binding" means immunospecifically binding to an antigen or a fragment thereof and not immunospecifically binding to other antigens. A binding molecule that immunospecifically binds to an antigen may bind to other peptides or polypeptides with lower affinity as determined by, *e.g.,* radioimmunoassays (RIA), enzyme-linked immunosorbent assays (ELISA), BIACORE, or other assays known in the art. Binding molecules or fragments thereof that immunospecifically bind to an antigen may be cross-reactive with related antigens. Preferably, binding molecules or fragments thereof that immunospecifically bind to an antigen do not cross-react with other antigens.

A "substitution", as used herein, denotes the replacement of one or more amino acids or nucleotides by different amino acids or nucleotides, respectively.

The term "therapeutically effective amount" refers to an amount of the binding molecule as defined herein that is effective for preventing, ameliorating and/or treating a condition resulting from infection with H5N1.

The term "treatment" refers to therapeutic treatment as well as prophylactic or preventative measures to cure or halt or at least retard disease progress. Those in need of treatment include those already inflicted with a condition resulting from infection with H5N1 as well as those in which infection with H5N1 is to be prevented. Subjects partially or totally recovered from infection with H5N1 might also be in need of treatment. Prevention encompasses inhibiting or reducing the spread of H5N1 or inhibiting or reducing the onset, development or progression of one or more of the symptoms associated with infection with H5N1.

The term "vector" denotes a nucleic acid molecule into which a second nucleic acid molecule can be inserted for introduction into a host where it will be replicated, and in some cases expressed. In other words, a vector is capable of transporting a nucleic acid molecule to which it has been linked. Cloning as well as expression vectors are contemplated by the term "vector", as used herein. Vectors include, but are not limited to, plasmids, cosmids, bacterial artificial chromosomes (BAC) and yeast artificial chromosomes (YAC) and vectors derived from bacteriophages or plant or animal (including human) viruses. Vectors comprise an origin of replication recognized by the proposed host and in case of expression vectors, promoter and other regulatory regions recognized by the host. A vector containing a second nucleic acid molecule is introduced into a cell by transformation, transfection, or by making use of viral entry mechanisms. Certain vectors are capable of autonomous replication in a host into which they are introduced (*e.g.,* vectors having a bacterial origin of replication can replicate in bacteria). Other vectors can be integrated into the genome of a host upon introduction into the host, and thereby are replicated along with the host genome.

### SUMMARY OF THE INVENTION

The invention provides human binding molecules capable of specifically binding to influenza virus H5N1 and exhibiting neutralizing activity against H5N1, as defined in the claims. The invention also provides binding molecules that bind to an epitope in the haemagglutinin protein that is shared between influenza subtypes and therefore relates to binding molecules that cross-react between H5-, H1-, H2-, H6- and H9 influenza based subtypes, such as H5N1, H1N1 and other influenza strains that contain the HA protein with these particular epitopes. The invention also pertains to nucleic acid molecules encoding at least the binding region of the human binding molecules. The invention further provides for the use of the human binding molecules of the invention in the prophylaxis and/or treatment of a subject having, or at risk of developing, an H5N1 infection. Besides that, the invention pertains to the use of the human binding molecules of the invention in the diagnosis/detection of H5N1.

### DETAILED DESCRIPTION

In a first aspect the present invention encompasses binding molecules capable of specifically binding to influenza virus A, particularly influenza virus A subtype H5N1, as defined in the claims. According to the invention, the binding molecules are human binding molecules. The binding molecules of the invention exhibit neutralizing activity against influenza virus A subtype H5N1. In a further aspect the binding molecules of the invention are capable of specifically binding to and/or have neutralizing activity against different influenza virus H5N1 strains. These strains may be a member of influenza virus H5N1 strains of clade 1, clade 2 or clade 3. Phylogenetic analyses of the HA genes from the 2004 and 2005 H5N1 outbreak showed two different lineages of HA genes, termed clades 1 and 2. Viruses in each of these clades are distributed in non-overlapping geographic regions of Asia. The H5N1 viruses from the Indochina peninsula are tightly clustered within clade 1, whereas H5N1 isolates from several surrounding countries are distinct from clade 1 isolates and belong in the more divergent clade 2. Clade 1 H5N1 viruses were isolated from humans and birds in Vietnam, Thailand, and Cambodia, but only from birds in Laos and Malaysia. The clade 2 viruses were found in viruses isolated exclusively from birds in China, Indonesia, Japan, and South Korea. Viruses isolated from birds and humans in Hong Kong in 2003 and 1997 made up clades 1' (also categorized in clade 1) and 3, respectively (see WHO Global Influenza Program Surveillance Network, 2005). The hemagglutinins of the clades differ in their amino acid sequences. Examples of clade 1 strains include, but are not limited to, A/Hong Kong/213/03, A/Vietnam/1194/04, A/Vietnam/1203/04 and A/Thailand/1(KAN-1)/2004. Examples of clade 2 strains include, but are not limited to, A/Indonesia/5/05, A/bar headed goose/Qinghai/1A/05, A/turkey/Turkey/1/05 and A/Anhui/1/05. Examples of clade 3 strains include, but are not limited to, A/Hong Kong/156/97 and A/goose/Guangdong/1/96. Other strains can be found, *e.g.,* in WHO Global Influenza Program Surveillance Network, 2005 and on http://www.who.int/csr/disease/avian_influenza/guidelines/recommendationvaccine.pdf Preferably, the binding molecules of the invention are capable of specifically binding to and have neutralizing activity against clade 1, clade 2 and clade 3 strains. The binding molecules of the invention may be capable of specifically binding to influenza virus H5N1 that are viable, living and/or infective or that are in inactivated/attenuated form. Methods for inactivating/attenuating influenza virus H5N1 are well known in the art and include, but are not limited to, treatment with formalin, β-propiolactone (BPL), merthiolate, and/or ultraviolet light.

The binding molecules of the invention may also be capable of specifically binding to one or more fragments of influenza virus H5N1 such as *inter alia* a preparation of one or more proteins and/or (poly)peptides derived from subtype H5N1 or one or more recombinantly produced proteins and/or polypeptides of H5N1. For methods of treatment and/or prevention of H5N1 infections the binding molecules are preferably capable of specifically binding to surface accessible proteins of H5N1 such as the surface glycoproteins, hemagglutinin (HA) and neuraminidase (NA), which are required for viral attachment and cellular release, or membrane proteins (M1 and M2). In a specific embodiment the binding molecules of the invention are capable of specifically binding to the HA molecule of H5N1 strains. They may be capable of specifically binding to the HA1 and/or HA2 subunit of the HA molecule. They may be capable of specifically binding to linear or structural and/or conformational epitopes on the HA1 and/or HA2 subunit of the HA molecule. The HA molecule may be purified from viruses or recombinantly produced and optionally isolated before use. Alternatively, HA may be expressed on the surface of cells.

For diagnostical purposes the binding molecules may also be capable of specifically binding to proteins not present on the surface of H5N1 including the nucleoprotein, the nucleocapsid structural protein, polymerases (PA, PB and PB2), and non-structural proteins (NS1 and NS2). The nucleotide and/or amino acid sequence of proteins of various H5N1 strains can be found in the GenBank-database, NCBI Influenza Virus Sequence Database, Influenza Sequence Database (ISD), EMBL-database and/or other databases. It is well within the reach of the skilled person to find such sequences in the respective databases.

In another embodiment the binding molecules of the invention are capable of specifically binding to a fragment of the above-mentioned proteins and/or polypeptides, wherein the fragment at least comprises an antigenic determinant recognized by the binding molecules of the invention. An "antigenic determinant" as used herein is a moiety that is capable of binding to a binding molecule of the invention with sufficiently high affinity to form a detectable antigen-binding molecule complex. The binding molecules of the invention may or may not be capable of specifically binding to the extracellular part of HA (also called herein soluble HA (sHA)).

The binding molecules of the invention can be intact immunoglobulin molecules such as polyclonal or monoclonal antibodies or the binding molecules can be antigen-binding fragments including, but not limited to, Fab, F(ab'), F(ab')₂, Fv, dAb, Fd, complementarity determining region (CDR) fragments, single-chain antibodies (scFv), bivalent single-chain antibodies, single-chain phage antibodies, diabodies, triabodies, tetrabodies, and (poly)peptides that contain at least a fragment of an immunoglobulin that is sufficient to confer specific antigen binding to influenza virus H5N1 strains or a fragment thereof. In a preferred embodiment the binding molecules of the invention are human monoclonal antibodies.

The binding molecules of the invention can be used in non-isolated or isolated form. Furthermore, the binding molecules of the invention can be used alone or in a mixture comprising at least one binding molecule (or variant or fragment thereof) of the invention. In other words, the binding molecules can be used in combination, *e.g.,* as a pharmaceutical composition comprising two or more binding molecules of the invention, variants or fragments thereof. For example, binding molecules having different, but complementary activities can be combined in a single therapy to achieve a desired prophylactic, therapeutic or diagnostic effect, but alternatively, binding molecules having identical activities can also be combined in a single therapy to achieve a desired prophylactic, therapeutic or diagnostic effect. Optionally, the mixture further comprises at least one other therapeutic agent. Preferably, the therapeutic agent such as, *e.g.,* M2 inhibitors (*e.g.,* amantidine, rimantadine) and/or neuraminidase inhibitors (*e.g*., zanamivir, oseltamivir) is useful in the prophylaxis and/or treatment of an influenza virus H5N1 infection.

Typically, binding molecules according to the invention can bind to their binding partners, *i.e.* influenza virus H5N1 or fragments thereof, with an affinity constant (K_{d}-value) that is lower than 0.2x10⁻⁴ M, 1.0x10⁻⁵ M, 1.0x10⁻⁶ M, 1.0x10⁻⁷ M, preferably lower than 1.0x10⁻⁸ M, more preferably lower than 1.0x10⁻⁹ M, more preferably lower than 1.0x10⁻¹⁰ M, even more preferably lower than 1.0x10⁻¹¹ M, and in particular lower than 1.0x10⁻¹² M. The affinity constants can vary for antibody isotypes. For example, affinity binding for an IgM isotype refers to a binding affinity of at least about 1.0x10⁻⁷ M. Affinity constants can for instance be measured using surface plasmon resonance, for example using the BIACORE system (Pharmacia Biosensor AB, Uppsala, Sweden).

The binding molecules according to the invention may bind to influenza virus H5N1 or a fragment thereof in soluble form such as for instance in a sample or in suspension or may bind to influenza virus H5N1 or a fragment thereof bound or attached to a carrier or substrate, e.g., microtiter plates, membranes and beads, etc. Carriers or substrates may be made of glass, plastic (*e.g.,* polystyrene), polysaccharides, nylon, nitrocellulose, or Teflon, etc. The surface of such supports may be solid or porous and of any convenient shape. Furthermore, the binding molecules may bind to influenza virus H5N1 in purified/isolated or non-purified/non-isolated form.

The binding molecules of the invention exhibit neutralizing activity. Neutralizing activity can for instance be measured as described herein. Alternative assays measuring neutralizing activity are described in for instance WHO Manual on Animal Influenza Diagnosis and Surveillance, Geneva: World Health Organisation, 2005, version 2002.5.

The present invention relates to an isolated human binding molecule that is able to recognize and bind to an epitope in the HA2 subunit of the influenza haemagglutinin protein (HA), characterized in that said binding molecule has neutralizing activity against an influenza virus comprising HA of the H5 subtype, as defined in the claims. Examples of influenza strains that contain such a HA of the H5 subtype and that are important strains in view of pandemic threats are H5N1, H5N2, H5N8, and H5N9. Particularly preferred are binding molecules that at least neutralize the H5N1 influenza strain. Preferably, said binding molecule according to the invention does not depend on an epitope in the HA1 subunit of the HA protein for binding to said HA protein. The known murine antibody that was described in the art (C 179) that also binds to the same epitope in the HA2 domain also depends on binding to an epitope in the HA1 domain of the HA protein. This is disadvantageous as it adds to the possibility of escape mutants that are no longer recognized by the antibody. Moreoever, a number of the antibodies of the present invention (such as CR6307 and CR6323) do not depend on conformational epitopes and recognize the HA2 epitope even in a reduced form (when used in western-blotting). This is an advantage over the antibodies from the art because when a conformational change is induced in the HA protein due to whatever mutation in another part of the protein, such conformational change will not most likely hamper the binding of the antibodies of the present invention to the HA2 epitope, whereas antibodies that do depend on conformation might very well be unable to bind when such mutations occur.

In another preferred embodiment, the binding molecule according to the invention also has neutralizing activity against an influenza virus comprising HA of the H1 subtype, and preferably wherein said binding molecule also has neutralizing activity against an influenza virus comprising HA of the H2, H6 and/or H9 subtype. It has been shown herein that the binding molecules of the present invention interact with an epitope present in the HA2 epitopes present in the H5, H1, H2, H6, and H9 subtypes, and it has been shown that the binding molecules of the present invention cross-neutralize between influenza subtypes because of this epitope-sharing. It is concluded that the binding molecules of the present invention as defined in the claims that depend on binding to that particular part in the HA2 domain (and not on another -mutational prone- epitope in HA1) can cross-neutralize between influenza virus subtypes as they do not appear to depend on binding to domains within HA1, which may be altered significantly due to antigenic drifts. The skilled person, based on what has been disclosed herein, can now determine whether an antibody indeed cross-reacts with HA proteins from different subtypes and also determine whether they are able to neutralize influenza viruses of different subtypes *in vivo.*

In another preferred aspect of the invention the binding molecule of the present invention binds to an epitope in the HA2 subunit that is selected from the group consisting of amino acid sequence: GVTNKVNSIIDK (SEQ ID NO:368), GVTNKVNSIINK (SEQ ID NO:369), GVTNKENSIIDK (SEQ ID NO:370 GVTNKVNRIIDK (SEQ ID NO:371), GITNKVNSVIEK (SEQ ID NO:372), GITNKENSVIEK (SEQ ID NO:373), GITNKVNSIIDK (SEQ ID NO:374), and KITSKVNNIVDK (SEQ ID NO:375). As can be concluded from the data shown in Table 13, certain binding molecules of the present invention, CR6261, CR6325, and CR6329 interact with the GVTNKVNSIIDK (SEQ ID NO:368) epitope present in H5N1, and are not hampered by a mutation in the TGLRN epitope in HA1 that do influence the binding of C179. Moreover, some binding molecules, such as CR6307 and CR6323 are not even hampered by a escape mutant, as disclosed in Okuno et al. (1993) with a valine -> glutamic acid mutation at position 6 (exemplified by GVTNKENSIIDK (SEQ ID NO:370)). This epitope is part of an extended alpha helix in the HA2 region. The residues in this putative epitope that are predicted to be most solvent exposed are underlined in bold: GV**T**NKE**N**SI**I**DK (SEQ ID NO:370). These amino acids would be most accessible to a binding molecule and thus may form the most important region of the epitope. Consistent with this notion the highlighted ammino acids are absolutly conserved in identity and position in all the sequences presented. This knowledge could be used to predict binding epitopes in influenza subtypes that do not carry the same sequence as above (i.e. H3, H7 and B strains).

The binding molecule according to the present invention comprises a heavy chain CDR1 region of SEQ ID NO:274, a heavy chain CDR2 region of SEQ ID NO:275, and a heavy chain CDR3 region of SEQ ID NO:276..

In a preferred embodiment, the binding molecule according to the invention is for a use as a medicament and preferably for the diagnostic, therapeutic and/or prophylactic treatment of influenza infection. In one aspect of such use, said influenza infection is caused by an influenza virus that is associated with a pandemic outbreak, or has the potential to be associated with a pandemic outbreak (see WO 2007/045674 and the tables therein). Preferably, the influenza virus strain that is associated with a pandemic outbreak and wherein the disease caused by these strains can be treated by the binding molecules of the present invention, is selected from the group consisting of H1N1, H5N1, H5N2, H5N8, H5N9, H2-based viruses, and H9N2.

The present invention also relates to a pharmaceutical composition comprising a binding molecule according to the invention, and a pharmaceutically acceptable excipient.

In yet another embodiment the invention relates to a use of a binding molecule according to the invention in the preparation of a medicament for the diagnosis, prophylaxis, and/or treatment of an influenza virus infection. Such infections can occur in small populations, but can also spread around the world in seasonal epidemics or, worse, in global pandemics where millions of individuals are at risk. The invention provides binding molecules that can neutralize the infection of influenza strains that cause such seasonal epidemics as well as potential pandemics. Importantly, protection and treatment can be envisioned now with the binding molecules of the present invention in relation to multiple influenza strains as it has been disclosed that due to the binding of an epitope that is shared between HA proteins of different influenza strains, cross-neutralization between such strains is now possible by using the binding molecules of the present invention. This is highly advantageous as the binding molecules can protect mammals when they are administered either before or after infection (as disclosed in the examples), and therefore also when it is unclear (in early stages of occurrence) whether the infection is caused by an H1, an H2, an H5, an H6 or an H9-based strain. Health workers, military forces as well as the general population may be treated prophylactically, or treated upon infection, with the binding molecules of the present invention. Potentially, the binding molecules of the present invention can be prepared and stored in huge stocks because it will provide protection against different pandemic strains, and this will be beneficial in the preparedness of possible influenza pandemics in the future.

In a preferred embodiment, the human binding molecule according to the invention comprises a heavy chain CDR1 region having the amino acid sequence of SEQ ID NO:274, a heavy chain CDR2 region having the amino acid sequence of SEQ ID NO:275, a heavy chain CDR3 region having the amino acid sequence of SEQ ID NO:276, a light chain CDR1 region having the amino acid sequence of SEQ ID NO:277, a light chain CDR2 region having the amino acid sequence of SEQ ID NO:278, and a light chain CDR3 region having the amino acid sequence of SEQ ID NO:279.

The CDR regions of the binding molecules are shown in Table 7. CDR regions are according to Kabat *et al.* (1991) as described in Sequences of Proteins of Immunological Interest. In an embodiment of th epresent invention binding molecules may comprise two, three, four, five or all six CDR regions as disclosed herein. Preferably, a binding molecule according to the present invention comprises at least two of the CDRs disclosed herein.

In an embodiment the binding molecules of the invention comprise the VH germline VH1-69 (see Tomlinson IM, Williams SC, Ignatovitch O, Corbett SJ, Winter G. V-BASE Sequence Directory. Cambridge United Kingdom: MRC Centre for Protein Engineering (1997)). In yet another embodiment, the binding molecules according to the invention comprise a heavy chain comprising the variable heavy chain of the amino acid sequence SEQ ID NO:341. In a further embodiment, the binding molecules according to the invention comprise a light chain comprising the variable light chain of the amino acid sequence SEQ ID NO:343. Table 8 specifies the heavy and light chain variable regions of the binding molecules of the invention.

Another aspect of the invention includes functional variants of the binding molecules as defined herein. Molecules are considered to be functional variants of a binding molecule according to the invention, if the variants are capable of competing for specifically binding to influenza virus H5N1 or a fragment thereof with the parental binding molecules. In other words, when the functional variants are still capable of binding to influenza virus H5N1 or a fragment thereof. Preferably, the functional variants are capable of competing for specifically binding to at least two (or more) different influenza virus H5N1 strains or fragments thereof that are specifically bound by the parental binding molecules. Furthermore, molecules are considered to be functional variants of a binding molecule according to the invention, if they have neutralizing activity against influenza virus H5N1, preferably against the at least two (or more) influenza virus H5N1 strains against which the parental binding molecule exhibits neutralizing activity. Functional variants include, but are not limited to, derivatives that are substantially similar in primary structural sequence, but which contain *e.g. in vitro* or *in vivo* modifications, chemical and/or biochemical, that are not found in the parental binding molecule. Such modifications include *inter alia* acetylation, acylation, covalent attachment of a nucleotide or nucleotide derivative, covalent attachment of a lipid or lipid derivative, cross-linking, disulfide bond formation, glycosylation, hydroxylation, methylation, oxidation, pegylation, proteolytic processing, phosphorylation, and the like.

Alternatively, functional variants can be binding molecules as defined in the present invention comprising an amino acid sequence containing substitutions, insertions, deletions or combinations thereof of one or more amino acids compared to the amino acid sequences of the parental binding molecules. Furthermore, functional variants can comprise truncations of the amino acid sequence at either or both the amino or carboxyl termini. Functional variants according to the invention may have the same or different, either higher or lower, binding affinities compared to the parental binding molecule but are still capable of binding to influenza virus H5N1 or a fragment thereof. For instance, functional variants according to the invention may have increased or decreased binding affinities for influenza virus H5N1 or a fragment thereof compared to the parental binding molecules. Preferably, the amino acid sequences of the variable regions, including, but not limited to, framework regions, hypervariable regions, in particular the CDR3 regions, are modified. Generally, the light chain and the heavy chain variable regions comprise three hypervariable regions, comprising three CDRs, and more conserved regions, the so-called framework regions (FRs). The hypervariable regions comprise amino acid residues from CDRs and amino acid residues from hypervariable loops. Functional variants intended to fall within the scope of the present invention have at least about 50% to about 99%, preferably at least about 60% to about 99%, more preferably at least about 70% to about 99%, even more preferably at least about 80% to about 99%, most preferably at least about 90% to about 99%, in particular at least about 95% to about 99%, and in particular at least about 97% to about 99% amino acid sequence homology with the parental binding molecules as defined herein. Computer algorithms such as *inter alia* Gap or Bestfit known to a person skilled in the art can be used to optimally align amino acid sequences to be compared and to define similar or identical amino acid residues. Functional variants can be obtained by altering the parental binding molecules or parts thereof by general molecular biology methods known in the art including, but not limited to, error-prone PCR, oligonucleotide-directed mutagenesis, site-directed mutagenesis and heavy and/or light chain shuffling. In an embodiment the functional variants of the invention have neutralizing activity against influenza virus H5N1. The neutralizing activity may either be identical, or be higher or lower compared to the parental binding molecules. Henceforth, when the term (human) binding molecule is used, this also encompasses functional variants of the (human) binding molecule.

In yet a further aspect, the invention includes immunoconjugates, *i.e.* molecules comprising at least one binding molecule as defined herein and further comprising at least one tag, such as *inter alia* a detectable moiety/agent. Also contemplated in the present invention are mixtures of immunoconjugates according to the invention or mixtures of at least one immunoconjugates according to the invention and another molecule, such as a therapeutic agent or another binding molecule or immunoconjugate. In a further embodiment, the immunoconjugates of the invention may comprise more than one tag. These tags can be the same or distinct from each other and can be joined/conjugated non-covalently to the binding molecules. The tag(s) can also be joined/conjugated directly to the human binding molecules through covalent bonding. Alternatively, the tag(s) can be joined/conjugated to the binding molecules by means of one or more linking compounds. Techniques for conjugating tags to binding molecules are well known to the skilled artisan.

The tags of the immunoconjugates of the present invention may be therapeutic agents, but they can also be detectable moieties/agents. Tags suitable in therapy and/or prevention may be toxins or functional parts thereof, antibiotics, enzymes, other binding molecules that enhance phagocytosis or immune stimulation. Immunoconjugates comprising a detectable agent can be used diagnostically to, for example, assess if a subject has been infected with an influenza virus H5N1 strain or monitor the development or progression of an influenza virus H5N1 infection as part of a clinical testing procedure to, *e.g.,* determine the efficacy of a given treatment regimen. However, they may also be used for other detection and/or analytical and/or diagnostic purposes. Detectable moieties/agents include, but are not limited to, enzymes, prosthetic groups, fluorescent materials, luminescent materials, bioluminescent materials, radioactive materials, positron emitting metals, and non-radioactive paramagnetic metal ions. The tags used to label the binding molecules for detection and/or analytical and/or diagnostic purposes depend on the specific detection/analysis/diagnosis techniques and/or methods used such as *inter alia* immunohistochemical staining of (tissue) samples, flow cytometric detection, scanning laser cytometric detection, fluorescent immunoassays, enzyme-linked immunosorbent assays (ELISAs), radioimmunoassays (RIAs), bioassays (*e.g.,* phagocytosis assays), Western blotting applications, etc. Suitable labels for the detection/analysis/diagnosis techniques and/or methods known in the art are well within the reach of the skilled artisan.

Furthermore, the human binding molecules or immunoconjugates of the invention can also be attached to solid supports, which are particularly useful for *in vitro* immunoassays or purification of influenza virus H5N1 or a fragment thereof. Such solid supports might be porous or nonporous, planar or non-planar. The binding molecules of the present invention can be fused to marker sequences, such as a peptide to facilitate purification. Examples include, but are not limited to, the hexa-histidine tag, the hemagglutinin (HA) tag, the myc tag or the flag tag. Alternatively, an antibody can be conjugated to a second antibody to form an antibody heteroconjugate. In another aspect the binding molecules of the invention may be conjugated/attached to one or more antigens. Preferably, these antigens are antigens which are recognized by the immune system of a subject to which the binding molecule-antigen conjugate is administered. The antigens may be identical, but may also differ from each other. Conjugation methods for attaching the antigens and binding molecules are well known in the art and include, but are not limited to, the use of cross-linking agents. The binding molecules of the invention will bind to influenza virus H5N1 and the antigens attached to the binding molecules will initiate a powerful T-cell attack on the conjugate, which will eventually lead to the destruction of the influenza virus H5N1.

Next to producing immunoconjugates chemically by conjugating, directly or indirectly, via for instance a linker, the immunoconjugates can be produced as fusion proteins comprising the binding molecules of the invention and a suitable tag. Fusion proteins can be produced by methods known in the art such as, *e.g.,* recombinantly by constructing nucleic acid molecules comprising nucleotide sequences encoding the binding molecules in frame with nucleotide sequences encoding the suitable tag(s) and then expressing the nucleic acid molecules.

It is another aspect of the present invention to provide a nucleic acid molecule encoding at least a binding molecule, functional variant or immunoconjugate according to the invention. Such nucleic acid molecules can be used as intermediates for cloning purposes, *e.g.* in the process of affinity maturation as described above. In a preferred embodiment, the nucleic acid molecules are isolated or purified.

The skilled man will appreciate that functional variants of these nucleic acid molecules are also intended to be a part of the present invention. Functional variants are nucleic acid sequences that can be directly translated, using the standard genetic code, to provide an amino acid sequence identical to that translated from the parental nucleic acid molecules.

Preferably, the nucleic acid molecules encode binding molecules comprising the CDR regions as described above. In a further embodiment the nucleic acid molecules encode binding molecules comprising two, three, four, five or even all six CDR regions of the binding molecules of the invention.

In another embodiment, the nucleic acid molecules encode binding molecules comprising a heavy chain comprising the variable heavy chain sequences as described above. In another embodiment the nucleic acid molecules encode binding molecules comprising a light chain comprising the variable light chain sequences as described above. The nucleotide sequences of the binding molecules of the invention are given in the Example section (see, *e.g.,* Tables 6 and 8).

It is another aspect of the invention to provide vectors, *i.e.* nucleic acid constructs, comprising one or more nucleic acid molecules according to the present invention. Vectors can be derived from plasmids such as *inter alia* F, R1, RP1, Col, pBR322, TOL, Ti, etc; cosmids; phages such as lambda, lambdoid, M13, Mu, P1, P22, Qβ, T-even, T-odd, T2, T4, T7, etc; plant viruses. Vectors can be used for cloning and/or for expression of the binding molecules of the invention and might even be used for gene therapy purposes. Vectors comprising one or more nucleic acid molecules according to the invention operably linked to one or more expression-regulating nucleic acid molecules are also covered by the present invention. The choice of the vector is dependent on the recombinant procedures followed and the host used. Introduction of vectors in host cells can be effected by *inter alia* calcium phosphate transfection, virus infection, DEAE-dextran mediated transfection, lipofectamin transfection or electroporation. Vectors may be autonomously replicating or may replicate together with the chromosome into which they have been integrated. Preferably, the vectors contain one or more selection markers. The choice of the markers may depend on the host cells of choice, although this is not critical to the invention as is well known to persons skilled in the art. They include, but are not limited to, kanamycin, neomycin, puromycin, hygromycin, zeocin, thymidine kinase gene from Herpes simplex virus (HSV-TK), dihydrofolate reductase gene from mouse (dhfr). Vectors comprising one or more nucleic acid molecules encoding the human binding molecules as described above operably linked to one or more nucleic acid molecules encoding proteins or peptides that can be used to isolate the human binding molecules are also covered by the invention. These proteins or peptides include, but are not limited to, glutathione-S-transferase, maltose binding protein, metal-binding polyhistidine, green fluorescent protein, luciferase and beta-galactosidase.

Hosts containing one or more copies of the vectors mentioned above are an additional subject of the present invention. Preferably, the hosts are host cells. Host cells include, but are not limited to, cells of mammalian, plant, insect, fungal or bacterial origin. Bacterial cells include, but are not limited to, cells from Gram-positive bacteria or Gram-negative bacteria such as several species of the genera *Escherichia,* such as *E. coli,* and *Pseudomonas.* In the group of fungal cells preferably yeast cells are used. Expression in yeast can be achieved by using yeast strains such as *inter alia Pichia pastoris, Saccharomyces cerevisiae* and *Hansenula polymorpha.* Furthermore, insect cells such as cells from Drosophila and Sf9 can be used as host cells. Besides that, the host cells can be plant cells such as *inter alia* cells from crop plants such as forestry plants, or cells from plants providing food and raw materials such as cereal plants, or medicinal plants, or cells from ornamentals, or cells from flower bulb crops. Transformed (transgenic) plants or plant cells are produced by known methods, for example, Agrobacterium-mediated gene transfer, transformation of leaf discs, protoplast transformation by polyethylene glycol-induced DNA transfer, electroporation, sonication, microinjection or bolistic gene transfer. Additionally, a suitable expression system can be a baculovirus system. Expression systems using mammalian cells such as Chinese Hamster Ovary (CHO) cells, COS cells, BHK cells, NSO cells or Bowes melanoma cells are preferred in the present invention. Mammalian cells provide expressed proteins with posttranslational modifications that are most similar to natural molecules of mammalian origin. Since the present invention deals with molecules that may have to be administered to humans, a completely human expression system would be particularly preferred. Therefore, even more preferably, the host cells are human cells. Examples of human cells are *inter alia* HeLa, 911, AT1080, A549, 293 and HEK293T cells. In preferred embodiments, the human producer cells comprise at least a functional part of a nucleic acid sequence encoding an adenovirus E1 region in expressible format. In even more preferred embodiments, said host cells are derived from a human retina and immortalized with nucleic acids comprising adenoviral E1 sequences, such as 911 cells or the cell line deposited at the European Collection of Cell Cultures (ECACC), CAMR, Salisbury, Wiltshire SP4 OJG, Great Britain on 29 February 1996 under number 96022940 and marketed under the trademark PER.C6® (PER.C6 is a registered trademark of Crucell Holland B.V.). For the purposes of this application "PER.C6 cells" refers to cells deposited under number 96022940 or ancestors, passages up-stream or downstream as well as descendants from ancestors of deposited cells, as well as derivatives of any of the foregoing. Production of recombinant proteins in host cells can be performed according to methods well known in the art. The use of the cells marketed under the trademark PER.C6® as a production platform for proteins of interest has been described in WO 00/63403.

A method of producing a binding molecule according to the invention is an additional part of the disclosure. The method comprises the steps of a) culturing a host according to the invention under conditions conducive to the expression of the binding molecule, and b) optionally, recovering the expressed binding molecule. The expressed binding molecules can be recovered from the cell free extract, but preferably they are recovered from the culture medium. The above method of producing can also be used to make functional variants of the binding molecules and/or immunoconjugates of the present invention. Methods to recover proteins, such as binding molecules, from cell free extracts or culture medium are well known to the man skilled in the art. Binding molecules, functional variants and/or immunoconjugates as obtainable by the above-described method are also a part of the present invention.

Alternatively, next to the expression in hosts, such as host cells, the binding molecules and immunoconjugates of the invention can be produced synthetically by conventional peptide synthesizers or in cell-free translation systems using RNA nucleic acid derived from DNA molecules according to the invention. Binding molecules and immunoconjugates as obtainable by the above described synthetic production methods or cell-free translation systems are also a part of the present invention.

In yet another embodiment, binding molecules of the present invention can also be produced in transgenic, non-human, mammals such as *inter alia* rabbits, goats or cows, and secreted into for instance the milk thereof.

In yet another alternative embodiment, binding molecules according to the present invention, preferably human binding molecules specifically binding to influenza virus H5N1 or a fragment thereof, may be generated by transgenic non-human mammals, such as for instance transgenic mice or rabbits, that express human immunoglobulin genes. Preferably, the transgenic non-human mammals have a genome comprising a human heavy chain transgene and a human light chain transgene encoding all or a portion of the human binding molecules as described above. The transgenic non-human mammals can be immunized with a purified or enriched preparation of influenza virus H5N1 or a fragment thereof. Protocols for immunizing non-human mammals are well established in the art. See Using Antibodies: A Laboratory Manual, Edited by: E. Harlow, D. Lane (1998), Cold Spring Harbor Laboratory, Cold Spring Harbor, New York and Current Protocols in Immunology, Edited by: J.E. Coligan, A.M. Kruisbeek, D.H. Margulies, E.M. Shevach, W. Strober (2001), John Wiley & Sons Inc., New York. Immunization protocols often include multiple immunizations, either with or without adjuvants such as Freund's complete adjuvant and Freund's incomplete adjuvant, but may also include naked DNA immunizations. In another embodiment, the human binding molecules are produced by B-cells, plasma and/or memory cells derived from the transgenic animals. In yet another embodiment, the human binding molecules are produced by hybridomas, which are prepared by fusion of B-cells obtained from the above-described transgenic non-human mammals to immortalized cells. B-cells, plasma cells and hybridomas as obtainable from the above-described transgenic non-human mammals and human binding molecules as obtainable from the above-described transgenic non-human mammals, B-cells, plasma and/or memory cells and hybridomas are also a part of the present invention.

In yet a further aspect, the invention provides compositions comprising at least a binding molecule preferably a human monoclonal antibody according to the invention, at least a functional variant thereof, at least an immunoconjugate according to the invention or a combination thereof. In addition to that, the compositions may comprise *inter alia* stabilizing molecules, such as albumin or polyethylene glycol, or salts. Preferably, the salts used are salts that retain the desired biological activity of the binding molecules and do not impart any undesired toxicological effects. If necessary, the human binding molecules of the invention may be coated in or on a material to protect them from the action of acids or other natural or non-natural conditions that may inactivate the binding molecules.

In yet a further aspect, the invention provides compositions comprising at least a nucleic acid molecule as defined in the present invention. The compositions may comprise aqueous solutions such as aqueous solutions containing salts (*e.g.,* NaCl or salts as described above), detergents (*e.g.,* SDS) and/or other suitable components.

Furthermore, the present invention pertains to pharmaceutical compositions comprising at least a binding molecule such as a human monoclonal antibody of the invention (or functional fragment or variant thereof), at least an immunoconjugate according to the invention, at least a composition according to the invention, or combinations thereof. The pharmaceutical composition of the invention further comprises at least one pharmaceutically acceptable excipient. Pharmaceutically acceptable excipients are well known to the skilled person. The pharmaceutical composition according to the invention may further comprise at least one other therapeutic agent. Suitable agents are also well known to the skilled artisan.

In an embodiment the pharmaceutical compositions may comprise two or more binding molecules that have neutralizing activity against influenza virus H5N1. In an embodiment, the binding molecules exhibit synergistic neutralizing activity, when used in combination. In other words, the compositions comprise at least two binding molecules having neutralizing activity, characterized in that the binding molecules act synergistically in neutralizing influenza virus H5N1. As used herein, the term "synergistic" means that the combined effect of the binding molecules when used in combination is greater than their additive effects when used individually. The synergistically acting binding molecules may bind to different structures on the same or distinct fragments of influenza virus H5N1. A way of calculating synergy is by means of the combination index. The concept of the combination index (CI) has been described by Chou and Talalay (1984). The compositions may also comprise one binding molecule having neutralizing activity and one non-neutralizing H5N1-specific binding molecule. The non-neutralizing and neutralizing H5N1-specific binding molecules may also act synergistically in neutralizing influenza virus H5N1.

A pharmaceutical composition according to the invention can further comprise at least one other therapeutic, prophylactic and/or diagnostic agent. Preferably, the pharmaceutical composition comprises at least one other prophylactic and/or therapeutic agent. Preferably, said further therapeutic and/or prophylactic agents are agents capable of preventing and/or treating an influenza virus H5N1 infection and/or a condition resulting from such an infection. Therapeutic and/or prophylactic agents include, but are not limited to, anti-viral agents. Such agents can be binding molecules, small molecules, organic or inorganic compounds, enzymes, polynucleotide sequences, anti-viral peptides, etc. Other agents that are currently used to treat patients infected with influenza virus H5N1 are M2 inhibitors (*e.g.,* amantidine, rimantadine) and/or neuraminidase inhibitors (*e.g.,* zanamivir, oseltamivir). These can be used in combination with the binding molecules of the invention. Agents capable of preventing and/or treating an infection with influenza virus H5N1 and/or a condition resulting from such an infection that are in the experimental phase might also be used as other therapeutic and/or prophylactic agents useful in the present invention.

The binding molecules or pharmaceutical compositions of the invention can be tested in suitable animal model systems prior to use in humans. Such animal model systems include, but are not limited to, mouse, ferret and monkey.

Typically, pharmaceutical compositions must be sterile and stable under the conditions of manufacture and storage. The binding molecules, immunoconjugates, nucleic acid molecules or compositions of the present invention can be in powder form for reconstitution in the appropriate pharmaceutically acceptable excipient before or at the time of delivery. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Alternatively, the binding molecules, immunoconjugates, nucleic acid molecules or compositions of the present invention can be in solution and the appropriate pharmaceutically acceptable excipient can be added and/or mixed before or at the time of delivery to provide a unit dosage injectable form. Preferably, the pharmaceutically acceptable excipient used in the present invention is suitable to high drug concentration, can maintain proper fluidity and, if necessary, can delay absorption.

The choice of the optimal route of administration of the pharmaceutical compositions will be influenced by several factors including the physico-chemical properties of the active molecules within the compositions, the urgency of the clinical situation and the relationship of the plasma concentrations of the active molecules to the desired therapeutic effect. For instance, if necessary, the binding molecules of the invention can be prepared with carriers that will protect them against rapid release, such as a controlled release formulation, including implants, transdermal patches, and microencapsulated delivery systems. Biodegradable, biocompatible polymers can *inter alia* be used, such as ethylene vinyl acetate, polyanhydrides, polyglycolic acid, collagen, polyorthoesters, and polylactic acid. Furthermore, it may be necessary to coat the binding molecules with, or co-administer the binding molecules with, a material or compound that prevents the inactivation of the human binding molecules. For example, the binding molecules may be administered to a subject in an appropriate carrier, for example, liposomes or a diluent.

The routes of administration can be divided into two main categories, oral and parenteral administration. The preferred administration route is intravenous or by inhalation.

Oral dosage forms can be formulated *inter alia* as tablets, troches, lozenges, aqueous or oily suspensions, dispersible powders or granules, emulsions, hard capsules, soft gelatin capsules, syrups or elixirs, pills, dragees, liquids, gels, or slurries. These formulations can contain pharmaceutically excipients including, but not limited to, inert diluents, granulating and disintegrating agents, binding agents, lubricating agents, preservatives, colouring, flavouring or sweetening agents, vegetable or mineral oils, wetting agents, and thickening agents.

The pharmaceutical compositions of the present invention can also be formulated for parenteral administration. Formulations for parenteral administration can be *inter alia* in the form of aqueous or non-aqueous isotonic sterile non-toxic injection or infusion solutions or suspensions. The solutions or suspensions may comprise agents that are non-toxic to recipients at the dosages and concentrations employed such as 1,3-butanediol, Ringer's solution, Hank's solution, isotonic sodium chloride solution, oils, fatty acids, local anaesthetic agents, preservatives, buffers, viscosity or solubility increasing agents, water-soluble antioxidants, oil-soluble antioxidants and metal chelating agents.

In a further aspect, the binding molecules such as human monoclonal antibodies (functional fragments and variants thereof), immunoconjugates, compositions, or pharmaceutical compositions of the invention can be used as a medicament. So, a method of treatment and/or prevention of an influenza virus H5N1 infection using the binding molecules, immunoconjugates, compositions, or pharmaceutical compositions of the invention is another part of the present invention. The above-mentioned molecules can *inter alia* be used in the diagnosis, prophylaxis, treatment, or combination thereof, of an influenza virus H5N1 infection. They are suitable for treatment of yet untreated patients suffering from an influenza virus H5N1 infection and patients who have been or are treated for an influenza virus H5N1 infection. They may be used for patients such as healthcare workers, relatives of infected subjects, (poultry-)farmers, etc.

The above-mentioned molecules or compositions may be employed in conjunction with other molecules useful in diagnosis, prophylaxis and/or treatment. They can be used *in vitro, ex vivo* or *in vivo.* For instance, the binding molecules such as human monoclonal antibodies (or functional variants thereof), immunoconjugates, compositions or pharmaceutical compositions of the invention can be co-administered with a vaccine against influenza virus H5N1 (if available). Alternatively, the vaccine may also be administered before or after administration of the molecules of the invention. Instead of a vaccine, anti-viral agents can also be employed in conjunction with the binding molecules of the present invention. Suitable anti-viral agents are mentioned above.

The molecules are typically formulated in the compositions and pharmaceutical compositions of the invention in a therapeutically or diagnostically effective amount. Alternatively, they may be formulated and administered separately. For instance the other molecules such as the anti-viral agents may be applied systemically, while the binding molecules of the invention may be applied intravenously.

Dosage regimens can be adjusted to provide the optimum desired response (*e.g.,* a therapeutic response). A suitable dosage range may for instance be 0.1-100 mg/kg body weight, preferably 0.5-15 mg/kg body weight. Furthermore, for example, a single bolus may be administered, several divided doses may be administered over time or the dose may be proportionally reduced or increased as indicated by the exigencies of the therapeutic situation. The molecules and compositions according to the present invention are preferably sterile. Methods to render these molecules and compositions sterile are well known in the art. The other molecules useful in diagnosis, prophylaxis and/or treatment can be administered in a similar dosage regimen as proposed for the binding molecules of the invention. If the other molecules are administered separately, they may be administered to a patient prior to (*e.g.,* 2 min, 5 min, 10 min, 15 min, 30 min, 45 min, 60 min, 2 hrs, 4 hrs, 6 hrs, 8 hrs, 10 hrs, 12 hrs, 14 hrs, 16 hrs, 18 hrs, 20 hrs, 22 hrs, 24 hrs, 2 days, 3 days, 4 days, 5 days, 7 days, 2 weeks, 4 weeks or 6 weeks before), concomitantly with, or subsequent to (*e.g.,* 2 min, 5 min, 10 min, 15 min, 30 min, 45 min, 60 min, 2 hrs, 4 hrs, 6 hrs, 8 hrs, 10 hrs, 12 hrs, 14 hrs, 16 hrs, 18 hrs, 20 hrs, 22 hrs, 24 hrs, 2 days, 3 days, 4 days, 5 days, 7 days, 2 weeks, 4 weeks or 6 weeks after) the administration of one or more of the human binding molecules or pharmaceutical compositions of the invention. The exact dosing regimen is usually sorted out during clinical trials in human patients.

Human binding molecules and pharmaceutical compositions comprising the human binding molecules are particularly useful, and often preferred, when to be administered to human beings as *in vivo* therapeutic agents, since recipient immune response to the administered antibody will often be substantially less than that occasioned by administration of a monoclonal murine, chimeric or humanized binding molecule.

In another aspect, the invention concerns the use of the binding molecules such as neutralizing human monoclonal antibodies (functional fragments and variants thereof), immunoconjugates, nucleic acid molecules, compositions or pharmaceutical compositions according to the invention in the preparation of a medicament for the diagnosis, prophylaxis, treatment, or combination thereof, of an influenza virus H5N1 infection.

Next to that, kits comprising at least a binding molecule such as a neutralizing human monoclonal antibody (functional fragments and variants thereof), at least an immunoconjugate, at least a nucleic acid molecule, at least a composition, at least a pharmaceutical composition, at least a vector, at least a host according to the invention or a combination thereof are also a part of the present invention. Optionally, the above-described components of the kits of the invention are packed in suitable containers and labelled for diagnosis, prophylaxis and/or treatment of the indicated conditions. The above-mentioned components may be stored in unit or multi-dose containers as an aqueous, preferably sterile, solution or as a lyophilised, preferably sterile, formulation for reconstitution. The containers may be formed from a variety of materials such as glass or plastic and may have a sterile access port (for example, the container may be an intravenous solution bag or a vial having a stopper pierceable by a hypodermic injection needle). The kit may further comprise more containers comprising a pharmaceutically acceptable buffer. It may further include other materials desirable from a commercial and user standpoint, including other buffers, diluents, filters, needles, syringes, culture medium for one or more of the suitable hosts and, possibly, even at least one other therapeutic, prophylactic or diagnostic agent. Associated with the kits can be instructions customarily included in commercial packages of therapeutic, prophylactic or diagnostic products, that contain information about for example the indications, usage, dosage, manufacture, administration, contra-indications and/or warnings concerning the use of such therapeutic, prophylactic or diagnostic products.

The disclosure further provides a method of detecting influenza virus H5N1 in a sample, wherein the method comprises the steps of (a) contacting a sample with a diagnostically effective amount of a binding molecule (functional fragments and variants thereof) or an immunoconjugate according to the invention, and (b) determining whether the binding molecule or immunoconjugate specifically binds to a molecule of the sample. The sample may be a biological sample including, but not limited to blood, serum, stool, sputum, nasophargyal aspirates, bronchial lavages, urine, tissue or other biological material from (potentially) infected subjects, or a non-biological sample such as water, drink, etc. The (potentially) infected subjects may be human subjects, but also animals that are suspected as carriers of influenza virus H5N1 might be tested for the presence of the virus using the human binding molecules or immunoconjugates of the invention. The sample may first be manipulated to make it more suitable for the method of detection. Manipulation means *inter alia* treating the sample suspected to contain and/or containing the virus in such a way that the virus will disintegrate into antigenic components such as proteins, (poly)peptides or other antigenic fragments. Preferably, the human binding molecules or immunoconjugates of the invention are contacted with the sample under conditions which allow the formation of an immunological complex between the human binding molecules and the virus or antigenic components thereof that may be present in the sample. The formation of an immunological complex, if any, indicating the presence of the virus in the sample, is then detected and measured by suitable means. Such methods include, *inter alia,* homogeneous and heterogeneous binding immunoassays, such as radio-immunoassays (RIA), ELISA, immunofluorescence, immunohistochemistry, FACS, BIACORE and Western blot analyses.

Preferred assay techniques, especially for large-scale clinical screening of patient sera and blood and blood-derived products are ELISA and Western blot techniques. ELISA tests are particularly preferred. For use as reagents in these assays, the binding molecules or immunoconjugates of the invention are conveniently bonded to the inside surface of microtiter wells. The binding molecules or immunoconjugates of the invention may be directly bonded to the microtiter well. However, maximum binding of the binding molecules or immunoconjugates of the invention to the wells might be accomplished by pre-treating the wells with polylysine prior to the addition of the binding molecules or immunoconjugates of the invention. Furthermore, the binding molecules or immunoconjugates of the invention may be covalently attached by known means to the wells. Generally, the binding molecules or immunoconjugates are used between 0.01 to 100 µg/ml for coating, although higher as well as lower amounts may also be used. Samples are then added to the wells coated with the binding molecules or immunoconjugates of the invention.

Furthermore, binding molecules of the invention can be used to identify specific binding structures of influenza virus H5N1. The binding structures can be epitopes on proteins and/or polypeptides. They can be linear, but also structural and/or conformational. In one embodiment, the binding structures can be analysed by means of PEPSCAN analysis (see *inter alia* WO 84/03564, WO 93/09872, Slootstra *et al.,* 1996). Alternatively, a random peptide library comprising peptides from a protein of influenza virus H5N1 can be screened for peptides capable of binding to the binding molecules of the invention. The binding structures/peptides/epitopes found can be used as vaccines and for the diagnosis of influenza virus H5N1 infections. In case fragments other than proteins and/or polypeptides are bound by the binding molecules, binding structures can be identified by mass spectrometry, high performance liquid chromatography and nuclear magnetic resonance.

In a further aspect, the disclosure provides a method of screening a binding molecule (or a functional fragment or variant thereof) for specific binding to the same epitope of influenza virus H5N1, as the epitope bound by a human binding molecule of the invention, wherein the method comprises the steps of (a) contacting a binding molecule to be screened, a binding molecule of the invention and influenza virus H5N1 or a fragment thereof, (b) measure if the binding molecule to be screened is capable of competing for specifically binding to influenza virus H5N1 or a fragment thereof with the binding molecule of the invention. In a further step it may be determined, if the screened binding molecules that are capable of competing for specifically binding to influenza virus H5N1 or a fragment thereof have neutralizing activity. A binding molecule that is capable of competing for specifically binding to influenza virus H5N1 or a fragment thereof with the binding molecule of the invention is another part of the present invention. In the above-described screening method, "specifically binding to the same epitope" also contemplates specific binding to substantially or essentially the same epitope as the epitope bound by the a binding molecule of the invention. The capacity to block, or compete with, the binding of the binding molecules of the invention to influenza virus H5N1 typically indicates that a binding molecule to be screened binds to an epitope or binding site on influenza virus H5N1 that structurally overlaps with the binding site on influenza virus H5N1 that is immunospecifically recognized by the binding molecules of the invention. Alternatively, this can indicate that a binding molecule to be screened binds to an epitope or binding site which is sufficiently proximal to the binding site immunospecifically recognized by the binding molecules of the invention to sterically or otherwise inhibit binding of the binding molecules of the invention to influenza virus H5N1.

In general, competitive inhibition is measured by means of an assay, wherein an antigen composition, *i.e.* a composition comprising influenza virus H5N1 or fragments thereof, is admixed with reference binding molecules, *i.e.* the binding molecules of the invention, and binding molecules to be screened. Usually, the binding molecules to be screened are present in excess. Protocols based upon ELISAs and Western blotting are suitable for use in such simple competition studies. By using species or isotype secondary antibodies one will be able to detect only the bound reference binding molecules, the binding of which will be reduced by the presence of a binding molecule to be screened that recognizes substantially the same epitope. In conducting a binding molecule competition study between a reference binding molecule and any binding molecule to be screened (irrespective of species or isotype), one may first label the reference binding molecule with a detectable label, such as, *e.g.,* biotin, an enzymatic, a radioactive or other label to enable subsequent identification. Binding molecules identified by these competition assays ("competitive binding molecules" or "cross-reactive binding molecules") include, but are not limited to, antibodies, antibody fragments and other binding agents that bind to an epitope or binding site bound by the reference binding molecule, *i.e.* a binding molecule of the invention, as well as antibodies, antibody fragments and other binding agents that bind to an epitope or binding site sufficiently proximal to an epitope bound by the reference binding molecule for competitive binding between the binding molecules to be screened and the reference binding molecule to occur. Preferably, competitive binding molecules of the invention will, when present in excess, inhibit specific binding of a reference binding molecule to a selected target species by at least 10%, preferably by at least 25%, more preferably by at least 50%, and most preferably by at least 75%-90% or even greater. The identification of one or more competitive binding molecules that bind to about, substantially, essentially or at the same epitope as the binding molecules of the invention is a straightforward technical matter. As the identification of competitive binding molecules is determined in comparison to a reference binding molecule, *i.e.* a binding molecule of the invention, it will be understood that actually determining the epitope to which the reference binding molecule and the competitive binding molecule bind is not in any way required in order to identify a competitive binding molecule that binds to the same or substantially the same epitope as the reference binding molecule.

### EXAMPLES

To illustrate the invention, the following examples are provided. The examples are not intended to limit the scope of the invention in any way.

### Example 1

*Constriction of scFv phage display libraries using RNA extracted from memory B cells* Peripheral blood was collected from normal healthy donors by venapuncture in EDTA anticoagulation sample tubes. A blood sample (45 ml) was diluted twice with PBS and 30 ml aliquots were underlayed with 10 ml Ficoll-Hypaque (Pharmacia) and centrifuged at 900xg for 20 min at room temperature without breaks. The supernatant was removed carefully to just above the white layer containing the lymphocytic and thrombocytic fraction. Next, this layer was carefully removed (∼10 ml), transferred to a fresh 50 ml tube and washed three times with 40 ml PBS and spun at 400xg for 10 min at room temperature to remove thrombocytes. The obtained pellet containing lymphocytes was resuspended in RPMI medium containing 2% FBS and the cell number was determined by cell counting. Approximately 1x10⁸ lymphocytes were stained for fluorescent cell sorting using CD24, CD27 and surface IgM as markers for the isolation of IgM memory B cells. A Becton Dickinson Digital Vantage apparatus set in Yield Mode was used for physical memory B cell sorting and isolation. Lymphocytes were gated as the small compact population from the FSC/SSC window. Memory B cells (CD24+/CD27+) were subsequently separated from naive B cells (CD24+/CD27-) and memory T cells (CD24-/CD27+). In a next step, IgM memory B cells (IgM+) were separated from switch memory B cells (IgM-) using IgM expression. In this step IgM memory B cells were sorted in a separate sample tube. 1x10⁵ cells were collected in DMEM/50% FBS and after completion of the sort they were centrifuged at 400xg for 10 min and lysed in 500 µl TRIZOL total RNA extraction solution (Invitrogen). The RNA was extracted from the lysis solution using 200 µl chloroform and isopropanol precipitation as detailed in the TRIZOL solution protocol. Next, 1 µl Pellet Paint (Novogen) was applied to enhance and visualize the pelleting process. The complete RNA preparation was dissolved in 23 µl DEPC treated ultrapure water (Invitrogen) and used for cDNA conversion with SuperScript III Reverse Transcriptase (Invitrogen). 1 µl Random Hexamers (500 ng/µl) (Promega) was added to the RNA sample and mixed and melted at 65°C for 5 min in a heated lid PCR machine. The sample was snap-cooled on wet-ice and the following components were added: 8 µl 5X RT buffer (250 mM Tris-HCl pH 8.3, 375 mM KCl, 15 mM MgCl₂), 2 µl dNTPs (10 mM of each) (Invitrogen), 2 µl DTT (100 mM), 2 µl RNAse Inhibitor (40 U/µl) (Promega), 2 µl SuperScript III (200 U/µl) (Invitrogen). The obtained mixture was first incubated for 5 min at room temperature and then transferred to a heated lid PCR machine at 50°C for one hr. The reaction was stopped by heating up to 75°C for 15 min. The cDNA obtained was diluted to 200 µl with ultrapure water and stored at -20°C until further use.

A two round PCR amplification approach was applied using the primer sets shown in Tables 1 and 2 to isolate the immunoglobulin VH and VL regions from the respective donor repertoire. The PCR formulation for amplification used throughout the procedure was as follows: 5 µl cDNA template, 32.75 µl ultrapure water, 2.5 µl of each primer (10 µM), 5 µl 10X PCR buffer (200 mM Tris-HCl pH 8.4, 500 mM KCl), 1.5 µl MgCl₂ (50 mM), 0.5 µl dNTP's (25 mM of each), 0.25 µl Taq polymerase (5 U/µl) (Invitrogen). First round amplification on the respective cDNA using the primer sets mentioned in Table 1 yielded 7, 6 and 9 products of about 650 base pairs for respectively VH, Vkappa and Vlambda regions. For IgM memory B cell VH region amplification the OCM constant primer was used in combination with OH1 to OH7. The thermal cycling program for first round amplifications was: 2 min 96°C (denaturation step), 30 cycles of 30 sec 96°C/ 30 sec 55°C/ 60 sec 72°C, 10 min 72°C final elongation and 4°C refrigeration. The products were loaded on and isolated from a 1% agarose gel using gel-extraction columns (Qiagen) and eluted in 50 µl 1 mM Tris-HCl pH 8.0. Ten percent of first round products (5 µl) was subjected to second round amplification using the primers mentioned in Table 2. These primers were extended with restriction sites enabling the directional cloning of the respective VL and VH regions into phage display vector PDV-C06. The PCR program for second round amplifications was as follows: 2 min 96°C (denaturation step), 30 cycles of 30 sec 96°C/ 30 sec 60°C/ 60 sec 72°C, 10 min 72°C final elongation and 4°C refrigeration. The second round products (∼350 base pairs) were first pooled according to natural occurrence of J segments found in immunoglobulin gene products, resulting in 7, 6 and 9 pools for respectively the VH, Vkappa and Vlambda variable regions (see Tables 3 and 4). To obtain a normalized distribution of immunoglobulin sequences in the immune library the 6 Vkappa and 9 Vlambda light chain pools were mixed according to the percentages mentioned in Table 3. This single final VL pool (3 µg) was digested overnight with SalI and NotI restriction enzymes, loaded on and isolated from a 1.5% agarose gel (∼350 base pairs) using Qiagen gel-extraction columns and ligated in SalI-NotI cut PDV-C06 vector (∼5000 base pairs) as follows: 10 µl PDV-C06 vector (50 ng/µl), 7 µl VL insert (10 ng/µl), 5 µl 10X ligation buffer (NEB), 2.5 T4 DNA Ligase (400 U/µl) (NEB), 25.5 µl ultrapure water (vector to insert ratio was 1:2). Ligation was performed overnight in a water bath of 16°C. Next, the volume was doubled with water, extracted with an equal volume of phenol-chloroform-isoamylalcohol (75:24:1) (Invitrogen) followed by chloroform (Merck) extraction and precipitated with 1 µl Pellet Paint (Novogen), 10 µl sodium acetate (3 M pH 5.0) and 100 µl isopropanol for 2 hrs at -20°C. The obtained sample was subsequently centrifuged at 20.000xg for 30 min at 4°C. The obtained precipitate was washed with 70% ethanol and centrifuged for 10 min at 20.000xg at room temperature. Ethanol was removed by vacuum aspiration and the pellet was air dried for several min and then dissolved in 50 µl buffer containing 10 mM Tris-HCl, pH 8.0. 1 µl ligation mixture was used for the transformation of 40 µl TG-1 electro-competent cells (Stratagene) in a chilled 0.1 cm electroporation cuvette (Biorad) using a Genepulser II apparatus (Biorad) set at 1.7 kV, 200 Ohm, 25 µF (time constant ∼4,5 msec). Directly after pulse, the bacteria were flushed from the cuvette with 1000 µl SOC medium (Invitrogen) containing 5% (w/v) glucose (Sigma) at 37°C and transferred to a 15 ml round bottom culture tube. Another 500 µl SOC/glucose was used to flush residual bacteria from the cuvette and was added to the culture tube. Bacteria were recovered by culturing for exactly one hr at 37°C in a shaker incubator at 220 rpm. The transformed bacteria were plated over large 240 mm square petridishes (NUNC) containing 200 ml 2TY agar (16 g/l bacto-tryptone, 10 g/l bacto-yeast extract, 5 g/l NaCl, 15 g/l agar, pH 7.0) supplemented with 50 µg/ml ampicillin and 5% (w/v) glucose (Sigma). A 1 to 1000 dilution was plated for counting purposes on 15 cm petridishes containing the same medium. This transformation procedure was repeated sequentially twenty times and the complete library was plated over a total of thirty large square petridishes and grown overnight in a 37°C culture stove. Typically, around 1x10⁷ cfu were obtained using the above protocol. The intermediate VL light chain library was harvested from the plates by mildly scraping the bacteria into 10 ml 2TY medium per plate. The cell mass was determined by OD600 measurement and two times 500 OD of bacteria was used for maxi plasmid DNA preparation using two P500 maxiprep columns (Qiagen) according to manufacturer's instructions.

Analogous to the VL variable regions, the second round VH-JH products were first mixed together to obtain the normal J segment usage distribution (see Table 4), resulting in 7 VH subpools called PH1 to PH7. The pools were mixed to acquire a normalized sequence distribution using the percentages depicted in Table 4, obtaining one VH fraction that was digested with SfiI and XhoI restriction enzymes and ligated in SfiI-XhoI cut PDV-VL intermediate library obtained as described above. The ligation set-up, purification method, subsequent transformation of TG1 and harvest of bacteria was exactly as described for the VL intermediate library (see above). The final library (approximately 5x10⁶ cfu) was checked for insert frequency with a colony PCR using a primer set flanking the inserted VH-VL regions. More than 95% of the colonies showed a correct length insert (see Table 5). The colony PCR products were used for subsequent DNA sequence analysis to check sequence variation and to assess the percentage of colonies showing a complete ORF. This was typically above 70% (see Table 5). The frequency of mutations in the V genes was also analysed. Out of 50 sequences, 47 (94%) were not in germline configuration indicative of a maturation process and consistent with the memory phenotype of the B cells used as an RNA source for the library. Finally, the library was rescued and amplified by using CT helper phages (see WO 02/103012) and was used for phage antibody selection by panning methods as described below.

### Example 2

### Selection of phages carrying single chain Fv fragments against H5N1

Antibody fragments were selected using antibody phage display libraries constructed essentially as described above and general phage display technology and MABSTRACT® technology essentially as described in US Patent Number 6,265,150 and in WO 98/15833. Furthermore, the methods and helper phages as described in WO 02/103012 were used in the present invention.

Selection was performed against recombinant hemagglutinin (HA) subtype H5 (A/Vietnam/1203/2004; see SEQ ID NO:110) produced using baculovirus vectors in insect cells (Protein Sciences, CT, USA). The amino acid sequence of HA from isolate A/Vietnam/1194/2004 (H5N1TV) and the consensus amino acid sequence representing HAs from strains isolated in Indonesia and China (H5N1IC) are shown in SEQ ID NO:111 and SEQ ID NO:112, respectively. Selections were also performed against soluble recombinant HA (sHA) from H5N1. The sequence encoding for the extracellular (soluble) part of HA (sHA) from isolate A/Vietnam/1194/2004 (H5N1), representing the HAs identified in influenza strains isolated in Thailand and Vietnam (sHA of H5N1TV, SEQ ID NO:113) in 2004 (clade 1) and a consensus sequence representing soluble parts of HAs of H5N1 strains isolated in Indonesia and China (sHA of H5N1IC, SEQ ID NO:114) in 2003/2004 (clade 2) were cloned in an expression vector containing a myc- and his-tag using standard DNA cloning techniques. HAs of H5N1TV and H5N1IC differ at 9 amino acid positions, all located in the HA1 subunit of the molecules. DNA transfections were performed in PER.C6 cells for transient and/or stable expression using standard techniques. sHA was purified from culture supernatant using metal chelate affinity chromatography using HisTrap™ FF Columns (Amersham Biosciences) according to the manufacturer's instructions.

HA antigens (recombinant HA and sHA of H5N1TV) were diluted in PBS (5.0 µg/ml), added to MaxiSorp™ Nunc-Immuno Tubes (Nunc) and incubated overnight at 4°C on a rotating wheel. The immunotubes were emptied and washed three times in block buffer (2% non-fat dry milk (ELK) in PBS). Subsequently, the immunotubes were filled completely with block buffer and incubated for 1-2 hrs at room temperature. In addition, immunotubes were coated overnight with anti-myc antibody and incubated with block buffer containing 5 µg/ml myc-tagged sHA of H5N1TV. Aliquots of pooled phage display library (500-1000 µl, 0.5x10¹³ - 1x10¹³ cfu, amplified using CT helper phage (see WO 02/103012)) were blocked in blocking buffer supplemented with 10% non-heat inactivated fetal bovine serum and 2% mouse serum for 1-2 hrs at room temperature. The blocked phage library was added to the immunotubes, incubated for 2 hrs at room temperature, and washed with wash buffer (0.05% (v/v) Tween-20 in PBS) to remove unbound phages. Bound phages were eluted from the respective antigen by incubation with 1 ml of 100 mM triethylamine (TEA) for 10 min at room temperature. Subsequently, the eluted phages were mixed with 0.5 ml of 1 M Tris-HCl pH 7.5 to neutralize the pH. This mixture was used to infect 5 ml of an XL1-Blue *E.coli* culture that had been grown at 37°C to an OD 600 nm of approximately 0.3. The phages were allowed to infect the XL1-Blue bacteria for 30 min at 37°C. Then, the mixture was centrifuged for 10 min at 3000xg at room temperature and the bacterial pellet was resuspended in 0.5 ml 2-trypton yeast extract (2TY) medium. The obtained bacterial suspension was divided over two 2TY agar plates supplemented with tetracycline, ampicillin and glucose. After incubation overnight of the plates at 37°C, the colonies were scraped from the plates and used to prepare an enriched phage library, essentially as described by De Kruif *et al.* (1995a) and WO 02/103012. Briefly, scraped bacteria were used to inoculate 2TY medium containing ampicillin, tetracycline and glucose and grown at a temperature of 37°C to an OD 600 nm of ∼0.3. CT helper phages were added and allowed to infect the bacteria after which the medium was changed to 2TY containing ampicillin, tetracycline and kanamycin. Incubation was continued overnight at 30°C. The next day, the bacteria were removed from the 2TY medium by centrifugation after which the phages in the medium were precipitated using polyethylene glycol (PEG) 6000/NaCl. Finally, the phages were dissolved in 2 ml of PBS with 1% bovine serum albumin (BSA), filter-sterilized and used for the next round of selection.

Alternatively, phage selections using full-length HA-expressing cell lines were performed. To this end, expression vectors containing the complete coding sequence of full-length HA from isolate A/Vietnam/1194/2004 (H5N1TV) and a consensus sequence representing full-length HAs of H5N1 strains isolated in Indonesia and China (H5N1IC) were used to transfect PER.C6 cells. Flow cytometry analysis using an anti-HA antibody was performed in parallel with each phage selection to assure HA expression by H5N1TV- and H5N1IC-transfected PER.C6 cells (data not shown). 10x10⁶ untransfected PER.C6 cells were resuspended in 0.5 ml pooled phage library and 0.5 ml PER.C6 culture medium supplemented with 4% ELK and incubated for 1 hr in an end-over-end rotor set at 5 rpm at 4°C. After 5 min centrifugation at 300xg at 4°C of the phage library/untransfected PER.C6 cell mixture, the supernatant containing the phages was subtracted for a second time with 10x10⁶ untransfected PER.C6 cells. The subtracted phage preparation was subsequently mixed with 1x10⁶ HA-expressing PER.C6 cells and incubated for 2 hrs in an end-over-end rotor set at 5 rpm at 4°C. Subsequently, the cells were spun for 2 min at 3000xg and the cell pellet was resuspended in 1 ml PBS/0.05% Tween-20 to wash away unbound phages. The cells were centrifuged for 2 min at 3000xg and the washing was repeated for an additional 5 times. After each wash the cells were transferred to a new tube. Bound phages were eluted from the antigen by incubation with 1 ml of 100 mM TEA for 10 min in an end-over-end rotor set at 5 rpm at room temperature. The cells were spun for 2 min at 3000xg and the eluted phages were transferred to a 50 ml tube containing 0.5 ml 1 M Tris-HCl, pH 7.5. Rescue and propagation of the eluted phages was performed as described above. Two rounds of selections were performed before isolation of individual single-chain phage antibodies. After the second round of selection, individual *E.coli* colonies were used to prepare monoclonal phage antibodies. Essentially, individual colonies were grown to log-phase in 96 well plate format and infected with VCS-M13 helper phages after which phage antibody production was allowed to proceed overnight. The supernatants containing phage antibodies were used directly in ELISA for binding to HA antigens. Alternatively, phage antibodies were PEG/NaCl-precipitated and filter-sterilized for flow cytometry analysis.

### Example 3

### Validation of the HA specific single-chain phage antibodies

Selected single-chain phage antibodies that were obtained in the screening described above were validated in ELISA for specificity, *i.e.* binding to HA antigens. For this purpose, baculovirus expressed recombinant HA (Protein Sciences, CT, USA) and purified sHAs of H5N1TV and H5N1IC were coated to Maxisorp™ ELISA plates. Anti-myc mA 9E10 (Roche) was immobilized on Maxisorp™ ELISA plates as negative control antigen. After coating, the plates were washed three times with PBS containing 0.1% v/v Tween-20 and blocked in PBS containing 3% BSA or 2% ELK for 1 hr at room temperature. The selected single-chain phage antibodies were incubated for 1 hr in an equal volume of PBS containing 4% ELK to obtain blocked phage antibodies. The plates were emptied, washed three times with PBS/0.1% Tween-20 and the blocked single-chain phage antibodies were added to the wells. Incubation was allowed to proceed for one hr, the plates were washed with PBS/0.1% Tween-20 and bound phage antibodies were detected (using OD 492nm measurement) using an anti-M13 antibody conjugated to peroxidase. As a control, the procedure was performed simultaneously without single-chain phage antibody and with a negative control single-chain phage antibody. From the selections on the different HA antigens with the IgM memory B cell library, 92 unique single-chain phage antibodies specific for either recombinant HA or sHA were obtained.

Alternatively, the reactivity of single chain antibodies that were selected for binding to HA-expressing PER.C6 cells was tested using flow cytometry analysis. PEG/NaCl precipitated phages were mixed with an equal volume of PBS/2% ELK blocked for 30 min on ice. The blocked phages were added to pelleted cells (untransfected PER.C6 and HA-expressing PER.C6 cells) and incubated for one hr on ice. The cells were washed three times with PBS/1% BSA, followed by a 1 minute centrifugation at 300xg, and binding of the single chain phage antibodies to the cells was visualized using a biotinylated anti-M13 antibody (Fitzgerald) followed by streptavidin-phycoerythrin conjugate (Caltag). The selections using HA-expressing PER.C6 cells provided 24 unique single-chain phage antibodies that were not identified during selections using the different recombinant HA proteins.

### Example 4

### Characterization of the HA specific scFvs

From the selected specific single-chain phage antibodies (scFv) clones plasmid DNA was obtained and nucleotide and amino acid sequences were determined according to standard techniques. The nucleotide and amino acid sequence and VH and VL gene identity (see Tomlinson IM et al. V-BASE Sequence Directory. Cambridge United Kingdom: MRC Centre for Protein Engineering (1997)) of the scFvs are depicted in Table 6. The CDR regions of the HA- specific immunoglobulins are shown in Table 7.

### Example 5

### Constriction of fully human immunoglobulin molecules (human monoclonal antibodies) from the selected single chain Fvs

Heavy and light chain variable regions of the scFvs were cloned directly by restriction digest for expression in the IgG expression vectors pIg-C911-HCgammal (see SEQ ID No:141), pIG-C909-Ckappa (see SEQ ID NO:142), or pIg-C910-Clambda (see SEQ ID No: 143). Nucleotide sequences for all constructs were verified according to standard techniques known to the skilled artisan. The resulting expression constructs encoding the human IgG1 heavy and light chains were transiently expressed in combination in 293T cells and supernatants containing human IgG1 antibodies were obtained and produced using standard purification procedures. The human IgG1 antibodies were titrated in a concentration range of between 10 and 0.003 µg/ml against H5 (data not shown). A SARS-CoV specific antibody was included as a control antibody. The IgG1 molecules showed the same pattern of reactivity as demonstrated for the single-chain phage antibodies.

The nucleotide and amino acid sequences of the heavy and light chain of the antibodies called CR6141, CR6255, CR6257, CR6260, CR6261, CR6262, CR6268, CR6272, CR6296, CR6301, CR6307, CR6310, CR6314, CR6323, CR6325, CR6327, CR6328, CR6329, CR6331, CR6332, CR6334, CR6336, CR6339, CR6342, CR6343 and CR6344 as well as their heavy and light chain variable regions are given in Table 8. Subsequently, binding of the anti-HA IgG to HA-expressing PER.C6 cells was investigated by flow cytometry. Flow cytometry analysis for antibody binding to HA demonstrated that antibodies CR6255, CR6257, CR6260, CR6261, CR6262, CR6268, CR6307, CR6310, CR6314, CR6323, CR6325, CR6331 and CR6344 bound to HA (H5N1TV)-expressing PER.C6 cells (see Table 9). Antibodies CR6261 and CR6344 also displayed binding to untransfected control cells, but the obtained signals were approximately 10-fold lower than the signals obtained for binding to HA (H5N1TV)-expressing PER.C6 cells (see Table 9). No binding of control antibody CR3014 to HA-expressing cells and untransfected control cells was observed.

### Example 6

### In vitro neutralization of H5N1 influenza virus by H5N1 specific IgGs (virus neutralization assay)

In order to determine whether the selected IgGs were capable of blocking H5N1 infection, *in vitro* virus neutralization assays (VNA) were performed. The VNA were performed on MDCK cells (ATCC CCL-34). MDCK cells were cultured in MDCK cell culture medium (MEM medium supplemented with antibiotics, 20 mM Hepes and 0.15% (w/v) sodium bicarbonate (complete MEM medium), supplemented with 10% (v/v) fetal bovine serum). The H5N1 reassortant strain NIBRG-14 which was used in the assay was diluted to a titer of 4x10³ TCID50/ml (50% tissue culture infective dose per ml), with the titer calculated according to the method of Spearman and Karber. The IgG preparations (200 µg/ml) were serially 2-fold diluted (1:2 - 1:16) in complete MEM medium in duplicate wells. 25 µl of the respective IgG dilution was mixed with 25 µl of virus suspension (100 TCID50/25 µl) and incubated for one hr at 37°C. The suspension was then transferred in duplicate onto 96-well plates containing confluent MDCK cultures in 50 µl complete MEM medium. Prior to use, MDCK cells were seeded at 3x10⁴ cells per well in MDCK cell culture medium, grown until cells had reached confluence, washed with 300-350 µl PBS, pH 7.4 and finally 50 µl complete MEM medium was added to each well. The inoculated cells were cultured for 3-4 days at 33°C and observed daily for the development of cytopathic effect (CPE). CPE was compared to the positive control (NIBRG-14-inoculated cells) and negative controls (mock-inoculated cells). The complete absence of CPE in an individual cell culture was defined as protection. Sheep anti-A/Vietnam/1194/04 H5N1 influenza virus HA (04/214, NIBSC) was used as a positive control in the assay.

In addition, cultures were tested for the presence of virus using immunohistochemistry. To this end, the culture supernatant was discarded and the cells were fixed with 40% (v/v) acetone and 60% (v/v) methanol for 15 min. Fixed cells were blocked for 30 min at 37°C in blocking buffer (200 mM NaCl, 0.2% (w/v) bovine serum albumin (BSA), 0.01% thimerosal, 0.2% (v/v) Tween-20, 20 mM Tris-HCl, pH 7.45) supplemented with 2% (w/v) BSA and 5% (v/v) goat serum. Subsequently, cells were incubated for one hr at 37°C with 50 µl mouse anti-influenza A monoclonal antibody blend (Chemicon) diluted 1:1000 in washing buffer. After three washes with washing buffer, 50 µl of biotin-conjugated goat anti-mouse IgG (Jackson) diluted 1:1000 in washing buffer was added and incubated for one hr at 37°C. After three washes with washing buffer, 50 µl of streptavidin-peroxidase conjugate (Calbiochem) diluted 1:3000 in washing buffer was added and incubated for 30 min at 37°C. After another three washes with washing buffer, staining was visualized using AEC solution (0.12% (w/v) 3-amino-9-ethylcarbazole, 30% (v/v) N-N-dimethylformamide and 70% (v/v) acetate buffer) containing 1 µl H₂O₂/1 ml AEC solution. After three washes with washing buffer, staining was analysed under a microscope.

The human anti-HA antibodies called CR6255, CR6257, CR6260, CR6261, CR6262, CR6268, CR6307, CR6310, CR6314, CR6323, CR6325, CR6331 and CR6344 were subjected to the above-described VNA. All antibodies neutralized H5N1 reassortant strain NIBRG-14. The concentrations (in µg/ml) at which these antibodies protect MDCK cultures against CPE are given in Table 10. In wells where CPE was observed, the presence of virus was confirmed by immunohistochemical staining (data not shown).

In order to determine the neutralizing potency of the H5N1 specific IgGs more accurately, the *in vitro* virus neutralization assays with NIBRG-14 was repeated, but this time the IgG preparations were further diluted. The IgG preparations (200 µg/ml) were serially 2-fold diluted (1:1 - 1:512) in complete MEM medium and tested in quadruplicate wells in the VNA as described above. The neutralizing potency of the human anti-H5N1 antibodies called CR6255, CR6257, CR6260, CR6261, CR6262, CR6268, CR6272, CR6307, CR6310, CR6314, CR6323, CR6325, CR6327, CR6328, CR6329, CR6331, CR6332, CR6334, CR6336, CR6339, CR6342, CR6343 and CR6344 was determined in the VNA. All antibodies, except CR6272 and CR6339, neutralized H5N1 reassortant strain NIBRG-14. The concentrations (in µg/ml, in the presence of 100 TCID50 of virus) at which these antibodies protect MDCK cultures against CPE are indicated in Table 11. In wells where CPE was observed, the presence of virus was confirmed by immunohistochemical staining (data not shown).

### Example 7

### Immunoblot analysis of H5N1 specific IgGs

To further investigate the specificity of the anti-HA antibodies, different recombinant hemagglutinin Influenza A antigens were subjected to SDS-PAGE under reducing conditions followed by anti-HA immunoblot analysis. The HA0 polypeptide is composed of the HA1 and HA2 subunit. Antibody CAR5111, a H5N1-specific control antibody, recognized the HA1 subunit and the intact (uncleaved) HA0 polypeptide of sHA of H5N1TV (see Fig 1, right part, lane 1). In addition, CR5111 recognized the HA1 subunit of recombinant HA, subtype H5 (A/Vietnam/1203/2004 (H5N1); see SEQ ID NO:110) produced using baculovirus vectors in insect cells (Protein Sciences, CT, USA) (Fig 1, right part, lane 2). Uncleaved HA0 polypeptide was not detected in this HA preparation. The difference in size between the HA subunits in lanes 1 and 2 might be explained by a different glycosylation of HA expressed in insect cells and PER.C6 cells. CR5 111 did not recognize the HA1 and/or HA0 polypeptides of recombinant HA, subtype H1 (A/New Caledonia/20/99 (H1N1)) (see Fig 1, right part, lane 4) or of recombinant HA, subtype H3 (A/Wyoming/3/2003(H3N2)) (Fig 1, right part, lane 3).

Antibodies CR6307 and CR6323 recognized the HA2 subunits of sHA of H5N1TV (see Fig 1, left and middle part, lanes 1) and recombinant HA, subtype H5 (A/Vietnam/1203/2004 (H5N1) (see Fig 1, left and middle part, lanes 2). Interestingly, the HA2 subunit as present in the intact, uncleaved HA0 polypeptides was not recognized. Apparently, the epitope recognized by CR6307 and CR6323 becomes accessible upon cleavage of HA0. In addition, antibodies CR6307 and CR6323 recognized the HA2 subunit of recombinant HA, subtype H1 (A/New Caledonia/20/99 (H1N1)) (see Fig 1, left and middle part, lanes 4), but not the HA2 subunit of recombinant HA, subtype H3 (A/Wyoming/3/2003(H3N2)) (see Fig 1, left and middle part, lanes 3), both recombinant HAs produced using baculovirus vectors in insect cells (Protein Sciences, CT, USA). Because the binding site of neutralizing antibodies CR6307 and CR6323 is conserved within influenza A strains of the subtype H1 and H5, a molecule comprising the binding site could be considered as (part of a) vaccine capable of inducing a broadly cross-reactive anti-influenza antibody response.

Next to antibodies CR6307 and CR6323, the antibodies called CR6141, CR6296, and CR6301 were used in immunoblot analysis. Each of the three antibodies was able to bind to the HA2 subunit of sHA of H5N1TV and recombinant HA, subtype H5 (A/Vietnam/1203/2004; H5N1) (data not shown).

### Example 8

### FACS analysis to determine the subunit specificity of H5N1 specific IgGs

In order to evaluate the contribution of the HA1 subunit to binding of anti-H5N1 antibodies, an assay was set up, wherein the HA1 subunit is released from HA-expressing PER.C6 cells. HA-expressing PER.C6 cells were washed three times with PBS, pH 7.4, and incubated for 10 min in acidified PBS, pH 4.9. Subsequently, the cells were washed with PBS, pH 7.4, and treated for 10 min with 10 µg/ml trypsin in PBS, pH 7.4, to cleave HA0 molecules into disulfide bond-linked HA1 and HA2 subunits. Finally, the cells were incubated for 20 min in 20 mM DTT in PBS, pH 7.4, to release the HA1 subunit from the membrane-anchored HA2 subunit. Untreated and acid/trypsin/DTT-treated cells were washed twice with PBS containing 1% w/v BSA. For flow cytometry analysis, 2.5x10⁵ cells were used per staining. No staining of treated and untreated cells with the negative control antibody CR3014 was observed. Antibody CR5111, which binds to the HA1 subunit in immunoblot analysis, stained the population of untreated HA-expressing PER.C6 cells, while treated cells were not recognized (data not shown). This provides further proof that CR5111 recognizes the HA1 subunit. Treated and untreated cells were stained to a similar extent by the antibodies CR6307 and CR6323 (data not shown). Release of the HA1 subunit obviously did not influence binding of these antibodies further substantiating the results from the immunoblot analysis that the antibodies bind to the HA2 subunit. Antibodies CR6325 and CR6331 bound to untreated cells, while binding to treated cells was markedly reduced compared with binding to untreated cells (data not shown). This suggests that the affinity of the antibodies for their epitope is significantly reduced by the conformational change induced upon acid treatment or by the reduction of the disulfide bond that links the HA1 and HA2 subunit.

### Example 9

### Hemagglutinin competition ELISA with phage antibodies and IgGs

To identify antibodies that bind to non-overlapping, non-competing epitopes, a hemagglutinin competition ELISA was performed. Nunc-Immuno™ Maxisorp F96 plates were coated overnight at 4°C with 0.5 µg/ml recombinant HA, subtype H5 (A/Vietnam/1203/2004 (H5N1) (Protein Sciences) in PBS. Uncoated protein was washed away before the wells were blocked with 300 µl PBS containing 2% w/v non-fat dry milk (blocking solution) for 1 hr at room temperature. The blocking solution was discarded and 50 µl of 10 µg/ml anti-H5N1 IgG in blocking solution was incubated per well for 1 hr at room temperature. Subsequently, 50 µl of phage antibody in blocking solution in a concentration twice of the concentration that resulted in 50% maximal binding (as determined in a previous assay) was added per well and incubated for another hr at room temperature. Wells were washed three times with PBS containing 0.1 % v/v Tween-20. Bound phage antibody was detected using a peroxidase-conjugated anti-M13 antibody. Wells were washed again as described above and the ELISA was further developed by the addition of 100 µl of OPD reagent (Sigma). The reaction was stopped by adding 50 µl 1 M H₂SO₄ and then the OD at 492 nm was measured. Binding of phage antibody, in the presence of IgG, was expressed as percentage of binding in the absence of IgG, which was set at 100%. Phage antibody SC05-111 and corresponding IgG CAR5111, which recognize an epitope in the HA1 subunit of H5 hemagglutinin were included as controls.

The results indicate that the majority of the phage antibodies and IgGs bind to an overlapping epitope on recombinant HA, subtype H5 (A/Vietnam/1203/2004 (H5N1)) (data not shown). Binding of SC05-111 phage antibody was blocked by CR5 111 IgG, but not by any other IgG, suggesting that the other IgGs recognize an epitope different from the binding region of CR5111. Five IgGs, CR6262, CR6272, CR6307, CR6339 and CR6343 competed to a lower extent for binding than the rest of the IgGs (more than 25% residual binding). For IgGs CR6262, CR6272, CR6339, and CR6343 this can be explained by a lower affinity for HA. This is supported by the observation that the phage antibodies corresponding to these antibodies were competed away more efficiently by the other IgGs. Binding of phage antibody SC06-307 is blocked by IgG CR6307, but less efficiently by the other IgGs. This suggests that SC06-307 and CR6307 recognize a unique epitope which differs from the epitope recognized by the other IgGs.

### Example 10

### Cross-reactivity ELISA using anti-H5N1 IgGs

To test whether the epitope of the anti-H5N1 antibodies is conserved among HAs other than subtype H5, a hemagglutinin cross-reactivity ELISA was performed. Nunc-Immuno™ Maxisorp F96 plates (Nunc) were coated overnight at 4°C with 0.5 µg/ml recombinant HA, subtype H1 (A/New Caledonia/20/99 (H1N1)), subtype H3 (A/Wyoming/3/03 (H3N2)), subtype H5 (A/Vietnam/1203/04 (H5N1)), subtype H7 (A/Netherlands/219/03 (H7N7)), and subtype H9 (A/Hong Kong/1073/99 (H9N2)) (Protein Sciences Corp.) in PBS. Additionally, BPL-inactivated virus preparations containing 0.5 µg/ml of HA of A/New Caledonia/20/99 (H1N1), and reassortant strain NIBRG-14 (A/Vietnam/1194/04 (H5N1)) were coated on the plates overnight in PBS. Wells were washed three times with PBS containing 0.1% v/v Tween-20 to remove uncoated protein and subsequently blocked with 300 µl PBS containing 2% w/v non-fat dry milk (blocking solution) for 1 hr at room temperature. The blocking solution was discarded and 100 µl per well of 5 µg/ml anti-H5N1 antibodies in blocking solution was incubated for 1 hr at room temperature. Wells were washed three times with PBS containing 0.1% v/v Tween-20 and bound antibodies were detected using a peroxidase-conjugated mouse anti-human IgG antibody (Jackson). The reaction was developed and measured as described *supra.* Table 12 shows that all anti-H5N1 IgGs bound to recombinant HA, subtype H5 (A/Vietnam/1203/2004 (H5N1)) and a BPL-inactivated virus preparation of NIBRG-14, which contains the HA of strain A/Vietnam/1194/2004 (H5N1). Recombinant HAs of subtype H3 and H7 were not recognized by any of the tested anti-H5N1 IgGs. Interestingly, all anti-H5N1 IgGs, with the exception of CR5111 and CR6307, bound to recombinant HA of subtypes H1 and H9, and a BPL-inactivated virus preparation of strain A/New Caledonia/20/99 (H1N1). This indicates that the epitope of the majority of the anti-H5N1 IgGs is conserved among HA molecules of different subtypes.

### Example 11

### Epitope mapping of anti-H5N1 IgGs

Okuno et al. (1993) and Smirnov et al. (1999) demonstrated the existence of a common epitope shared by the HAs of the influenza A virus subtypes H1, H2, and H5 and neutralization of these subtypes by the murine monoclonal antibody C179 directed against this epitope. This conformational epitope is composed of two different sites which are located in the HA1 and HA2 subunit. Both sites are located in close proximity in the middle of the stem region of the HA molecule. In order to evaluate whether the anti-HA antibodies described *supra* recognized this epitope, HA molecules containing amino acid substitutions in this region were made.

The epitope recognized by antibody C179 (Takara Bio Inc.) has been attributed to regions encompassing residues 318-322 of the HA1 subunit and residues 47-58 of the HA2 subunit (amino acid numbering as described by Okuno et al.1999). Escape viruses containing HAs that carry a Thr to Lys substitution at position 318 in the HA1 subunit or a Val to Glu substitution at position 52 in the HA2 subunit were no longer recognized and neutralized by C179. These mutations (position 318 Thr to Lys, mutant I; position 52 Val to Glu, mutant II), a Leu to Met substitution at position 320 in the HA1 subunit (mutant III, Met320 is present in region 318-322 of HA1 of subtype H3 and H7), a Ser to Arg substitution at position 54 in the HA2 subunit (mutant IV, Arg54 is present in region 47-58 of HA2 of subtype H3 and H7) and an Asp to Asn substitution at position 57 in the HA2 subunit (mutant V, Asn57 is present in region 47-58 of HA2 of strain A/Hong Kong/156/97 (H5N1)) were introduced in full-length HA from isolate A/Vietnam/1194/2004 (H5N1TV) and tranfected in PER.C6 cells. Binding of C179 and the human anti-HA antibodies to PER.C6 cells expressing these mutants was evaluated by FACS analysis as described *supra.* Antibody CAR5111, which is directed against an epitope in the HA1 subunit, and a polyclonal anti-H5 sheep serum confirmed the expression of H5N1TV and HA mutants by transfected PER.C6 cells (data not shown). No staining with the negative control antibody CR3014 or in the absence of antibody was observed (data not shown). As expected, antibody C 179 did not recognize mutants I and II, which carried the same amino acid substitutions as the HAs in the C 179 escape viruses (see Okuno *et al.,* 1993). Furthermore, C179 did not bind to mutant IV, whereas binding of C179 to mutants III and V was unaffected. A similar pattern of reactivity was observed for antibody CR6342, which suggests that antibody C179 and CR6342 recognize a similar epitope. Antibodies CR6261, CR6325 and CR6329 recognized all mutants, with the exception of mutant II. This suggests that the epitope of antibodies CR6261, CR6325 and CR6329 is different from that of C179. Since substitutions in the HA1 subunit did not abrogate binding of these antibodies, their epitope is most likely located in the HA2 subunit. Antibodies CR6307 and CR6323 recognized all mutants, which suggests that also the epitope of these antibodies is different from that of C179. A summary of the sequence of the mutants and binding of the antibodies is given in Table 13. In conclusion, the results indicate that antibodies CR6261, CR6325, CR6329, CR6307 and CR6323 are ideal candidates to bind to and neutralize influenza viruses that have mutations in the epitope recognized by the murine monoclonal antibody C179 and as a consequence thereof are no longer neutralized by this antibody.

### Example 12

### Prophylactic activity of human IgG monoclonal antibodies against lethal H5N1 challenge in vivo

A lethal dose of influenza H5N1 strain A/HongKong/156/97 was administered to mice in order to study the prophylactic effect of human monoclonal IgG antibodies CR6261, CR6323 and CR6325. One day prior to infection 8 groups of 10 mice each were injected intraperitoneally with different doses of antibody. As a negative control one group of mice was injected with a non-relevant control antibody (CR3014). Clinical signs, weight loss and mortality were monitored until 21 days after infection. This study was conducted to assess the prophylactic effect of the monoclonal human anti-H5N1 IgG antibodies in vivo.

The H5N1 strain was originally obtained from a 3-year-old child suffering from respiratory disease. The virus was passaged two times on MDCK cells. The batch [Titre 8.1 log TCID₅₀ /ml] used to infect mice was propagated once in embryonated eggs.

80 female 7-week-old Balb/c mice were divided in the 8 groups of 10 mice each with the following injections prior to challenge with the H5N1 virus:
1. 15 mg/kg CR6261.
2. 5 mg/kg CR6261.
3. 2 mg/kg CR6261.
4. 0.7 mg/kg CR6261.
5. 15 mg/kg CR6323.
6. 15 mg/kg CR6325
7. 500µl rabbit anti-H5N3 immune serum (100x diluted).
8. 15 mg/kg CR3014.

All animals were acclimatized and maintained for a period of 6 days prior to the start of the study. One day prior to infection with H5N1 virus, 500 µl of antibody was administered by intraperitoneal injection. The animals were inoculated intranasally on day 0 with 25 LD₅₀ of virus (approximately 50 µl), and followed for 21 days. The actual dose of the virus administered was estimated by titrating a few replicate samples from the inoculum remaining after inoculation of the animals was completed. Virus titers (TCID50/mL) of the inoculum were determined on MDCK cells. The results showed that no inactivation of virus had unintentionally occurred during preparation or administration of the inoculum. Group 8 acted as negative control. The animals in this group were injected with an irrelevant monoclonal antibody (CR3014) on day 0. Group 7 was supposed to act as positive control. The mice in this group were injected with a rabbit polyclonal serum antibody raised against H5N3 influenza virus.

Clinical signs and weights were assessed daily from day -1 until 21 days after virus inoculation. Clinical signs were scored with a scoring system (0 = no clinical signs; 1 = rough coat; 2 = rough coat, less reactive, passive during handling; 3 = rough coat, rolled up, laboured breathing, passive during handling; 4 = rough coat, rolled up, laboured breathing, does not roll back on stomach when laid down on its back) and recorded. Surviving animals were euthanised and bled on day 21. For analysis of serum IgG antibody levels blood samples were collected from each mouse on day 0. Sera were prepared according to standard procedure. To generate post infection sera, blood was collected from surviving animals on day 21. Sera were stored at -20 °C ± 2°C until assayed for the presence of virus specific antibodies. Sera were tested in duplicate using 4 HAU of the H5N1 HK/97 substrate. Titers were expressed as the reciprocal of the highest serum dilution showing HI, starting at a dilution of 1/10.

All mice were active and appeared healthy without showing signs of disease during the acclimatization period. The average clinical score (total clinical score divided by number of surviving animals in each group) was calculated per day and the results are indicated in Fig 2 (average clinical score per group), Table 14 (clinical scores) and Table 15 (respiratory distress). Onset of negative clinical signs was observed at day 3 after infection in the group that was inoculated with the negative control Ab CR3014 (group 8). Respiratory distress was first recorded on day 6, and lasted for 1 to 4 days. No clinical signs were observed in mice that were treated with 15 mg/kg of CR6325 (group 6). In group 5 (CR6323), one mouse showed mild clinical signs (score 1) from day 5 to 8 and died the next day. In group 1, one mice died on day 13 without having shown any previous clinical signs. Higher Ab doses of CR6261 correlated with later onset and lower clinical scores on average. Respiratory distress was noticed in the lowest dose group (0.7 mg/kg), but not in the higher dose groups (2, 5 and 15 mg/kg). All mice showed improvement in their clinical condition between day 9 and 13 (group 2, 3 and 5) or from day 17 on (group 4). Mice that were injected with the rabbit polyclonal antibody (group 7) developed severe illness within 3 days and then died, demonstrating that the rabbit antibody did not protect against infection in vivo. Two animals (one in group 4 and one in group 8) were euthanized on day 10 and removed from the study, because the animals were considered to be severely ill (score 4).

The animals infected with H5N1 showed varying degrees of weight loss (Fig 3). Proportional weight loss and the moment of onset were related to antibody dose. In the groups treated with the highest dose of antibodies weight steadily increased over time consistent with age-related weight gain. The group of animals inoculated with the lowest dose of CR6261 lost weight more quickly than the groups of animals receiving higher doses of antibody. The total amount of weight loss was greater in the lower dose groups, with average weight loss of about 15 and 40% of starting weight in the groups inoculated with 2 and 0.7 mg/kg CR6261, respectively. In the lower dose groups average body weight appeared to increase again at the same time as animals showed some clinical improvement.

Fig 4 shows the number of mice surviving per group on each day. A clear dose-response relationship between the amount of antibody administered and average survival time was present. Fig 5 shows the mortality in a dose-responsive manner. The first mice died 7 days after inoculation in the lowest dose group (0.7 mg/kg) and in the group of mice that received the negative control: CR3014. Less than 50% of the animals were protected against death when 0.7 mg/kg of CR6261 antibody was administered. However, 9 to 10 mice survived when the highest dose of antibody was administered. No mice of the negative control group (CR3014) survived, showing that indeed a 100% lethal challenge dose was used in this study.

To assess whether IgG antibodies are able to render complete protection against infection, an HI assay is performed with sera collected at day 21 from mice that had received 15 mg/kg of CR6261 (group 1). This data should indicate that the mice experienced an H5N1 infection albeit without clinical manifestation.

These results show that at least three human anti-H5N1 antibodies, identified and developed as disclosed herein (CR6261, CR6323 and CR6325) are each separately able to provide protection against a lethal dose of influenza H5N1 in vivo. A clear dose-response relationship between the amount of CR6261 antibody administered and average survival time was observed. The results show that each monoclonal anti-H5N1 IgG antibody tested was able to prevent clinical manifestation of H5N1 infection in mice when administered one day prior to infection at a dose of 15 mg/kg.

### Example 13

### Projective and therapeutic, effects of human monoclonal anti-H5N1 antibodies administered after an infection with a lethal dose of influenza H5N1 virus in vivo

A study was performed to test the therapeutic effect of the monoclonal antibodies as disclosed herein, exemplified by CR6261, in a post-infection model, against a lethal H5N1 A/HK/97 influenza virus challenge in vivo. The virus batch and the type, and age of mice were the same as used in example 12. As a negative control one group of mice was injected with a non-relevant control antibody (CR2006). Clinical signs, weight loss and mortality were monitored until 21 days after infection.

58 female 7-week-old Balb/c mice were divided in 5 groups that received the antibody at different stages after infection, as follows:
1. 10 mice; 15 mg/kg CR6261 at 4 hr post infection
2. 14 mice; 15 mg/kg CR6261 at 1 day post infection
3. 10 mice; 15 mg/kg CR6261 at 2 days post infection
4. 10 mice; 15 mg/kg CR6261 at 3 days post infection
5. 14 mice; 15 mg/kg CR2006 at 1 day post infection

All animals were acclimatized and maintained for a period of 6 days prior to the start of the study. The animals were inoculated intranasally on day 0 with 25 LD₅₀ of H5N1 influenza virus (approximately 50 µl), and monitored for 21 days. The actual dose of the virus administered was estimated by titrating a few replicate samples from the inoculum remaining after inoculation of the animals was completed. Virus titers (TCID50/mL) of the inoculum were determined on MDCK cells. The results showed that no inactivation of virus had unintentionally occurred during preparation or administration of the inoculum. At the specified time points after inoculation, 500 µl of antibody was administered by intraperitoneal injection. Group 5 acted as negative control. The animals in this group were injected with an irrelevant monoclonal antibody (CR2006) day 1 post-infection.

Clinical signs and weights were assessed each day from day -1 until day 21. Clinical signs were scored as described in example 12, with a scoring range from 0 to 4. Surviving animals were euthanised and bled on day 21. For assessment of pathological changes, 4 animals of group 2 and 5 were killed on day 6 after challenge. These animals were already pre-selected on day 0, and set apart from the others. For that reason, groups 2 and 5 started with 14 animals, with 10 mice remaining after the selection. Clinical signs and weights were assessed daily from day -1 until 21 days after virus inoculation.

All mice were active and appeared healthy without showing signs of disease during the acclimatization period. The average clinical score (total clinical score divided by number of surviving animals in each group) was calculated per day and the results are indicated in Fig 6 (average clinical score per group), Table 16 (clinical scores) and Table 17 (respiratory distress). Clearly, all groups contained mice that showed clinical signs already at day 1 after infection. Depending on the time at which the antibody was administered, the clinical signs diminished and in all groups clinical signs were absent again at day 15. In the control group 5, all animals suffered from severe clinical signs and all animals had died, or were euthanized because they reached level 4 in the clinical scores, at day 9. This shows that again a lethal dose of influenza virus was administered to the animals. In Group 1, wherein the animals already received the antibody 4 hr after infection, some animals did not develop clinical signs, whereas others did. The number of animals that did exhibit clinical signs that could be scored are provided in Table 16. Since influenza virus can have a dramatic effect on the respiratory organs, also the respiratory distress was measured and here provided in Table 17. Fig 7 and Table 18 show the number of surviving animals and the mortality rate respectively for all groups. For unknown reasons, one animal in group 1 that received the antibody 4 hr after infection died at day 10. All remaining animals in the groups that received the antibody after the influenza infection survived and were healthy at day 21. This is clearly shown in the body weight data that was obtained from all mice. Fig 8 shows the mean body weight in each group of mice during the 21 days of the study. Although the body weight of all mice decreased upon infection, the body weight did return to normal levels after administration of the antibody. Clearly, the return to normal body weight levels depended on the timing of the antibody treatment, where animals that were treated 4 hr after exposure, recovered rapidly and reached normal levels at day 7, the animals that were treated 3 days after infection, reached their normal body weight at day 17. All animals reached a similar and healthy body weight at the end of the study, at day 21. Clearly, all animals that received the negative control antibody did not regain body weight and measurements stopped at death at day 9.

These results show that a post-infection treatment with a monoclonal antibody directed against H5N1 influenza virus, as disclosed herein and exemplified by antibody CR6261, can rescue mammalian subjects, as showed herein in mice, after challenge with a lethal dose of H5N1 influenza virus. Even at a late stage, 3 days post-infection, the antibody is able to reverse the clinical symptoms to a level in which no clinical signs could be monitored anymore. Strikingly, at day 21 post-infection, all antibody-treated animals reached normal body weight levels and did not show any remaining clinical signs.

### Example 14

### In vivo cross-protection against lethal challenge by heterologous influenza subtypes using monoclonal antibodies directed against HA of H5N1.

As disclosed supra (example 11), some of the antibodies of the present invention apparently recognize a single epitope in the HA2 domain of the H5 haemagglutinin protein. In example 7 it was shown that certain binding molecules of the present invention could interact with the HA2 epitope in a non-conformational manner, in other words, the form in which the haemagglutinin protein is folded does not seem to hinder the neutralizing activity and the protective effects of these antibodies as disclosed herein.

The sequences of the influenza haemagglutinin protein parts that are not prone to antigenic drift (= the change of immunodominant regions within the HA1 region of the protein, resulting in the need for yearly updated influenza vaccines) are known in the art. The epitope in HA2 that is recognized by CR6261, CR6325 and CR6329 is contained in the amino acid sequence GVTNKVNSIIDK (SEQ ID NO:368, see Table 13) and is also present in the haemagglutinin proteins of H1 and H9 subtypes, see Table 22. It was shown in example 10 that interaction of the binding molecules of the present invention to the epitope was not limited to HA from H5, but that also HA from H1 and H9 were recognized (see Table 12). Hence, the binding molecules of the present invention recognize an epitope that is present in multiple HA proteins from different influenza serotypes.

To study the cross-applicability of these antibodies for therapy in vivo, an experiment was performed in mice that was in line with the dosing schedule of the experiment described in example 13. In the present experiment, a very high, lethal dose of another influenza virus related to the 1918 pandemic outbreak and now circulating seasonally in humans, namely H1N1, was administered in mice. Subsequently, the mice were treated with an antibody of the present invention, exemplified by CR6261, and clinical signs, respiratory distress and body weight were monitored for 3 weeks after infection. It turned out that these binding molecules that could rescue mice when infected with H5N1, were also able to rescue mammalian subjects when infected with H1N1, as outlined below.

A study was performed to test the therapeutic effect of the monoclonal antibodies, exemplified by CR6261, in a post-infection model against a lethal H1N1 A/WSN/33 influenza virus challenge in vivo. The virus batch was obtained from ATCC (VR-219) and was once propagated in embryonated eggs. The titre was 8.5 log TCID₅₀/ml. As a negative control one group of mice was injected with an irrelevant monoclonal antibody (IgG1, λ named 'CR57', isotype matched negative control antibody). Clinical signs, weight loss and mortality were monitored until 21 days after infection.

50 female 6 to 8-week-old Balb/c mice were divided in 5 groups that received the antibody at different stages related to the infection, as follows:
1. 10 mice; 15 mg/kg CR6261 at 1 day prior to infection
2. 10 mice; 15 mg/kg CR6261 at 1 day post infection
3. 10 mice; 15 mg/kg CR6261 at 2 days post infection
4. 10 mice; 15 mg/kg CR6261 at 3 days post infection
5. 10 mice; 15 mg/kg neg. contr. CR57 at 1 day post infection

All animals were acclimatized and maintained for a period of at least 4 days prior to the start of the study. The animals were inoculated intranasally on day 0 with a lethal dose of H1N1 virus (6.6 log TCID₅₀; equivalent of 25x the LD₅₀) in approximately 50 µl, and monitored. At the specified time points before/after inoculation, 500 µl of antibody was administered by intraperitoneal injection. General health of the mice was monitored throughout the study. Clinical signs and weights were assessed each day from day -1 until day 21. Clinical signs were scored with the scoring rates as disclosed in example 12 and 13, ranging from 0 to 4. Surviving animals were euthanised and bled on day 21.

The mortality rate for each group is provided in Table 19, showing the number of living mice in each study group throughout the study. Two mice died shortly after the inoculation event (at day 1, one in group 2 and one in group 3) and were excluded from the analysis as pre-defined in the study plan. All mice in the control group 5 were dead at day 9 like in the previous study with H5N1 (see Table 18). The percentage of animals surviving this lethal challenge dose of H1N1 is also plotted in Fig 9. The number of mice showing relevant clinical signs in each group is provided in Table 20. No clinical signs were observed in Group 1, whereas in Group 2, 3 and 4, some mice displayed clinical signs that disappeared completely after day 14 upon inoculation. All animals in Group 5 started showing clinical signs at day 2. No mice in this group recovered. The number of mice showing respiratory distress in category 2 or 3 is given in Table 21. No clinical distress was observed anymore after day 13 in Groups 1-4, whereas all remaining mice in control Group 5 did suffer from severe respiratory distress.

Fig 10 shows the average body weight of the mice in each study group. Clearly, no measurements are provided for the mice in Group 5 after day 8. As can be seen from this figure, all mice that received the anti-H5N1 antibody and that recovered from the clinical signs did get to their expected body weight level at day 21.

The antibodies could protect these mice when administered before infection or after infection. Notably, the infection dose was rather high: 25x the LD₅₀ dose, indicating that the antibodies provide a very strong protection against the virus even when present at high titers in the lung. This is clinically relevant as highly pathogenic viruses like H5N1 replicate to high titer after infection and these high viral loads have been linked to the frequently severe outcome in infected humans. Moreover, all protected mice recovered completely from this lethal infection over time. It is concluded that the anti-H5N1 antibodies of the present invention (such as CR6261, CR6325, and CR6329) that bind to (single) epitopes in the HA2 region, a region that is not prone to antigenic drift, provide cross-protection in vivo against multiple influenza serotypes, circumventing the need for antibodies against the highly mutation-sensitive HA1 region. It is to be understood that the binding molecules of the present invention that are not limited by epitopes that are only present in HA from an H5 influenza serotype, can also be used in the prophylactic-, or therapeutic treatment of all influenza serotypes that contain the same epitope in the stable region of HA2, such as H1N1, and influenza viruses comprising H2, H6 and H9 haemagglutinin proteins.

### Example 15

### Affinity studies

Affinity studies were performed using surface plasmon resonance analysis with a BIAcore3000 analytical system at 25°C and 37°C, using HBS-EP (Biacore AB, Sweden) as running buffer at a flow rate of 75 µl/min. IgGs were immobilized on a research grade CM5 4-flow channel (Fc) sensor chip (Biacore AB, Sweden) using amine coupling. A varying amount of HA from an H5N1 virus (A/Vietnam/1203/2004) was injected to analyse the binding interaction between the HA protein and the immobilized IgGs. Regeneration using 20 mM NaOH was performed at the end of each measurement to remove bound HA, while leaving the immobilized IgG on the chip.

Affinity constants were determined for CR6261, CR6323 and CR6325 antibodies. Five concentrations in 4-fold dilutions of HA were injected (100 µl per injection), followed by a dissociation phase of 3600 sec, and regeneration using 10 µl 20 mM NaOH. The resulting data were fitted using a 1:1 (Langmuir) model. However, an accurate dissociation constant (KD) could not be calculated. This was due to extremely low dissociation rates at 25°C (even with an extended measurement) leading to unacceptable error in the calculation. When the experiments were repeated at 37°C discernible dissociation did occur but still not sufficiently enough to accurately measure the KD. Experiments are performed to establish a definitive KD for the antibodies. These are estimated to be at least in the single digit nM range and most likely in the pM range of affinity. These experiments show that the binding molecules of the present invention have a very high affinity for their epitope present in the HA protein of influenza virus.

Below, the details of the nucleic acid and amino acid sequences referred to herein as SEQ ID NO:58-143, SEQ ID NO:212-237, and SEQ ID NO:316-367 are provided.
SEQ ID NO:58
SEQ ID NO:59
SEQ ID NO:60
SEQ ID NO:61
SEQ ID NO:62
SEQ ID NO:63
SEQ ID NO:64
SEQ ID NO:65
SEQ ID NO:66
SEQ ID NO:67
SEQ ID NO:68
SEQ ID NO:69
SEQ ID NO:70
SEQ ID NO:71
SEQ ID NO:72
SEQ ID NO:73
SEQ ID NO:74
SEQ ID NO:75
SEQ ID NO:76
SEQ ID NO:77
SEQ ID NO:78
SEQ ID NO:79
SEQ ID NO:80
SEQ ID NO:81
SEQ ID NO:82
SEQ ID NO:83
SEQ ID NO:84
SEQ ID NO:85
SEQ ID NO:86
SEQ ID NO:87
SEQ ID NO:88
SEQ ID NO:89
SEQ ID NO:90
SEQ ID NO:91
SEQ ID NO:92
SEQ ID NO:93
SEQ ID NO:94
SEQ ID NO:95
SEQ ID NO:96
SEQ ID NO:97
SEQ ID NO:98
SEQ ID NO:99
SEQ ID NO:100
SEQ ID NO:101
SEQ ID NO:102
SEQ ID NO:103
SEQ ID NO:104
SEQ ID NO:105
SEQ ID NO:106
SEQ ID NO:107
SEQ ID NO:108
SEQ ID NO:109
SEQ ID NO:110
SEQ ID NO:111
SEQ ID NO:112
SEQ ID NO:113
SEQ ID NO:114
SEQ ID NO:115
SEQ ID NO:116
SEQ ID NO:117
SEQ ID NO:118
SEQ ID NO:119
SEQ ID NO:120
SEQ ID NO:121
SEQ ID NO:122
SEQ ID NO:123
SEQ ID NO:124
SEQ ID NO:125
SEQ ID NO:126
SEQ ID NO:127
SEQ ID NO:128
SEQ ID NO:129
SEQ ID NO:130
SEQ ID NO:131
SEQ ID NO:132
SEQ ID NO:133
SEQ ID NO:134
SEQ ID NO:135
SEQ ID NO:136
SEQ ID NO:137
SEQ ID NO:138
   SEQ ID NO:139
SEQ ID NO:140
SEQ ID NO:141
SEQ ID NO:142
SEQ ID NO:143
SEQ ID NO:212
SEQ ID NO:213
SEQ ID NO:214
SEQ ID NO:215
SEQ ID NO:216
SEQ ID NO:217
SEQ ID NO:218
SEQ ID NO:219
SEQ ID NO:220
SEQ ID NO:221
SEQ ID NO:222
SEQ ID NO:223
SEQ ID NO:224
SEQ ID NO:225
SEQ ID NO:226
SEQ ID NO:227
SEQ ID NO:228
SEQ ID NO:229
SEQ ID NO:230
SEQ ID NO:231
SEQ ID NO:232
SEQ ID NO:233
SEQ ID NO:234
SEQ ID NO:235
SEQ ID NO:236
SEQ ID NO:237
SEQ ID NO:316
SEQ ID NO:317
SEQ ID NO:318
SEQ ID NO:319
SEQ ID NO:320
SEQ ID NO:321
SEQ ID NO:322
SEQ ID NO:323
SEQ ID NO:324
SEQ ID NO:325
SEQ ID NO:326
SEQ ID NO:327
SEQ ID NO:328
SEQ ID NO:329
SEQ ID NO:330
SEQ ID NO:331
SEQ ID NO:332
SEQ ID NO:333
SEQ ID NO:334
SEQ ID NO:335
SEQ ID NO:336
SEQ ID NO:337
SEQ ID NO:338
SEQ ID NO:339
   SEQ ID NO:340
SEQ ID NO:341
SEQ ID NO:342
SEQ ID NO:343
SEQ ID NO:344
SEQ ID NO:345
SEQ ID NO:346
SEQ ID NO:347
SEQ ID NO:348
SEQ ID NO:349
SEQ ID NO:350
SEQ ID NO:351
SEQ ID NO:352
SEQ ID NO:353
SEQ ID NO:354
SEQ ID NO:355
SEQ ID NO:356
SEQ ID NO:357
SEQ ID NO:358
SEQ ID NO:359
SEQ ID NO:360
SEQ ID NO:361
SEQ ID NO:362
SEQ ID NO:363
SEQ ID NO:364
SEQ ID NO:365
SEQ ID NO:366
SEQ ID NO:367

**Table 1: First round Vkappa, Vlambda and VH amplifications**

| Primer name | Primer nucleotide sequence | SEQ ID NO: |
|---|---|---|
| OK1 (HuVK1B) | GAC ATC CAG WTG ACC CAG TCT CC | 144 |
| OK2 (HuVK2) | GAT GTT GTG ATG ACT CAG TCT CC | 145 |
| OK3 (HuVK2B2) | GAT ATT GTG ATG ACC CAG ACT CC | 146 |
| OK4 (HuVK3B) | GAA ATT GTG WTG ACR CAG TCT CC | 147 |
| OK5 (HuVK5) | GAA ACG ACA CTC ACG CAG TCT CC | 148 |
| OK6 (HuVK6) | GAA ATT GTG CTG ACT CAG TCT CC | 149 |
| OCK (HuCK) | ACA CTC TCC CCT GTT GAA GCT CTT | 150 |
| OL1 (HuVL1A)* | CAG TCT GTG CTG ACT CAG CCA CC | 151 |
| OL1 (HuVL1B)* | CAG TCT GTG YTG ACG CAG CCG CC | 152 |
| OL1 (HuVL1C)* | CAG TCT GTC GTG ACG CAG CCG CC | 153 |
| OL2 (HuVL2B) | CAG TCT GCC CTG ACT CAG CC | 154 |
| OL3 (HuVL3A) | TCC TAT GWG CTG ACT CAG CCA CC | 155 |
| OL4 (HuVL3B) | TCT TCT GAG CTG ACT CAG GAC CC | 156 |
| OL5 (HuVL4B) | CAG CYT GTG CTG ACT CAA TC | 157 |
| OL6 (HuVL5) | CAG GCT GTG CTG ACT CAG CCG TC | 158 |
| OL7 (HuVL6) | AAT TTT ATG CTG ACT CAG CCC CA | 159 |
| OL8 (HuVL7/8) | CAG RCT GTG GTG ACY CAG GAG CC | 160 |
| OL9 (HuVL9)# | CWG CCT GTG CTG ACT CAG CCM CC | 161 |
| OL9 (HuVL10)# | CAG GCA GGG CTG ACT CAG | 162 |
| OCL (HuCL2)X | TGA ACA TTC TGT AGG GGC CAC TG | 163 |
| OCL (HuCL7)X | AGA GCA TTC TGC AGG GGC CAC TG | 164 |
| OH1(HuVH1B7A)+ | CAG RTG CAG CTG GTG CAR TCT GG | 165 |
| OH1 (HuVH1C)+ | SAG GTC CAG CTG GTR CAG TCT GG | 166 |
| OH2 (HuVH2B) | CAG RTC ACC TTG AAG GAG TCT GG | 167 |
| OH3 (HuVH3A) | GAG GTG CAG CTG GTG GAG | 168 |
| OH4 (HuVH3C) | GAG GTG CAG CTG GTG GAG WCY GG | 169 |
| OH5 (HuVH4B) | CAG GTG CAG CTA CAG CAG TGG GG | 170 |
| OH6 (HuVH4C) | CAG STG CAG CTG CAG GAG TCS GG | 171 |
| OH7 (HuVH6A) | CAG GTA CAG CTG CAG CAG TCA GG | 172 |
| OCM (HuCIgM) | TGG AAG AGG CAC GTT CTT TTC TTT | 173 |

| | | |
|---|---|---|
| * Mix in 1:1:1 ratio # Mix in 1:1 ratio X Mix in 1:1 ratio + Mix in 1:1 ratio | | |

**Table 2: Second round Vkappa, Vlambda and VH amplifications**

| Primer name | Primer nucleotide sequence | SEQ ID NO |
|---|---|---|
| OK1S (HuVK1B-SAL) | | 174 |
| OK2S (HuVK2-SAL) | | 175 |
| OK3S (HuVK2B2-SAL) | | 176 |
| OK4S (HuVK3B-SAL) | | 177 |
| OK5S (HuVK5-SAL) | | 178 |
| OK6S (HuVK6-SAL) | | 179 |
| OJK1 (HuJK1-NOT) | | 180 |
| OJK2 (HuJK2-NOT) | | 181 |
| OJK3 (HuJK3-NOT) | | 182 |
| OJK4 (HuJK4-NOT) | | 183 |
| OJK5 (HuJK5-NOT) | | 184 |
| OL1S (HuVL1A-SAL)* | | 185 |
| OL1S (HuVL1B-SAL)* | | 186 |
| OL1S (HuVL1C-SAL)* | | 187 |
| OL2S (HuVL2B-SAL) | | 188 |
| OL3S (HuVL3A-SAL) | | 189 |
| OL4S (HuVL3B-SAL) | | 190 |
| OL5S (HuVL4B-SAL) | | 191 |
| OL6S (HuVL5-SAL) | | 192 |
| OL7S (HuVL6-SAL) | | 193 |
| OL8S (HuVL7/8-SAL) | | 194 |
| OL9S (HuVL9-SAL)# | | 195 |
| OL9S (HuVL10-SAL)# | | 196 |
| OJL1 (HuJL1-NOT) | | 197 |
| OJL2 (HuJL2/3-NOT) | | 198 |
| OJL3 (HuJL7-NOT) | | 199 |
| OH1S (HuVH1B-SFI)+ | | 200 |
| OH1S (HuVH1C-SFI)+ | | 201 |
| OH2S (HuVH2B-SFI) | | 202 |
| OH3S (HuVH3A-SFI) | | 203 |
| OH4S (HuVH3C-SFI) | | 204 |
| OH5S (HuVH4B-SFI) | | 205 |
| OH6S (HuVH4C-SFI) | | 206 |
| OH7S (HuVH6A-SFI) | | 207 |
| OJH1 (HuJH1/2-XHO) | | 208 |
| OJH2 (HuJH3-XHO) | | 209 |
| OJH3 (HuJH4/5-XHO) | | 210 |
| OJH4 (HuJH6-XHO) | | 211 |

| | | |
|---|---|---|
| * Mix in 1:1:1 ratio # Mix in 1:1 ratio + Mix in 1:1 ratio | | |

**Table 3. Second round VL regions amplification overview**

| Template | 5' primer | 3' primer | Product | Share in PK/PL(%) | Pool | Share in VL (%) |
|---|---|---|---|---|---|---|
| K1 | OK1S | OJK1 | K1J1 | 25 | PK1 | 30 |
| | OK1S | OJK2 | K1J2 | 25 | | |
| | OK1S | OJK3 | K1J3 | 10 | | |
| | OK1S | OJK4 | K1J4 | 25 | | |
| | OK1S | OJK5 | K1J5 | 15 | | |
| K2 | OK2S | OJK1 | K2J1 | 25 | PK2 | 4 |
| | OK2S | OJK2 | K2J2 | 25 | | |
| | OK2S | OJK3 | K2J3 | 10 | | |
| | OK2S | OJK4 | K2J4 | 25 | | |
| | OK2S | OJK5 | K2J5 | 15 | | |
| K3 | OK3S | OJK1 | K3J1 | 25 | PK3 | 1 |
| | OK3S | OJK2 | K3J2 | 25 | | |
| | OK3S | OJK3 | K3J3 | 10 | | |
| | OK3S | OJK4 | K3J4 | 25 | | |
| | OK3S | OJK5 | K3J5 | 15 | | |
| K4 | OK4S | OJK1 | K4J1 | 25 | PK4 | 19 |
| | OK4S | OJK2 | K4J2 | 25 | | |
| | OK4S | OJK3 | K4J3 | 10 | | |
| | OK4S | OJK4 | K4J4 | 25 | | |
| | OK4S | OJK5 | K4J5 | 15 | | |
| K5 | OK5S | OJK1 | K5J1 | 25 | PK5 | 1 |
| | OK5S | OJK2 | K5J2 | 25 | | |
| | OK5S | OJK3 | K5J3 | 10 | | |
| | OK5S | OJK4 | K5J4 | 25 | | |
| | OK5S | OJK5 | K5J5 | 15 | | |
| K6 | OK6S | OJK1 | K6J1 | 25 | PK6 | 5 |
| | OK6S | OJK2 | K6J2 | 25 | | |
| | OK6S | OJK3 | K6J3 | 10 | | |
| | OK6S | OJK4 | K6J4 | 25 | | |
| | OK6S | OJK5 | K6J5 | 15 | | |
| L1 | OL1S | OJL1 | L1J1 | 30 | PL1 | 14 |
| | OL1S | OJL2 | L1J2 | 60 | | |
| | OL1S | OJL3 | L1J3 | 10 | | |
| L2 | OL2S | OJL1 | L2J1 | 30 | PL2 | 10 |
| | OL2S | OJL2 | L2J2 | 60 | | |
| | OL2S | OJL3 | L2J3 | 10 | | |
| L3 | OL3S | OJL1 | L3J1 | 30 | PL3 | 10 |
| | OL3S | OJL2 | L3J2 | 60 | | |
| | OL3S | OJL3 | L3J3 | 10 | | |
| L4 | OL4S | OJL1 | L4J1 | 30 | PL4 | 1 |
| | OL4S | OJL2 | L4J2 | 60 | | |
| | OL4S | OJL3 | L4J3 | 10 | | |
| L5 | OL5S | OJL1 | L5J1 | 30 | PL5 | 1 |
| | OL5S | OJL2 | L5J2 | 60 | | |
| | OL5S | OJL3 | L5J3 | 10 | | |
| L6 | OL6S | OJL1 | L6J1 | 30 | PL6 | 1 |
| | OL6S | OJL2 | L6J2 | 60 | | |
| | OL6S | OJL3 | L6J3 | 10 | | |
| L7 | OL7S | OJL1 | L7J1 | 30 | PL7 | 1 |
| | OL7S | OJL2 | L7J2 | 60 | | |
| | OL7S | OJL3 | L7J3 | 10 | | |
| L8 | OL8S | OJL1 | L8J1 | 30 | PL8 | 1 |
| | OL8S | OJL2 | L8J2 | 60 | | |
| | OL8S | OJL3 | L8J3 | 10 | | |
| L9 | OL9S | OJL1 | L9J1 | 30 | PL9 | 1 |
| | OL9S | OJL2 | L9J2 | 60 | | |
| | OL9S | OJL3 | L9J3 | 10 | | |
| | | | | | VL | 100% |

**Table 4. Second round VH regions amplification overview**

| Template | 5' primer | 3' primer | Product | Share in PK/PL (%) | Pool | Share in VH (%) |
|---|---|---|---|---|---|---|
| H1 | OH1S | OJH1 | H1J1 | 10 | PH1 | 25 |
| | OH1S | OJH2 | H1J2 | 10 | | |
| | OH1S | OJH3 | H1J3 | 60 | | |
| | OH1S | OJH4 | H1J4 | 20 | | |
| H2 | OH2S | OJH1 | H2J1 | 10 | PH2 | 2 |
| | OH2S | OJH2 | H2J2 | 10 | | |
| | OH2S | OJH3 | H2J3 | 60 | | |
| | OH2S | OJH4 | H2J4 | 20 | | |
| H3 | OH3S | OJH1 | H3J1 | 10 | PH3 | 25 |
| | OH3S | OJH2 | H3J2 | 10 | | |
| | OH3S | OJH3 | H3J3 | 60 | | |
| | OH3S | OJH4 | H3J4 | 20 | | |
| H4 | OH4S | OJH1 | H4J1 | 10 | PH4 | 25 |
| | OH4S | OJH2 | H4J2 | 10 | | |
| | OH4S | OJH3 | H4J3 | 60 | | |
| | OH4S | OJH4 | H4J4 | 20 | | |
| H5 | OH5S | OJH1 | H5J1 | 10 | PH5 | 2 |
| | OH5S | OJH2 | H5J2 | 10 | | |
| | OH5S | OJH3 | H5J3 | 60 | | |
| | OH5S | OJH4 | H5J4 | 20 | | |
| H6 | OH6S | OJH1 | H6J1 | 10 | PH6 | 20 |
| | OH6S | OJH2 | H6J2 | 10 | | |
| | OH6S | OJH3 | H6J3 | 60 | | |
| | OH6S | OJH4 | H6J4 | 20 | | |
| H7 | OH7S | OJH1 | H7J1 | 10 | PH7 | 1 |
| | OH7S | OJH2 | H7J2 | 10 | | |
| | OH7S | OJH3 | H7J3 | 60 | | |
| | OH7S | OJH4 | H7J4 | 20 | | |
| | | | | | VH | 100% |

**Table 5: Characteristics of the individual IgM memory B cell libraries.**

| IgM memory libraries | | | | | | |
|---|---|---|---|---|---|---|
| Donor | Cells | | Libraries | | | |
| | Total PBL (x10⁶) | % memory B cells | Size (x10⁶) | % Insert frequency | % ORF | % Unique |
| Individual 1 | | | 3 | 96 | 74 | 98 |
| Individual 2 | 72.5 | 1.7 | 5 | 98 | 79 | 98 |
| Individual 3 | 67.5 | 1.4 | 3 | 96 | 79 | 98 |
| Individual 4 | 132.5 | 2.3 | 6 | 98 | 69 | 99 |

**Table 6: Data of the HA-specific single-chain Fvs.**

| Name scFv | SEQ ID NO (nucl sequence) | SEQ ID NO (amino acid sequence)* | VH-locus | VL-locus |
|---|---|---|---|---|
| SC06-141 | 212 | 213 (Vh 1-115; Vl 132-245) | VH1 (1-18) | VKIV (B3) |
| SC06-255 | 115 | 116 (Vh 1-121; Vl 138-248) | VH1 (1-69) | VL1 (V1-16) |
| SC06-257 | 117 | 118 (Vh 1-121; Vl 138-248) | VH1 (1-69) | VL2 (V1-4) |
| SC06-260 | 119 | 120 (Vh 1-121; Vl 138-248) | VH1 (1-69) | VL1 (V1-17) |
| SC06-261 | 121 | 122 (Vh 1-121; Vl 138-249) | VH1 (1-69) | VL1 (V1-19) |
| SC06-262 | 123 | 124 (Vh 1-120; Vl 137-245) | VH1 (1-69) | VKI (A20) |
| SC06-268 | 125 | 126 (Vh 1-120; Vl 137-243) | VH1 (1-69) | VL3 (V2-1) |
| SC06-272 | 214 | 215 (Vh 1-120; Vl 137-247) | VH1 (1-69) | VL2 (V1-3) |
| SC06-296 | 216 | 217 (Vh 1-121; Vl 138-246) | VH1 (1-2) | VKIII (A27) |
| SC06-301 | 218 | 219 (Vh 1-117; Vl 134-246) | VH1 (3-23) | VKII (A3) |
| SC06-307 | 127 | 128 (Vh 1-122; Vl 139-246) | VH3 (3-21) | VKIII (A27) |
| SC06-310 | 129 | 130 (Vh 1-121; Vl 138-246) | VH1 (1-69) | VL3 (V2-14) |
| SC06-314 | 131 | 132 (Vh 1-121; Vl 138-248) | VH1 (1-69) | VL1 (V1-17) |
| SC06-323 | 133 | 134 (Vh 1-120; Vl 137-245) | VH1 (1-69) | VKIII (A27) |
| SC06-325 | 135 | 136 (Vh 1-121; Vl 138-248) | VH1 (1-69) | VL2 (V1-4) |
| SC06-327 | 220 | 221 (Vh 1-121; Vl 138-246) | VH1 (1-69) | VL3 (V2-14) |
| SC06-328 | 222 | 223 (Vh 1-128; Vl 145-252) | VH1 (1-69) | VKIII (A27) |
| SC06-329 | 224 | 225 (Vh 1-121; Vl 138-246) | VH1 (1-69) | VKIII (A27) |
| SC06-331 | 137 | 138 (Vh 1-120; Vl 137-245) | VH1 (1-69) | VL3 (V2-14) |
| SC06-332 | 226 | 227 (Vh 1-120; Vl 137-243) | VH1 (1-69) | VKI (A20) |
| SC06-334 | 228 | 229 (Vh 1-120; Vl 137-245) | VH1 (1-69) | VL3 (V2-14) |
| SC06-336 | 230 | 231 (Vh 1-120; Vl 137-245) | VH1 (1-69) | VKIII (A27) |
| SC06-339 | 232 | 233 (Vh 1-121; Vl 138-246) | VH1 (1-69) | VL3 (V2-14) |
| SC06-342 | 234 | 235 (Vh 1-126; Vl 143-256) | VH1 (1-69) | VKIV (B3) |
| SC06-343 | 236 | 237 (Vh 1-120; Vl 137-245) | VH1 (1-69) | VL3 (V2-14) |
| SC06-344 | 139 | 140 (Vh 1-123; Vl 140-250) | VH1 (1-69) | VL1 (V1-13) |

| | | | | |
|---|---|---|---|---|
| * between brackets the amino acids making up the heavy chain variable region (VH) and the light chain variable region (VL) is shown | | | | |

**Table 7: Data of the CDR regions of the HA specific immunoglobulins. The SEQ ID NO is given between brackets.**

| Name scFv | HCDR1 | HCDR2 | HCDR3 | LCDR1 | LCDR2 | LCDR3 |
|---|---|---|---|---|---|---|
| SC06-141 | GYYVY (238) | | SRSLDV (240) | | WASTRES (242) | QQYYSTPLT (243) |
| SC06-255 | SYAIS (1) | | | | SNNQRPS (5) | |
| SC06-257 | SYAIS (1) | | | | EVSNRPS (8) | |
| SC06-260 | SYAIS (1) | | | | KNTQRPS (11) | |
| SC06-261 | SYAIS (1) | | | | DNNKRPS (14) | |
| SC06-262 | GSAIS (16) | | | RASQGISSYLA (19) | DASTLRS (20) | |
| SC06-268 | SYAIS (1) | | | SGHKLGDKYVS (24) | QDNRRPS (25) | QAWDSSTAV (26) |
| SC06-272 | SYAIT (244) | | | | DVSKRPS (248) | |
| SC06-296 | SYYMH (250) | | | | DASSRAT (254) | QQYGSSLW (255) |
| SC06-301 | IYAMS (256) | | AYGYTFDP (258) | | LGSNRAS (260) | MQALQTPL (261) |
| SC06-307 | SYSMN (27) | | | RASQRVSSYLA (30) | GASTRAA (31) | QQYGRTPLT (32) |
| SC06-310 | SYAIS (1) | | | GGNNIGSKSVH (33) | DDSDRPS (34) | |
| SC06-314 | SYAIS (1) | | | | RDGQRPS (37) | |
| SC06-323 | SYGIS (39) | | | | GASSRAT (43) | QQYGSSPRT (44) |
| SC06-325 | FYSMS (45) | | | | EVSNRPS (8) | |
| SC06-327 | THAIS (262) | | | GGNNIGSKGVH (265) | DDSDRPS (266) | |
| SC06-328 | GYAIS (268) | | | | GASSRAT (272) | QQYGSSLT (273) |
| SC06-329 | SNSIS (274) | | | | GASSRAS (278) | QQYGSSPLT (279) |
| SC06-331 | SYAIS (1) | | | GGNNIGSKSVH (33) | DDSDRPS (34) | |
| SC06-332 | NFAIN (280) | | | RASQGISTYLA (283) | AASTLQS (284) | QKYNSAPS (285) |
| SC06-334 | SNAVS (286) | | | GGNNIGRNSVH (289) | DDSDRPS (290) | |
| SC06-336 | SYAIS (292) | | | | GASSRAT (296) | QQYGSSSLT (297) |
| SC06-339 | SYAIS (298) | | | GGNNIGSKSVH (301) | DDSDRPS (302) | |
| SC06-342 | SYAIS (304) | | | | WASTRES (308) | QQYYSSPPT (309) |
| SC06-343 | YYAMS (310) | | | GGHNIGSNSVH (313) | DNSDRPS (314) | |
| SC06-344 | NYAMS (52) | | | | GNSNRPS (56) | |

**Table 8. Data of the HA-specific IgGs.**

| Name IgG | SEQ ID NO of nucl. sequence heavy chain | SEQ ID NO of amino acid sequence* heavy chain | SEQ ID NO of nucl. sequence light chain | SEQ ID NO of amino acid sequence* light chain |
|---|---|---|---|---|
| CR6141 | 316 | 317 (Vh 1-115) | 318 | 319 (Vl 1-114) |
| CR6255 | 58 | 59 (Vh 1-121) | 84 | 85 (Vl 1-111) |
| CR6257 | 60 | 61 (Vh 1-121) | 86 | 87 (Vl 1-111) |
| CR6260 | 62 | 63 (Vh 1-121) | 88 | 89 (Vl 1-111) |
| CR6261 | 64 | 65 (Vh 1-121) | 90 | 91 (Vl 1-112) |
| CR6262 | 66 | 67 (Vh 1-120) | 92 | 93 (Vl 1-109) |
| CR6268 | 68 | 69 (Vh 1-120) | 94 | 95 (Vl 1-107) |
| CR6272 | 320 | 321 (Vh 1-120) | 322 | 323 (Vl 1-111) |
| CR6296 | 324 | 325 (Vh 1-121) | 326 | 327 (Vl 1-109) |
| CR6301 | 328 | 329 (Vh 1-117) | 330 | 331 (Vl 1-113) |
| CR6307 | 70 | 71 (Vh 1-122) | 96 | 97 (Vl 1-108) |
| CR6310 | 72 | 73 (Vh 1-121) | 98 | 99 (Vl 1-109) |
| CR6314 | 74 | 75 (Vh 1-121) | 100 | 101 (Vl 1-111) |
| CR6323 | 76 | 77 (Vh 1-120) | 102 | 103 (Vl 1-109) |
| CR6325 | 78 | 79 (Vh 1-121) | 104 | 105 (Vl 1-111) |
| CR6327 | 332 | 333 (Vh 1-121) | 334 | 335 (Vl 1-109) |
| CR6328 | 336 | 337 (Vh 1-128) | 338 | 339 (Vl 1-108) |
| CR6329 | 340 | 341 (Vh 1-121) | 342 | 343 (Vl 1-109) |
| CR6331 | 80 | 81 (Vh 1-120) | 106 | 107 (Vl 1-109) |
| CR6332 | 344 | 345 (Vh 1-120) | 346 | 347 (Vl 1-107) |
| CR6334 | 348 | 349 (Vh 1-120) | 350 | 351 (Vl 1-109) |
| CR6336 | 352 | 353 (Vh 1-120) | 354 | 355 (Vl 1-109) |
| CR6339 | 356 | 357 (Vh 1-121) | 358 | 359 (Vl 1-109) |
| CR6342 | 360 | 361 (Vh 1-126) | 362 | 363 (Vl 1-114) |
| CR6343 | 364 | 365 (Vh 1-120) | 366 | 367 (Vl 1-109) |
| CR6344 | 82 | 83 (Vh 1-123) | 108 | 109 (Vl 1-111) |

| | | | | |
|---|---|---|---|---|
| * between brackets the amino acids making up the heavy chain variable region (VH) and the light chain variable region (VL) is shown | | | | |

**Table 9. Binding of IgGs to HA (H5N1TV)-expressing PER.C6 cells.**

| | HA (H5N1TV)-expressing PER.C6 cells | | | Control PER.C6 cells | | |
|---|---|---|---|---|---|---|
| Antibody | 10 µg/ml | 1 µg/ml | 0.1 µg/ml | 10 µg/ml | 1 µg/ml | 0.1 µg/ml |
| CR6255 | 414.18 | 257.13 | 60.43 | 3.16 | 2.64 | 2.48 |
| CR6257 | 365.17 | 283.87 | 62.08 | 2.59 | 2.44 | 2.53 |
| CR6260 | 323.42 | 168.49 | 31.06 | 5.42 | 3.34 | 2.57 |
| CR6261 | 330.77 | 278.81 | 85.82 | 29.43 | 13.22 | 3.89 |
| CR6262 | 84.29 | 20.91 | 8.06 | 2.71 | 2.53 | 2.48 |
| CR6268 | 421.70 | 218.70 | 43.71 | 3.82 | 2.74 | 2.50 |
| CR6307 | 484.78 | 266.55 | 82.42 | 4.87 | 3.02 | 2.48 |
| CR6314 | 399.54 | 166.98 | 44.51 | 5.99 | 3.49 | 2.64 |
| CR6323 | 445.08 | 116.52 | 33.38 | 5.05 | 2.92 | 2.71 |
| CR6325 | 478.29 | 239.28 | 64.36 | 4.00 | 3.11 | 2.57 |
| CR6344 | 768.25 | 328.16 | 106.65 | 80.90 | 26.33 | 10.17 |
| CR3014 | 13.10 | 10.00 | 6.21 | 3.11 | 2.69 | 2.55 |
| | | | | | | |
| CR6310* | 597.04 | 290.93 | 86.91 | 14.92 | 6.05 | 4.42 |
| CR6331* | 421.14 | 165.43 | 41.04 | 7.87 | 4.59 | 4.55 |
| CR3014* | 9.15 | 7.95 | 10.51 | 4.74 | 4.12 | 4.57 |

| | | | | | | |
|---|---|---|---|---|---|---|
| * Indicates FACS analyses that were performed in a separate experiment | | | | | | |

**Table 10. Potency of the anti-HA antibodies in the neutralizing antibody titer assay.**

| Antibody | Concentration (µg/ml) |
|---|---|
| CR6255 | 25 |
| CR6257 | 12.5 |
| CR6260 | 12.5 |
| CR6261 | 12.5 |
| CR6262 | 100 |
| CR6268 | 50 |
| CR6307 | 50 |
| CR6314 | 12.5 |
| CR6323 | 50 |
| CR6325 | 12.5 |
| CR6344 | 25 |
| CR6310 | 25 |
| CR6331 | 100 |
| CR4098 | -* |

| | |
|---|---|
| *At 50 µg/ml no neutralization was observed | |

**Table 11. Potency of the anti-HA antibodies in the neutralizing antibody titer assay.**

| Antibody | Concentration (µg/ml) |
|---|---|
| CR6255 | 3.12 |
| CR6257 | 1.56 |
| CR6260 | 3.12 |
| CR6261 | 0.78 |
| CR6262 | 25 |
| CR6268 | 6.25 |
| CR6272 | - |
| CR6307 | 25 |
| CR6310 | 6.25 |
| CR6314 | 3.12 |
| CR6323 | 6.25 |
| CR6325 | 6.25 |
| CR6327 | 6.25 |
| CR6328 | 25 |
| CR6329 | 3.12 |
| CR6331 | 25 |
| CR6332 | 12.5 |
| CR6334 | 6.25 |
| CR6336 | 25 |
| CR6339 | - |
| CR6342 | 6.25 |
| CR6343 | 50 |
| CR6344 | 25 |

| | |
|---|---|
| - means at 100 µg/ml no neutralization was observed | |

**Table 12. Cross-reactivity of anti-H5N1 IgGs to HA molecules of different HA subtypes as measured by ELISA (OD 492nm). ND: Not determined**

| | H1 | H3 | H5 | H7 | H9 | BPL-inact. A/NC/20/99 (H1N1) | BPL-inact. NIBRG14 (H5N1) |
|---|---|---|---|---|---|---|---|
| CR6255 | 1.91 | 0.08 | 1.44 | 0.22 | 1.97 | 1.38 | 1.18 |
| CR6257 | 1.93 | 0.08 | 1.37 | 0.16 | 2.06 | 1.34 | 1.21 |
| CR6260 | 1.88 | 0.08 | 1.45 | 0.19 | 2.00 | 1.34 | 1.17 |
| CR6261 | 2.04 | 0.07 | 1.44 | 0.31 | 2.32 | 1.46 | 1.41 |
| CR6262 | 1.48 | 0.09 | 1.02 | 0.17 | 1.93 | 0.51 | 0.35 |
| CR6268 | 1.78 | 0.08 | 1.30 | 0.15 | 2.16 | 1.39 | 1.13 |
| CR6272 | 0.81 | 0.07 | 0.58 | 0.15 | 0.96 | 0.92 | 0.79 |
| CR6307 | 0.22 | 0.11 | 0.99 | 0.22 | 0.17 | 0.23 | 0.50 |
| CR6310 | 1.92 | 0.08 | 1.37 | 0.18 | 2.17 | 1.31 | 1.00 |
| CR6314 | 1.93 | 0.07 | 1.48 | 0.25 | 2.21 | 1.37 | 1.42 |
| CR6323 | 2.32 | 0.07 | 1.89 | 0.15 | 2.27 | 1.40 | 0.93 |
| CR6325 | 1.42 | 0.07 | 1.30 | 0.17 | 2.04 | 1.09 | 1.20 |
| CR6327 | 1.75 | 0.08 | 1.11 | 0.14 | 1.93 | 1.16 | 0.73 |
| CR6328 | 2.43 | 0.07 | 1.78 | 0.16 | 2.38 | 1.39 | 0.98 |
| CR6329 | 1.98 | 0.08 | 1.43 | 0.17 | 2.16 | 1.04 | 1.01 |
| CR6331 | 1.75 | 0.06 | 1.32 | 0.16 | 2.02 | 1.25 | 0.92 |
| CR6332 | 2.20 | 0.15 | 1.65 | 0.26 | 2.11 | 1.20 | 1.11 |
| CR6334 | 2.04 | 0.07 | 1.19 | 0.15 | 1.82 | 1.13 | 0.91 |
| CR6336 | 1.92 | 0.10 | 1.41 | 0.18 | 2.02 | 1.09 | 0.94 |
| CR6339 | 1.25 | 0.07 | 0.81 | 0.14 | 1.96 | 0.94 | 0.50 |
| CR6342 | 1.99 | 0.09 | 1.25 | 0.17 | 2.13 | 1.32 | 0.90 |
| CR6343 | 1.28 | 0.07 | 0.76 | 0.15 | 1.88 | 0.91 | 0.64 |
| CR6344 | 1.80 | 0.09 | 1.31 | 0.18 | 2.15 | 1.31 | 0.96 |
| CR5111 | 0.08 | 0.07 | 1.57 | 0.15 | 0.17 | 0.09 | 0.81 |
| CR3014 | 0.08 | 0.07 | 0.09 | 0.17 | 0.16 | 0.09 | 0.11 |
| No IgG | 0.13 | 0.08 | 0.09 | 0.15 | 0.16 | 0.09 | 0.14 |
| Sheep anti-H5 | ND | ND | ND | 0.90 | 2.07 | 2.62 | 2.93 |

**Table 13. Epitope mapping of human anti-HA antibodies.**

| | HA1 | HA2 | CR5111 | CR6342 C179 | CR6307 CR6323 | CR6261 CR6325 CR6329 |
|---|---|---|---|---|---|---|
| H5N1TV | TGLRN | GVTNKVNSIIDK | + | + | + | + |
| Mutant I | K---- | ------------ | + | - | + | + |
| Mutant II | ----- | -----E------ | + | - | + | - |
| Mutant III | --M-- | ------------ | + | + | + | + |
| Mutant IV | ----- | -------R---- | + | - | + | + |
| Mutant V | ----- | ----------N- | + | + | + | + |

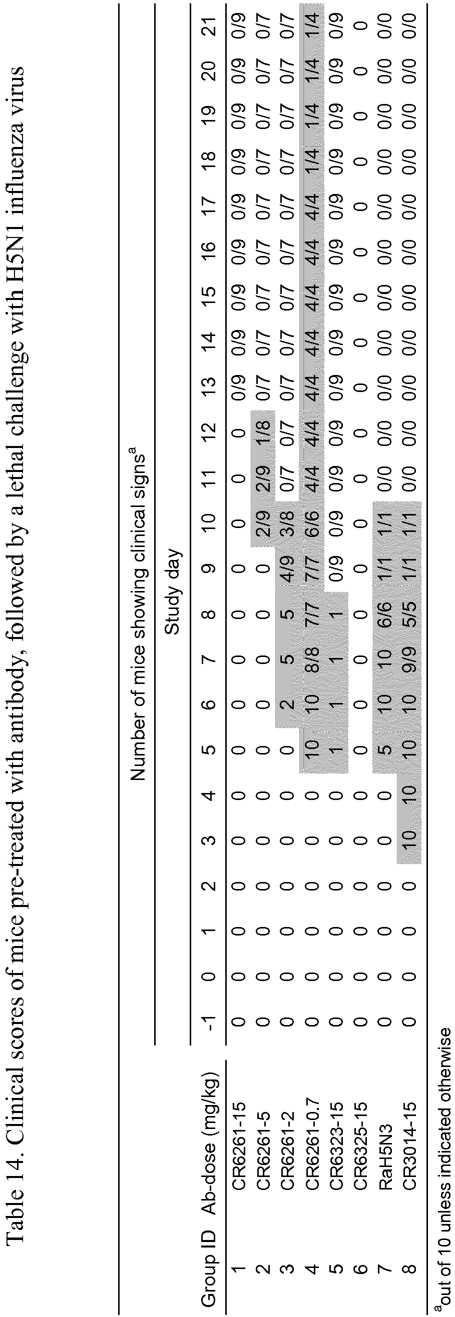

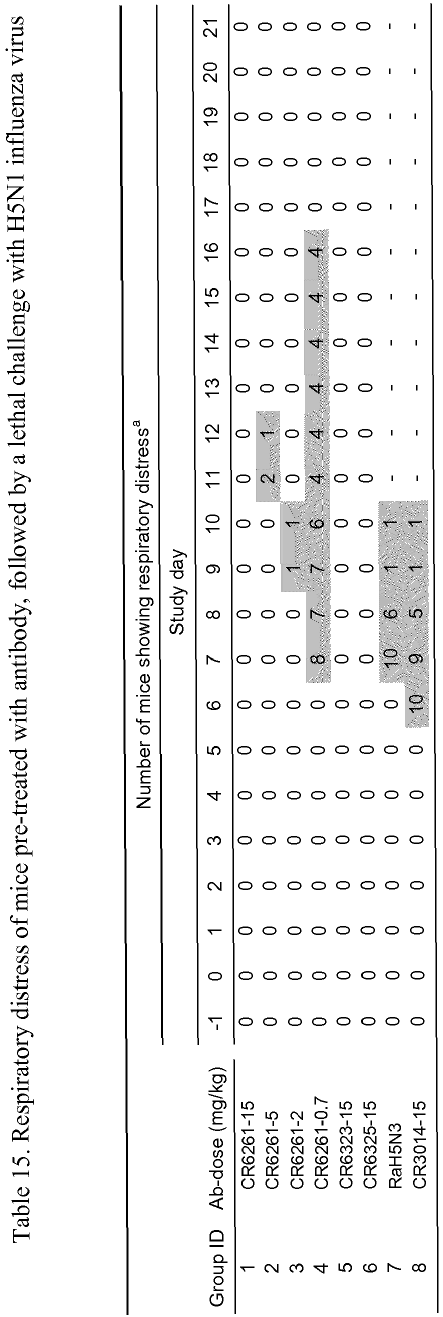

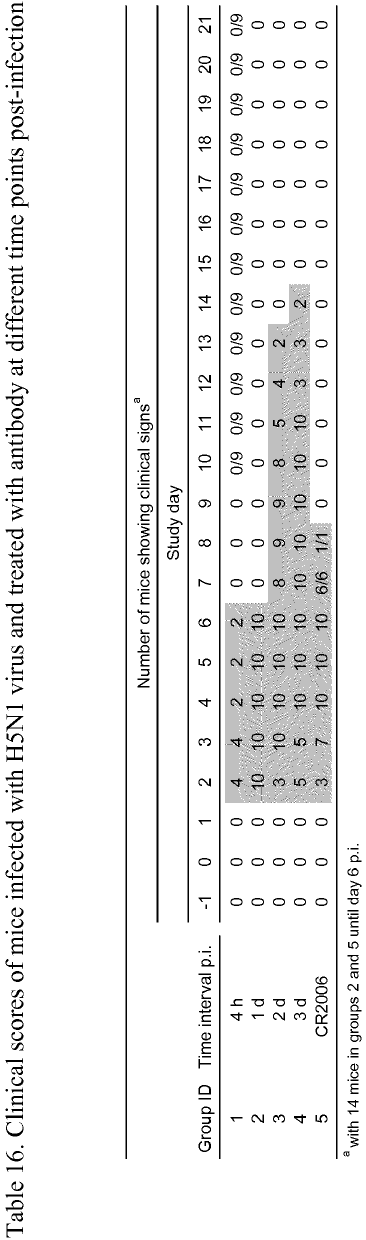

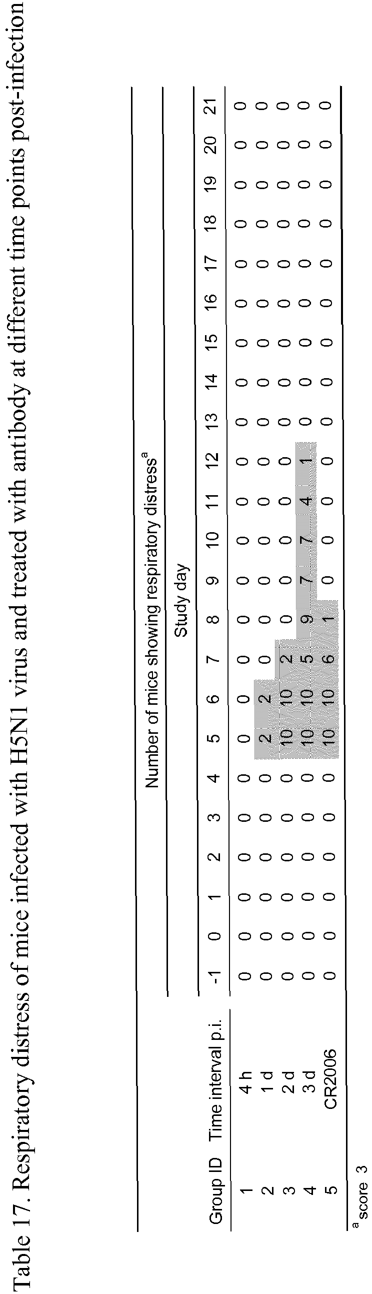

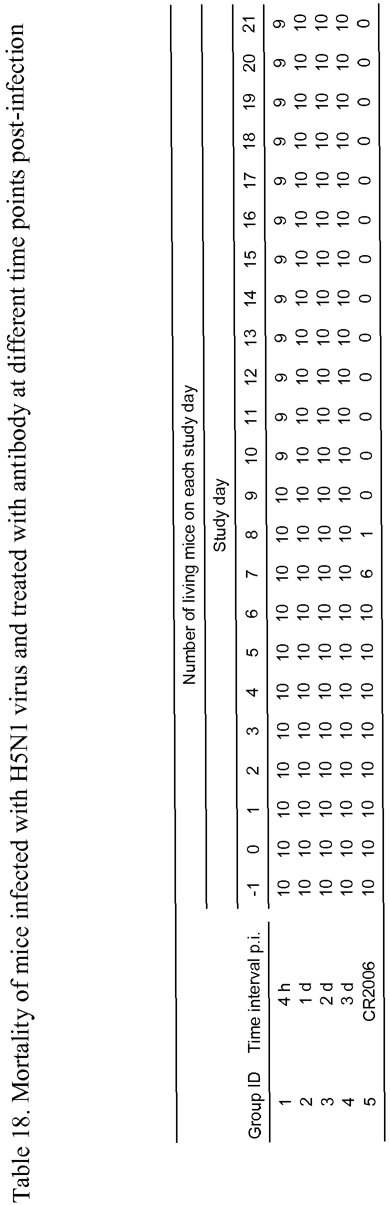

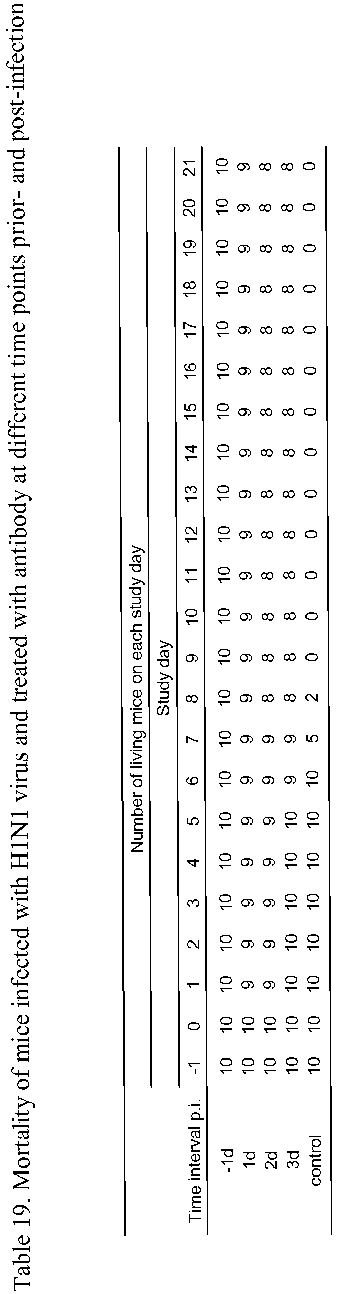

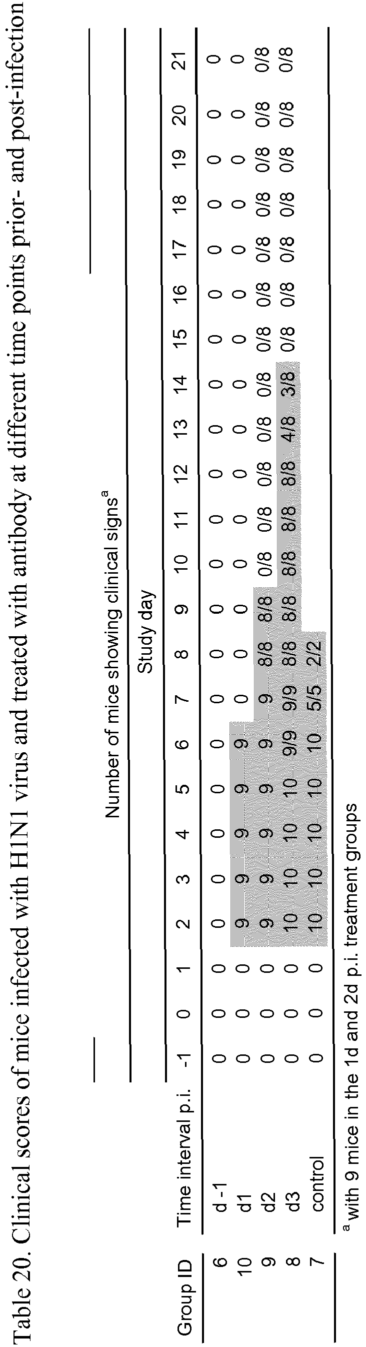

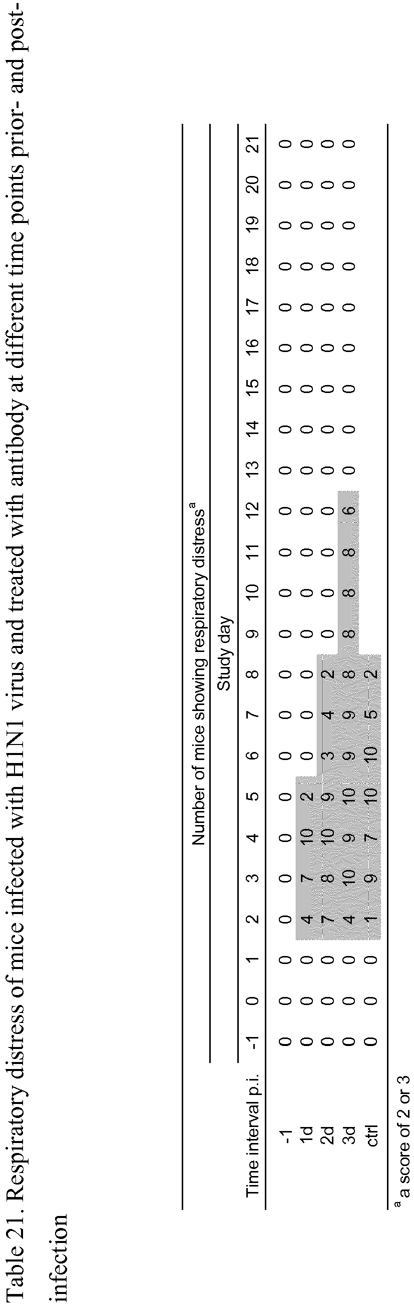

**Table 22. Sequence of HA2 epitope in different influenza subtypes. The underlined amino acid represents the valine (V) -> glutamic acid (E) substitution in the H2N2 escape mutant (mutant II; Table 13)**

| | HA2 | SEQ ID NO: |
|---|---|---|
| H5N1: | | |
| A/Vietnam/1203/2004 | GVTNKVNSIIDK | 368 |
| H5N1: | | |
| A/Hong Kong/156/97 | GVTNKVNSIINK | 369 |
| H5N2: | | |
| A/mald/PA/84 | GVTNKVNSIIDK | 368 |
| H1N1: | | |
| A/PR/8/34 | GITNKVNSVIEK | 372 |
| A/South Carolina/1/1918 | | |
| A/WSN/33 | | |
| A/New Caledonia/20/99 | | |
| A/Bangkok/10/83 | | |
| A/Yamagata/120/86 | | |
| H2N2: | | |
| A/Okuda/57 | GITNKVNSVIEK | 372 |
| A/Kumamoto/1/65 | | |
| A/Korea/426/68 | | |
| A/Izumi/5/65 | | |
| H2N2: | | |
| A/Izumi/5/65 (R) | GITNKENSVIEK | 373 |
| H6N2: | | |
| A/Mallard/Netherlands/16/99 | GITNKVNSIIDK | 374 |
| H9N2: | | |
| A/Hong Kong/1073/99 | KITSKVNNIVDK | 375 |
| H3N2: | | |
| A/Aichi/2/68 | QINGKLNRVIEK | 376 |
| H3N2: | | |
| A/Fukuoka/C29/85 | QINGKLNRLIEK | 377 |

### REFERENCES

Boel E et al. (2000), Functional human monoclonal antibodies of all isotypes constructed from phage display library-derived single-chain Fv antibody fragments. J. Immunol. Methods 239:153-166
Burton DR and Barbas CF (1994), Human antibodies from combinatorial libraries. Adv. Immunol. 57:191-280
Chou TC and P Talalay (1984) Quantitative analysis of dose-effect relationships: the combined effects of multiple drugs or enzyme inhibitors. Adv Enzyme Regul 22:27-55
De Kruif J et al. (1995a), Rapid selection of cell subpopulation-specific human monoclonal antibodies from a synthetic phage antibody library. Proc Natl Acad Sci USA 92:3938
De Kruif J et al. (1995b) Selection and application of human single-chain Fv antibody fragments from a semi-synthetic phage antibody display library with designed CDR3 regions. J. Mol. Biol. 248:97-105.
Huls G et al. (1999) Antitumor immune effector mechanisms recruited by phage display-derived fully human IgG1 and IgA1 monoclonal antibodies. Cancer Res 59:5778-5784
Okuno Y et al (1993) A common neutralizing epitope conserved between the hemagglutinins of Influenza A virus H1 and H2 strains. J. Virol. 67:2552-2558.
Slootstra JW et al. (1996) Structural aspects of antibody-antigen interaction revealed through small random peptide libraries. Mol. Divers. 1:87-96.
Smirnov YA et al (1999) An epitope shared by the hemagglutinins of H1, H2, H5 and H6 subtypes of influenza A virus. Acta Virol. 43:237-244.
The World Health Organization Global Influenza Program Surveillance Network (2005), Evolution of H5N1 Avian Influenza Viruses in Asia. Emerg Infect Dis 11:1515-1521

## Claims

1. A human antibody, or antigen-binding fragment thereof, capable of recognizing and binding to an epitope in the HA2 subunit of the influenza haemagglutinin protein (HA), **characterized in that** said antibody or antigen binding fragment has neutralizing activity against an influenza virus comprising HA of the H5 subtype and wherein said antibody or antigen binding fragment also has neutralizing activity against an influenza virus comprising HA of the H1 subtype, wherein the antibody or antigen binding fragment comprises a heavy chain CDR1 region of SEQ ID NO:274, a heavy chain CDR2 region of SEQ ID NO:275, a heavy chain CDR3 region of SEQ ID NO:276, a light chain CDR1 region of SEQ ID NO:277, a light chain CDR2 region of SEQ ID NO:278, and a light chain CDR3 region of SEQ ID NO:279.

2. A human antibody, or antigen-binding fragment thereof according to claim 1, wherein said human antibody, or antigen-binding fragment thereof also has neutralizing activity against an influenza virus comprising HA of the H2, H6 and/or H9 subtype.

3. A binding molecule according to claim 1 or 2 for use as a medicament.

4. A binding molecule according to any one of claims 1-3 for use in the diagnosis, prophylaxis and/or treatment of influenza infection.

5. A nucleic acid molecule encoding a binding molecule according to any one of claims 1-4.

6. A pharmaceutical composition comprising a binding molecule according to any one of claims 1-4, and a pharmaceutically acceptable excipient.

## Patentansprüche

1. Menschlicher Antikörper oder antigenbindendes Fragment davon mit der Fähigkeit zur Erkennung eines Epitops in der HA2-Untereinheit des Influenza-Hämagglutininproteins (HA) und zur Bindung daran, **dadurch gekennzeichnet, dass** der Antikörper bzw. das antigenbindende Fragment Neutralisierungsaktivität gegen ein HA des H5-Subtyps umfassendes Influenzavirus aufweist und wobei der Antikörper bzw. das antigenbindende Fragment auch Neutralisierungsaktivität gegen ein HA des H1-Subtyps umfassendes Influenzavirus aufweist, wobei der Antikörper bzw. das antigenbindende Fragment eine Schwere-Kette-CDR1-Region unter SEQ ID NO:274, eine Schwere-Kette-CDR2-Region unter SEQ ID NO:275, eine Schwere-Kette-CDR3-Region unter SEQ ID NO:276, eine Leichte-Kette-CDR1-Region unter SEQ ID NO:277, eine Leichte-Kette-CDR2-Region unter SEQ ID NO:278 und eine Leichte-Kette-CDR3-Region unter SEQ ID NO:279 umfasst.

2. Menschlicher Antikörper oder antigenbindendes Fragment davon nach Anspruch 1, wobei der menschliche Antikörper bzw. das antigenbindende Fragment davon auch Neutralisierungsaktivität gegen ein HA des Subtyps H2, H6 und/oder H9 umfassendes Influenzavirus aufweist.

3. Bindungsmolekül nach Anspruch 1 oder 2 zur Verwendung als Arzneimittel.

4. Bindungsmolekül nach einem der Ansprüche 1-3 zur Verwendung bei der Diagnose, Prophylaxe und/oder Behandlung einer Influenzainfektion.

5. Nukleinsäuremolekül, codierend ein Bindungsmolekül nach einem der Ansprüche 1-4.

6. Pharmazeutische Zusammensetzung, umfassend ein Bindungsmolekül nach einem der Ansprüche 1-4 und einen pharmazeutisch unbedenklichen Hilfsstoff.

## Revendications

1. Anticorps humain, ou fragment se liant à l'antigène de celui-ci, capable de reconnaître et de se lier à un épitope de la sous-unité HA2 de la protéine d'hémagglutinine (HA) du virus influenza, **caractérisé en ce que** ledit anticorps ou fragment se liant à l'antigène présente une activité neutralisante vis-à-vis d'un virus influenza comprenant une HA du sous-type H5 et où ledit anticorps ou fragment se liant à l'antigène présente également une activité neutralisante vis-à-vis d'un virus influenza comprenant une HA du sous-type H1, où l'anticorps ou le fragment se liant à l'antigène comprend une région CDR1 de chaîne lourde de SEQ ID NO:274, une région CDR2 de chaîne lourde de SEQ ID NO:275, une région CDR3 de chaîne lourde de SEQ ID NO:276, une région CDR1 de chaîne légère de SEQ ID NO:277, une région CDR2 de chaîne légère de SEQ ID NO:278, et une région CDR3 de chaîne légère de SEQ ID NO:279.

2. Anticorps humain, ou fragment se liant à l'antigène de celui-ci selon la revendication 1, où ledit anticorps humain, ou fragment se liant à l'antigène de celui-ci, présente également une activité neutralisante vis-à-vis d'un virus influenza comprenant une HA du sous-type H2, H6 et/ou H9.

3. Molécule de liaison selon la revendication 1 ou 2, pour utilisation en tant que médicament.

4. Molécule de liaison selon l'une quelconque des revendications 1 à 3, pour utilisation dans le diagnostic, la prophylaxie et/ou le traitement d'une infection par influenza.

5. Molécules d'acide nucléique codant une molécule de liaison selon l'une quelconque des revendications 1 à 4.

6. Composition pharmaceutique comprenant une molécule de liaison selon l'une quelconque des revendications 1 à 4 ainsi qu'un excipient pharmaceutiquement acceptable.


### SEQUENCE LISTING

<110> Crucell Holland B.V.
   Van den Brink, Edward N
   Throsby, Mark
   De Kruif, Cornelis A
<120> Human binding molecules capable of neutralizing influenza virus H5N1 and uses thereof
<130> 0145 WO 00 ORD
<140> PCT/EP2007/059356
   <141> 2007-09-06
<150> US 60/842,930
   <151> 2006-09-07
<150> EP 06120316.2
   <151> 2006-09-07
<150> EP 06120644.7
   <151> 2006-09-14
<150> EP 06125107.0
   <151> 2006-11-30
<150> EP 07111235.3
   <151> 2007-06-28
<160> 377
<170> PatentIn version 3.3
<210> 1
   <211> 5
   <212> PRT
   <213> Homo sapiens
<400> 1
   Ser Tyr Ala Ile Ser
   1               5
<210> 2
   <211> 17
   <212> PRT
   <213> Homo sapiens
<400> 2
   Gly Ile Ile Pro Ile Phe Gly Thr Thr Lys Tyr Ala Pro Lys Phe Gln
   1               5                   10                  15
   Gly
<210> 3
   <211> 12
   <212> PRT
   <213> Homo sapiens
<400> 3
   His Met Gly Tyr Gln Val Arg Glu Thr Met Asp Val
   1               5                   10
<210> 4
   <211> 13
   <212> PRT
   <213> Homo sapiens
<400> 4
   Ser Gly Ser Thr Phe Asn Ile Gly Ser Asn Ala Val Asp
   1               5                   10
<210> 5
   <211> 7
   <212> PRT
   <213> Homo sapiens
<400> 5
   Ser Asn Asn Gln Arg Pro Ser
   1               5
<210> 6
   <211> 11
   <212> PRT
   <213> Homo sapiens
<400> 6
   Ala Ala Trp Asp Asp Ile Leu Asn Val Pro Val
   1               5                   10
<210> 7
   <211> 14
   <212> PRT
   <213> Homo sapiens
<400> 7
   Thr Gly Thr Ser Ser Asp Val Gly Gly Tyr Asn Tyr Val Ser
   1               5                   10
<210> 8
   <211> 7
   <212> PRT
   <213> Homo sapiens
<400> 8
   Glu Val Ser Asn Arg Pro Ser
   1 5
<210> 9
   <211> 10
   <212> PRT
   <213> Homo sapiens
<400> 9
   Ser Ser Tyr Thr Ser Ser Ser Thr Tyr Val
   1 5 10
<210> 10
   <211> 13
   <212> PRT
   <213> Homo sapiens
<400> 10
   Ser Gly Ser Arg Ser Asn Val Gly Asp Asn Ser Val Tyr
   1 5 10
<210> 11
   <211> 7
   <212> PRT
   <213> Homo sapiens
<400> 11
   Lys Asn Thr Gln Arg Pro Ser
   1 5
<210> 12
   <211> 11
   <212> PRT
   <213> Homo sapiens
<400> 12
   Val Ala Trp Asp Asp Ser Val Asp Gly Tyr Val
   1 5 10
<210> 13
   <211> 13
   <212> PRT
   <213> Homo sapiens
<400> 13
   Ser Gly Ser Ser Ser Asn Ile Gly Asn Asp Tyr Val Ser
   1 5 10
<210> 14
   <211> 7
   <212> PRT
   <213> Homo sapiens
<400> 14
   Asp Asn Asn Lys Arg Pro Ser
   1               5
<210> 15
   <211> 12
   <212> PRT
   <213> Homo sapiens
<400> 15
   Ala Thr Trp Asp Arg Arg Pro Thr Ala Tyr Val Val
   1               5                   10
<210> 16
   <211> 5
   <212> PRT
   <213> Homo sapiens
<400> 16
   Gly Ser Ala Ile Ser
   1               5
<210> 17
   <211> 17
   <212> PRT
   <213> Homo sapiens
<400> 17
   Gly Ile Ser Pro Leu Phe Gly Thr Thr Asn Tyr Ala Gln Lys Phe Gln
   1               5                   10                  15
   Gly
<210> 18
   <211> 11
   <212> PRT
   <213> Homo sapiens
<400> 18
   Gly Pro Lys Tyr Tyr Ser Glu Tyr Met Asp Val
   1               5                   10
<210> 19
   <211> 11
   <212> PRT
   <213> Homo sapiens
<400> 19
   Arg Ala Ser Gln Gly Ile Ser Ser Tyr Leu Ala
   1 5 10
<210> 20
   <211> 7
   <212> PRT
   <213> Homo sapiens
<400> 20
   Asp Ala Ser Thr Leu Arg Ser
   1 5
<210> 21
   <211> 10
   <212> PRT
   <213> Homo sapiens
<400> 21
   Gln Arg Tyr Asn Ser Ala Pro Pro Ile Thr
   1 5 10
<210> 22
   <211> 17
   <212> PRT
   <213> Homo sapiens
<400> 22
   Gly Ile Met Gly Met Phe Gly Thr Thr Asn Tyr Ala Gln Lys Phe Gln
   1 5 10 15
   Gly
<210> 23
   <211> 11
   <212> PRT
   <213> Homo sapiens
<400> 23
   Ser Ser Gly Tyr Tyr Pro Glu Tyr Phe Gln Asp
   1 5 10
<210> 24
   <211> 11
   <212> PRT
   <213> Homo sapiens
<400> 24
   Ser Gly His Lys Leu Gly Asp Lys Tyr Val Ser
   1 5 10
<210> 25
   <211> 7
   <212> PRT
   <213> Homo sapiens
<400> 25
   Gln Asp Asn Arg Arg Pro Ser
   1               5
<210> 26
   <211> 9
   <212> PRT
   <213> Homo sapiens
<400> 26
   Gln Ala Trp Asp Ser Ser Thr Ala Val
   1               5
<210> 27
   <211> 5
   <212> PRT
   <213> Homo sapiens
<400> 27
   Ser Tyr Ser Met Asn
   1               5
<210> 28
   <211> 17
   <212> PRT
   <213> Homo sapiens
<400> 28
   Ser Ile Ser Ser Ser Ser Ser Tyr Ile Tyr Tyr Val Asp Ser Val Lys
   1               5                   10                  15
   Gly
<210> 29
   <211> 13
   <212> PRT
   <213> Homo sapiens
<400> 29
   Gly Gly Gly Ser Tyr Gly Ala Tyr Glu Gly Phe Asp Tyr
   1               5                   10
<210> 30
   <211> 11
   <212> PRT
   <213> Homo sapiens
<400> 30
   Arg Ala Ser Gln Arg Val Ser Ser Tyr Leu Ala
   1               5                   10
<210> 31
   <211> 7
   <212> PRT
   <213> Homo sapiens
<400> 31
   Gly Ala Ser Thr Arg Ala Ala
   1               5
<210> 32
   <211> 9
   <212> PRT
   <213> Homo sapiens
<400> 32
   Gln Gln Tyr Gly Arg Thr Pro Leu Thr
   1               5
<210> 33
   <211> 11
   <212> PRT
   <213> Homo sapiens
<400> 33
   Gly Gly Asn Asn Ile Gly Ser Lys Ser Val His
   1               5                   10
<210> 34
   <211> 7
   <212> PRT
   <213> Homo sapiens
<400> 34
   Asp Asp Ser Asp Arg Pro Ser
   1               5
<210> 35
   <211> 11
   <212> PRT
   <213> Homo sapiens
<400> 35
   Gln Val Trp Asp Ser Ser Ser Asp His Ala Val
   1               5                   10
<210> 36
   <211> 13
   <212> PRT
   <213> Homo sapiens
<400> 36
   Ser Gly Ser Ser Ser Asn Ile Gly Ser Asn Tyr Val Tyr
   1 5 10
<210> 37
   <211> 7
   <212> PRT
   <213> Homo sapiens
<400> 37
   Arg Asp Gly Gln Arg Pro Ser
   1 5
<210> 38
   <211> 11
   <212> PRT
   <213> Homo sapiens
<400> 38
   Ala Thr Trp Asp Asp Asn Leu Ser Gly Pro Val
   1 5 10
<210> 39
   <211> 5
   <212> PRT
   <213> Homo sapiens
<400> 39
   Ser Tyr Gly Ile Ser
   1 5
<210> 40
   <211> 17
   <212> PRT
   <213> Homo sapiens
<400> 40
   Asp Ile Ile Gly Met Phe Gly Ser Thr Asn Tyr Ala Gln Asn Phe Gln
   1 5 10 15
   Gly
<210> 41
   <211> 11
   <212> PRT
   <213> Homo sapiens
<400> 41
   Ser Ser Gly Tyr Tyr Pro Ala Tyr Leu Pro His
   1 5 10
<210> 42
   <211> 12
   <212> PRT
   <213> Homo sapiens
<400> 42
   Arg Ala Ser Gln Ser Val Ser Ser Ser Tyr Leu Ala
   1 5 10
<210> 43
   <211> 7
   <212> PRT
   <213> Homo sapiens
<400> 43
   Gly Ala Ser Ser Arg Ala Thr
   1 5
<210> 44
   <211> 9
   <212> PRT
   <213> Homo sapiens
<400> 44
   Gln Gln Tyr Gly Ser Ser Pro Arg Thr
   1 5
<210> 45
   <211> 5
   <212> PRT
   <213> Homo sapiens
<400> 45
   Phe Tyr Ser Met Ser
   1 5
<210> 46
   <211> 17
   <212> PRT
   <213> Homo sapiens
<400> 46
   Gly Ile Ile Pro Met Phe Gly Thr Thr Asn Tyr Ala Gln Lys Phe Gln
   1 5 10 15
   Gly
<210> 47
   <211> 12
   <212> PRT
   <213> Homo sapiens
<400> 47
   Gly Asp Lys Gly Ile Tyr Tyr Tyr Tyr Met Asp Val
   1               5                   10
<210> 48
   <211> 10
   <212> PRT
   <213> Homo sapiens
<400> 48
   Ser Ser Tyr Thr Ser Ser Ser Thr Leu Val
   1               5                   10
<210> 49
   <211> 17
   <212> PRT
   <213> Homo sapiens
<400> 49
   Gly Ile Ile Gly Met Phe Gly Thr Ala Asn Tyr Ala Gln Lys Phe Gln
   1               5                   10                  15
   Gly
<210> 50
   <211> 11
   <212> PRT
   <213> Homo sapiens
<400> 50
   Gly Asn Tyr Tyr Tyr Glu Ser Ser Leu Asp Tyr
   1               5                   10
<210> 51
   <211> 11
   <212> PRT
   <213> Homo sapiens
<400> 51
   Gln Val Trp Asp Ser Ser Ser Asp His Tyr Val
   1               5                   10
<210> 52
   <211> 5
   <212> PRT
   <213> Homo sapiens
<400> 52
   Asn Tyr Ala Met Ser
   1 5
<210> 53
   <211> 17
   <212> PRT
   <213> Homo sapiens
<400> 53
   Gly Ile Ile Ala Ile Phe Gly Thr Pro Lys Tyr Ala Gln Lys Phe Gln
   1 5 10 15
   Gly
<210> 54
   <211> 14
   <212> PRT
   <213> Homo sapiens
<400> 54
   Ile Pro His Tyr Asn Phe Gly Ser Gly Ser Tyr Phe Asp Tyr
   1 5 10
<210> 55
   <211> 14
   <212> PRT
   <213> Homo sapiens
<400> 55
   Thr Gly Ser Ser Ser Asn Ile Gly Ala Gly Tyr Asp Val His
   1 5 10
<210> 56
   <211> 7
   <212> PRT
   <213> Homo sapiens
<400> 56
   Gly Asn Ser Asn Arg Pro Ser
   1 5
<210> 57
   <211> 11
   <212> PRT
   <213> Homo sapiens
<400> 57
   Gly Thr Trp Asp Ser Ser Leu Ser Ala Tyr Val
   1 5 10
<210> 58
   <211> 1353
   <212> DNA
   <213> Homo sapiens
<220>
   <221> CDS
   <222> (1)..(1353)
<400> 58
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc      384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg     1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc     1344
   His Glu Ala Leu His Asn His Tyr Thr Gln Lys Ser Leu Ser Leu Ser
           435                 440                 445
   ccc ggc aag                                                         1353
   Pro Gly Lys
       450
<210> 59
   <211> 451
   <212> PRT
   <213> Homo sapiens
<400> 59
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65 70 75 80
   Met Glu Leu Ser Ser Leu Arg Ser Glu Asp Thr Ala Met Tyr Tyr Cys
   85 90 95
   Ala Lys His Met Gly Tyr Gln Val Arg Glu Thr Met Asp Val Trp Gly
   100 105 110
   Lys Gly Thr Thr Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 60
   <211> 1353
   <212> DNA
   <213> Homo sapiens
<220>
   <221> CDS
   <222> (1)..(1353)
<400> 60
   cag gtc cag ctg gtg cag tct ggg gct gag gtg aag aag cct ggg tcc        48
   Gln Val Gln Leu Val Gln Ser Gly Ala Glu Val Lys Lys Pro Gly Ser
   1               5                   10                  15
   tcg gtg aaa gtc tct tgc aag gct tct gga ggc ccc ttc cgc agc tat        96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg       144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc       192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac       240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc      384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc      1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag      1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct      1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg      1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 61
   <211> 451
   <212> PRT
   <213> Homo sapiens
<400> 61
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 62
   <211> 1353
   <212> DNA
   <213> Homo sapiens
<220>
   <221> CDS
   <222> (1)..(1353)
<400> 62
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc      384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct      1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg      1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 63
   <211> 451
   <212> PRT
   <213> Homo sapiens
<400> 63
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 64
   <211> 1353
   <212> DNA
   <213> Homo sapiens
<220>
   <221> CDS
   <222> (1)..(1353)
<400> 64
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc      384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 65
   <211> 451
   <212> PRT
   <213> Homo sapiens
<400> 65
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 66
   <211> 1350
   <212> DNA
   <213> Homo sapiens
<220>
   <221> CDS
   <222> (1)..(1350)
<400> 66
   cag gta cag ctg cag cag tca ggg gct gag gtg aag aag cct ggg tcc       48
   Gln Val Gln Leu Gln Gln Ser Gly Ala Glu Val Lys Lys Pro Gly Ser
   1               5                   10                  15
   tcg gtg aag gtc tcc tgc aag gtt tcc gga gtc att ttc agc ggc agt       96
   Ser Val Lys Val Ser Cys Lys Val Ser Gly Val Ile Phe Ser Gly Ser
               20                  25                  30
   gcg atc agc tgg gtg cga cag gcc cct gga caa ggc ctt gag tgg atg      144
   Ala Ile Ser Trp Val Arg Gln Ala Pro Gly Gln Gly Leu Glu Trp Met
           35                  40                  45
   gga ggg atc agc cct ctc ttt ggc aca aca aat tac gca caa aag ttc      192
   Gly Gly Ile Ser Pro Leu Phe Gly Thr Thr Asn Tyr Ala Gln Lys Phe
       50                  55                  60
   cag ggc aga gtc acg att acc gcg gac caa tcc acg aac aca acc tac      240
   Gln Gly Arg Val Thr Ile Thr Ala Asp Gln Ser Thr Asn Thr Thr Tyr
   65                  70                  75                  80
   atg gag gtg aac agc ctg aga tat gag gac acg gcc gtg tat ttc tgt      288
   Met Glu Val Asn Ser Leu Arg Tyr Glu Asp Thr Ala Val Tyr Phe Cys
                   85                  90                  95
   gcg cga ggt cca aaa tat tac agt gag tac atg gac gtc tgg ggc aaa      336
   Ala Arg Gly Pro Lys Tyr Tyr Ser Glu Tyr Met Asp Val Trp Gly Lys
               100                 105                 110
   ggg acc acg gtc acc gtc tcg agt gct agc acc aag ggc ccc agc gtg      384
   Gly Thr Thr Val Thr Val Ser Ser Ala Ser Thr Lys Gly Pro Ser Val
           115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag        624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac        672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga        720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc        768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag        816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac        864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg        912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag        960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag       1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac       1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc       1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg       1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg       1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac       1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac       1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc       1344
   Glu Ala Leu His Asn His Tyr Thr Gln Lys Ser Leu Ser Leu Ser Pro
           435                 440                 445
   ggc aag                                                                  1350
   Gly Lys
       450
<210> 67
   <211> 450
   <212> PRT
   <213> Homo sapiens
<400> 67
   Gln Val Gln Leu Gln Gln Ser Gly Ala Glu Val Lys Lys Pro Gly Ser
   1               5                   10                  15
   Ser Val Lys Val Ser Cys Lys Val Ser Gly Val Ile Phe Ser Gly Ser
               20                  25                  30
   Ala Ile Ser Trp Val Arg Gln Ala Pro Gly Gln Gly Leu Glu Trp Met
           35                  40                  45
   Gly Gly Ile Ser Pro Leu Phe Gly Thr Thr Asn Tyr Ala Gln Lys Phe
       50                  55                  60
   Gln Gly Arg Val Thr Ile Thr Ala Asp Gln Ser Thr Asn Thr Thr Tyr
   65                  70                  75                  80
   Met Glu Val Asn Ser Leu Arg Tyr Glu Asp Thr Ala Val Tyr Phe Cys
                   85                  90                  95
   Ala Arg Gly Pro Lys Tyr Tyr Ser Glu Tyr Met Asp Val Trp Gly Lys
               100                 105                 110
   Gly Thr Thr Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 68
   <211> 1350
   <212> DNA
   <213> Homo sapiens
<220>
   <221> CDS
   <222> (1)..(1350)
<400> 68
   cag gtc cag ctg gta cag tct ggg gct gag gtg aag aag cct ggg tcc      48
   Gln Val Gln Leu Val Gln Ser Gly Ala Glu Val Lys Lys Pro Gly Ser
   1               5                   10                  15
   tcg gtg aag gtc tcc tgc aag gct tct gga ggc acc ttc agt agt tat      96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga gga atc atg ggt atg ttt ggc aca act aac tac gca cag aag ttc      192
   Gly Gly Ile Met Gly Met Phe Gly Thr Thr Asn Tyr Ala Gln Lys Phe
       50                  55                  60
   cag ggc aga gtc acg att acc gcg gac gaa ttc acg agc gca gcc tac      240
   Gln Gly Arg Val Thr Ile Thr Ala Asp Glu Phe Thr Ser Ala Ala Tyr
   65                  70                  75                  80
   atg gag ctg agg agc ctg aga tct gag gac acg gcc gtc tac tac tgt      288
   Met Glu Leu Arg Ser Leu Arg Ser Glu Asp Thr Ala Val Tyr Tyr Cys
                   85                  90                  95
   gcg agg tct agt ggt tat tac ccc gaa tac ttc cag gac tgg ggc cag      336
   Ala Arg Ser Ser Gly Tyr Tyr Pro Glu Tyr Phe Gln Asp Trp Gly Gln
               100                 105                 110
   ggc acc ctg gtc acc gtc tcg agt gct agc acc aag ggc ccc agc gtg      384
   Gly Thr Leu Val Thr Val Ser Ser Ala Ser Thr Lys Gly Pro Ser Val
           115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg     1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg     1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac     1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac     1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc     1344
   Glu Ala Leu His Asn His Tyr Thr Gln Lys Ser Leu Ser Leu Ser Pro
           435                 440                 445
   ggc aag                                                             1350
   Gly Lys
       450
<210> 69
   <211> 450
   <212> PRT
   <213> Homo sapiens
<400> 69
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Met Gly Met Phe Gly Thr Thr Asn Tyr Ala Gln Lys Phe
       50                  55                  60
   Gln Gly Arg Val Thr Ile Thr Ala Asp Glu Phe Thr Ser Ala Ala Tyr
   65                  70                  75                  80
   Met Glu Leu Arg Ser Leu Arg Ser Glu Asp Thr Ala Val Tyr Tyr Cys
                   85                  90                  95
   Ala Arg Ser Ser Gly Tyr Tyr Pro Glu Tyr Phe Gln Asp Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 70
   <211> 1356
   <212> DNA
   <213> Homo sapiens
<220>
   <221> CDS
   <222> (1)..(1356)
<400> 70
   cag gtc cag ctg gtg cag tct ggg gga ggc ctg gtc aag cct ggg ggg      48
   Gln Val Gln Leu Val Gln Ser Gly Gly Gly Leu Val Lys Pro Gly Gly
   1               5                   10                  15
   tcc ctg aga ctc tcc tgt gca gcc tct gga ttc acc ttc agt agc tat      96
   Ser Leu Arg Leu Ser Cys Ala Ala Ser Gly Phe Thr Phe Ser Ser Tyr
               20                  25                  30
   agc atg aac tgg gtc cgc cag gct cca ggg aag ggg ctg gag tgg gtc     144
   Ser Met Asn Trp Val Arg Gln Ala Pro Gly Lys Gly Leu Glu Trp Val
           35                  40                  45
   tca tcc att agt agt agt agt agt tac ata tac tac gta gac tca gtg     192
   Ser Ser Ile Ser Ser Ser Ser Ser Tyr Ile Tyr Tyr Val Asp Ser Val
       50                  55                  60
   aag ggc cga ttc acc atc tcc aga gac aac gcc aag aac tca ctg tat     240
   Lys Gly Arg Phe Thr Ile Ser Arg Asp Asn Ala Lys Asn Ser Leu Tyr
   65                  70                  75                  80
   ctg caa atg aac agc ctg aga gcc gag gac acg gct gtg tat tac tgt     288
   Leu Gln Met Asn Ser Leu Arg Ala Glu Asp Thr Ala Val Tyr Tyr Cys
                   85                  90                  95
   gcg aga ggt ggt ggg agc tac ggg gcc tac gaa ggc ttt gac tac tgg     336
   Ala Arg Gly Gly Gly Ser Tyr Gly Ala Tyr Glu Gly Phe Asp Tyr Trp
               100                 105                 110
   ggc cag ggc acc ctg gtc acc gtc tcg agt gct agc acc aag ggc ccc     384
   Gly Gln Gly Thr Leu Val Thr Val Ser Ser Ala Ser Thr Lys Gly Pro
           115                 120                 125
   agc gtg ttc ccc ctg gcc ccc agc agc aag agc acc agc ggc ggc aca     432
   Ser Val Phe Pro Leu Ala Pro Ser Ser Lys Ser Thr Ser Gly Gly Thr
       130                 135                 140
   gcc gcc ctg ggc tgc ctg gtg aag gac tac ttc ccc gag ccc gtg acc     480
   Ala Ala Leu Gly Cys Leu Val Lys Asp Tyr Phe Pro Glu Pro Val Thr
   145                 150                 155                 160
   gtg agc tgg aac agc ggc gcc ttg acc agc ggc gtg cac acc ttc ccc     528
   Val Ser Trp Asn Ser Gly Ala Leu Thr Ser Gly Val His Thr Phe Pro
                   165                 170                 175
   gcc gtg ctg cag agc agc ggc ctg tac agc ctg agc agc gtg gtg acc     576
   Ala Val Leu Gln Ser Ser Gly Leu Tyr Ser Leu Ser Ser Val Val Thr
               180                 185                 190
   gtg ccc agc agc agc ctg ggc acc cag acc tac atc tgc aac gtg aac     624
   Val Pro Ser Ser Ser Leu Gly Thr Gln Thr Tyr Ile Cys Asn Val Asn
           195                 200                 205
   cac aag ccc agc aac acc aag gtg gac aaa cgc gtg gag ccc aag agc     672
   His Lys Pro Ser Asn Thr Lys Val Asp Lys Arg Val Glu Pro Lys Ser
       210                 215                 220
   tgc gac aag acc cac acc tgc ccc ccc tgc cct gcc ccc gag ctg ctg     720
   Cys Asp Lys Thr His Thr Cys Pro Pro Cys Pro Ala Pro Glu Leu Leu
   225                 230                 235                 240
   ggc gga ccc tcc gtg ttc ctg ttc ccc ccc aag ccc aag gac acc ctc     768
   Gly Gly Pro Ser Val Phe Leu Phe Pro Pro Lys Pro Lys Asp Thr Leu
                   245                 250                 255
   atg atc agc cgg acc ccc gag gtg acc tgc gtg gtg gtg gac gtg agc     816
   Met Ile Ser Arg Thr Pro Glu Val Thr Cys Val Val Val Asp Val Ser
               260                 265                 270
   cac gag gac ccc gag gtg aag ttc aac tgg tac gtg gac ggc gtg gag      864
   His Glu Asp Pro Glu Val Lys Phe Asn Trp Tyr Val Asp Gly Val Glu
           275                 280                 285
   gtg cac aac gcc aag acc aag ccc cgg gag gag cag tac aac agc acc      912
   Val His Asn Ala Lys Thr Lys Pro Arg Glu Glu Gln Tyr Asn Ser Thr
       290                 295                 300
   tac cgg gtg gtg agc gtg ctc acc gtg ctg cac cag gac tgg ctg aac      960
   Tyr Arg Val Val Ser Val Leu Thr Val Leu His Gln Asp Trp Leu Asn
   305                 310                 315                 320
   ggc aag gag tac aag tgc aag gtg agc aac aag gcc ctg cct gcc ccc     1008
   Gly Lys Glu Tyr Lys Cys Lys Val Ser Asn Lys Ala Leu Pro Ala Pro
                   325                 330                 335
   atc gag aag acc atc agc aag gcc aag ggc cag ccc cgg gag ccc cag     1056
   Ile Glu Lys Thr Ile Ser Lys Ala Lys Gly Gln Pro Arg Glu Pro Gln
               340                 345                 350
   gtg tac acc ctg ccc ccc agc cgg gag gag atg acc aag aac cag gtg     1104
   Val Tyr Thr Leu Pro Pro Ser Arg Glu Glu Met Thr Lys Asn Gln Val
           355                 360                 365
   tcc ctc acc tgt ctg gtg aag ggc ttc tac ccc agc gac atc gcc gtg     1152
   Ser Leu Thr Cys Leu Val Lys Gly Phe Tyr Pro Ser Asp Ile Ala Val
       370                 375                 380
   gag tgg gag agc aac ggc cag ccc gag aac aac tac aag acc acc ccc     1200
   Glu Trp Glu Ser Asn Gly Gln Pro Glu Asn Asn Tyr Lys Thr Thr Pro
   385                 390                 395                 400
   cct gtg ctg gac agc gac ggc agc ttc ttc ctg tac agc aag ctc acc     1248
   Pro Val Leu Asp Ser Asp Gly Ser Phe Phe Leu Tyr Ser Lys Leu Thr
                   405                 410                 415
   gtg gac aag agc cgg tgg cag cag ggc aac gtg ttc agc tgc agc gtg     1296
   Val Asp Lys Ser Arg Trp Gln Gln Gly Asn Val Phe Ser Cys Ser Val
               420                 425                 430
   atg cac gag gcc ctg cac aac cac tac acc cag aag agc ctg agc ctg     1344
   Met His Glu Ala Leu His Asn His Tyr Thr Gln Lys Ser Leu Ser Leu
           435                 440                 445
   agc ccc ggc aag                                                     1356
   Ser Pro Gly Lys
       450
<210> 71
   <211> 452
   <212> PRT
   <213> Homo sapiens
<400> 71
   Gln Val Gln Leu Val Gln Ser Gly Gly Gly Leu Val Lys Pro Gly Gly
   1               5                   10                  15
   Ser Leu Arg Leu Ser Cys Ala Ala Ser Gly Phe Thr Phe Ser Ser Tyr
               20                  25                  30
   Ser Met Asn Trp Val Arg Gln Ala Pro Gly Lys Gly Leu Glu Trp Val
           35                  40                  45
   Ser Ser Ile Ser Ser Ser Ser Ser Tyr Ile Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Gly Ser Tyr Gly Ala Tyr Glu Gly Phe Asp Tyr Trp
               100                 105                 110
   Gly Gln Gly Thr Leu Val Thr Val Ser Ser Ala Ser Thr Lys Gly Pro
           115                 120                 125
   Ser Val Phe Pro Leu Ala Pro Ser Ser Lys Ser Thr Ser Gly Gly Thr
       130                 135                 140
   Ala Ala Leu Gly Cys Leu Val Lys Asp Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ala Val Leu Gln Ser Ser Gly Leu Tyr Ser Leu Ser Ser Val Val Thr
               180                 185                 190
   Val Pro Ser Ser Ser Leu Gly Thr Gln Thr Tyr Ile Cys Asn Val Asn
           195                 200                 205
   His Lys Pro Ser Asn Thr Lys Val Asp Lys Arg Val Glu Pro Lys Ser
       210                 215                 220
   Cys Asp Lys Thr His Thr Cys Pro Pro Cys Pro Ala Pro Glu Leu Leu
   225                 230                 235                 240
   Gly Gly Pro Ser Val Phe Leu Phe Pro Pro Lys Pro Lys Asp Thr Leu
                   245                 250                 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
   290 295 300
   Tyr Arg Val Val Ser Val Leu Thr Val Leu His Gln Asp Trp Leu Asn
   305 310 315 320
   Gly Lys Glu Tyr Lys Cys Lys Val Ser Asn Lys Ala Leu Pro Ala Pro
   325 330 335
   Ile Glu Lys Thr Ile Ser Lys Ala Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 72
   <211> 1353
   <212> DNA
   <213> Homo sapiens
<220>
   <221> CDS
   <222> (1)..(1353)
<400> 72
   gag gtg cag ctg gtg gag tct ggg gct gag gtg aag aag cct ggg tcc      48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat      96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg     144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc      384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg     1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc     1344
   His Glu Ala Leu His Asn His Tyr Thr Gln Lys Ser Leu Ser Leu Ser
           435                 440                 445
   ccc ggc aag                                                         1353
   Pro Gly Lys
       450
<210> 73
   <211> 451
   <212> PRT
   <213> Homo sapiens
<400> 73
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 74
   <211> 1353
   <212> DNA
   <213> Homo sapiens
<220>
   <221> CDS
   <222> (1)..(1353)
<400> 74
   gag gtg cag ctg gtg gag tct ggg gct gag gtg aag aag cct ggg tcc        48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat        96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg       144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc       192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac       240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt        288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc        336
   Ala Lys His Met Gly Tyr Gln Val Arg Glu Thr Met Asp Val Trp Gly
               100                 105                 110
   aaa ggg acc acg gtc acc gtc tcg agt gct agc acc aag ggc ccc agc        384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc        432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg        480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc        528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg        576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac        624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc        672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc        720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg        768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac        816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg        864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac        912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc        960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc       1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg      1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc      1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag      1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct      1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg      1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 75
   <211> 451
   <212> PRT
   <213> Homo sapiens
<400> 75
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 76
   <211> 1350
   <212> DNA
   <213> Homo sapiens
<220>
   <221> CDS
   <222> (1)..(1350)
<400> 76
   gag gtg cag ctg gtg gag tct ggg gct gag gtg aag aag cca ggg tcc      48
   Glu Val Gln Leu Val Glu Ser Gly Ala Glu Val Lys Lys Pro Gly Ser
   1               5                   10                  15
   tcg gtg aag gtc tcc tgt aag gcc tct gga ggc acc ttc tcc agc tat      96
   Ser Val Lys Val Ser Cys Lys Ala Ser Gly Gly Thr Phe Ser Ser Tyr
               20                  25                  30
   ggt atc agc tgg gtg cga cag gcc cct gga caa ggg ctt gag tgg atg     144
   Gly Ile Ser Trp Val Arg Gln Ala Pro Gly Gln Gly Leu Glu Trp Met
           35                  40                  45
   gga gac atc atc ggt atg ttt ggt tca aca aac tac gca cag aac ttc     192
   Gly Asp Ile Ile Gly Met Phe Gly Ser Thr Asn Tyr Ala Gln Asn Phe
       50                  55                  60
   cag ggc aga ctc acg att acc gcg gac gaa tcc acg agc aca gcc tac     240
   Gln Gly Arg Leu Thr Ile Thr Ala Asp Glu Ser Thr Ser Thr Ala Tyr
   65                  70                  75                  80
   atg gag ctg agc agc ctg aga tct gag gac acg gcc gtg tat tac tgt     288
   Met Glu Leu Ser Ser Leu Arg Ser Glu Asp Thr Ala Val Tyr Tyr Cys
                   85                  90                  95
   gcg aga agt agt ggt tat tac cct gca tac ctc ccc cac tgg ggc cag     336
   Ala Arg Ser Ser Gly Tyr Tyr Pro Ala Tyr Leu Pro His Trp Gly Gln
               100                 105                 110
   ggc acc ttg gtc acc gtc tcg agt gct agc acc aag ggc ccc agc gtg      384
   Gly Thr Leu Val Thr Val Ser Ser Ala Ser Thr Lys Gly Pro Ser Val
           115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg      1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg      1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac      1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac      1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc      1344
   Glu Ala Leu His Asn His Tyr Thr Gln Lys Ser Leu Ser Leu Ser Pro
           435                 440                 445
   ggc aag                                                              1350
   Gly Lys
       450
<210> 77
   <211> 450
   <212> PRT
   <213> Homo sapiens
<400> 77
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Gly Ile Ser Trp Val Arg Gln Ala Pro Gly Gln Gly Leu Glu Trp Met
           35                  40                  45
   Gly Asp Ile Ile Gly Met Phe Gly Ser Thr Asn Tyr Ala Gln Asn Phe
       50                  55                  60
   Gln Gly Arg Leu Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ser Gly Tyr Tyr Pro Ala Tyr Leu Pro His Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 78
   <211> 1353
   <212> DNA
   <213> Homo sapiens
<220>
   <221> CDS
   <222> (1)..(1353)
<400> 78
   gag gtg cag ctg gtg gag tct ggg gct gag gtg aag aag ccg ggg tcc      48
   Glu Val Gln Leu Val Glu Ser Gly Ala Glu Val Lys Lys Pro Gly Ser
   1               5                   10                  15
   tcg gtg aag gtc tcc tgc aag gct tct gga ggc acc ttc agc ttc tat      96
   Ser Val Lys Val Ser Cys Lys Ala Ser Gly Gly Thr Phe Ser Phe Tyr
               20                  25                  30
   tct atg agc tgg gtg cga cag gcc cct gga caa gga ctt gag tgg atg     144
   Ser Met Ser Trp Val Arg Gln Ala Pro Gly Gln Gly Leu Glu Trp Met
           35                  40                  45
   gga ggg atc atc cct atg ttt ggt aca aca aac tac gca cag aag ttc     192
   Gly Gly Ile Ile Pro Met Phe Gly Thr Thr Asn Tyr Ala Gln Lys Phe
       50                  55                  60
   cag ggc aga gtc acg att acc gcg gtc gaa tcc acg agc aca gcc tac     240
   Gln Gly Arg Val Thr Ile Thr Ala Val Glu Ser Thr Ser Thr Ala Tyr
   65                  70                  75                  80
   atg gag gtg agc agc ctg aga tct gag gac acg gcc gtt tat tac tgt     288
   Met Glu Val Ser Ser Leu Arg Ser Glu Asp Thr Ala Val Tyr Tyr Cys
                   85                  90                  95
   gcg aga ggt gat aag ggt atc tac tac tac tac atg gac gtc tgg ggc     336
   Ala Arg Gly Asp Lys Gly Ile Tyr Tyr Tyr Tyr Met Asp Val Trp Gly
               100                 105                 110
   aaa ggg acc acg gtc acc gtc tcg agt gct agc acc aag ggc ccc agc     384
   Lys Gly Thr Thr Val Thr Val Ser Ser Ala Ser Thr Lys Gly Pro Ser
           115                 120                 125
   gtg ttc ccc ctg gcc ccc agc agc aag agc acc agc ggc ggc aca gcc     432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 79
   <211> 451
   <212> PRT
   <213> Homo sapiens
<400> 79
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Phe Tyr
               20                  25                  30
   Ser Met Ser Trp Val Arg Gln Ala Pro Gly Gln Gly Leu Glu Trp Met
           35                  40                  45
   Gly Gly Ile Ile Pro Met Phe Gly Thr Thr Asn Tyr Ala Gln Lys Phe
       50                  55                  60
   Gln Gly Arg Val Thr Ile Thr Ala Val Glu Ser Thr Ser Thr Ala Tyr
   65                  70                  75                  80
   Met Glu Val Ser Ser Leu Arg Ser Glu Asp Thr Ala Val Tyr Tyr Cys
                   85                  90                  95
   Ala Arg Gly Asp Lys Gly Ile Tyr Tyr Tyr Tyr Met Asp Val Trp Gly
               100                 105                 110
   Lys Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 80
   <211> 1350
   <212> DNA
   <213> Homo sapiens
<220>
   <221> CDS
   <222> (1)..(1350)
<400> 80
   gag gtg cag ctg gtg gag tct ggg gct gag gtg aag aag cct ggg tcc      48
   Glu Val Gln Leu Val Glu Ser Gly Ala Glu Val Lys Lys Pro Gly Ser
   1               5                   10                  15
   tcg gtg aag gtc tcc tgc aag gct tct gga ggc acc ttc agc agc tat      96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg     144
   Ala Ile Ser Trp Val Arg Gln Ala Pro Gly Gln Gly Leu Glu Trp Met
           35                  40                  45
   gga ggg atc atc ggt atg ttc ggt aca gca aac tac gca cag aag ttc     192
   Gly Gly Ile Ile Gly Met Phe Gly Thr Ala Asn Tyr Ala Gln Lys Phe
       50                  55                  60
   cag ggc aga gtc acg att acc gcg gac gaa ttt acg agc aca gcc tac     240
   Gln Gly Arg Val Thr Ile Thr Ala Asp Glu Phe Thr Ser Thr Ala Tyr
   65                  70                  75                  80
   atg gag ctg agc agc ctg aga tct gag gac acg gcc gtg tat tac tgt     288
   Met Glu Leu Ser Ser Leu Arg Ser Glu Asp Thr Ala Val Tyr Tyr Cys
                   85                  90                  95
   gcg aga gga aat tat tac tat gag agt agt ctc gac tac tgg ggc cag     336
   Ala Arg Gly Asn Tyr Tyr Tyr Glu Ser Ser Leu Asp Tyr Trp Gly Gln
               100                 105                 110
   gga acc ctg gtc acc gtc tcg agt gct agc acc aag ggc ccc agc gtg     384
   Gly Thr Leu Val Thr Val Ser Ser Ala Ser Thr Lys Gly Pro Ser Val
           115                 120                 125
   ttc ccc ctg gcc ccc agc agc aag agc acc agc ggc ggc aca gcc gcc     432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc     480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg     528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc     576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg     1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg     1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac     1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac     1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc     1344
   Glu Ala Leu His Asn His Tyr Thr Gln Lys Ser Leu Ser Leu Ser Pro
   435 440 445
   ggc aag 1350
   Gly Lys
   450
<210> 81
   <211> 450
   <212> PRT
   <213> Homo sapiens
<400> 81
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Gly Met Phe Gly Thr Ala Asn Tyr Ala Gln Lys Phe
       50                  55                  60
   Gln Gly Arg Val Thr Ile Thr Ala Asp Glu Phe Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Asn Tyr Tyr Tyr Glu Ser Ser Leu Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 82
   <211> 1359
   <212> DNA
   <213> Homo sapiens
<220>
   <221> CDS
   <222> (1)..(1359)
<400> 82
   cag gtg cag ctg gtg cag tct ggg gct gag gtg aag aag cct ggg tcc      48
   Gln Val Gln Leu Val Gln Ser Gly Ala Glu Val Lys Lys Pro Gly Ser
   1               5                   10                  15
   tcg gtg aga gtc tcc tgc aag gct tct gga agc atc ttc aga aac tat      96
   Ser Val Arg Val Ser Cys Lys Ala Ser Gly Ser Ile Phe Arg Asn Tyr
               20                  25                  30
   gct atg agc tgg gtg cga cag gcc cct gga caa ggg ctt gag tgg atg     144
   Ala Met Ser Trp Val Arg Gln Ala Pro Gly Gln Gly Leu Glu Trp Met
           35                  40                  45
   gga ggg atc atc gct att ttt ggg aca cca aag tac gca cag aag ttc     192
   Gly Gly Ile Ile Ala Ile Phe Gly Thr Pro Lys Tyr Ala Gln Lys Phe
       50                  55                  60
   cag ggc aga gtc acg att acc gcg gac gaa tcg acg agc act gtc tac     240
   Gln Gly Arg Val Thr Ile Thr Ala Asp Glu Ser Thr Ser Thr Val Tyr
   65                  70                  75                  80
   atg gaa ctg agc gga ctg aga tct gag gac acg gcc atg tat tac tgt     288
   Met Glu Leu Ser Gly Leu Arg Ser Glu Asp Thr Ala Met Tyr Tyr Cys
                   85                  90                  95
   gcg agg att ccc cac tat aat ttt ggt tcg ggg agt tat ttc gac tac     336
   Ala Arg Ile Pro His Tyr Asn Phe Gly Ser Gly Ser Tyr Phe Asp Tyr
               100                 105                 110
   tgg ggc cag gga acc ctg gtc acc gtc tcg agt gct agc acc aag ggc     384
   Trp Gly Gln Gly Thr Leu Val Thr Val Ser Ser Ala Ser Thr Lys Gly
           115                 120                 125
   ccc agc gtg ttc ccc ctg gcc ccc agc agc aag agc acc agc ggc ggc     432
   Pro Ser Val Phe Pro Leu Ala Pro Ser Ser Lys Ser Thr Ser Gly Gly
       130                 135                 140
   aca gcc gcc ctg ggc tgc ctg gtg aag gac tac ttc ccc gag ccc gtg     480
   Thr Ala Ala Leu Gly Cys Leu Val Lys Asp Tyr Phe Pro Glu Pro Val
   145                 150                 155                 160
   acc gtg agc tgg aac agc ggc gcc ttg acc agc ggc gtg cac acc ttc     528
   Thr Val Ser Trp Asn Ser Gly Ala Leu Thr Ser Gly Val His Thr Phe
                   165                 170                 175
   ccc gcc gtg ctg cag agc agc ggc ctg tac agc ctg agc agc gtg gtg     576
   Pro Ala Val Leu Gln Ser Ser Gly Leu Tyr Ser Leu Ser Ser Val Val
               180                 185                 190
   acc gtg ccc agc agc agc ctg ggc acc cag acc tac atc tgc aac gtg     624
   Thr Val Pro Ser Ser Ser Leu Gly Thr Gln Thr Tyr Ile Cys Asn Val
           195                 200                 205
   aac cac aag ccc agc aac acc aag gtg gac aaa cgc gtg gag ccc aag     672
   Asn His Lys Pro Ser Asn Thr Lys Val Asp Lys Arg Val Glu Pro Lys
   210 215 220
   agc tgc gac aag acc cac acc tgc ccc ccc tgc cct gcc ccc gag ctg 720
   Ser Cys Asp Lys Thr His Thr Cys Pro Pro Cys Pro Ala Pro Glu Leu
   225 230 235 240
   ctg ggc gga ccc tcc gtg ttc ctg ttc ccc ccc aag ccc aag gac acc 768
   Leu Gly Gly Pro Ser Val Phe Leu Phe Pro Pro Lys Pro Lys Asp Thr
   245 250 255
   ctc atg atc agc cgg acc ccc gag gtg acc tgc gtg gtg gtg gac gtg 816
   Leu Met Ile Ser Arg Thr Pro Glu Val Thr Cys Val Val Val Asp Val
   260 265 270
   agc cac gag gac ccc gag gtg aag ttc aac tgg tac gtg gac ggc gtg 864
   Ser His Glu Asp Pro Glu Val Lys Phe Asn Trp Tyr Val Asp Gly Val
   275 280 285
   gag gtg cac aac gcc aag acc aag ccc cgg gag gag cag tac aac agc 912
   Glu Val His Asn Ala Lys Thr Lys Pro Arg Glu Glu Gln Tyr Asn Ser
   290 295 300
   acc tac cgg gtg gtg agc gtg ctc acc gtg ctg cac cag gac tgg ctg 960
   Thr Tyr Arg Val Val Ser Val Leu Thr Val Leu His Gln Asp Trp Leu
   305 310 315 320
   aac ggc aag gag tac aag tgc aag gtg agc aac aag gcc ctg cct gcc 1008
   Asn Gly Lys Glu Tyr Lys Cys Lys Val Ser Asn Lys Ala Leu Pro Ala
   325 330 335
   ccc atc gag aag acc atc agc aag gcc aag ggc cag ccc cgg gag ccc 1056
   Pro Ile Glu Lys Thr Ile Ser Lys Ala Lys Gly Gln Pro Arg Glu Pro
   340 345 350
   cag gtg tac acc ctg ccc ccc agc cgg gag gag atg acc aag aac cag 1104
   Gln Val Tyr Thr Leu Pro Pro Ser Arg Glu Glu Met Thr Lys Asn Gln
   355 360 365
   gtg tcc ctc acc tgt ctg gtg aag ggc ttc tac ccc agc gac atc gcc 1152
   Val Ser Leu Thr Cys Leu Val Lys Gly Phe Tyr Pro Ser Asp Ile Ala
   370 375 380
   gtg gag tgg gag agc aac ggc cag ccc gag aac aac tac aag acc acc 1200
   Val Glu Trp Glu Ser Asn Gly Gln Pro Glu Asn Asn Tyr Lys Thr Thr
   385 390 395 400
   ccc cct gtg ctg gac agc gac ggc agc ttc ttc ctg tac agc aag ctc 1248
   Pro Pro Val Leu Asp Ser Asp Gly Ser Phe Phe Leu Tyr Ser Lys Leu
   405 410 415
   acc gtg gac aag agc cgg tgg cag cag ggc aac gtg ttc agc tgc agc 1296
   Thr Val Asp Lys Ser Arg Trp Gln Gln Gly Asn Val Phe Ser Cys Ser
   420 425 430
   gtg atg cac gag gcc ctg cac aac cac tac acc cag aag agc ctg agc 1344
   Val Met His Glu Ala Leu His Asn His Tyr Thr Gln Lys Ser Leu Ser
   435 440 445
   ctg agc ccc ggc aag 1359
   Leu Ser Pro Gly Lys
   450
<210> 83
   <211> 453
   <212> PRT
   <213> Homo sapiens
<400> 83
   Gln Val Gln Leu Val Gln Ser Gly Ala Glu Val Lys Lys Pro Gly Ser
   1               5                   10                  15
   Ser Val Arg Val Ser Cys Lys Ala Ser Gly Ser Ile Phe Arg Asn Tyr
               20                  25                  30
   Ala Met Ser Trp Val Arg Gln Ala Pro Gly Gln Gly Leu Glu Trp Met
           35                  40                  45
   Gly Gly Ile Ile Ala Ile Phe Gly Thr Pro Lys Tyr Ala Gln Lys Phe
       50                  55                  60
   Gln Gly Arg Val Thr Ile Thr Ala Asp Glu Ser Thr Ser Thr Val Tyr
   65                  70                  75                  80
   Met Glu Leu Ser Gly Leu Arg Ser Glu Asp Thr Ala Met Tyr Tyr Cys
                   85                  90                  95
   Ala Arg Ile Pro His Tyr Asn Phe Gly Ser Gly Ser Tyr Phe Asp Tyr
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Arg Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
   245 250 255
   Leu Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly Val
   275 280 285
   Glu Val His Asn Ala Lys Thr Lys Pro Arg Glu Glu Gln Tyr Asn Ser
   290 295 300
   Thr Tyr Arg Val Val Ser Val Leu Thr Val Leu His Gln Asp Trp Leu
   305 310 315 320
   Asn Gly Lys Glu Tyr Lys Cys Lys Val Ser Asn Lys Ala Leu Pro Ala
   325 330 335
   Pro Ile Glu Lys Thr Ile Ser Lys Ala Lys Gly Gln Pro Arg Glu Pro
   340 345 350
   Gln Val Tyr Thr Leu Pro Pro Ser Arg Glu Glu Met Thr Lys Asn Gln
   355 360 365
   Val Ser Leu Thr Cys Leu Val Lys Gly Phe Tyr Pro Ser Asp Ile Ala
   370 375 380
   Val Glu Trp Glu Ser Asn Gly Gln Pro Glu Asn Asn Tyr Lys Thr Thr
   385 390 395 400
   Pro Pro Val Leu Asp Ser Asp Gly Ser Phe Phe Leu Tyr Ser Lys Leu
   405 410 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 84
   <211> 660
   <212> DNA
   <213> Homo sapiens
<220>
   <221> CDS
   <222> (1)..(660)
<400> 84
   tcc tat gtg ctg act cag cca ccc tca gcg tct ggg acc ccc ggg cag        48
   Ser Tyr Val Leu Thr Gln Pro Pro Ser Ala Ser Gly Thr Pro Gly Gln
   1               5                   10                  15
   agg gtc acc atc tct tgt tct gga agc acg ttc aac atc gga agt aat        96
   Arg Val Thr Ile Ser Cys Ser Gly Ser Thr Phe Asn Ile Gly Ser Asn
               20                  25                  30
   gct gta gac tgg tac cgg cag ctc cca gga acg gcc ccc aaa ctc ctc       144
   Ala Val Asp Trp Tyr Arg Gln Leu Pro Gly Thr Ala Pro Lys Leu Leu
           35                  40                  45
   atc tat agt aat aat cag cgg ccc tca ggg gtc cct gac cga ttc tct       192
   Ile Tyr Ser Asn Asn Gln Arg Pro Ser Gly Val Pro Asp Arg Phe Ser
       50                  55                  60
   ggc tcc agg tct ggc acc tca gcc tcc ctg gcc atc agt ggg ctc cag       240
   Gly Ser Arg Ser Gly Thr Ser Ala Ser Leu Ala Ile Ser Gly Leu Gln
   65                  70                  75                  80
   tct gag gat gag gct gat tat tac tgt gca gca tgg gat gac atc ctg       288
   Ser Glu Asp Glu Ala Asp Tyr Tyr Cys Ala Ala Trp Asp Asp Ile Leu
                   85                  90                  95
   aat gtt ccg gta ttc ggc gga ggg acc aag ctg acc gtc cta ggt gcg       336
   Asn Val Pro Val Phe Gly Gly Gly Thr Lys Leu Thr Val Leu Gly Ala
               100                 105                 110
   gcc gca ggc cag ccc aag gcc gct ccc agc gtg acc ctg ttc ccc ccc       384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc       432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc       480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc       528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag       576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc       624
   Trp Lys Ser His Arg Ser Tyr Ser Cys Gln Val Thr His Glu Gly Ser
           195                 200                 205
   acc gtg gag aag acc gtg gcc ccc acc gag tgc agc                       660
   Thr Val Glu Lys Thr Val Ala Pro Thr Glu Cys Ser
       210                 215                 220
<210> 85
   <211> 220
   <212> PRT
   <213> Homo sapiens
<400> 85
   Ser Tyr Val Leu Thr Gln Pro Pro Ser Ala Ser Gly Thr Pro Gly Gln
   1               5                   10                  15
   Arg Val Thr Ile Ser Cys Ser Gly Ser Thr Phe Asn Ile Gly Ser Asn
               20                  25                  30
   Ala Val Asp Trp Tyr Arg Gln Leu Pro Gly Thr Ala Pro Lys Leu Leu
           35                  40                  45
   Ile Tyr Ser Asn Asn Gln Arg Pro Ser Gly Val Pro Asp Arg Phe Ser
       50                  55                  60
   Gly Ser Arg Ser Gly Thr Ser Ala Ser Leu Ala Ile Ser Gly Leu Gln
   65                  70                  75                  80
   Ser Glu Asp Glu Ala Asp Tyr Tyr Cys Ala Ala Trp Asp Asp Ile Leu
                   85                  90                  95
   Asn Val Pro Val Phe Gly Gly Gly Thr Lys Leu Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 86
   <211> 660
   <212> DNA
   <213> Homo sapiens
<220>
   <221> CDS
   <222> (1)..(660)
<400> 86
   cag tct gcc ctg act cag cct gcc gcc gtg tct ggg tct cct gga cag          48
   Gln Ser Ala Leu Thr Gln Pro Ala Ala Val Ser Gly Ser Pro Gly Gln
   1               5                   10                  15
   tcg atc acc atc tcc tgc act gga acc agc agt gac gtt ggt ggt tat          96
   Ser Ile Thr Ile Ser Cys Thr Gly Thr Ser Ser Asp Val Gly Gly Tyr
               20                  25                  30
   aac tat gtc tcc tgg tac caa cag cac cca ggc aaa gcc ccc aaa ctc         144
   Asn Tyr Val Ser Trp Tyr Gln Gln His Pro Gly Lys Ala Pro Lys Leu
           35                  40                  45
   atg att tat gag gtc agt aat cgg ccc tca ggg gtt tct aat cgc ttc         192
   Met Ile Tyr Glu Val Ser Asn Arg Pro Ser Gly Val Ser Asn Arg Phe
       50                  55                  60
   tct ggc tcc aag tct ggc aac acg gcc tcc ctg acc atc tct ggg ctc         240
   Ser Gly Ser Lys Ser Gly Asn Thr Ala Ser Leu Thr Ile Ser Gly Leu
   65                  70                  75                  80
   cag gct gag gac gag gct gat tat tac tgc agc tca tat aca agc agc         288
   Gln Ala Glu Asp Glu Ala Asp Tyr Tyr Cys Ser Ser Tyr Thr Ser Ser
                   85                  90                  95
   agc act tat gtc ttc gga act ggg acc aag gtc acc gtc cta ggt gcg         336
   Ser Thr Tyr Val Phe Gly Thr Gly Thr Lys Val Thr Val Leu Gly Ala
               100                 105                 110
   gcc gca ggc cag ccc aag gcc gct ccc agc gtg acc ctg ttc ccc ccc         384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc         432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc         480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc         528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag         576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc         624
   Trp Lys Ser His Arg Ser Tyr Ser Cys Gln Val Thr His Glu Gly Ser
           195                 200                 205
   acc gtg gag aag acc gtg gcc ccc acc gag tgc agc                         660
   Thr Val Glu Lys Thr Val Ala Pro Thr Glu Cys Ser
       210                 215                 220
<210> 87
   <211> 220
   <212> PRT
   <213> Homo sapiens
<400> 87
   Gln Ser Ala Leu Thr Gln Pro Ala Ala Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Glu Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Tyr Val Phe Gly Thr Gly Thr Lys Val Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 88
   <211> 660
   <212> DNA
   <213> Homo sapiens
<220>
   <221> CDS
   <222> (1)..(660)
<400> 88
   tcc tat gtg ctg act cag cca ccc tca gtc tct ggg acc ccc ggg cag       48
   Ser Tyr Val Leu Thr Gln Pro Pro Ser Val Ser Gly Thr Pro Gly Gln
   1               5                   10                  15
   agg gtc acc atc tct tgc tct gga agc cgc tcc aac gtc gga gat aat       96
   Arg Val Thr Ile Ser Cys Ser Gly Ser Arg Ser Asn Val Gly Asp Asn
               20                  25                  30
   tct gta tat tgg tat caa cac gtc cca gaa atg gcc ccc aaa ctc ctc      144
   Ser Val Tyr Trp Tyr Gln His Val Pro Glu Met Ala Pro Lys Leu Leu
           35                  40                  45
   gtc tat aag aat act caa cgg ccc tca gga gtc cct gcc cgg ttt tcc      192
   Val Tyr Lys Asn Thr Gln Arg Pro Ser Gly Val Pro Ala Arg Phe Ser
       50                  55                  60
   ggc tcc aag tct ggc act tca gcc tcc ctg gcc atc att ggc ctc cag      240
   Gly Ser Lys Ser Gly Thr Ser Ala Ser Leu Ala Ile Ile Gly Leu Gln
   65                  70                  75                  80
   tcc ggc gat gag gct gat tat tat tgt gtg gca tgg gat gac agc gta      288
   Ser Gly Asp Glu Ala Asp Tyr Tyr Cys Val Ala Trp Asp Asp Ser Val
                   85                  90                  95
   gat ggc tat gtc ttc gga tct ggg acc aag gtc acc gtc cta ggt gcg      336
   Asp Gly Tyr Val Phe Gly Ser Gly Thr Lys Val Thr Val Leu Gly Ala
               100                 105                 110
   gcc gca ggc cag ccc aag gcc gct ccc agc gtg acc ctg ttc ccc ccc      384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc      432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc      480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc      528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag      576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc      624
   Trp Lys Ser His Arg Ser Tyr Ser Cys Gln Val Thr His Glu Gly Ser
           195                 200                 205
   acc gtg gag aag acc gtg gcc ccc acc gag tgc agc                      660
   Thr Val Glu Lys Thr Val Ala Pro Thr Glu Cys Ser
       210                 215                 220
<210> 89
   <211> 220
   <212> PRT
   <213> Homo sapiens
<400> 89
   Ser Tyr Val Leu Thr Gln Pro Pro Ser Val Ser Gly Thr Pro Gly Gln
   1               5                   10                  15
   Arg Val Thr Ile Ser Cys Ser Gly Ser Arg Ser Asn Val Gly Asp Asn
               20                  25                  30
   Ser Val Tyr Trp Tyr Gln His Val Pro Glu Met Ala Pro Lys Leu Leu
           35                  40                  45
   Val Tyr Lys Asn Thr Gln Arg Pro Ser Gly Val Pro Ala Arg Phe Ser
       50                  55                  60
   Gly Ser Lys Ser Gly Thr Ser Ala Ser Leu Ala Ile Ile Gly Leu Gln
   65                  70                  75                  80
   Ser Gly Asp Glu Ala Asp Tyr Tyr Cys Val Ala Trp Asp Asp Ser Val
                   85                  90                  95
   Asp Gly Tyr Val Phe Gly Ser Gly Thr Lys Val Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 90
   <211> 663
   <212> DNA
   <213> Homo sapiens
<220>
   <221> CDS
   <222> (1)..(663)
<400> 90
   cag tct gtg ttg acg cag ccg ccc tca gtg tct gcg gcc cca gga cag        48
   Gln Ser Val Leu Thr Gln Pro Pro Ser Val Ser Ala Ala Pro Gly Gln
   1               5                   10                  15
   aag gtc acc atc tcc tgc tct gga agc agc tcc aac att ggg aat gat        96
   Lys Val Thr Ile Ser Cys Ser Gly Ser Ser Ser Asn Ile Gly Asn Asp
               20                  25                  30
   tat gta tcc tgg tac cag cag ctc cca gga aca gcc ccc aaa ctc ctc       144
   Tyr Val Ser Trp Tyr Gln Gln Leu Pro Gly Thr Ala Pro Lys Leu Leu
           35                  40                  45
   att tat gac aat aat aag cga ccc tca ggg att cct gac cga ttc tct       192
   Ile Tyr Asp Asn Asn Lys Arg Pro Ser Gly Ile Pro Asp Arg Phe Ser
       50                  55                  60
   ggc tcc aag tct ggc acg tca gcc acc ctg ggc atc acc gga ctc cag       240
   Gly Ser Lys Ser Gly Thr Ser Ala Thr Leu Gly Ile Thr Gly Leu Gln
   65                  70                  75                  80
   act ggg gac gag gcc aac tat tac tgc gca aca tgg gat cgc cgc ccg       288
   Thr Gly Asp Glu Ala Asn Tyr Tyr Cys Ala Thr Trp Asp Arg Arg Pro
                   85                  90                  95
   act gct tat gtt gtc ttc ggc gga ggg acc aag ctg acc gtc cta ggt       336
   Thr Ala Tyr Val Val Phe Gly Gly Gly Thr Lys Leu Thr Val Leu Gly
               100                 105                 110
   gcg gcc gca ggc cag ccc aag gcc gct ccc agc gtg acc ctg ttc ccc       384
   Ala Ala Ala Gly Gln Pro Lys Ala Ala Pro Ser Val Thr Leu Phe Pro
           115                 120                 125
   ccc tcc tcc gag gag ctg cag gcc aac aag gcc acc ctg gtg tgc ctc       432
   Pro Ser Ser Glu Glu Leu Gln Ala Asn Lys Ala Thr Leu Val Cys Leu
       130                 135                 140
   atc agc gac ttc tac cct ggc gcc gtg acc gtg gcc tgg aag gcc gac       480
   Ile Ser Asp Phe Tyr Pro Gly Ala Val Thr Val Ala Trp Lys Ala Asp
   145                 150                 155                 160
   agc agc ccc gtg aag gcc ggc gtg gag acc acc acc ccc agc aag cag       528
   Ser Ser Pro Val Lys Ala Gly Val Glu Thr Thr Thr Pro Ser Lys Gln
                   165                 170                 175
   agc aac aac aag tac gcc gcc agc agc tac ctg agc ctc acc ccc gag       576
   Ser Asn Asn Lys Tyr Ala Ala Ser Ser Tyr Leu Ser Leu Thr Pro Glu
               180                 185                 190
   cag tgg aag agc cac cgg agc tac agc tgc cag gtg acc cac gag ggc       624
   Gln Trp Lys Ser His Arg Ser Tyr Ser Cys Gln Val Thr His Glu Gly
           195                 200                 205
   agc acc gtg gag aag acc gtg gcc ccc acc gag tgc agc                   663
   Ser Thr Val Glu Lys Thr Val Ala Pro Thr Glu Cys Ser
       210                 215                 220
<210> 91
   <211> 221
   <212> PRT
   <213> Homo sapiens
<400> 91
   Gln Ser Val Leu Thr Gln Pro Pro Ser Val Ser Ala Ala Pro Gly Gln
   1               5                   10                  15
   Lys Val Thr Ile Ser Cys Ser Gly Ser Ser Ser Asn Ile Gly Asn Asp
               20                  25                  30
   Tyr Val Ser Trp Tyr Gln Gln Leu Pro Gly Thr Ala Pro Lys Leu Leu
           35                  40                  45
   Ile Tyr Asp Asn Asn Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asn Tyr Tyr Cys Ala Thr Trp Asp Arg Arg Pro
                   85                  90                  95
   Thr Ala Tyr Val Val Phe Gly Gly Gly Thr Lys Leu Thr Val Leu Gly
               100                 105                 110
   Ala Ala Ala Gly Gln Pro Lys Ala Ala Pro Ser Val Thr Leu Phe Pro
           115                 120                 125
   Pro Ser Ser Glu Glu Leu Gln Ala Asn Lys Ala Thr Leu Val Cys Leu
       130                 135                 140
   Ile Ser Asp Phe Tyr Pro Gly Ala Val Thr Val Ala Trp Lys Ala Asp
   145                 150                 155                 160
   Ser Ser Pro Val Lys Ala Gly Val Glu Thr Thr Thr Pro Ser Lys Gln
                   165                 170                 175
   Ser Asn Asn Lys Tyr Ala Ala Ser Ser Tyr Leu Ser Leu Thr Pro Glu
               180                 185                 190
   Gln Trp Lys Ser His Arg Ser Tyr Ser Cys Gln Val Thr His Glu Gly
           195                 200                 205
   Ser Thr Val Glu Lys Thr Val Ala Pro Thr Glu Cys Ser
       210                 215                 220
<210> 92
   <211> 642
   <212> DNA
   <213> Homo sapiens
<220>
   <221> CDS
   <222> (1)..(642)
<400> 92
   gac atc cag atg acc cag tct cca tcc tcc ctg tct gca tct gta gga        48
   Asp Ile Gln Met Thr Gln Ser Pro Ser Ser Leu Ser Ala Ser Val Gly
   1               5                   10                  15
   gac aga gtc acc atc act tgc cgg gcg agt cag ggc att agc agt tat        96
   Asp Arg Val Thr Ile Thr Cys Arg Ala Ser Gln Gly Ile Ser Ser Tyr
               20                  25                  30
   tta gcc tgg tat cag cag aag cca ggg aaa gtt cct aca ctc ctg atc       144
   Leu Ala Trp Tyr Gln Gln Lys Pro Gly Lys Val Pro Thr Leu Leu Ile
           35                  40                  45
   tat gat gca tcc act ttg cga tca ggg gtc cca tct cgc ttc agt ggc       192
   Tyr Asp Ala Ser Thr Leu Arg Ser Gly Val Pro Ser Arg Phe Ser Gly
       50                  55                  60
   agt gga tct gcg aca gat ttc act ctc acc atc agc agc ctg cag cct       240
   Ser Gly Ser Ala Thr Asp Phe Thr Leu Thr Ile Ser Ser Leu Gln Pro
   65                  70                  75                  80
   gaa gat gtt gca act tat tac tgt caa agg tat aac agt gcc ccc ccg       288
   Glu Asp Val Ala Thr Tyr Tyr Cys Gln Arg Tyr Asn Ser Ala Pro Pro
                   85                  90                  95
   atc acc ttc ggc caa ggg aca cga ctg gag att aaa cgt gcg gcc gca       336
   Ile Thr Phe Gly Gln Gly Thr Arg Leu Glu Ile Lys Arg Ala Ala Ala
               100                 105                 110
   ccc agc gtg ttc atc ttc ccc ccc tcc gac gag cag ctg aag agc ggc       384
   Pro Ser Val Phe Ile Phe Pro Pro Ser Asp Glu Gln Leu Lys Ser Gly
           115                 120                 125
   acc gcc agc gtg gtg tgc ctg ctg aac aac ttc tac ccc cgg gag gcc       432
   Thr Ala Ser Val Val Cys Leu Leu Asn Asn Phe Tyr Pro Arg Glu Ala
       130                 135                 140
   aag gtg cag tgg aag gtg gac aac gcc ctg cag agc ggc aac agc cag       480
   Lys Val Gln Trp Lys Val Asp Asn Ala Leu Gln Ser Gly Asn Ser Gln
   145                 150                 155                 160
   gag agc gtg acc gag cag gac agc aag gac tcc acc tac agc ctg agc       528
   Glu Ser Val Thr Glu Gln Asp Ser Lys Asp Ser Thr Tyr Ser Leu Ser
                   165                 170                 175
   agc acc ctc acc ctg agc aag gcc gac tac gag aag cac aag gtg tac       576
   Ser Thr Leu Thr Leu Ser Lys Ala Asp Tyr Glu Lys His Lys Val Tyr
               180                 185                 190
   gcc tgc gag gtg acc cac cag ggc ctg agc agc ccc gtg acc aag agc       624
   Ala Cys Glu Val Thr His Gln Gly Leu Ser Ser Pro Val Thr Lys Ser
           195                 200                 205
   ttc aac cgg ggc gag tgt                                               642
   Phe Asn Arg Gly Glu Cys
       210
<210> 93
   <211> 214
   <212> PRT
   <213> Homo sapiens
<400> 93
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Val Pro Thr Leu Leu Ile
           35                  40                  45
   Tyr Asp Ala Ser Thr Leu Arg Ser Gly Val Pro Ser Arg Phe Ser Gly
       50                  55                  60
   Ser Gly Ser Ala Thr Asp Phe Thr Leu Thr Ile Ser Ser Leu Gln Pro
   65                  70                  75                  80
   Glu Asp Val Ala Thr Tyr Tyr Cys Gln Arg Tyr Asn Ser Ala Pro Pro
                   85                  90                  95
   Ile Thr Phe Gly Gln Gly Thr Arg Leu Glu Ile Lys Arg Ala Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 94
   <211> 648
   <212> DNA
   <213> Homo sapiens
<220>
   <221> CDS
   <222> (1)..(648)
<400> 94
   cag tct gtg ctg act cag cca ccc tca gag tcc gtg tcc cca gga cag         48
   Gln Ser Val Leu Thr Gln Pro Pro Ser Glu Ser Val Ser Pro Gly Gln
   1               5                   10                  15
   aca gcc agc gtc acc tgc tct gga cat aaa ttg ggg gat aaa tat gtt         96
   Thr Ala Ser Val Thr Cys Ser Gly His Lys Leu Gly Asp Lys Tyr Val
               20                  25                  30
   tcg tgg tat cag cag aag cca ggc cag tcc cct gta tta ctc atc tat        144
   Ser Trp Tyr Gln Gln Lys Pro Gly Gln Ser Pro Val Leu Leu Ile Tyr
           35                  40                  45
   caa gat aac agg cgg ccc tca ggg atc cct gag cga ttc ata ggc tcc        192
   Gln Asp Asn Arg Arg Pro Ser Gly Ile Pro Glu Arg Phe Ile Gly Ser
       50                  55                  60
   aac tct ggg aac aca gcc act ctg acc atc agc ggg acc cag gct ctg        240
   Asn Ser Gly Asn Thr Ala Thr Leu Thr Ile Ser Gly Thr Gln Ala Leu
   65                  70                  75                  80
   gat gag gct gac tat tac tgt cag gcg tgg gac agc agc act gcg gtt        288
   Asp Glu Ala Asp Tyr Tyr Cys Gln Ala Trp Asp Ser Ser Thr Ala Val
                   85                  90                  95
   ttc ggc gga ggg acc aag ctg acc gtc cta ggt gcg gcc gca ggc cag        336
   Phe Gly Gly Gly Thr Lys Leu Thr Val Leu Gly Ala Ala Ala Gly Gln
               100                 105                 110
   ccc aag gcc gct ccc agc gtg acc ctg ttc ccc ccc tcc tcc gag gag        384
   Pro Lys Ala Ala Pro Ser Val Thr Leu Phe Pro Pro Ser Ser Glu Glu
           115                 120                 125
   ctg cag gcc aac aag gcc acc ctg gtg tgc ctc atc agc gac ttc tac        432
   Leu Gln Ala Asn Lys Ala Thr Leu Val Cys Leu Ile Ser Asp Phe Tyr
       130                 135                 140
   cct ggc gcc gtg acc gtg gcc tgg aag gcc gac agc agc ccc gtg aag        480
   Pro Gly Ala Val Thr Val Ala Trp Lys Ala Asp Ser Ser Pro Val Lys
   145                 150                 155                 160
   gcc ggc gtg gag acc acc acc ccc agc aag cag agc aac aac aag tac        528
   Ala Gly Val Glu Thr Thr Thr Pro Ser Lys Gln Ser Asn Asn Lys Tyr
                   165                 170                 175
   gcc gcc agc agc tac ctg agc ctc acc ccc gag cag tgg aag agc cac        576
   Ala Ala Ser Ser Tyr Leu Ser Leu Thr Pro Glu Gln Trp Lys Ser His
               180                 185                 190
   cgg agc tac agc tgc cag gtg acc cac gag ggc agc acc gtg gag aag        624
   Arg Ser Tyr Ser Cys Gln Val Thr His Glu Gly Ser Thr Val Glu Lys
           195                 200                 205
   acc gtg gcc ccc acc gag tgc agc                                        648
   Thr Val Ala Pro Thr Glu Cys Ser
       210                 215
<210> 95
   <211> 216
   <212> PRT
   <213> Homo sapiens
<400> 95
   Gln Ser Val Leu Thr Gln Pro Pro Ser Glu Ser Val Ser Pro Gly Gln
   1               5                   10                  15
   Thr Ala Ser Val Thr Cys Ser Gly His Lys Leu Gly Asp Lys Tyr Val
               20                  25                  30
   Ser Trp Tyr Gln Gln Lys Pro Gly Gln Ser Pro Val Leu Leu Ile Tyr
           35                  40                  45
   Gln Asp Asn Arg Arg Pro Ser Gly Ile Pro Glu Arg Phe Ile Gly Ser
       50                  55                  60
   Asn Ser Gly Asn Thr Ala Thr Leu Thr Ile Ser Gly Thr Gln Ala Leu
   65                  70                  75                  80
   Asp Glu Ala Asp Tyr Tyr Cys Gln Ala Trp Asp Ser Ser Thr Ala Val
                   85                  90                  95
   Phe Gly Gly Gly Thr Lys Leu Thr Val Leu Gly Ala Ala Ala Gly Gln
               100                 105                 110
   Pro Lys Ala Ala Pro Ser Val Thr Leu Phe Pro Pro Ser Ser Glu Glu
           115                 120                 125
   Leu Gln Ala Asn Lys Ala Thr Leu Val Cys Leu Ile Ser Asp Phe Tyr
       130                 135                 140
   Pro Gly Ala Val Thr Val Ala Trp Lys Ala Asp Ser Ser Pro Val Lys
   145                 150                 155                 160
   Ala Gly Val Glu Thr Thr Thr Pro Ser Lys Gln Ser Asn Asn Lys Tyr
                   165                 170                 175
   Ala Ala Ser Ser Tyr Leu Ser Leu Thr Pro Glu Gln Trp Lys Ser His
               180                 185                 190
   Arg Ser Tyr Ser Cys Gln Val Thr His Glu Gly Ser Thr Val Glu Lys
           195                 200                 205
   Thr Val Ala Pro Thr Glu Cys Ser
       210                 215
<210> 96
   <211> 639
   <212> DNA
   <213> Homo sapiens
<220>
   <221> CDS
   <222> (1)..(639)
<400> 96
   gaa att gtg ctg act cag tct cca ggc acc ctg tct ttg tct cca ggg      48
   Glu Ile Val Leu Thr Gln Ser Pro Gly Thr Leu Ser Leu Ser Pro Gly
   1               5                   10                  15
   gaa aga gcc acc ctc tcc tgc agg gcc agt cag cgt gtt agc agc tac      96
   Glu Arg Ala Thr Leu Ser Cys Arg Ala Ser Gln Arg Val Ser Ser Tyr
               20                  25                  30
   tta gcc tgg tac caa cag aaa cct ggc cag gct ccc agg ctc ctc atc     144
   Leu Ala Trp Tyr Gln Gln Lys Pro Gly Gln Ala Pro Arg Leu Leu Ile
           35                  40                  45
   tat ggt gca tcc acc agg gcc gct ggc atc cca gac agg ttc agt ggc     192
   Tyr Gly Ala Ser Thr Arg Ala Ala Gly Ile Pro Asp Arg Phe Ser Gly
       50                  55                  60
   agt ggg tct ggg aca gac ttc act ctc acc atc agc aga ctg gag cct     240
   Ser Gly Ser Gly Thr Asp Phe Thr Leu Thr Ile Ser Arg Leu Glu Pro
   65                  70                  75                  80
   gaa gat tct gca gtg tat tac tgt cag cag tat ggt agg aca ccg ctc     288
   Glu Asp Ser Ala Val Tyr Tyr Cys Gln Gln Tyr Gly Arg Thr Pro Leu
                   85                  90                  95
   act ttc ggc gga ggg acc aag gtg gag atc aaa cgt gcg gcc gca ccc     336
   Thr Phe Gly Gly Gly Thr Lys Val Glu Ile Lys Arg Ala Ala Ala Pro
               100                 105                 110
   agc gtg ttc atc ttc ccc ccc tcc gac gag cag ctg aag agc ggc acc     384
   Ser Val Phe Ile Phe Pro Pro Ser Asp Glu Gln Leu Lys Ser Gly Thr
           115                 120                 125
   gcc agc gtg gtg tgc ctg ctg aac aac ttc tac ccc cgg gag gcc aag     432
   Ala Ser Val Val Cys Leu Leu Asn Asn Phe Tyr Pro Arg Glu Ala Lys
       130                 135                 140
   gtg cag tgg aag gtg gac aac gcc ctg cag agc ggc aac agc cag gag     480
   Val Gln Trp Lys Val Asp Asn Ala Leu Gln Ser Gly Asn Ser Gln Glu
   145                 150                 155                 160
   agc gtg acc gag cag gac agc aag gac tcc acc tac agc ctg agc agc     528
   Ser Val Thr Glu Gln Asp Ser Lys Asp Ser Thr Tyr Ser Leu Ser Ser
                   165                 170                 175
   acc ctc acc ctg agc aag gcc gac tac gag aag cac aag gtg tac gcc     576
   Thr Leu Thr Leu Ser Lys Ala Asp Tyr Glu Lys His Lys Val Tyr Ala
               180                 185                 190
   tgc gag gtg acc cac cag ggc ctg agc agc ccc gtg acc aag agc ttc     624
   Cys Glu Val Thr His Gln Gly Leu Ser Ser Pro Val Thr Lys Ser Phe
           195                 200                 205
   aac cgg ggc gag tgt                                                 639
   Asn Arg Gly Glu Cys
       210
<210> 97
   <211> 213
   <212> PRT
   <213> Homo sapiens
<400> 97
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Arg Val Ser Ser Tyr
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Ala Gly Ile Pro Asp Arg Phe Ser Gly
       50                  55                  60
   Ser Gly Ser Gly Thr Asp Phe Thr Leu Thr Ile Ser Arg Leu Glu Pro
   65                  70                  75                  80
   Glu Asp Ser Ala Val Tyr Tyr Cys Gln Gln Tyr Gly Arg Thr Pro Leu
                   85                  90                  95
   Thr Phe Gly Gly Gly Thr Lys Val Glu Ile Lys Arg Ala Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
       210
<210> 98
   <211> 654
   <212> DNA
   <213> Homo sapiens
<220>
   <221> CDS
   <222> (1)..(654)
<400> 98
   tcc tat gtg ctg act cag cca ccc tcg gtg tca gtg gcc cca gga cag        48
   Ser Tyr Val Leu Thr Gln Pro Pro Ser Val Ser Val Ala Pro Gly Gln
   1               5                   10                  15
   acg gcc agg att acc tgt ggg gga aac aac att gga agt aaa agt gtg        96
   Thr Ala Arg Ile Thr Cys Gly Gly Asn Asn Ile Gly Ser Lys Ser Val
               20                  25                  30
   cac tgg tac cag cag aag cca ggc cag gcc cct gtg ctg gtc gtc tat       144
   His Trp Tyr Gln Gln Lys Pro Gly Gln Ala Pro Val Leu Val Val Tyr
           35                  40                  45
   gat gat agc gac cgg ccc tca ggg atc cct gag cga ttc tct ggc tcc       192
   Asp Asp Ser Asp Arg Pro Ser Gly Ile Pro Glu Arg Phe Ser Gly Ser
       50                  55                  60
   aac tct ggg aac acg gcc acc ctg acc atc agc agg gtc gaa gcc ggg       240
   Asn Ser Gly Asn Thr Ala Thr Leu Thr Ile Ser Arg Val Glu Ala Gly
   65                  70                  75                  80
   gat gag gcc gac tat tac tgt cag gtg tgg gat agt agt agt gat cat       288
   Asp Glu Ala Asp Tyr Tyr Cys Gln Val Trp Asp Ser Ser Ser Asp His
                   85                  90                  95
   gct gtg ttc gga gga ggc acc cag ctg acc gtc ctc ggt gcg gcc gca       336
   Ala Val Phe Gly Gly Gly Thr Gln Leu Thr Val Leu Gly Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 99
   <211> 218
   <212> PRT
   <213> Homo sapiens
<400> 99
   Ser Tyr Val Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Ala Val Phe Gly Gly Gly Thr Gln Leu Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 100
   <211> 660
   <212> DNA
   <213> Homo sapiens
<220>
   <221> CDS
   <222> (1)..(660)
<400> 100
   tcc tat gtg ctg act cag cca ccc tca gcg tct ggg acc ccc ggg cag       48
   Ser Tyr Val Leu Thr Gln Pro Pro Ser Ala Ser Gly Thr Pro Gly Gln
   1               5                   10                  15
   agg gtc acc atc tct tgt tct gga agc agc tcc aac atc gga agt aat       96
   Arg Val Thr Ile Ser Cys Ser Gly Ser Ser Ser Asn Ile Gly Ser Asn
               20                  25                  30
   tat gta tac tgg tac cag cag ctc cca ggc acg gcc ccc aaa ctc ctc      144
   Tyr Val Tyr Trp Tyr Gln Gln Leu Pro Gly Thr Ala Pro Lys Leu Leu
           35                  40                  45
   atc tat agg gat ggt cag cgg ccc tca ggg gtc cct gac cga ttc tct      192
   Ile Tyr Arg Asp Gly Gln Arg Pro Ser Gly Val Pro Asp Arg Phe Ser
       50                  55                  60
   ggc tcc aag tct ggc acc tca gcc tcc ctg gcc atc agt gga ctc cgg      240
   Gly Ser Lys Ser Gly Thr Ser Ala Ser Leu Ala Ile Ser Gly Leu Arg
   65                  70                  75                  80
   tcc gat gat gag gct gat tat tac tgt gca aca tgg gat gac aac ctg      288
   Ser Asp Asp Glu Ala Asp Tyr Tyr Cys Ala Thr Trp Asp Asp Asn Leu
                   85                  90                  95
   agt ggt cca gta ttc ggc gga ggg acc aag ctg acc gtc cta ggt gcg      336
   Ser Gly Pro Val Phe Gly Gly Gly Thr Lys Leu Thr Val Leu Gly Ala
               100                 105                 110
   gcc gca ggc cag ccc aag gcc gct ccc agc gtg acc ctg ttc ccc ccc      384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc      432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc      480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc      528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag      576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc      624
   Trp Lys Ser His Arg Ser Tyr Ser Cys Gln Val Thr His Glu Gly Ser
           195                 200                 205
   acc gtg gag aag acc gtg gcc ccc acc gag tgc agc                      660
   Thr Val Glu Lys Thr Val Ala Pro Thr Glu Cys Ser
       210                 215                 220
<210> 101
   <211> 220
   <212> PRT
   <213> Homo sapiens
<400> 101
   Ser Tyr Val Leu Thr Gln Pro Pro Ser Ala Ser Gly Thr Pro Gly Gln
   1               5                   10                  15
   Arg Val Thr Ile Ser Cys Ser Gly Ser Ser Ser Asn Ile Gly Ser Asn
               20                  25                  30
   Tyr Val Tyr Trp Tyr Gln Gln Leu Pro Gly Thr Ala Pro Lys Leu Leu
           35                  40                  45
   Ile Tyr Arg Asp Gly Gln Arg Pro Ser Gly Val Pro Asp Arg Phe Ser
       50                  55                  60
   Gly Ser Lys Ser Gly Thr Ser Ala Ser Leu Ala Ile Ser Gly Leu Arg
   65                  70                  75                  80
   Ser Asp Asp Glu Ala Asp Tyr Tyr Cys Ala Thr Trp Asp Asp Asn Leu
                   85                  90                  95
   Ser Gly Pro Val Phe Gly Gly Gly Thr Lys Leu Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 102
   <211> 642
   <212> DNA
   <213> Homo sapiens
<220>
   <221> CDS
   <222> (1)..(642)
<400> 102
   gaa att gtg ttg acc cag tct cca ggc acc ctg tct ttg tct cca ggg      48
   Glu Ile Val Leu Thr Gln Ser Pro Gly Thr Leu Ser Leu Ser Pro Gly
   1               5                   10                  15
   gaa aga gcc acc ctc tcc tgc agg gcc agt cag agt gtt agc agc agc      96
   Glu Arg Ala Thr Leu Ser Cys Arg Ala Ser Gln Ser Val Ser Ser Ser
               20                  25                  30
   tac tta gcc tgg tac cag cag aaa cct ggc cag gct ccc agg ctc ctc     144
   Tyr Leu Ala Trp Tyr Gln Gln Lys Pro Gly Gln Ala Pro Arg Leu Leu
           35                  40                  45
   atc tat ggt gca tcc agc agg gcc act ggc atc cca gac agg ttc agt     192
   Ile Tyr Gly Ala Ser Ser Arg Ala Thr Gly Ile Pro Asp Arg Phe Ser
       50                  55                  60
   ggc agt ggg tct ggg aca gac ttc act ctc acc atc agc aga ctg gag     240
   Gly Ser Gly Ser Gly Thr Asp Phe Thr Leu Thr Ile Ser Arg Leu Glu
   65                  70                  75                  80
   cct gaa gat ttt gca gtg tat tac tgt cag cag tat ggt agc tca ccc     288
   Pro Glu Asp Phe Ala Val Tyr Tyr Cys Gln Gln Tyr Gly Ser Ser Pro
                   85                  90                  95
   aga act ttc ggc gga ggg acc aag gtg gag atc aaa cgt gcg gcc gca     336
   Arg Thr Phe Gly Gly Gly Thr Lys Val Glu Ile Lys Arg Ala Ala Ala
               100                 105                 110
   ccc agc gtg ttc atc ttc ccc ccc tcc gac gag cag ctg aag agc ggc     384
   Pro Ser Val Phe Ile Phe Pro Pro Ser Asp Glu Gln Leu Lys Ser Gly
           115                 120                 125
   acc gcc agc gtg gtg tgc ctg ctg aac aac ttc tac ccc cgg gag gcc     432
   Thr Ala Ser Val Val Cys Leu Leu Asn Asn Phe Tyr Pro Arg Glu Ala
       130                 135                 140
   aag gtg cag tgg aag gtg gac aac gcc ctg cag agc ggc aac agc cag     480
   Lys Val Gln Trp Lys Val Asp Asn Ala Leu Gln Ser Gly Asn Ser Gln
   145                 150                 155                 160
   gag agc gtg acc gag cag gac agc aag gac tcc acc tac agc ctg agc     528
   Glu Ser Val Thr Glu Gln Asp Ser Lys Asp Ser Thr Tyr Ser Leu Ser
                   165                 170                 175
   agc acc ctc acc ctg agc aag gcc gac tac gag aag cac aag gtg tac     576
   Ser Thr Leu Thr Leu Ser Lys Ala Asp Tyr Glu Lys His Lys Val Tyr
               180                 185                 190
   gcc tgc gag gtg acc cac cag ggc ctg agc agc ccc gtg acc aag agc     624
   Ala Cys Glu Val Thr His Gln Gly Leu Ser Ser Pro Val Thr Lys Ser
           195                 200                 205
   ttc aac cgg ggc gag tgt                                             642
   Phe Asn Arg Gly Glu Cys
   210
<210> 103
   <211> 214
   <212> PRT
   <213> Homo sapiens
<400> 103
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Arg Thr Phe Gly Gly Gly Thr Lys Val Glu Ile Lys Arg Ala Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 104
   <211> 660
   <212> DNA
   <213> Homo sapiens
<220>
   <221> CDS
   <222> (1)..(660)
<400> 104
   cag tct gcc ctg act cag cct gcc tcc gtg tct ggg tct cct gga cag        48
   Gln Ser Ala Leu Thr Gln Pro Ala Ser Val Ser Gly Ser Pro Gly Gln
   1               5                   10                  15
   tcg atc acc atc tcc tgc act gga acc agc agt gac gtt ggt ggt tat        96
   Ser Ile Thr Ile Ser Cys Thr Gly Thr Ser Ser Asp Val Gly Gly Tyr
               20                  25                  30
   aac tat gtc tcc tgg tac caa cag cac cca ggc aaa gcc ccc aaa ctc       144
   Asn Tyr Val Ser Trp Tyr Gln Gln His Pro Gly Lys Ala Pro Lys Leu
           35                  40                  45
   atg att tat gag gtc agt aat cgg ccc tca ggg gtt tct aat cgc ttc       192
   Met Ile Tyr Glu Val Ser Asn Arg Pro Ser Gly Val Ser Asn Arg Phe
       50                  55                  60
   tct ggc tcc aag tct ggc aac acg gcc tcc ctg acc atc tct ggg ctc       240
   Ser Gly Ser Lys Ser Gly Asn Thr Ala Ser Leu Thr Ile Ser Gly Leu
   65                  70                  75                  80
   cag gct gag gac gag gct gat tat tac tgc agc tca tat aca agc agc       288
   Gln Ala Glu Asp Glu Ala Asp Tyr Tyr Cys Ser Ser Tyr Thr Ser Ser
                   85                  90                  95
   agc act ctt gtc ttc gga act ggg acc aag gtc acc gtc cta ggt gcg       336
   Ser Thr Leu Val Phe Gly Thr Gly Thr Lys Val Thr Val Leu Gly Ala
               100                 105                 110
   gcc gca ggc cag ccc aag gcc gct ccc agc gtg acc ctg ttc ccc ccc       384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc       432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc       480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc       528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag       576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc       624
   Trp Lys Ser His Arg Ser Tyr Ser Cys Gln Val Thr His Glu Gly Ser
           195                 200                 205
   acc gtg gag aag acc gtg gcc ccc acc gag tgc agc                       660
                   Thr Val Glu Lys Thr Val Ala Pro Thr Glu Cys Ser
                       210                 215                 220
<210> 105
   <211> 220
   <212> PRT
   <213> Homo sapiens
<400> 105
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Glu Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr Leu Val Phe Gly Thr Gly Thr Lys Val Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 106
   <211> 654
   <212> DNA
   <213> Homo sapiens
<220>
   <221> CDS
   <222> (1)..(654)
<400> 106
   cag tct gtc gtg acg cag ccg ccc tcg gtg tca gtg gcc cca gga cag        48
   Gln Ser Val Val Thr Gln Pro Pro Ser Val Ser Val Ala Pro Gly Gln
   1               5                   10                  15
   acg gcc agg att acc tgt ggg gga aac aac att gga agt aaa agt gtg        96
   Thr Ala Arg Ile Thr Cys Gly Gly Asn Asn Ile Gly Ser Lys Ser Val
               20                  25                  30
   cac tgg tac cag cag aag cca ggc cag gcc cct gtg ctg gtc gtc tat       144
   His Trp Tyr Gln Gln Lys Pro Gly Gln Ala Pro Val Leu Val Val Tyr
           35                  40                  45
   gat gat agc gac cgg ccc tca ggg atc cct gag cga ttc tct ggc tcc       192
   Asp Asp Ser Asp Arg Pro Ser Gly Ile Pro Glu Arg Phe Ser Gly Ser
       50                  55                  60
   aac tct ggg aac acg gcc acc ctg acc atc agc agg gtc gaa gcc ggg       240
   Asn Ser Gly Asn Thr Ala Thr Leu Thr Ile Ser Arg Val Glu Ala Gly
   65                  70                  75                  80
   gat gag gcc gac tat tac tgt cag gtg tgg gat agt agt agt gat cat       288
   Asp Glu Ala Asp Tyr Tyr Cys Gln Val Trp Asp Ser Ser Ser Asp His
                   85                  90                  95
   tat gtc ttc gga act ggg acc aag gtc acc gtc cta ggt gcg gcc gca       336
   Tyr Val Phe Gly Thr Gly Thr Lys Val Thr Val Leu Gly Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 107
   <211> 218
   <212> PRT
   <213> Homo sapiens
<400> 107
   Gln Ser Val Val Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Tyr Val Phe Gly Thr Gly Thr Lys Val Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
   210 215
<210> 108
   <211> 660
   <212> DNA
   <213> Homo sapiens
<220>
   <221> CDS
   <222> (1)..(660)
<400> 108
   act gtg ttg aca cag ccg ccc tca gtg tct ggg gcc cca ggg cag agg      48
   Thr Val Leu Thr Gln Pro Pro Ser Val Ser Gly Ala Pro Gly Gln Arg
   1               5                   10                  15
   gtc acc atc tcc tgc act ggg agc agc tcc aac atc ggg gca ggt tat      96
   Val Thr Ile Ser Cys Thr Gly Ser Ser Ser Asn Ile Gly Ala Gly Tyr
               20                  25                  30
   gat gta cac tgg tac cag cag ctt cca gga aca gcc ccc aaa ctc ctc     144
   Asp Val His Trp Tyr Gln Gln Leu Pro Gly Thr Ala Pro Lys Leu Leu
           35                  40                  45
   atc tat ggt aac agc aat cgg ccc tca ggg gtc cct gac cga ttc tct     192
   Ile Tyr Gly Asn Ser Asn Arg Pro Ser Gly Val Pro Asp Arg Phe Ser
       50                  55                  60
   ggc tcc aag tct ggc acg tca gcc acc ctg ggc atc acc gga ctc cag     240
   Gly Ser Lys Ser Gly Thr Ser Ala Thr Leu Gly Ile Thr Gly Leu Gln
   65                  70                  75                  80
   act ggg gac gag gcc gat tat tac tgc gga aca tgg gat agc agc ctg     288
   Thr Gly Asp Glu Ala Asp Tyr Tyr Cys Gly Thr Trp Asp Ser Ser Leu
                   85                  90                  95
   agt gct tat gtc ttc gga act ggg acc aag gtc acc gtc cta ggt gcg     336
   Ser Ala Tyr Val Phe Gly Thr Gly Thr Lys Val Thr Val Leu Gly Ala
               100                 105                 110
   gcc gca ggc cag ccc aag gcc gct ccc agc gtg acc ctg ttc ccc ccc     384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc     432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc     480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc     528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag     576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc     624
   Trp Lys Ser His Arg Ser Tyr Ser Cys Gln Val Thr His Glu Gly Ser
           195                 200                 205
   acc gtg gag aag acc gtg gcc ccc acc gag tgc agc                     660
   Thr Val Glu Lys Thr Val Ala Pro Thr Glu Cys Ser
       210                 215                 220
<210> 109
   <211> 220
   <212> PRT
   <213> Homo sapiens
<400> 109
   Thr Val Leu Thr Gln Pro Pro Ser Val Ser Gly Ala Pro Gly Gln Arg
   1               5                   10                  15
   Val Thr Ile Ser Cys Thr Gly Ser Ser Ser Asn Ile Gly Ala Gly Tyr
               20                  25                  30
   Asp Val His Trp Tyr Gln Gln Leu Pro Gly Thr Ala Pro Lys Leu Leu
           35                  40                  45
   Ile Tyr Gly Asn Ser Asn Arg Pro Ser Gly Val Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Asp Tyr Tyr Cys Gly Thr Trp Asp Ser Ser Leu
                   85                  90                  95
   Ser Ala Tyr Val Phe Gly Thr Gly Thr Lys Val Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 110
   <211> 565
   <212> PRT
   <213> Influenza A virus
<220>
   <221> MISC_FEATURE
   <222> (1)..(565)
   <223> HA protein of influenza virus H5N1 isolate A/Vietnam/1203/2004
<400> 110
   Met Glu Lys Ile Val Leu Leu Phe Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Lys His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Val Asn Asp Leu Cys Tyr Pro Gly Asp Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Ser His Glu Ala Ser
       130                 135                 140
   Leu Gly Val Ser Ser Ala Cys Pro Tyr Gln Gly Lys Ser Ser Phe Phe
   145                 150                 155                 160
   Arg Asn Val Val Trp Leu Ile Lys Lys Asn Ser Thr Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Arg Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
           450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Ile Tyr Gln Ile Leu Ser Ile Tyr Ser Thr Val Ala Ser Ser Leu Ala
       530                 535                 540
   Leu Ala Ile Met Val Ala Gly Leu Ser Leu Trp Met Cys Ser Asn Gly
   545                 550                 555                 560
   Ser Leu Gln Cys Arg
                   565
<210> 111
   <211> 565
   <212> PRT
   <213> Influenza A virus
<220>
   <221> MISC_FEATURE
   <223> HA protein of influenza virus H5N1 isolate A/Vietnam/1194/2004 (H5N1TV)
<400> 111
   Met Glu Lys Ile Val Leu Leu Phe Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
   85 90 95
   Glu Lys Ala Asn Pro Val Asn Asp Leu Cys Tyr Pro Gly Asp Phe Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Ser His Glu Ala Ser
   130 135 140
   Leu Gly Val Ser Ser Ala Cys Pro Tyr Gln Gly Lys Ser Ser Phe Phe
   145 150 155 160
   Arg Asn Val Val Trp Leu Ile Lys Lys Asn Ser Thr Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Lys Leu Tyr Gln
   195 200 205
   Asn Pro Thr Thr Tyr Ile Ser Val Gly Thr Ser Thr Leu Asn Gln Arg
   210 215 220
   Leu Val Pro Arg Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Ile Tyr Gln Ile Leu Ser Ile Tyr Ser Thr Val Ala Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg
   565
<210> 112
   <211> 565
   <212> PRT
   <213> Influenza A virus
<220>
   <221> MISC_FEATURE
   <223> Consensus amino acid sequence representing HAs from strains isolated in Indonesia and China (H5N1IC)
<400> 112
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Ala Asn Asp Leu Cys Tyr Pro Gly Asn Phe Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Gln Gly Lys Ser Ser Phe Phe
   145 150 155 160
   Arg Asn Val Val Trp Leu Ile Lys Lys Asn Ser Ser Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Arg Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Ile Tyr Gln Ile Leu Ser Ile Tyr Ser Thr Val Ala Ser Ser Leu Ala
       530                 535                 540
   Leu Ala Ile Met Val Ala Gly Leu Ser Leu Trp Met Cys Ser Asn Gly
   545                 550                 555                 560
   Ser Leu Gln Cys Arg
                   565
<210> 113
   <211> 567
   <212> PRT
   <213> Influenza A virus
<220>
   <221> MISC_FEATURE
   <223> Soluble part of HA from H5N1TV
<220>
   <221> MISC_FEATURE
   <222> (533)..(546)
   <223> Spacer
<220>
   <221> MISC_FEATURE
   <222> (547)..(556)
   <223> Myc-tag
<220>
   <221> MISC_FEATURE
   <222> (557)..(561)
   <223> Spacer
<220>
   <221> MISC_FEATURE
   <222> (562)..(567)
   <223> His-tag
<400> 113
   Met Glu Lys Ile Val Leu Leu Phe Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Val Asn Asp Leu Cys Tyr Pro Gly Asp Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Ser His Glu Ala Ser
       130                 135                 140
   Leu Gly Val Ser Ser Ala Cys Pro Tyr Gln Gly Lys Ser Ser Phe Phe
   145                 150                 155                 160
   Arg Asn Val Val Trp Leu Ile Lys Lys Asn Ser Thr Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Lys Leu Tyr Gln
           195                 200                 205
   Asn Pro Thr Thr Tyr Ile Ser Val Gly Thr Ser Thr Leu Asn Gln Arg
       210                 215                 220
   Leu Val Pro Arg Ile Ala Thr Arg Ser Lys Val Asn Gly Gln Ser Gly
   225                 230                 235                 240
   Arg Met Glu Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Arg Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Ile Tyr Gln Ile Gln His Ser Gly Gly Arg Ser Ser Leu Glu Gly Pro
       530                 535                 540
   Arg Phe Glu Gln Lys Leu Ile Ser Glu Glu Asp Leu Asn Met His Thr
   545                 550                 555                 560
   Gly His His His His His His
                   565
<210> 114
   <211> 567
   <212> PRT
   <213> Influenza A virus
<220>
   <221> MISC_FEATURE
   <223> Soluble part of HA from H5N1IC
<220>
   <221> MISC_FEATURE
   <222> (533)..(546)
   <223> Spacer
<220>
   <221> MISC_FEATURE
   <222> (547)..(556)
   <223> Myc-tag
<220>
   <221> MISC_FEATURE
   <222> (557)..(561)
   <223> Spacer
<220>
   <221> MISC_FEATURE
   <222> (562)..(567)
   <223> His-tag
<400> 114
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Ser Gly Val Ser Ser Ala Cys Pro Tyr Gln Gly Lys Ser Ser Phe Phe
   145                 150                 155                 160
   Arg Asn Val Val Trp Leu Ile Lys Lys Asn Ser Ser Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Met Glu Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Leu Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Arg Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Arg Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Ile Tyr Gln Ile Gln His Ser Gly Gly Arg Ser Ser Leu Glu Gly Pro
   530 535 540
   Arg Phe Glu Gln Lys Leu Ile Ser Glu Glu Asp Leu Asn Met His Thr
   545 550 555 560
   Gly His His His His His His
   565
<210> 115
   <211> 744
   <212> DNA
   <213> Artificial
<220>
   <223> SC06-255
<220>
   <221> CDS
   <222> (1)..(744)
<400> 115
   gag gtg cag ctg gtg gag tct ggg gct gag gtg aag aag cct ggg tcc        48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat        96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg       144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc       192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac       240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt       288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc       336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga       384
   Lys Gly Thr Thr Val Thr Val Ser Ser Gly Thr Gly Gly Ser Gly Gly
           115                 120                 125
   acc ggc agc ggc act ggc ggg tcg acg tcc tat gtg ctg act cag cca       432
   Thr Gly Ser Gly Thr Gly Gly Ser Thr Ser Tyr Val Leu Thr Gln Pro
       130                 135                 140
   ccc tca gcg tct ggg acc ccc ggg cag agg gtc acc atc tct tgt tct       480
   Pro Ser Ala Ser Gly Thr Pro Gly Gln Arg Val Thr Ile Ser Cys Ser
   145                 150                 155                 160
   gga agc acg ttc aac atc gga agt aat gct gta gac tgg tac cgg cag       528
   Gly Ser Thr Phe Asn Ile Gly Ser Asn Ala Val Asp Trp Tyr Arg Gln
                   165                 170                 175
   ctc cca gga acg gcc ccc aaa ctc ctc atc tat agt aat aat cag cgg       576
   Leu Pro Gly Thr Ala Pro Lys Leu Leu Ile Tyr Ser Asn Asn Gln Arg
               180                 185                 190
   ccc tca ggg gtc cct gac cga ttc tct ggc tcc agg tct ggc acc tca       624
   Pro Ser Gly Val Pro Asp Arg Phe Ser Gly Ser Arg Ser Gly Thr Ser
           195                 200                 205
   gcc tcc ctg gcc atc agt ggg ctc cag tct gag gat gag gct gat tat       672
   Ala Ser Leu Ala Ile Ser Gly Leu Gln Ser Glu Asp Glu Ala Asp Tyr
       210                 215                 220
   tac tgt gca gca tgg gat gac atc ctg aat gtt ccg gta ttc ggc gga       720
   Tyr Cys Ala Ala Trp Asp Asp Ile Leu Asn Val Pro Val Phe Gly Gly
   225                 230                 235                 240
   ggg acc aag ctg acc gtc cta ggt                                       744
   Gly Thr Lys Leu Thr Val Leu Gly
                   245
<210> 116
   <211> 248
   <212> PRT
   <213> Artificial
<220>
   <223> Synthetic Construct
<400> 116
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Gly Thr Gly Gly Ser Gly Gly
           115                 120                 125
   Thr Gly Ser Gly Thr Gly Gly Ser Thr Ser Tyr Val Leu Thr Gln Pro
       130                 135                 140
   Pro Ser Ala Ser Gly Thr Pro Gly Gln Arg Val Thr Ile Ser Cys Ser
   145                 150                 155                 160
   Gly Ser Thr Phe Asn Ile Gly Ser Asn Ala Val Asp Trp Tyr Arg Gln
                   165                 170                 175
   Leu Pro Gly Thr Ala Pro Lys Leu Leu Ile Tyr Ser Asn Asn Gln Arg
               180                 185                 190
   Pro Ser Gly Val Pro Asp Arg Phe Ser Gly Ser Arg Ser Gly Thr Ser
           195                 200                 205
   Ala Ser Leu Ala Ile Ser Gly Leu Gln Ser Glu Asp Glu Ala Asp Tyr
       210                 215                 220
   Tyr Cys Ala Ala Trp Asp Asp Ile Leu Asn Val Pro Val Phe Gly Gly
   225                 230                 235                 240
   Gly Thr Lys Leu Thr Val Leu Gly
                   245
<210> 117
   <211> 744
   <212> DNA
   <213> Artificial
<220>
   <223> SC06-257
<220>
   <221> CDS
   <222> (1)..(744)
<400> 117
   cag gtc cag ctg gtg cag tct ggg gct gag gtg aag aag cct ggg tcc      48
   Gln Val Gln Leu Val Gln Ser Gly Ala Glu Val Lys Lys Pro Gly Ser
   1               5                   10                  15
   tcg gtg aaa gtc tct tgc aag gct tct gga ggc ccc ttc cgc agc tat      96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg     144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc     192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac     240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt     288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc     336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga     384
   Lys Gly Thr Thr Val Thr Val Ser Ser Gly Thr Gly Gly Ser Gly Gly
           115                 120                 125
   acc ggc agc ggc act ggc ggg tcg acg cag tct gcc ctg act cag cct     432
   Thr Gly Ser Gly Thr Gly Gly Ser Thr Gln Ser Ala Leu Thr Gln Pro
       130                 135                 140
   gcc gcc gtg tct ggg tct cct gga cag tcg atc acc atc tcc tgc act     480
   Ala Ala Val Ser Gly Ser Pro Gly Gln Ser Ile Thr Ile Ser Cys Thr
   145                 150                 155                 160
   gga acc agc agt gac gtt ggt ggt tat aac tat gtc tcc tgg tac caa     528
   Gly Thr Ser Ser Asp Val Gly Gly Tyr Asn Tyr Val Ser Trp Tyr Gln
                   165                 170                 175
   cag cac cca ggc aaa gcc ccc aaa ctc atg att tat gag gtc agt aat     576
   Gln His Pro Gly Lys Ala Pro Lys Leu Met Ile Tyr Glu Val Ser Asn
               180                 185                 190
   cgg ccc tca ggg gtt tct aat cgc ttc tct ggc tcc aag tct ggc aac        624
   Arg Pro Ser Gly Val Ser Asn Arg Phe Ser Gly Ser Lys Ser Gly Asn
           195                 200                 205
   acg gcc tcc ctg acc atc tct ggg ctc cag gct gag gac gag gct gat        672
   Thr Ala Ser Leu Thr Ile Ser Gly Leu Gln Ala Glu Asp Glu Ala Asp
       210                 215                 220
   tat tac tgc agc tca tat aca agc agc agc act tat gtc ttc gga act        720
   Tyr Tyr Cys Ser Ser Tyr Thr Ser Ser Ser Thr Tyr Val Phe Gly Thr
   225                 230                 235                 240
   ggg acc aag gtc acc gtc cta ggt                                        744
   Gly Thr Lys Val Thr Val Leu Gly
                   245
<210> 118
   <211> 248
   <212> PRT
   <213> Artificial
<220>
   <223> Synthetic Construct
<400> 118
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Gly Thr Gly Gly Ser Gly Gly
           115                 120                 125
   Thr Gly Ser Gly Thr Gly Gly Ser Thr Gln Ser Ala Leu Thr Gln Pro
       130                 135                 140
   Ala Ala Val Ser Gly Ser Pro Gly Gln Ser Ile Thr Ile Ser Cys Thr
   145                 150                 155                 160
   Gly Thr Ser Ser Asp Val Gly Gly Tyr Asn Tyr Val Ser Trp Tyr Gln
                   165                 170                 175
   Gln His Pro Gly Lys Ala Pro Lys Leu Met Ile Tyr Glu Val Ser Asn
               180                 185                 190
   Arg Pro Ser Gly Val Ser Asn Arg Phe Ser Gly Ser Lys Ser Gly Asn
           195                 200                 205
   Thr Ala Ser Leu Thr Ile Ser Gly Leu Gln Ala Glu Asp Glu Ala Asp
       210                 215                 220
   Tyr Tyr Cys Ser Ser Tyr Thr Ser Ser Ser Thr Tyr Val Phe Gly Thr
   225                 230                 235                 240
   Gly Thr Lys Val Thr Val Leu Gly
                   245
<210> 119
   <211> 744
   <212> DNA
   <213> Artificial
<220>
   <223> SC06-260
<220>
   <221> CDS
   <222> (1)..(744)
<400> 119
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga      384
   Lys Gly Thr Thr Val Thr Val Ser Ser Gly Thr Gly Gly Ser Gly Gly
           115                 120                 125
   acc ggc agc ggc act ggc ggg tcg acg tcc tat gtg ctg act cag cca      432
   Thr Gly Ser Gly Thr Gly Gly Ser Thr Ser Tyr Val Leu Thr Gln Pro
       130                 135                 140
   ccc tca gtc tct ggg acc ccc ggg cag agg gtc acc atc tct tgc tct      480
   Pro Ser Val Ser Gly Thr Pro Gly Gln Arg Val Thr Ile Ser Cys Ser
   145                 150                 155                 160
   gga agc cgc tcc aac gtc gga gat aat tct gta tat tgg tat caa cac      528
   Gly Ser Arg Ser Asn Val Gly Asp Asn Ser Val Tyr Trp Tyr Gln His
                   165                 170                 175
   gtc cca gaa atg gcc ccc aaa ctc ctc gtc tat aag aat act caa cgg      576
   Val Pro Glu Met Ala Pro Lys Leu Leu Val Tyr Lys Asn Thr Gln Arg
               180                 185                 190
   ccc tca gga gtc cct gcc cgg ttt tcc ggc tcc aag tct ggc act tca      624
   Pro Ser Gly Val Pro Ala Arg Phe Ser Gly Ser Lys Ser Gly Thr Ser
           195                 200                 205
   gcc tcc ctg gcc atc att ggc ctc cag tcc ggc gat gag gct gat tat      672
   Ala Ser Leu Ala Ile Ile Gly Leu Gln Ser Gly Asp Glu Ala Asp Tyr
       210                 215                 220
   tat tgt gtg gca tgg gat gac agc gta gat ggc tat gtc ttc gga tct      720
   Tyr Cys Val Ala Trp Asp Asp Ser Val Asp Gly Tyr Val Phe Gly Ser
   225                 230                 235                 240
   ggg acc aag gtc acc gtc cta ggt                                      744
   Gly Thr Lys Val Thr Val Leu Gly
                   245
<210> 120
   <211> 248
   <212> PRT
   <213> Artificial
<220>
   <223> Synthetic Construct
<400> 120
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
   50 55 60
   Gln Gly Arg Val Thr Ile Thr Ala Asp Asp Phe Ala Gly Thr Val Tyr
   65 70 75 80
   Met Glu Leu Ser Ser Leu Arg Ser Glu Asp Thr Ala Met Tyr Tyr Cys
   85 90 95
   Ala Lys His Met Gly Tyr Gln Val Arg Glu Thr Met Asp Val Trp Gly
   100 105 110
   Lys Gly Thr Thr Val Thr Val Ser Ser Gly Thr Gly Gly Ser Gly Gly
   115 120 125
   Thr Gly Ser Gly Thr Gly Gly Ser Thr Ser Tyr Val Leu Thr Gln Pro
   130 135 140
   Pro Ser Val Ser Gly Thr Pro Gly Gln Arg Val Thr Ile Ser Cys Ser
   145 150 155 160
   Gly Ser Arg Ser Asn Val Gly Asp Asn Ser Val Tyr Trp Tyr Gln His
   165 170 175
   Val Pro Glu Met Ala Pro Lys Leu Leu Val Tyr Lys Asn Thr Gln Arg
   180 185 190
   Pro Ser Gly Val Pro Ala Arg Phe Ser Gly Ser Lys Ser Gly Thr Ser
   195 200 205
   Ala Ser Leu Ala Ile Ile Gly Leu Gln Ser Gly Asp Glu Ala Asp Tyr
   210 215 220
   Tyr Cys Val Ala Trp Asp Asp Ser Val Asp Gly Tyr Val Phe Gly Ser
   225 230 235 240
   Gly Thr Lys Val Thr Val Leu Gly
   245
<210> 121
   <211> 747
   <212> DNA
   <213> Artificial
<220>
   <223> SC06-261
<220>
   <221> CDS
   <222> (1)..(747)
<400> 121
   gag gtg cag ctg gtg gag tct ggg gct gag gtg aag aag cct ggg tcc      48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat      96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg     144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc     192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac     240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt     288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc     336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga     384
   Lys Gly Thr Thr Val Thr Val Ser Ser Gly Thr Gly Gly Ser Gly Gly
           115                 120                 125
   acc ggc agc ggc act ggc ggg tcg acg cag tct gtg ttg acg cag ccg     432
   Thr Gly Ser Gly Thr Gly Gly Ser Thr Gln Ser Val Leu Thr Gln Pro
       130                 135                 140
   ccc tca gtg tct gcg gcc cca gga cag aag gtc acc atc tcc tgc tct     480
   Pro Ser Val Ser Ala Ala Pro Gly Gln Lys Val Thr Ile Ser Cys Ser
   145                 150                 155                 160
   gga agc agc tcc aac att ggg aat gat tat gta tcc tgg tac cag cag     528
   Gly Ser Ser Ser Asn Ile Gly Asn Asp Tyr Val Ser Trp Tyr Gln Gln
                   165                 170                 175
   ctc cca gga aca gcc ccc aaa ctc ctc att tat gac aat aat aag cga     576
   Leu Pro Gly Thr Ala Pro Lys Leu Leu Ile Tyr Asp Asn Asn Lys Arg
               180                 185                 190
   ccc tca ggg att cct gac cga ttc tct ggc tcc aag tct ggc acg tca     624
   Pro Ser Gly Ile Pro Asp Arg Phe Ser Gly Ser Lys Ser Gly Thr Ser
           195                 200                 205
   gcc acc ctg ggc atc acc gga ctc cag act ggg gac gag gcc aac tat     672
   Ala Thr Leu Gly Ile Thr Gly Leu Gln Thr Gly Asp Glu Ala Asn Tyr
       210                 215                 220
   tac tgc gca aca tgg gat cgc cgc ccg act gct tat gtt gtc ttc ggc     720
   Tyr Cys Ala Thr Trp Asp Arg Arg Pro Thr Ala Tyr Val Val Phe Gly
   225                 230                 235                 240
   gga ggg acc aag ctg acc gtc cta ggt                                 747
   Gly Gly Thr Lys Leu Thr Val Leu Gly
                   245
<210> 122
   <211> 249
   <212> PRT
   <213> Artificial
<220>
   <223> Synthetic Construct
<400> 122
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
               20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Gly Thr Gly Gly Ser Gly Gly
           115                 120                 125
   Thr Gly Ser Gly Thr Gly Gly Ser Thr Gln Ser Val Leu Thr Gln Pro
       130                 135                 140
   Pro Ser Val Ser Ala Ala Pro Gly Gln Lys Val Thr Ile Ser Cys Ser
   145                 150                 155                 160
   Gly Ser Ser Ser Asn Ile Gly Asn Asp Tyr Val Ser Trp Tyr Gln Gln
                   165                 170                 175
   Leu Pro Gly Thr Ala Pro Lys Leu Leu Ile Tyr Asp Asn Asn Lys Arg
               180                 185                 190
   Pro Ser Gly Ile Pro Asp Arg Phe Ser Gly Ser Lys Ser Gly Thr Ser
           195                 200                 205
   Ala Thr Leu Gly Ile Thr Gly Leu Gln Thr Gly Asp Glu Ala Asn Tyr
       210                 215                 220
   Tyr Cys Ala Thr Trp Asp Arg Arg Pro Thr Ala Tyr Val Val Phe Gly
   225                 230                 235                 240
   Gly Gly Thr Lys Leu Thr Val Leu Gly
                   245
<210> 123
   <211> 735
   <212> DNA
   <213> Artificial
<220>
   <223> SC06-262
<220>
   <221> CDS
   <222> (1)..(735)
<400> 123
   cag gta cag ctg cag cag tca ggg gct gag gtg aag aag cct ggg tcc       48
   Gln Val Gln Leu Gln Gln Ser Gly Ala Glu Val Lys Lys Pro Gly Ser
   1               5                   10                  15
   tcg gtg aag gtc tcc tgc aag gtt tcc gga gtc att ttc agc ggc agt       96
   Ser Val Lys Val Ser Cys Lys Val Ser Gly Val Ile Phe Ser Gly Ser
               20                  25                  30
   gcg atc agc tgg gtg cga cag gcc cct gga caa ggc ctt gag tgg atg      144
   Ala Ile Ser Trp Val Arg Gln Ala Pro Gly Gln Gly Leu Glu Trp Met
           35                  40                  45
   gga ggg atc agc cct ctc ttt ggc aca aca aat tac gca caa aag ttc      192
   Gly Gly Ile Ser Pro Leu Phe Gly Thr Thr Asn Tyr Ala Gln Lys Phe
       50                  55                  60
   cag ggc aga gtc acg att acc gcg gac caa tcc acg aac aca acc tac      240
   Gln Gly Arg Val Thr Ile Thr Ala Asp Gln Ser Thr Asn Thr Thr Tyr
   65                  70                  75                  80
   atg gag gtg aac agc ctg aga tat gag gac acg gcc gtg tat ttc tgt      288
   Met Glu Val Asn Ser Leu Arg Tyr Glu Asp Thr Ala Val Tyr Phe Cys
                   85                  90                  95
   gcg cga ggt cca aaa tat tac agt gag tac atg gac gtc tgg ggc aaa      336
   Ala Arg Gly Pro Lys Tyr Tyr Ser Glu Tyr Met Asp Val Trp Gly Lys
               100                 105                 110
   ggg acc acg gtc acc gtc tcg agc ggt acg ggc ggt tca ggc gga acc      384
   Gly Thr Thr Val Thr Val Ser Ser Gly Thr Gly Gly Ser Gly Gly Thr
           115                 120                 125
   ggc agc ggc act ggc ggg tcg acg gac atc cag atg acc cag tct cca      432
   Gly Ser Gly Thr Gly Gly Ser Thr Asp Ile Gln Met Thr Gln Ser Pro
       130                 135                 140
   tcc tcc ctg tct gca tct gta gga gac aga gtc acc atc act tgc cgg      480
   Ser Ser Leu Ser Ala Ser Val Gly Asp Arg Val Thr Ile Thr Cys Arg
   145                 150                 155                 160
   gcg agt cag ggc att agc agt tat tta gcc tgg tat cag cag aag cca      528
   Ala Ser Gln Gly Ile Ser Ser Tyr Leu Ala Trp Tyr Gln Gln Lys Pro
                   165                 170                 175
   ggg aaa gtt cct aca ctc ctg atc tat gat gca tcc act ttg cga tca        576
   Gly Lys Val Pro Thr Leu Leu Ile Tyr Asp Ala Ser Thr Leu Arg Ser
               180                 185                 190
   ggg gtc cca tct cgc ttc agt ggc agt gga tct gcg aca gat ttc act        624
   Gly Val Pro Ser Arg Phe Ser Gly Ser Gly Ser Ala Thr Asp Phe Thr
           195                 200                 205
   ctc acc atc agc agc ctg cag cct gaa gat gtt gca act tat tac tgt        672
   Leu Thr Ile Ser Ser Leu Gln Pro Glu Asp Val Ala Thr Tyr Tyr Cys
       210                 215                 220
   caa agg tat aac agt gcc ccc ccg atc acc ttc ggc caa ggg aca cga        720
   Gln Arg Tyr Asn Ser Ala Pro Pro Ile Thr Phe Gly Gln Gly Thr Arg
   225                 230                 235                 240
   ctg gag att aaa cgt                                                    735
   Leu Glu Ile Lys Arg
                   245
<210> 124
   <211> 245
   <212> PRT
   <213> Artificial
<220>
   <223> Synthetic Construct
<400> 124
   Gln Val Gln Leu Gln Gln Ser Gly Ala Glu Val Lys Lys Pro Gly Ser
   1               5                   10                  15
   Ser Val Lys Val Ser Cys Lys Val Ser Gly Val Ile Phe Ser Gly Ser
               20                  25                  30
   Ala Ile Ser Trp Val Arg Gln Ala Pro Gly Gln Gly Leu Glu Trp Met
           35                  40                  45
   Gly Gly Ile Ser Pro Leu Phe Gly Thr Thr Asn Tyr Ala Gln Lys Phe
       50                  55                  60
   Gln Gly Arg Val Thr Ile Thr Ala Asp Gln Ser Thr Asn Thr Thr Tyr
   65                  70                  75                  80
   Met Glu Val Asn Ser Leu Arg Tyr Glu Asp Thr Ala Val Tyr Phe Cys
                   85                  90                  95
   Ala Arg Gly Pro Lys Tyr Tyr Ser Glu Tyr Met Asp Val Trp Gly Lys
               100                 105                 110
   Gly Thr Thr Val Thr Val Ser Ser Gly Thr Gly Gly Ser Gly Gly Thr
           115                 120                 125
   Gly Ser Gly Thr Gly Gly Ser Thr Asp Ile Gln Met Thr Gln Ser Pro
   130 135 140
   Ser Ser Leu Ser Ala Ser Val Gly Asp Arg Val Thr Ile Thr Cys Arg
   145 150 155 160
   Ala Ser Gln Gly Ile Ser Ser Tyr Leu Ala Trp Tyr Gln Gln Lys Pro
   165 170 175
   Gly Lys Val Pro Thr Leu Leu Ile Tyr Asp Ala Ser Thr Leu Arg Ser
   180 185 190
   Gly Val Pro Ser Arg Phe Ser Gly Ser Gly Ser Ala Thr Asp Phe Thr
   195 200 205
   Leu Thr Ile Ser Ser Leu Gln Pro Glu Asp Val Ala Thr Tyr Tyr Cys
   210 215 220
   Gln Arg Tyr Asn Ser Ala Pro Pro Ile Thr Phe Gly Gln Gly Thr Arg
   225 230 235 240
   Leu Glu Ile Lys Arg
   245
<210> 125
   <211> 729
   <212> DNA
   <213> Artificial
<220>
   <223> SC06-268
<220>
   <221> CDS
   <222> (1)..(729)
<400> 125
   cag gtc cag ctg gta cag tct ggg gct gag gtg aag aag cct ggg tcc      48
   Gln Val Gln Leu Val Gln Ser Gly Ala Glu Val Lys Lys Pro Gly Ser
   1               5                   10                  15
   tcg gtg aag gtc tcc tgc aag gct tct gga ggc acc ttc agt agt tat      96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg     144
   Ala Ile Ser Trp Val Arg Gln Ala Pro Gly Gln Gly Leu Glu Trp Met
           35                  40                  45
   gga gga atc atg ggt atg ttt ggc aca act aac tac gca cag aag ttc     192
   Gly Gly Ile Met Gly Met Phe Gly Thr Thr Asn Tyr Ala Gln Lys Phe
       50                  55                  60
   cag ggc aga gtc acg att acc gcg gac gaa ttc acg agc gca gcc tac     240
   Gln Gly Arg Val Thr Ile Thr Ala Asp Glu Phe Thr Ser Ala Ala Tyr
   65                  70                  75                  80
   atg gag ctg agg agc ctg aga tct gag gac acg gcc gtc tac tac tgt        288
   Met Glu Leu Arg Ser Leu Arg Ser Glu Asp Thr Ala Val Tyr Tyr Cys
                   85                  90                  95
   gcg agg tct agt ggt tat tac ccc gaa tac ttc cag gac tgg ggc cag        336
   Ala Arg Ser Ser Gly Tyr Tyr Pro Glu Tyr Phe Gln Asp Trp Gly Gln
               100                 105                 110
   ggc acc ctg gtc acc gtc tcg agc ggt acg ggc ggt tca ggc gga acc        384
   Gly Thr Leu Val Thr Val Ser Ser Gly Thr Gly Gly Ser Gly Gly Thr
           115                 120                 125
   ggc agc ggc act ggc ggg tcg acg cag tct gtg ctg act cag cca ccc        432
   Gly Ser Gly Thr Gly Gly Ser Thr Gln Ser Val Leu Thr Gln Pro Pro
       130                 135                 140
   tca gag tcc gtg tcc cca gga cag aca gcc agc gtc acc tgc tct gga        480
   Ser Glu Ser Val Ser Pro Gly Gln Thr Ala Ser Val Thr Cys Ser Gly
   145                 150                 155                 160
   cat aaa ttg ggg gat aaa tat gtt tcg tgg tat cag cag aag cca ggc        528
   His Lys Leu Gly Asp Lys Tyr Val Ser Trp Tyr Gln Gln Lys Pro Gly
                   165                 170                 175
   cag tcc cct gta tta ctc atc tat caa gat aac agg cgg ccc tca ggg        576
   Gln Ser Pro Val Leu Leu Ile Tyr Gln Asp Asn Arg Arg Pro Ser Gly
               180                 185                 190
   atc cct gag cga ttc ata ggc tcc aac tct ggg aac aca gcc act ctg        624
   Ile Pro Glu Arg Phe Ile Gly Ser Asn Ser Gly Asn Thr Ala Thr Leu
           195                 200                 205
   acc atc agc ggg acc cag gct ctg gat gag gct gac tat tac tgt cag        672
   Thr Ile Ser Gly Thr Gln Ala Leu Asp Glu Ala Asp Tyr Tyr Cys Gln
       210                 215                 220
   gcg tgg gac agc agc act gcg gtt ttc ggc gga ggg acc aag ctg acc        720
   Ala Trp Asp Ser Ser Thr Ala Val Phe Gly Gly Gly Thr Lys Leu Thr
   225                 230                 235                 240
   gtc cta ggt                                                            729
   Val Leu Gly
<210> 126
   <211> 243
   <212> PRT
   <213> Artificial
<220>
   <223> Synthetic Construct
<400> 126
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
               35                  40                  45
   Gly Gly Ile Met Gly Met Phe Gly Thr Thr Asn Tyr Ala Gln Lys Phe
       50                  55                  60
   Gln Gly Arg Val Thr Ile Thr Ala Asp Glu Phe Thr Ser Ala Ala Tyr
   65                  70                  75                  80
   Met Glu Leu Arg Ser Leu Arg Ser Glu Asp Thr Ala Val Tyr Tyr Cys
                   85                  90                  95
   Ala Arg Ser Ser Gly Tyr Tyr Pro Glu Tyr Phe Gln Asp Trp Gly Gln
               100                 105                 110
   Gly Thr Leu Val Thr Val Ser Ser Gly Thr Gly Gly Ser Gly Gly Thr
           115                 120                 125
   Gly Ser Gly Thr Gly Gly Ser Thr Gln Ser Val Leu Thr Gln Pro Pro
       130                 135                 140
   Ser Glu Ser Val Ser Pro Gly Gln Thr Ala Ser Val Thr Cys Ser Gly
   145                 150                 155                 160
   His Lys Leu Gly Asp Lys Tyr Val Ser Trp Tyr Gln Gln Lys Pro Gly
                   165                 170                 175
   Gln Ser Pro Val Leu Leu Ile Tyr Gln Asp Asn Arg Arg Pro Ser Gly
               180                 185                 190
   Ile Pro Glu Arg Phe Ile Gly Ser Asn Ser Gly Asn Thr Ala Thr Leu
           195                 200                 205
   Thr Ile Ser Gly Thr Gln Ala Leu Asp Glu Ala Asp Tyr Tyr Cys Gln
       210                 215                 220
   Ala Trp Asp Ser Ser Thr Ala Val Phe Gly Gly Gly Thr Lys Leu Thr
   225                 230                 235                 240
   Val Leu Gly
<210> 127
   <211> 738
   <212> DNA
   <213> Artificial
<220>
   <223> SC06-307
<220>
   <221> CDS
   <222> (1)..(738)
<400> 127
   cag gtc cag ctg gtg cag tct ggg gga ggc ctg gtc aag cct ggg ggg      48
   Gln Val Gln Leu Val Gln Ser Gly Gly Gly Leu Val Lys Pro Gly Gly
   1               5                   10                  15
   tcc ctg aga ctc tcc tgt gca gcc tct gga ttc acc ttc agt agc tat      96
   Ser Leu Arg Leu Ser Cys Ala Ala Ser Gly Phe Thr Phe Ser Ser Tyr
               20                  25                  30
   agc atg aac tgg gtc cgc cag gct cca ggg aag ggg ctg gag tgg gtc     144
   Ser Met Asn Trp Val Arg Gln Ala Pro Gly Lys Gly Leu Glu Trp Val
           35                  40                  45
   tca tcc att agt agt agt agt agt tac ata tac tac gta gac tca gtg     192
   Ser Ser Ile Ser Ser Ser Ser Ser Tyr Ile Tyr Tyr Val Asp Ser Val
       50                  55                  60
   aag ggc cga ttc acc atc tcc aga gac aac gcc aag aac tca ctg tat     240
   Lys Gly Arg Phe Thr Ile Ser Arg Asp Asn Ala Lys Asn Ser Leu Tyr
   65                  70                  75                  80
   ctg caa atg aac agc ctg aga gcc gag gac acg gct gtg tat tac tgt     288
   Leu Gln Met Asn Ser Leu Arg Ala Glu Asp Thr Ala Val Tyr Tyr Cys
                   85                  90                  95
   gcg aga ggt ggt ggg agc tac ggg gcc tac gaa ggc ttt gac tac tgg     336
   Ala Arg Gly Gly Gly Ser Tyr Gly Ala Tyr Glu Gly Phe Asp Tyr Trp
               100                 105                 110
   ggc cag ggc acc ctg gtc acc gtc tcg agc ggt acg ggc ggt tca ggc     384
   Gly Gln Gly Thr Leu Val Thr Val Ser Ser Gly Thr Gly Gly Ser Gly
           115                 120                 125
   gga acc ggc agc ggc act ggc ggg tcg acg gaa att gtg ctg act cag     432
   Gly Thr Gly Ser Gly Thr Gly Gly Ser Thr Glu Ile Val Leu Thr Gln
       130                 135                 140
   tct cca ggc acc ctg tct ttg tct cca ggg gaa aga gcc acc ctc tcc     480
   Ser Pro Gly Thr Leu Ser Leu Ser Pro Gly Glu Arg Ala Thr Leu Ser
   145                 150                 155                 160
   tgc agg gcc agt cag cgt gtt agc agc tac tta gcc tgg tac caa cag     528
   Cys Arg Ala Ser Gln Arg Val Ser Ser Tyr Leu Ala Trp Tyr Gln Gln
                   165                 170                 175
   aaa cct ggc cag gct ccc agg ctc ctc atc tat ggt gca tcc acc agg     576
   Lys Pro Gly Gln Ala Pro Arg Leu Leu Ile Tyr Gly Ala Ser Thr Arg
               180                 185                 190
   gcc gct ggc atc cca gac agg ttc agt ggc agt ggg tct ggg aca gac     624
   Ala Ala Gly Ile Pro Asp Arg Phe Ser Gly Ser Gly Ser Gly Thr Asp
           195                 200                 205
   ttc act ctc acc atc agc aga ctg gag cct gaa gat tct gca gtg tat     672
   Phe Thr Leu Thr Ile Ser Arg Leu Glu Pro Glu Asp Ser Ala Val Tyr
       210                 215                 220
   tac tgt cag cag tat ggt agg aca ccg ctc act ttc ggc gga ggg acc     720
   Tyr Cys Gln Gln Tyr Gly Arg Thr Pro Leu Thr Phe Gly Gly Gly Thr
   225                 230                 235                 240
   aag gtg gag atc aaa cgt                                             738
   Lys Val Glu Ile Lys Arg
   245
<210> 128
   <211> 246
   <212> PRT
   <213> Artificial
<220>
   <223> Synthetic Construct
<400> 128
   Gln Val Gln Leu Val Gln Ser Gly Gly Gly Leu Val Lys Pro Gly Gly
   1 5 10 15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Ser Tyr Ile Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Gly Gly Gly Ser Tyr Gly Ala Tyr Glu Gly Phe Asp Tyr Trp
   100 105 110
   Gly Gln Gly Thr Leu Val Thr Val Ser Ser Gly Thr Gly Gly Ser Gly
   115 120 125
   Gly Thr Gly Ser Gly Thr Gly Gly Ser Thr Glu Ile Val Leu Thr Gln
   130 135 140
   Ser Pro Gly Thr Leu Ser Leu Ser Pro Gly Glu Arg Ala Thr Leu Ser
   145 150 155 160
   Cys Arg Ala Ser Gln Arg Val Ser Ser Tyr Leu Ala Trp Tyr Gln Gln
   165 170 175
   Lys Pro Gly Gln Ala Pro Arg Leu Leu Ile Tyr Gly Ala Ser Thr Arg
   180 185 190
   Ala Ala Gly Ile Pro Asp Arg Phe Ser Gly Ser Gly Ser Gly Thr Asp
   195 200 205
   Phe Thr Leu Thr Ile Ser Arg Leu Glu Pro Glu Asp Ser Ala Val Tyr
   210 215 220
   Tyr Cys Gln Gln Tyr Gly Arg Thr Pro Leu Thr Phe Gly Gly Gly Thr
   225 230 235 240
   Lys Val Glu Ile Lys Arg
   245
<210> 129
   <211> 738
   <212> DNA
   <213> Artificial
<220>
   <223> SC06-310
<220>
   <221> CDS
   <222> (1)..(738)
<400> 129
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac      240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt      288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc      336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga      384
   Lys Gly Thr Thr Val Thr Val Ser Ser Gly Thr Gly Gly Ser Gly Gly
           115                 120                 125
   acc ggc agc ggc act ggc ggg tcg acg tcc tat gtg ctg act cag cca      432
   Thr Gly Ser Gly Thr Gly Gly Ser Thr Ser Tyr Val Leu Thr Gln Pro
       130                 135                 140
   ccc tcg gtg tca gtg gcc cca gga cag acg gcc agg att acc tgt ggg      480
   Pro Ser Val Ser Val Ala Pro Gly Gln Thr Ala Arg Ile Thr Cys Gly
   145                 150                 155                 160
   gga aac aac att gga agt aaa agt gtg cac tgg tac cag cag aag cca        528
   Gly Asn Asn Ile Gly Ser Lys Ser Val His Trp Tyr Gln Gln Lys Pro
                   165                 170                 175
   ggc cag gcc cct gtg ctg gtc gtc tat gat gat agc gac cgg ccc tca        576
   Gly Gln Ala Pro Val Leu Val Val Tyr Asp Asp Ser Asp Arg Pro Ser
               180                 185                 190
   ggg atc cct gag cga ttc tct ggc tcc aac tct ggg aac acg gcc acc        624
   Gly Ile Pro Glu Arg Phe Ser Gly Ser Asn Ser Gly Asn Thr Ala Thr
           195                 200                 205
   ctg acc atc agc agg gtc gaa gcc ggg gat gag gcc gac tat tac tgt        672
   Leu Thr Ile Ser Arg Val Glu Ala Gly Asp Glu Ala Asp Tyr Tyr Cys
       210                 215                 220
   cag gtg tgg gat agt agt agt gat cat gct gtg ttc gga gga ggc acc        720
   Gln Val Trp Asp Ser Ser Ser Asp His Ala Val Phe Gly Gly Gly Thr
   225                 230                 235                 240
   cag ctg acc gtc ctc ggt                                                738
   Gln Leu Thr Val Leu Gly
                   245
<210> 130
   <211> 246
   <212> PRT
   <213> Artificial
<220>
   <223> Synthetic Construct
<400> 130
   Glu Val Gln Leu Val Glu Ser Gly Ala Glu Val Lys Lys Pro Gly Ser
   1 5 10 15
   Ser Val Lys Val Ser Cys Lys Ala Ser Gly Gly Pro Phe Arg Ser Tyr
   20 25 30
   Ala Ile Ser Trp Val Arg Gln Ala Pro Gly Gln Gly Pro Glu Trp Met
   35 40 45
   Gly Gly Ile Ile Pro Ile Phe Gly Thr Thr Lys Tyr Ala Pro Lys Phe
   50 55 60
   Gln Gly Arg Val Thr Ile Thr Ala Asp Asp Phe Ala Gly Thr Val Tyr
   65 70 75 80
   Met Glu Leu Ser Ser Leu Arg Ser Glu Asp Thr Ala Met Tyr Tyr Cys
   85 90 95
   Ala Lys His Met Gly Tyr Gln Val Arg Glu Thr Met Asp Val Trp Gly
   100 105 110
   Lys Gly Thr Thr Val Thr Val Ser Ser Gly Thr Gly Gly Ser Gly Gly
   115 120 125
   Thr Gly Ser Gly Thr Gly Gly Ser Thr Ser Tyr Val Leu Thr Gln Pro
   130 135 140
   Pro Ser Val Ser Val Ala Pro Gly Gln Thr Ala Arg Ile Thr Cys Gly
   145 150 155 160
   Gly Asn Asn Ile Gly Ser Lys Ser Val His Trp Tyr Gln Gln Lys Pro
   165 170 175
   Gly Gln Ala Pro Val Leu Val Val Tyr Asp Asp Ser Asp Arg Pro Ser
   180 185 190
   Gly Ile Pro Glu Arg Phe Ser Gly Ser Asn Ser Gly Asn Thr Ala Thr
   195 200 205
   Leu Thr Ile Ser Arg Val Glu Ala Gly Asp Glu Ala Asp Tyr Tyr Cys
   210 215 220
   Gln Val Trp Asp Ser Ser Ser Asp His Ala Val Phe Gly Gly Gly Thr
   225 230 235 240
   Gln Leu Thr Val Leu Gly
   245
<210> 131
   <211> 744
   <212> DNA
   <213> Artificial
<220>
   <223> SC06-314
<220>
   <221> CDS
   <222> (1)..(744)
<400> 131
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aaa gtc tct tgc aag gct tct gga ggc ccc ttc cgc agc tat       96
   Ser Val Lys Val Ser Cys Lys Ala Ser Gly Gly Pro Phe Arg Ser Tyr
               20                  25                  30
   gct atc agc tgg gtg cga cag gcc cct gga caa ggg cct gag tgg atg      144
   Ala Ile Ser Trp Val Arg Gln Ala Pro Gly Gln Gly Pro Glu Trp Met
           35                  40                  45
   gga ggg atc atc cct att ttt ggt aca aca aaa tac gca ccg aag ttc      192
   Gly Gly Ile Ile Pro Ile Phe Gly Thr Thr Lys Tyr Ala Pro Lys Phe
       50                  55                  60
   cag ggc aga gtc acg att acc gcg gac gat ttc gcg ggc aca gtt tac        240
   Gln Gly Arg Val Thr Ile Thr Ala Asp Asp Phe Ala Gly Thr Val Tyr
   65                  70                  75                  80
   atg gag ctg agc agc ctg cga tct gag gac acg gcc atg tac tac tgt        288
   Met Glu Leu Ser Ser Leu Arg Ser Glu Asp Thr Ala Met Tyr Tyr Cys
                   85                  90                  95
   gcg aaa cat atg ggg tac cag gtg cgc gaa act atg gac gtc tgg ggc        336
   Ala Lys His Met Gly Tyr Gln Val Arg Glu Thr Met Asp Val Trp Gly
               100                 105                 110
   aaa ggg acc acg gtc acc gtc tcg agc ggt acg ggc ggt tca ggc gga        384
   Lys Gly Thr Thr Val Thr Val Ser Ser Gly Thr Gly Gly Ser Gly Gly
           115                 120                 125
   acc ggc agc ggc act ggc ggg tcg acg tcc tat gtg ctg act cag cca        432
   Thr Gly Ser Gly Thr Gly Gly Ser Thr Ser Tyr Val Leu Thr Gln Pro
       130                 135                 140
   ccc tca gcg tct ggg acc ccc ggg cag agg gtc acc atc tct tgt tct        480
   Pro Ser Ala Ser Gly Thr Pro Gly Gln Arg Val Thr Ile Ser Cys Ser
   145                 150                 155                 160
   gga agc agc tcc aac atc gga agt aat tat gta tac tgg tac cag cag        528
   Gly Ser Ser Ser Asn Ile Gly Ser Asn Tyr Val Tyr Trp Tyr Gln Gln
                   165                 170                 175
   ctc cca ggc acg gcc ccc aaa ctc ctc atc tat agg gat ggt cag cgg        576
   Leu Pro Gly Thr Ala Pro Lys Leu Leu Ile Tyr Arg Asp Gly Gln Arg
               180                 185                 190
   ccc tca ggg gtc cct gac cga ttc tct ggc tcc aag tct ggc acc tca        624
   Pro Ser Gly Val Pro Asp Arg Phe Ser Gly Ser Lys Ser Gly Thr Ser
           195                 200                 205
   gcc tcc ctg gcc atc agt gga ctc cgg tcc gat gat gag gct gat tat        672
   Ala Ser Leu Ala Ile Ser Gly Leu Arg Ser Asp Asp Glu Ala Asp Tyr
       210                 215                 220
   tac tgt gca aca tgg gat gac aac ctg agt ggt cca gta ttc ggc gga        720
   Tyr Cys Ala Thr Trp Asp Asp Asn Leu Ser Gly Pro Val Phe Gly Gly
   225                 230                 235                 240
   ggg acc aag ctg acc gtc cta ggt                                        744
   Gly Thr Lys Leu Thr Val Leu Gly
                   245
<210> 132
   <211> 248
   <212> PRT
   <213> Artificial
<220>
   <223> Synthetic Construct
<400> 132
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Pro Phe Arg Ser Tyr
                   20                  25                  30
   Ala Ile Ser Trp Val Arg Gln Ala Pro Gly Gln Gly Pro Glu Trp Met
           35                  40                  45
   Gly Gly Ile Ile Pro Ile Phe Gly Thr Thr Lys Tyr Ala Pro Lys Phe
       50                  55                  60
   Gln Gly Arg Val Thr Ile Thr Ala Asp Asp Phe Ala Gly Thr Val Tyr
   65                  70                  75                  80
   Met Glu Leu Ser Ser Leu Arg Ser Glu Asp Thr Ala Met Tyr Tyr Cys
                   85                  90                  95
   Ala Lys His Met Gly Tyr Gln Val Arg Glu Thr Met Asp Val Trp Gly
               100                 105                 110
   Lys Gly Thr Thr Val Thr Val Ser Ser Gly Thr Gly Gly Ser Gly Gly
           115                 120                 125
   Thr Gly Ser Gly Thr Gly Gly Ser Thr Ser Tyr Val Leu Thr Gln Pro
       130                 135                 140
   Pro Ser Ala Ser Gly Thr Pro Gly Gln Arg Val Thr Ile Ser Cys Ser
   145                 150                 155                 160
   Gly Ser Ser Ser Asn Ile Gly Ser Asn Tyr Val Tyr Trp Tyr Gln Gln
                   165                 170                 175
   Leu Pro Gly Thr Ala Pro Lys Leu Leu Ile Tyr Arg Asp Gly Gln Arg
               180                 185                 190
   Pro Ser Gly Val Pro Asp Arg Phe Ser Gly Ser Lys Ser Gly Thr Ser
           195                 200                 205
   Ala Ser Leu Ala Ile Ser Gly Leu Arg Ser Asp Asp Glu Ala Asp Tyr
       210                 215                 220
   Tyr Cys Ala Thr Trp Asp Asp Asn Leu Ser Gly Pro Val Phe Gly Gly
   225                 230                 235                 240
   Gly Thr Lys Leu Thr Val Leu Gly
                   245
<210> 133
   <211> 735
   <212> DNA
   <213> Artificial
<220>
   <223> SC06-323
<220>
   <221> CDS
   <222> (1)..(735)
<400> 133
   gag gtg cag ctg gtg gag tct ggg gct gag gtg aag aag cca ggg tcc      48
   Glu Val Gln Leu Val Glu Ser Gly Ala Glu Val Lys Lys Pro Gly Ser
   1               5                   10                  15
   tcg gtg aag gtc tcc tgt aag gcc tct gga ggc acc ttc tcc agc tat      96
   Ser Val Lys Val Ser Cys Lys Ala Ser Gly Gly Thr Phe Ser Ser Tyr
               20                  25                  30
   ggt atc agc tgg gtg cga cag gcc cct gga caa ggg ctt gag tgg atg      144
   Gly Ile Ser Trp Val Arg Gln Ala Pro Gly Gln Gly Leu Glu Trp Met
           35                  40                  45
   gga gac atc atc ggt atg ttt ggt tca aca aac tac gca cag aac ttc      192
   Gly Asp Ile Ile Gly Met Phe Gly Ser Thr Asn Tyr Ala Gln Asn Phe
       50                  55                  60
   cag ggc aga ctc acg att acc gcg gac gaa tcc acg agc aca gcc tac      240
   Gln Gly Arg Leu Thr Ile Thr Ala Asp Glu Ser Thr Ser Thr Ala Tyr
   65                  70                  75                  80
   atg gag ctg agc agc ctg aga tct gag gac acg gcc gtg tat tac tgt      288
   Met Glu Leu Ser Ser Leu Arg Ser Glu Asp Thr Ala Val Tyr Tyr Cys
                   85                  90                  95
   gcg aga agt agt ggt tat tac cct gca tac ctc ccc cac tgg ggc cag      336
   Ala Arg Ser Ser Gly Tyr Tyr Pro Ala Tyr Leu Pro His Trp Gly Gln
               100                 105                 110
   ggc acc ttg gtc acc gtc tcg agc ggt acg ggc ggt tca ggc gga acc      384
   Gly Thr Leu Val Thr Val Ser Ser Gly Thr Gly Gly Ser Gly Gly Thr
           115                 120                 125
   ggc agc ggc act ggc ggg tcg acg gaa att gtg ttg acc cag tct cca      432
   Gly Ser Gly Thr Gly Gly Ser Thr Glu Ile Val Leu Thr Gln Ser Pro
       130                 135                 140
   ggc acc ctg tct ttg tct cca ggg gaa aga gcc acc ctc tcc tgc agg      480
   Gly Thr Leu Ser Leu Ser Pro Gly Glu Arg Ala Thr Leu Ser Cys Arg
   145                 150                 155                 160
   gcc agt cag agt gtt agc agc agc tac tta gcc tgg tac cag cag aaa      528
   Ala Ser Gln Ser Val Ser Ser Ser Tyr Leu Ala Trp Tyr Gln Gln Lys
                   165                 170                 175
   cct ggc cag gct ccc agg ctc ctc atc tat ggt gca tcc agc agg gcc      576
   Pro Gly Gln Ala Pro Arg Leu Leu Ile Tyr Gly Ala Ser Ser Arg Ala
               180                 185                 190
   act ggc atc cca gac agg ttc agt ggc agt ggg tct ggg aca gac ttc      624
   Thr Gly Ile Pro Asp Arg Phe Ser Gly Ser Gly Ser Gly Thr Asp Phe
           195                 200                 205
   act ctc acc atc agc aga ctg gag cct gaa gat ttt gca gtg tat tac      672
   Thr Leu Thr Ile Ser Arg Leu Glu Pro Glu Asp Phe Ala Val Tyr Tyr
       210                 215                 220
   tgt cag cag tat ggt agc tca ccc aga act ttc ggc gga ggg acc aag      720
   Cys Gln Gln Tyr Gly Ser Ser Pro Arg Thr Phe Gly Gly Gly Thr Lys
   225                 230                 235                 240
   gtg gag atc aaa cgt                                                  735
   Val Glu Ile Lys Arg
                   245
<210> 134
   <211> 245
   <212> PRT
   <213> Artificial
<220>
   <223> Synthetic Construct
<400> 134
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Gly Ile Ser Trp Val Arg Gln Ala Pro Gly Gln Gly Leu Glu Trp Met
           35                  40                  45
   Gly Asp Ile Ile Gly Met Phe Gly Ser Thr Asn Tyr Ala Gln Asn Phe
       50                  55                  60
   Gln Gly Arg Leu Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Ser Gly Tyr Tyr Pro Ala Tyr Leu Pro His Trp Gly Gln
               100                 105                 110
   Gly Thr Leu Val Thr Val Ser Ser Gly Thr Gly Gly Ser Gly Gly Thr
           115                 120                 125
   Gly Ser Gly Thr Gly Gly Ser Thr Glu Ile Val Leu Thr Gln Ser Pro
       130                 135                 140
   Gly Thr Leu Ser Leu Ser Pro Gly Glu Arg Ala Thr Leu Ser Cys Arg
   145                 150                 155                 160
   Ala Ser Gln Ser Val Ser Ser Ser Tyr Leu Ala Trp Tyr Gln Gln Lys
                   165                 170                 175
   Pro Gly Gln Ala Pro Arg Leu Leu Ile Tyr Gly Ala Ser Ser Arg Ala
               180                 185                 190
   Thr Gly Ile Pro Asp Arg Phe Ser Gly Ser Gly Ser Gly Thr Asp Phe
           195                 200                 205
   Thr Leu Thr Ile Ser Arg Leu Glu Pro Glu Asp Phe Ala Val Tyr Tyr
       210                 215                 220
   Cys Gln Gln Tyr Gly Ser Ser Pro Arg Thr Phe Gly Gly Gly Thr Lys
   225                 230                 235                 240
   Val Glu Ile Lys Arg
                   245
<210> 135
   <211> 744
   <212> DNA
   <213> Artificial
<220>
   <223> SC06-325
<220>
   <221> CDS
   <222> (1)..(744)
<400> 135
   gag gtg cag ctg gtg gag tct ggg gct gag gtg aag aag ccg ggg tcc        48
   Glu Val Gln Leu Val Glu Ser Gly Ala Glu Val Lys Lys Pro Gly Ser
   1               5                   10                  15
   tcg gtg aag gtc tcc tgc aag gct tct gga ggc acc ttc agc ttc tat        96
   Ser Val Lys Val Ser Cys Lys Ala Ser Gly Gly Thr Phe Ser Phe Tyr
               20                  25                  30
   tct atg agc tgg gtg cga cag gcc cct gga caa gga ctt gag tgg atg       144
   Ser Met Ser Trp Val Arg Gln Ala Pro Gly Gln Gly Leu Glu Trp Met
           35                  40                  45
   gga ggg atc atc cct atg ttt ggt aca aca aac tac gca cag aag ttc       192
   Gly Gly Ile Ile Pro Met Phe Gly Thr Thr Asn Tyr Ala Gln Lys Phe
       50                  55                  60
   cag ggc aga gtc acg att acc gcg gtc gaa tcc acg agc aca gcc tac       240
   Gln Gly Arg Val Thr Ile Thr Ala Val Glu Ser Thr Ser Thr Ala Tyr
   65                  70                  75                  80
   atg gag gtg agc agc ctg aga tct gag gac acg gcc gtt tat tac tgt       288
   Met Glu Val Ser Ser Leu Arg Ser Glu Asp Thr Ala Val Tyr Tyr Cys
                   85                  90                  95
   gcg aga ggt gat aag ggt atc tac tac tac tac atg gac gtc tgg ggc       336
   Ala Arg Gly Asp Lys Gly Ile Tyr Tyr Tyr Tyr Met Asp Val Trp Gly
               100                 105                 110
   aaa ggg acc acg gtc acc gtc tcg agc ggt acg ggc ggt tca ggc gga       384
   Lys Gly Thr Thr Val Thr Val Ser Ser Gly Thr Gly Gly Ser Gly Gly
           115                 120                 125
   acc ggc agc ggc act ggc ggg tcg acg cag tct gcc ctg act cag cct       432
   Thr Gly Ser Gly Thr Gly Gly Ser Thr Gln Ser Ala Leu Thr Gln Pro
           130                 135                 140
   gcc tcc gtg tct ggg tct cct gga cag tcg atc acc atc tcc tgc act      480
   Ala Ser Val Ser Gly Ser Pro Gly Gln Ser Ile Thr Ile Ser Cys Thr
   145                 150                 155                 160
   gga acc agc agt gac gtt ggt ggt tat aac tat gtc tcc tgg tac caa      528
   Gly Thr Ser Ser Asp Val Gly Gly Tyr Asn Tyr Val Ser Trp Tyr Gln
                   165                 170                 175
   cag cac cca ggc aaa gcc ccc aaa ctc atg att tat gag gtc agt aat      576
   Gln His Pro Gly Lys Ala Pro Lys Leu Met Ile Tyr Glu Val Ser Asn
               180                 185                 190
   cgg ccc tca ggg gtt tct aat cgc ttc tct ggc tcc aag tct ggc aac      624
   Arg Pro Ser Gly Val Ser Asn Arg Phe Ser Gly Ser Lys Ser Gly Asn
           195                 200                 205
   acg gcc tcc ctg acc atc tct ggg ctc cag gct gag gac gag gct gat      672
   Thr Ala Ser Leu Thr Ile Ser Gly Leu Gln Ala Glu Asp Glu Ala Asp
       210                 215                 220
   tat tac tgc agc tca tat aca agc agc agc act ctt gtc ttc gga act      720
   Tyr Tyr Cys Ser Ser Tyr Thr Ser Ser Ser Thr Leu Val Phe Gly Thr
   225                 230                 235                 240
   ggg acc aag gtc acc gtc cta ggt                                      744
   Gly Thr Lys Val Thr Val Leu Gly
                   245
<210> 136
   <211> 248
   <212> PRT
   <213> Artificial
<220>
   <223> Synthetic Construct
<400> 136
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Phe Tyr
               20                  25                  30
   Ser Met Ser Trp Val Arg Gln Ala Pro Gly Gln Gly Leu Glu Trp Met
           35                  40                  45
   Gly Gly Ile Ile Pro Met Phe Gly Thr Thr Asn Tyr Ala Gln Lys Phe
       50                  55                  60
   Gln Gly Arg Val Thr Ile Thr Ala Val Glu Ser Thr Ser Thr Ala Tyr
   65                  70                  75                  80
   Met Glu Val Ser Ser Leu Arg Ser Glu Asp Thr Ala Val Tyr Tyr Cys
                   85                  90                  95
   Ala Arg Gly Asp Lys Gly Ile Tyr Tyr Tyr Tyr Met Asp Val Trp Gly
               100                 105                 110
   Lys Gly Thr Thr Val Thr Val Ser Ser Gly Thr Gly Gly Ser Gly Gly
           115                 120                 125
   Thr Gly Ser Gly Thr Gly Gly Ser Thr Gln Ser Ala Leu Thr Gln Pro
       130                 135                 140
   Ala Ser Val Ser Gly Ser Pro Gly Gln Ser Ile Thr Ile Ser Cys Thr
   145                 150                 155                 160
   Gly Thr Ser Ser Asp Val Gly Gly Tyr Asn Tyr Val Ser Trp Tyr Gln
                   165                 170                 175
   Gln His Pro Gly Lys Ala Pro Lys Leu Met Ile Tyr Glu Val Ser Asn
               180                 185                 190
   Arg Pro Ser Gly Val Ser Asn Arg Phe Ser Gly Ser Lys Ser Gly Asn
           195                 200                 205
   Thr Ala Ser Leu Thr Ile Ser Gly Leu Gln Ala Glu Asp Glu Ala Asp
       210                 215                 220
   Tyr Tyr Cys Ser Ser Tyr Thr Ser Ser Ser Thr Leu Val Phe Gly Thr
   225                 230                 235                 240
   Gly Thr Lys Val Thr Val Leu Gly
                   245
<210> 137
   <211> 735
   <212> DNA
   <213> Artificial
<220>
   <223> SC06-331
<220>
   <221> CDS
   <222> (1)..(735)
<400> 137
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aag gtc tcc tgc aag gct tct gga ggc acc ttc agc agc tat       96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ggg atc atc ggt atg ttc ggt aca gca aac tac gca cag aag ttc      192
   Gly Gly Ile Ile Gly Met Phe Gly Thr Ala Asn Tyr Ala Gln Lys Phe
       50                  55                  60
   cag ggc aga gtc acg att acc gcg gac gaa ttt acg agc aca gcc tac      240
   Gln Gly Arg Val Thr Ile Thr Ala Asp Glu Phe Thr Ser Thr Ala Tyr
   65                  70                  75                  80
   atg gag ctg agc agc ctg aga tct gag gac acg gcc gtg tat tac tgt      288
   Met Glu Leu Ser Ser Leu Arg Ser Glu Asp Thr Ala Val Tyr Tyr Cys
                   85                  90                  95
   gcg aga gga aat tat tac tat gag agt agt ctc gac tac tgg ggc cag      336
   Ala Arg Gly Asn Tyr Tyr Tyr Glu Ser Ser Leu Asp Tyr Trp Gly Gln
               100                 105                 110
   gga acc ctg gtc acc gtc tcg agc ggt acg ggc ggt tca ggc gga acc      384
   Gly Thr Leu Val Thr Val Ser Ser Gly Thr Gly Gly Ser Gly Gly Thr
           115                 120                 125
   ggc agc ggc act ggc ggg tcg acg cag tct gtc gtg acg cag ccg ccc      432
   Gly Ser Gly Thr Gly Gly Ser Thr Gln Ser Val Val Thr Gln Pro Pro
       130                 135                 140
   tcg gtg tca gtg gcc cca gga cag acg gcc agg att acc tgt ggg gga      480
   Ser Val Ser Val Ala Pro Gly Gln Thr Ala Arg Ile Thr Cys Gly Gly
   145                 150                 155                 160
   aac aac att gga agt aaa agt gtg cac tgg tac cag cag aag cca ggc      528
   Asn Asn Ile Gly Ser Lys Ser Val His Trp Tyr Gln Gln Lys Pro Gly
                   165                 170                 175
   cag gcc cct gtg ctg gtc gtc tat gat gat agc gac cgg ccc tca ggg      576
   Gln Ala Pro Val Leu Val Val Tyr Asp Asp Ser Asp Arg Pro Ser Gly
               180                 185                 190
   atc cct gag cga ttc tct ggc tcc aac tct ggg aac acg gcc acc ctg      624
   Ile Pro Glu Arg Phe Ser Gly Ser Asn Ser Gly Asn Thr Ala Thr Leu
           195                 200                 205
   acc atc agc agg gtc gaa gcc ggg gat gag gcc gac tat tac tgt cag      672
   Thr Ile Ser Arg Val Glu Ala Gly Asp Glu Ala Asp Tyr Tyr Cys Gln
       210                 215                 220
   gtg tgg gat agt agt agt gat cat tat gtc ttc gga act ggg acc aag      720
   Val Trp Asp Ser Ser Ser Asp His Tyr Val Phe Gly Thr Gly Thr Lys
   225                 230                 235                 240
   gtc acc gtc cta ggt                                                  735
   Val Thr Val Leu Gly
                   245
<210> 138
   <211> 245
   <212> PRT
   <213> Artificial
<220>
   <223> Synthetic Construct
<400> 138
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Ile Gly Met Phe Gly Thr Ala Asn Tyr Ala Gln Lys Phe
       50                  55                  60
   Gln Gly Arg Val Thr Ile Thr Ala Asp Glu Phe Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Gly Asn Tyr Tyr Tyr Glu Ser Ser Leu Asp Tyr Trp Gly Gln
               100                 105                 110
   Gly Thr Leu Val Thr Val Ser Ser Gly Thr Gly Gly Ser Gly Gly Thr
           115                 120                 125
   Gly Ser Gly Thr Gly Gly Ser Thr Gln Ser Val Val Thr Gln Pro Pro
       130                 135                 140
   Ser Val Ser Val Ala Pro Gly Gln Thr Ala Arg Ile Thr Cys Gly Gly
   145                 150                 155                 160
   Asn Asn Ile Gly Ser Lys Ser Val His Trp Tyr Gln Gln Lys Pro Gly
                   165                 170                 175
   Gln Ala Pro Val Leu Val Val Tyr Asp Asp Ser Asp Arg Pro Ser Gly
               180                 185                 190
   Ile Pro Glu Arg Phe Ser Gly Ser Asn Ser Gly Asn Thr Ala Thr Leu
           195                 200                 205
   Thr Ile Ser Arg Val Glu Ala Gly Asp Glu Ala Asp Tyr Tyr Cys Gln
       210                 215                 220
   Val Trp Asp Ser Ser Ser Asp His Tyr Val Phe Gly Thr Gly Thr Lys
   225                 230                 235                 240
   Val Thr Val Leu Gly
                   245
<210> 139
   <211> 750
   <212> DNA
   <213> Artificial
<220>
   <223> SC06-344
<220>
   <221> CDS
   <222> (1)..(750)
<400> 139
   cag gtg cag ctg gtg cag tct ggg gct gag gtg aag aag cct ggg tcc       48
   Gln Val Gln Leu Val Gln Ser Gly Ala Glu Val Lys Lys Pro Gly Ser
   1               5                   10                  15
   tcg gtg aga gtc tcc tgc aag gct tct gga agc atc ttc aga aac tat       96
   Ser Val Arg Val Ser Cys Lys Ala Ser Gly Ser Ile Phe Arg Asn Tyr
               20                  25                  30
   gct atg agc tgg gtg cga cag gcc cct gga caa ggg ctt gag tgg atg      144
   Ala Met Ser Trp Val Arg Gln Ala Pro Gly Gln Gly Leu Glu Trp Met
           35                  40                  45
   gga ggg atc atc gct att ttt ggg aca cca aag tac gca cag aag ttc      192
   Gly Gly Ile Ile Ala Ile Phe Gly Thr Pro Lys Tyr Ala Gln Lys Phe
       50                  55                  60
   cag ggc aga gtc acg att acc gcg gac gaa tcg acg agc act gtc tac      240
   Gln Gly Arg Val Thr Ile Thr Ala Asp Glu Ser Thr Ser Thr Val Tyr
   65                  70                  75                  80
   atg gaa ctg agc gga ctg aga tct gag gac acg gcc atg tat tac tgt      288
   Met Glu Leu Ser Gly Leu Arg Ser Glu Asp Thr Ala Met Tyr Tyr Cys
                   85                  90                  95
   gcg agg att ccc cac tat aat ttt ggt tcg ggg agt tat ttc gac tac      336
   Ala Arg Ile Pro His Tyr Asn Phe Gly Ser Gly Ser Tyr Phe Asp Tyr
               100                 105                 110
   tgg ggc cag gga acc ctg gtc acc gtc tcg agc ggt acg ggc ggt tca      384
   Trp Gly Gln Gly Thr Leu Val Thr Val Ser Ser Gly Thr Gly Gly Ser
           115                 120                 125
   ggc gga acc ggc agc ggc act ggc ggg tcg acg act gtg ttg aca cag      432
   Gly Gly Thr Gly Ser Gly Thr Gly Gly Ser Thr Thr Val Leu Thr Gln
       130                 135                 140
   ccg ccc tca gtg tct ggg gcc cca ggg cag agg gtc acc atc tcc tgc      480
   Pro Pro Ser Val Ser Gly Ala Pro Gly Gln Arg Val Thr Ile Ser Cys
   145                 150                 155                 160
   act ggg agc agc tcc aac atc ggg gca ggt tat gat gta cac tgg tac      528
   Thr Gly Ser Ser Ser Asn Ile Gly Ala Gly Tyr Asp Val His Trp Tyr
                   165                 170                 175
   cag cag ctt cca gga aca gcc ccc aaa ctc ctc atc tat ggt aac agc      576
   Gln Gln Leu Pro Gly Thr Ala Pro Lys Leu Leu Ile Tyr Gly Asn Ser
               180                 185                 190
   aat cgg ccc tca ggg gtc cct gac cga ttc tct ggc tcc aag tct ggc      624
   Asn Arg Pro Ser Gly Val Pro Asp Arg Phe Ser Gly Ser Lys Ser Gly
           195                 200                 205
   acg tca gcc acc ctg ggc atc acc gga ctc cag act ggg gac gag gcc      672
   Thr Ser Ala Thr Leu Gly Ile Thr Gly Leu Gln Thr Gly Asp Glu Ala
       210                 215                 220
   gat tat tac tgc gga aca tgg gat agc agc ctg agt gct tat gtc ttc      720
   Asp Tyr Tyr Cys Gly Thr Trp Asp Ser Ser Leu Ser Ala Tyr Val Phe
   225                 230                 235                 240
   gga act ggg acc aag gtc acc gtc cta ggt                              750
   Gly Thr Gly Thr Lys Val Thr Val Leu Gly
                   245                 250
<210> 140
   <211> 250
   <212> PRT
   <213> Artificial
<220>
   <223> Synthetic Construct
<400> 140
   Gln Val Gln Leu Val Gln Ser Gly Ala Glu Val Lys Lys Pro Gly Ser
   1               5                   10                  15
   Ser Val Arg Val Ser Cys Lys Ala Ser Gly Ser Ile Phe Arg Asn Tyr
               20                  25                  30
   Ala Met Ser Trp Val Arg Gln Ala Pro Gly Gln Gly Leu Glu Trp Met
           35                  40                  45
   Gly Gly Ile Ile Ala Ile Phe Gly Thr Pro Lys Tyr Ala Gln Lys Phe
       50                  55                  60
   Gln Gly Arg Val Thr Ile Thr Ala Asp Glu Ser Thr Ser Thr Val Tyr
   65                  70                  75                  80
   Met Glu Leu Ser Gly Leu Arg Ser Glu Asp Thr Ala Met Tyr Tyr Cys
                   85                  90                  95
   Ala Arg Ile Pro His Tyr Asn Phe Gly Ser Gly Ser Tyr Phe Asp Tyr
               100                 105                 110
   Trp Gly Gln Gly Thr Leu Val Thr Val Ser Ser Gly Thr Gly Gly Ser
           115                 120                 125
   Gly Gly Thr Gly Ser Gly Thr Gly Gly Ser Thr Thr Val Leu Thr Gln
       130                 135                 140
   Pro Pro Ser Val Ser Gly Ala Pro Gly Gln Arg Val Thr Ile Ser Cys
   145                 150                 155                 160
   Thr Gly Ser Ser Ser Asn Ile Gly Ala Gly Tyr Asp Val His Trp Tyr
                   165                 170                 175
   Gln Gln Leu Pro Gly Thr Ala Pro Lys Leu Leu Ile Tyr Gly Asn Ser
               180                 185                 190
   Asn Arg Pro Ser Gly Val Pro Asp Arg Phe Ser Gly Ser Lys Ser Gly
           195                 200                 205
   Thr Ser Ala Thr Leu Gly Ile Thr Gly Leu Gln Thr Gly Asp Glu Ala
       210                 215                 220
   Asp Tyr Tyr Cys Gly Thr Trp Asp Ser Ser Leu Ser Ala Tyr Val Phe
   225                 230                 235                 240
   Gly Thr Gly Thr Lys Val Thr Val Leu Gly
                   245                 250
<210> 141
   <211> 10515
   <212> DNA
   <213> Artificial
<220>
   <223> Vector pIg-C911-HCgamma1
<220>
   <221> misc_feature
   <222> (1326)..(5076)
   <223> Stuffer
<400> 141
   tcgacggatc gggagatctc ccgatcccct atggtgcact ctcagtacaa tctgctctga      60
   tgccgcatag ttaagccagt atctgctccc tgcttgtgtg ttggaggtcg ctgagtagtg     120
   cgcgagcaaa atttaagcta caacaaggca aggcttgacc gacaattgca tgaagaatct     180
   gcttagggtt aggcgttttg cgctgcttcg ctaggtggtc aatattggcc attagccata     240
   ttattcattg gttatatagc ataaatcaat attggctatt ggccattgca tacgttgtat     300
   ccatatcata atatgtacat ttatattggc tcatgtccaa cattaccgcc atgttgacat     360
   tgattattga ctagttatta atagtaatca attacggggt cattagttca tagcccatat     420
   atggagttcc gcgttacata acttacggta aatggcccgc ctggctgacc gcccaacgac     480
   ccccgcccat tgacgtcaat aatgacgtat gttcccatag taacgccaat agggactttc     540
   cattgacgtc aatgggtgga gtatttacgg taaactgccc acttggcagt acatcaagtg     600
   tatcatatgc caagtacgcc ccctattgac gtcaatgacg gtaaatggcc cgcctggcat     660
   tatgcccagt acatgacctt atgggacttt cctacttggc agtacatcta cgtattagtc     720
   atcgctatta ccatggtgat gcggttttgg cagtacatca atgggcgtgg atagcggttt     780
   gactcacggg gatttccaag tctccacccc attgacgtca atgggagttt gttttggcac     840
   caaaatcaac gggactttcc aaaatgtcgt aacaactccg ccccattgac gcaaatgggc     900
   ggtaggcgtg tacggtggga ggtctatata agcagagctc gtttagtgaa ccgtcagatc 960
   gcctggagac gccatccacg ctgttttgac ctccatagaa gacaccggga ccgatccagc 1020
   ctccgcggcc gggaacggtg cattggaagc tggcctggat atcctgactc tcttaggtag 1080
   ccttgcagaa gttggtcgtg aggcactggg caggtaagta tcaaggttac aagacaggtt 1140
   taaggagatc aatagaaact gggcttgtcg agacagagaa gactcttgcg tttctgatag 1200
   gcacctattg gtcttactga catccacttt gcctttctct ccacaggtgt ccactcccag 1260
   ttcaattaca gctcgccacc atgggatgga gctgtatcat cctcttcttg gtactgctgc 1320
   tggcccagcc ggccagtgac cttgaccggt gcaccacttt tgatgatgtt caagctccta 1380
   attacactca acatacttca tctatgaggg gggtttacta tcctgatgaa atttttagat 1440
   cggacactct ttatttaact caggatttat ttcttccatt ttattctaat gttacagggt 1500
   ttcatactat taatcatacg tttggcaacc ctgtcatacc ttttaaggat ggtatttatt 1560
   ttgctgccac agagaaatca aatgttgtcc gtggttgggt ttttggttct accatgaaca 1620
   acaagtcaca gtcggtgatt attattaaca attctactaa tgttgttata cgagcatgta 1680
   actttgaatt gtgtgacaac cctttctttg ctgtttctaa acccatgggt acacagacac 1740
   atactatgat attcgataat gcatttaatt gcactttcga gtacatatct gatgcctttt 1800
   cgcttgatgt ttcagaaaag tcaggtaatt ttaaacactt acgagagttt gtgtttaaaa 1860
   ataaagatgg gtttctctat gtttataagg gctatcaacc tatagatgta gttcgtgatc 1920
   taccttctgg ttttaacact ttgaaaccta tttttaagtt gcctcttggt attaacatta 1980
   caaattttag agccattctt acagcctttt cacctgctca agacatttgg ggcacgtcag 2040
   ctgcagccta ttttgttggc tatttaaagc caactacatt tatgctcaag tatgatgaaa 2100
   atggtacaat cacagatgct gttgattgtt ctcaaaatcc acttgctgaa ctcaaatgct 2160
   ctgttaagag ctttgagatt gacaaaggaa tttaccagac ctctaatttc agggttgttc 2220
   cctcaggaga tgttgtgaga ttccctaata ttacaaactt gtgtcctttt ggagaggttt 2280
   ttaatgctac taaattccct tctgtctatg catgggagag aaaaaaaatt tctaattgtg 2340
   ttgctgatta ctctgtgctc tacaactcaa catttttttc aacctttaag tgctatggcg 2400
   tttctgccac taagttgaat gatctttgct tctccaatgt ctatgcagat tcttttgtag 2460
   tcaagggaga tgatgtaaga caaatagcgc caggacaaac tggtgttatt gctgattata 2520
   attataaatt gccagatgat ttcatgggtt gtgtccttgc ttggaatact aggaacattg 2580
   atgctacttc aactggtaat tataattata aatataggta tcttagacat ggcaagctta 2640
   ggccctttga gagagacata tctaatgtgc ctttctcccc tgatggcaaa ccttgcaccc 2700
   cacctgctct taattgttat tggccattaa atgattatgg tttttacacc actactggca 2760
   ttggctacca accttacaga gttgtagtac tttcttttga acttttaaat gcaccggcca 2820
   cggtttgtgg accaaaatta tccactgacc ttattaagaa ccagtgtgtc aattttaatt 2880
   ttaatggact cactggtact ggtgtgttaa ctccttcttc aaagagattt caaccatttc 2940
   aacaatttgg ccgtgatgtt tctgatttca ctgattccgt tcgagatcct aaaacatctg 3000
   aaatattaga catttcacct tgctcttttg ggggtgtaag tgtaattaca cctggaacaa 3060
   atgcttcatc tgaagttgct gttctatatc aagatgttaa ctgcactgat gtttctacag 3120
   caattcatgc agatcaactc acaccagctt ggcgcatata ttctactgga aacaatgtat 3180
   tccagactca ggcaggctgt cttataggag ctgagcatgt cgacacttct tatgagtgcg 3240
   acattcctat tggagctggc atttgtgcta gttaccatac agtttcttta ttacgtagta 3300
   ctagccaaaa atctattgtg gcttatacta tgtctttagg tgctgatagt tcaattgctt 3360
   actctaataa caccattgct atacctacta acttttcaat tagcattact acagaagtaa 3420
   tgcctgtttc tatggctaaa acctccgtag attgtaatat gtacatctgc ggagattcta 3480
   ctgaatgtgc taatttgctt ctccaatatg gtagcttttg cacacaacta aatcgtgcac 3540
   tctcaggtat tgctgctgaa caggatcgca acacacgtga agtgttcgct caagtcaaac 3600
   aaatgtacaa aaccccaact ttgaaatatt ttggtggttt taatttttca caaatattac 3660
   ctgaccctct aaagccaact aagaggtctt ttattgagga cttgctcttt aataaggtga 3720
   cactcgctga tgctggcttc atgaagcaat atggcgaatg cctaggtgat attaatgcta 3780
   gagatctcat ttgtgcgcag aagttcaatg gacttacagt gttgccacct ctgctcactg 3840
   atgatatgat tgctgcctac actgctgctc tagttagtgg tactgccact gctggatgga 3900
   catttggtgc tggcgctgct cttcaaatac cttttgctat gcaaatggca tataggttca 3960
   atggcattgg agttacccaa aatgttctct atgagaacca aaaacaaatc gccaaccaat 4020
   ttaacaaggc gattagtcaa attcaagaat cacttacaac aacatcaact gcattgggca 4080
   agctgcaaga cgttgttaac cagaatgctc aagcattaaa cacacttgtt aaacaactta 4140
   gctctaattt tggtgcaatt tcaagtgtgc taaatgatat cctttcgcga cttgataaag 4200
   tcgaggcgga ggtacaaatt gacaggttaa ttacaggcag acttcaaagc cttcaaacct 4260
   atgtaacaca acaactaatc agggctgctg aaatcagggc ttctgctaat cttgctgcta 4320
   ctaaaatgtc tgagtgtgtt cttggacaat caaaaagagt tgacttttgt ggaaagggct 4380
   accaccttat gtccttccca caagcagccc cgcatggtgt tgtcttccta catgtcacgt 4440
   atgtgccatc ccaggagagg aacttcacca cagcgccagc aatttgtcat gaaggcaaag 4500
   catacttccc tcgtgaaggt gtttttgtgt ttaatggcac ttcttggttt attacacaga 4560
   ggaacttctt ttctccacaa ataattacta cagacaatac atttgtctca ggaaattgtg 4620
   atgtcgttat tggcatcatt aacaacacag tttatgatcc tctgcaacct gagcttgact 4680
   cattcaaaga agagctggac aagtacttca aaaatcatac atcaccagat gttgattttg 4740
   gcgacatttc aggcattaac gcttctgtcg tcaacattca aaaagaaatt gaccgcctca 4800
   atgaggtcgc taaaaattta aatgaatcac tcattgacct tcaagaactg ggaaaatatg 4860
   agcaatatat taaatggcct ctcgacgaac aaaaactcat ctcagaagag gatctgaatg 4920
   ctgtgggcca ggacacgcag gaggtcatcg tggtgccaca ctccttgccc tttaaggtgg 4980
   tggtgatctc agccatcctg gccctggtgg tgctcaccat catctccctt atcatcctca 5040
   tcatgctttg gcagaagaag ccacgttagg cggccgctcg agtgctagca ccaagggccc 5100
   cagcgtgttc cccctggccc ccagcagcaa gagcaccagc ggcggcacag ccgccctggg 5160
   ctgcctggtg aaggactact tccccgagcc cgtgaccgtg agctggaaca gcggcgcctt 5220
   gaccagcggc gtgcacacct tccccgccgt gctgcagagc agcggcctgt acagcctgag 5280
   cagcgtggtg accgtgccca gcagcagcct gggcacccag acctacatct gcaacgtgaa 5340
   ccacaagccc agcaacacca aggtggacaa acgcgtggag cccaagagct gcgacaagac 5400
   ccacacctgc cccccctgcc ctgcccccga gctgctgggc ggaccctccg tgttcctgtt 5460
   cccccccaag cccaaggaca ccctcatgat cagccggacc cccgaggtga cctgcgtggt 5520
   ggtggacgtg agccacgagg accccgaggt gaagttcaac tggtacgtgg acggcgtgga 5580
   ggtgcacaac gccaagacca agccccggga ggagcagtac aacagcacct accgggtggt 5640
   gagcgtgctc accgtgctgc accaggactg gctgaacggc aaggagtaca agtgcaaggt 5700
   gagcaacaag gccctgcctg cccccatcga gaagaccatc agcaaggcca agggccagcc 5760
   ccgggagccc caggtgtaca ccctgccccc cagccgggag gagatgacca agaaccaggt 5820
   gtccctcacc tgtctggtga agggcttcta ccccagcgac atcgccgtgg agtgggagag 5880
   caacggccag cccgagaaca actacaagac caccccccct gtgctggaca gcgacggcag 5940
   cttcttcctg tacagcaagc tcaccgtgga caagagccgg tggcagcagg gcaacgtgtt 6000
   cagctgcagc gtgatgcacg aggccctgca caaccactac acccagaaga gcctgagcct 6060
   gagccccggc aagtgataat ctagagggcc cgtttaaacc cgctgatcag cctcgactgt 6120
   gccttctagt tgccagccat ctgttgtttg cccctccccc gtgccttcct tgaccctgga 6180
   aggtgccact cccactgtcc tttcctaata aaatgaggaa attgcatcgc attgtctgag 6240
   taggtgtcat tctattctgg ggggtggggt ggggcaggac agcaaggggg aggattggga 6300
   agacaatagc aggcatgctg gggatgcggt gggctctatg gcttctgagg cggaaagaac 6360
   cagctggggc tctagggggt atccccacgc gccctgtagc ggcgcattaa gcgcggcggg 6420
   tgtggtggtt acgcgcagcg tgaccgctac acttgccagc gccctagcgc ccgctccttt 6480
   cgctttcttc ccttcctttc tcgccacgtt cgccggcttt ccccgtcaag ctctaaatcg 6540
   ggggctccct ttagggttcc gatttagtgc tttacggcac ctcgacccca aaaaacttga 6600
   ttagggtgat ggttcacgta gtgggccatc gccctgatag acggtttttc gccctttgac 6660
   gttggagtcc acgttcttta atagtggact cttgttccaa actggaacaa cactcaaccc 6720
   tatctcggtc tattcttttg atttataagg gattttgccg atttcggcct attggttaaa 6780
   aaatgagctg atttaacaaa aatttaacgc gaattaattc tgtggaatgt gtgtcagtta 6840
   gggtgtggaa agtccccagg ctccccagca ggcagaagta tgcaaagcat gcatctcaat 6900
   tagtcagcaa ccaggtgtgg aaagtcccca ggctccccag caggcagaag tatgcaaagc 6960
   atgcatctca attagtcagc aaccatagtc ccgcccctaa ctccgcccat cccgccccta 7020
   actccgccca gttccgccca ttctccgccc catggctgac taattttttt tatttatgca 7080
   gaggccgagg ccgcctctgc ctctgagcta ttccagaagt agtgaggagg cttttttgga 7140
   ggcctaggct tttgcaaaaa gctcccggga gcttgtatat ccattttcgg atctgatcaa 7200
   gagacaggat gaggatcgtt tcgcatgatt gaacaagatg gattgcacgc aggttctccg 7260
   gccgcttggg tggagaggct attcggctat gactgggcac aacagacaat cggctgctct 7320
   gatgccgccg tgttccggct gtcagcgcag gggcgcccgg ttctttttgt caagaccgac 7380
   ctgtccggtg ccctgaatga actgcaggac gaggcagcgc ggctatcgtg gctggccacg 7440
   acgggcgttc cttgcgcagc tgtgctcgac gttgtcactg aagcgggaag ggactggctg 7500
   ctattgggcg aagtgccggg gcaggatctc ctgtcatctc accttgctcc tgccgagaaa 7560
   gtatccatca tggctgatgc aatgcggcgg ctgcatacgc ttgatccggc tacctgccca 7620
   ttcgaccacc aagcgaaaca tcgcatcgag cgagcacgta ctcggatgga agccggtctt 7680
   gtcgatcagg atgatctgga cgaagagcat caggggctcg cgccagccga actgttcgcc 7740
   aggctcaagg cgcgcatgcc cgacggcgag gatctcgtcg tgacccatgg cgatgcctgc 7800
   ttgccgaata tcatggtgga aaatggccgc ttttctggat tcatcgactg tggccggctg 7860
   ggtgtggcgg accgctatca ggacatagcg ttggctaccc gtgatattgc tgaagagctt 7920
   ggcggcgaat gggctgaccg cttcctcgtg ctttacggta tcgccgctcc cgattcgcag 7980
   cgcatcgcct tctatcgcct tcttgacgag ttcttctgag cgggactctg gggttcgaaa 8040
   tgaccgacca agcgacgccc aacctgccat cacgagattt cgattccacc gccgccttct 8100
   atgaaaggtt gggcttcgga atcgttttcc gggacgccgg ctggatgatc ctccagcgcg 8160
   gggatctcat gctggagttc ttcgcccacc ccaacttgtt tattgcagct tataatggtt 8220
   acaaataaag caatagcatc acaaatttca caaataaagc atttttttca ctgcattcta 8280
   gttgtggttt gtccaaactc atcaatgtat cttatcatgt ctgtataccg tcgacctcta 8340
   gctagagctt ggcgtaatca tggtcatagc tgtttcctgt gtgaaattgt tatccgctca 8400
   caattccaca caacatacga gccggaagca taaagtgtaa agcctggggt gcctaatgag 8460
   tgagctaact cacattaatt gcgttgcgct cactgcccgc tttccagtcg ggaaacctgt 8520
   cgtgccagct gcattaatga atcggccaac gcgcggggag aggcggtttg cgtattgggc 8580
   gctcttccgc ttcctcgctc actgactcgc tgcgctcggt cgttcggctg cggcgagcgg 8640
   tatcagctca ctcaaaggcg gtaatacggt tatccacaga atcaggggat aacgcaggaa 8700
   agaacatgtg agcaaaaggc cagcaaaagg ccaggaaccg taaaaaggcc gcgttgctgg 8760
   cgtttttcca taggctccgc ccccctgacg agcatcacaa aaatcgacgc tcaagtcaga 8820
   ggtggcgaaa cccgacagga ctataaagat accaggcgtt tccccctgga agctccctcg 8880
   tgcgctctcc tgttccgacc ctgccgctta ccggatacct gtccgccttt ctcccttcgg 8940
   gaagcgtggc gctttctcat agctcacgct gtaggtatct cagttcggtg taggtcgttc 9000
   gctccaagct gggctgtgtg cacgaacccc ccgttcagcc cgaccgctgc gccttatccg 9060
   gtaactatcg tcttgagtcc aacccggtaa gacacgactt atcgccactg gcagcagcca 9120
   ctggtaacag gattagcaga gcgaggtatg taggcggtgc tacagagttc ttgaagtggt 9180
   ggcctaacta cggctacact agaagaacag tatttggtat ctgcgctctg ctgaagccag 9240
   ttaccttcgg aaaaagagtt ggtagctctt gatccggcaa acaaaccacc gctggtagcg 9300
   gtttttttgt ttgcaagcag cagattacgc gcagaaaaaa aggatctcaa gaagatcctt 9360
   tgatcttttc tacggggtct gacgctcagt ggaacgaaaa ctcacgttaa gggattttgg 9420
   tcatgagatt atcaaaaagg atcttcacct agatcctttt aaattaaaaa tgaagtttta 9480
   aatcaatcta aagtatatat gagtaaactt ggtctgacag ttaccaatgc ttaatcagtg 9540
   aggcacctat ctcagcgatc tgtctatttc gttcatccat agttgcctga ctccccgtcg 9600
   tgtagataac tacgatacgg gagggcttac catctggccc cagtgctgca atgataccgc 9660
   gagacccacg ctcaccggct ccagatttat cagcaataaa ccagccagcc ggaagggccg 9720
   agcgcagaag tggtcctgca actttatccg cctccatcca gtctattaat tgttgccggg 9780
   aagctagagt aagtagttcg ccagttaata gtttgcgcaa cgttgttgcc attgctacag 9840
   gcatcgtggt gtcacgctcg tcgtttggta tggcttcatt cagctccggt tcccaacgat 9900
   caaggcgagt tacatgatcc cccatgttgt gcaaaaaagc ggttagctcc ttcggtcctc 9960
   cgatcgttgt cagaagtaag ttggccgcag tgttatcact catggttatg gcagcactgc 10020
   ataattctct tactgtcatg ccatccgtaa gatgcttttc tgtgactggt gagtactcaa 10080
   ccaagtcatt ctgagaatag tgtatgcggc gaccgagttg ctcttgcccg gcgtcaatac 10140
   gggataatac cgcgccacat agcagaactt taaaagtgct catcattgga aaacgttctt 10200
   cggggcgaaa actctcaagg atcttaccgc tgttgagatc cagttcgatg taacccactc 10260
   gtgcacccaa ctgatcttca gcatctttta ctttcaccag cgtttctggg tgagcaaaaa 10320
   caggaaggca aaatgccgca aaaaagggaa taagggcgac acggaaatgt tgaatactca 10380
   tactcttcct ttttcaatat tattgaagca tttatcaggg ttattgtctc atgagcggat 10440
   acatatttga atgtatttag aaaaataaac aaataggggt tccgcgcaca tttccccgaa 10500
   aagtgccacc tgacg 10515
<210> 142
   <211> 8777
   <212> DNA
   <213> Artificial
<220>
   <223> Vector pIg-C909-Ckappa
<220>
   <221> misc_feature
   <222> (1328)..(3860)
   <223> Stuffer
<400> 142
   tcgacggatc gggagatctc ccgatcccct atggtgcact ctcagtacaa tctgctctga 60
   tgccgcatag ttaagccagt atctgctccc tgcttgtgtg ttggaggtcg ctgagtagtg 120
   cgcgagcaaa atttaagcta caacaaggca aggcttgacc gacaattgtt aattaacatg 180
   aagaatctgc ttagggttag gcgttttgcg ctgcttcgct aggtggtcaa tattggccat 240
   tagccatatt attcattggt tatatagcat aaatcaatat tggctattgg ccattgcata 300
   cgttgtatcc atatcataat atgtacattt atattggctc atgtccaaca ttaccgccat 360
   gttgacattg attattgact agttattaat agtaatcaat tacggggtca ttagttcata 420
   gcccatatat ggagttccgc gttacataac ttacggtaaa tggcccgcct ggctgaccgc 480
   ccaacgaccc ccgcccattg acgtcaataa tgacgtatgt tcccatagta acgccaatag 540
   ggactttcca ttgacgtcaa tgggtggagt atttacggta aactgcccac ttggcagtac 600
   atcaagtgta tcatatgcca agtacgcccc ctattgacgt caatgacggt aaatggcccg 660
   cctggcatta tgcccagtac atgaccttat gggactttcc tacttggcag tacatctacg 720
   tattagtcat cgctattacc atggtgatgc ggttttggca gtacatcaat gggcgtggat 780
   agcggtttga ctcacgggga tttccaagtc tccaccccat tgacgtcaat gggagtttgt 840
   tttggcacca aaatcaacgg gactttccaa aatgtcgtaa caactccgcc ccattgacgc 900
   aaatgggcgg taggcgtgta cggtgggagg tctatataag cagagctcgt ttagtgaacc 960
   gtcagatcgc ctggagacgc catccacgct gttttgacct ccatagaaga caccgggacc 1020
   gatccagcct ccgcggccgg gaacggtgca ttggaatcga tgactctctt aggtagcctt 1080
   gcagaagttg gtcgtgaggc actgggcagg taagtatcaa ggttacaaga caggtttaag 1140
   gagatcaata gaaactgggc ttgtcgagac agagaagact cttgcgtttc tgataggcac 1200
   ctattggtct tactgacatc cactttgcct ttctctccac aggtgtccac tcccagttca 1260
   attacagctc gccaccatgc ggctgcccgc ccagctgctg ggccttctca tgctgtgggt 1320
   gcccgcctcg agatctatcg atgcatgcca tggtaccaag cttgccacca tgagcagcag 1380
   ctcttggctg ctgctgagcc tggtggccgt gacagccgcc cagagcacca tcgaggagca 1440
   ggccaagacc ttcctggaca agttcaacca cgaggccgag gacctgttct accagagcag 1500
   cctggccagc tggaactaca acaccaacat caccgaggag aacgtgcaga acatgaacaa 1560
   cgccggcgac aagtggagcg ccttcctgaa ggagcagagc acactggccc agatgtaccc 1620
   cctgcaggag atccagaacc tgaccgtgaa gctgcagctg caggccctgc agcagaacgg 1680
   cagcagcgtg ctgagcgagg acaagagcaa gcggctgaac accatcctga acaccatgtc 1740
   caccatctac agcaccggca aagtgtgcaa ccccgacaac ccccaggagt gcctgctgct 1800
   ggagcccggc ctgaacgaga tcatggccaa cagcctggac tacaacgagc ggctgtgggc 1860
   ctgggagagc tggcggagcg aagtgggcaa gcagctgcgg cccctgtacg aggagtacgt 1920
   ggtgctgaag aacgagatgg ccagggccaa ccactacgag gactacggcg actactggag 1980
   aggcgactac gaagtgaacg gcgtggacgg ctacgactac agcagaggcc agctgatcga 2040
   ggacgtggag cacaccttcg aggagatcaa gcctctgtac gagcacctgc acgcctacgt 2100
   gcgggccaag ctgatgaacg cctaccccag ctacatcagc cccatcggct gcctgcccgc 2160
   ccacctgctg ggcgacatgt ggggccggtt ctggaccaac ctgtacagcc tgaccgtgcc 2220
   cttcggccag aagcccaaca tcgacgtgac cgacgccatg gtggaccagg cctgggacgc 2280
   ccagcggatc ttcaaggagg ccgagaagtt cttcgtgagc gtgggcctgc ccaacatgac 2340
   ccagggcttt tgggagaaca gcatgctgac cgaccccggc aatgtgcaga aggccgtgtg 2400
   ccaccccacc gcctgggacc tgggcaaggg cgacttccgg atcctgatgt gcaccaaagt 2460
   gaccatggac gacttcctga ccgcccacca cgagatgggc cacatccagt acgacatggc 2520
   ctacgccgcc cagcccttcc tgctgcggaa cggcgccaac gagggctttc acgaggccgt 2580
   gggcgagatc atgagcctga gcgccgccac ccccaagcac ctgaagagca tcggcctgct 2640
   gagccccgac ttccaggagg acaacgagac cgagatcaac ttcctgctga agcaggccct 2700
   gaccatcgtg ggcaccctgc ccttcaccta catgctggag aagtggcggt ggatggtgtt 2760
   taagggcgag atccccaagg accagtggat gaagaagtgg tgggagatga agcgggagat 2820
   cgtgggcgtg gtggagcccg tgccccacga cgagacctac tgcgaccccg ccagcctgtt 2880
   ccacgtgagc aacgactact ccttcatccg gtactacacc cggaccctgt accagttcca 2940
   gttccaggag gccctgtgcc aggccgccaa gcacgagggc cccctgcaca agtgcgacat 3000
   cagcaacagc accgaggccg gacagaaact gttcaacatg ctgcggctgg gcaagagcga 3060
   gccctggacc ctggccctgg agaatgtggt gggcgccaag aacatgaatg tgcgccccct 3120
   gctgaactac ttcgagcccc tgttcacctg gctgaaggac cagaacaaga acagcttcgt 3180
   gggctggagc accgactgga gcccctacgc cgaccagagc atcaaagtgc ggatcagcct 3240
   gaagagcgcc ctgggcgaca aggcctacga gtggaacgac aacgagatgt acctgttccg 3300
   gagcagcgtg gcctatgcca tgcggcagta cttcctgaaa gtgaagaacc agatgatcct 3360
   gttcggcgag gaggacgtga gagtggccaa cctgaagccc cggatcagct tcaacttctt 3420
   cgtgaccgcc cccaagaacg tgagcgacat catcccccgg accgaagtgg agaaggccat 3480
   ccggatgagc cggagccgga tcaacgacgc cttccggctg aacgacaact ccctggagtt 3540
   cctgggcatc cagcccaccc tgggccctcc caaccagccc cccgtgagca tctggctgat 3600
   cgtgtttggc gtggtgatgg gcgtgatcgt ggtgggaatc gtgatcctga tcttcaccgg 3660
   catccgggac cggaagaaga agaacaaggc ccggagcggc gagaacccct acgccagcat 3720
   cgatatcagc aagggcgaga acaaccccgg cttccagaac accgacgacg tgcagaccag 3780
   cttctgataa tctagaacga gctcgaattc gaagcttctg cagacgcgtc gacgtcatat 3840
   ggatccgata tcgccgtggc ggccgcaccc agcgtgttca tcttcccccc ctccgacgag 3900
   cagctgaaga gcggcaccgc cagcgtggtg tgcctgctga acaacttcta cccccgggag 3960
   gccaaggtgc agtggaaggt ggacaacgcc ctgcagagcg gcaacagcca ggagagcgtg 4020
   accgagcagg acagcaagga ctccacctac agcctgagca gcaccctcac cctgagcaag 4080
   gccgactacg agaagcacaa ggtgtacgcc tgcgaggtga cccaccaggg cctgagcagc 4140
   cccgtgacca agagcttcaa ccggggcgag tgttaataga cttaagttta aaccgctgat 4200
   cagcctcgac tgtgccttct agttgccagc catctgttgt ttgcccctcc cccgtgcctt 4260
   ccttgaccct ggaaggtgcc actcccactg tcctttccta ataaaatgag gaaattgcat 4320
   cgcattgtct gagtaggtgt cattctattc tggggggtgg ggtggggcag gacagcaagg 4380
   gggaggattg ggaagacaat agcaggcatg ctggggatgc ggtgggctct atggcttctg 4440
   aggcggaaag aaccagctgg ggctctaggg ggtatcccca cgcgccctgt agcggcgcat 4500
   taagcgcggc gggtgtggtg gttacgcgca gcgtgaccgc tacacttgcc agcgccctag 4560
   cgcccgctcc tttcgctttc ttcccttcct ttctcgccac gttcgccggc tttccccgtc 4620
   aagctctaaa tcgggggctc cctttagggt tccgatttag tgctttacgg cacctcgacc 4680
   ccaaaaaact tgattagggt gatggttcac gtagtgggcc atcgccctga tagacggttt 4740
   ttcgcccttt gacgttggag tccacgttct ttaatagtgg actcttgttc caaactggaa 4800
   caacactcaa ccctatctcg gtctattctt ttgatttata agggattttg gccatttcgg 4860
   cctattggtt aaaaaatgag ctgatttaac aaaaatttaa cgcgaattaa ttctgtggaa 4920
   tgtgtgtcag ttagggtgtg gaaagtcccc aggctcccca gcaggcagaa gtatgcaaag 4980
   catgcatctc aattagtcag caaccaggtg tggaaagtcc ccaggctccc cagcaggcag 5040
   aagtatgcaa agcatgcatc tcaattagtc agcaaccata gtcccgcccc taactccgcc 5100
   catcccgccc ctaactccgc ccagttccgc ccattctccg ccccatggct gactaatttt 5160
   ttttatttat gcagaggccg aggccgcctc tgcctctgag ctattccaga agtagtgagg 5220
   aggctttttt ggaggcctag gcttttgcaa aaagctcccg ggagcttgta tatccatttt 5280
   cggatctgat cagcacgtga tgaaaaagcc tgaactcacc gcgacgtctg tcgagaagtt 5340
   tctgatcgaa aagttcgaca gcgtctccga cctgatgcag ctctcggagg gcgaagaatc 5400
   tcgtgctttc agcttcgatg taggagggcg tggatatgtc ctgcgggtaa atagctgcgc 5460
   cgatggtttc tacaaagatc gttatgttta tcggcacttt gcatcggccg cgctcccgat 5520
   tccggaagtg cttgacattg gggaattcag cgagagcctg acctattgca tctcccgccg 5580
   tgcacagggt gtcacgttgc aagacctgcc tgaaaccgaa ctgcccgctg ttctgcagcc 5640
   ggtcgcggag gccatggatg cgatcgctgc ggccgatctt agccagacga gcgggttcgg 5700
   cccattcgga ccacaaggaa tcggtcaata cactacatgg cgtgatttca tatgcgcgat 5760
   tgctgatccc catgtgtatc actggcaaac tgtgatggac gacaccgtca gtgcgtccgt 5820
   cgcgcaggct ctcgatgagc tgatgctttg ggccgaggac tgccccgaag tccggcacct 5880
   cgtgcacgcg gatttcggct ccaacaatgt cctgacggac aatggccgca taacagcggt 5940
   cattgactgg agcgaggcga tgttcgggga ttcccaatac gaggtcgcca acatcttctt 6000
   ctggaggccg tggttggctt gtatggagca gcagacgcgc tacttcgagc ggaggcatcc 6060
   ggagcttgca ggatcgccgc ggctccgggc gtatatgctc cgcattggtc ttgaccaact 6120
   ctatcagagc ttggttgacg gcaatttcga tgatgcagct tgggcgcagg gtcgatgcga 6180
   cgcaatcgtc cgatccggag ccgggactgt cgggcgtaca caaatcgccc gcagaagcgc 6240
   ggccgtctgg accgatggct gtgtagaagt actcgccgat agtggaaacc gacgccccag 6300
   cactcgtccg agggcaaagg aatagcacgt gctacgagat ttcgattcca ccgccgcctt 6360
   ctatgaaagg ttgggcttcg gaatcgtttt ccgggacgcc ggctggatga tcctccagcg 6420
   cggggatctc atgctggagt tcttcgccca ccccaacttg tttattgcag cttataatgg 6480
   ttacaaataa agcaatagca tcacaaattt cacaaataaa gcattttttt cactgcattc 6540
   tagttgtggt ttgtccaaac tcatcaatgt atcttatcat gtctgtatac cgtcgacctc 6600
   tagctagagc ttggcgtaat catggtcata gctgtttcct gtgtgaaatt gttatccgct 6660
   cacaattcca cacaacatac gagccggaag cataaagtgt aaagcctggg gtgcctaatg 6720
   agtgagctaa ctcacattaa ttgcgttgcg ctcactgccc gctttccagt cgggaaacct 6780
   gtcgtgccag ctgcattaat gaatcggcca acgcgcgggg agaggcggtt tgcgtattgg 6840
   gcgctcttcc gcttcctcgc tcactgactc gctgcgctcg gtcgttcggc tgcggcgagc 6900
   ggtatcagct cactcaaagg cggtaatacg gttatccaca gaatcagggg ataacgcagg 6960
   aaagaacatg tgagcaaaag gccagcaaaa ggccaggaac cgtaaaaagg ccgcgttgct 7020
   ggcgtttttc cataggctcc gcccccctga cgagcatcac aaaaatcgac gctcaagtca 7080
   gaggtggcga aacccgacag gactataaag ataccaggcg tttccccctg gaagctccct 7140
   cgtgcgctct cctgttccga ccctgccgct taccggatac ctgtccgcct ttctcccttc 7200
   gggaagcgtg gcgctttctc atagctcacg ctgtaggtat ctcagttcgg tgtaggtcgt 7260
   tcgctccaag ctgggctgtg tgcacgaacc ccccgttcag cccgaccgct gcgccttatc 7320
   cggtaactat cgtcttgagt ccaacccggt aagacacgac ttatcgccac tggcagcagc 7380
   cactggtaac aggattagca gagcgaggta tgtaggcggt gctacagagt tcttgaagtg 7440
   gtggcctaac tacggctaca ctagaagaac agtatttggt atctgcgctc tgctgaagcc 7500
   agttaccttc ggaaaaagag ttggtagctc ttgatccggc aaacaaacca ccgctggtag 7560
   cggttttttt gtttgcaagc agcagattac gcgcagaaaa aaaggatctc aagaagatcc 7620
   tttgatcttt tctacggggt ctgacgctca gtggaacgaa aactcacgtt aagggatttt 7680
   ggtcatgaga ttatcaaaaa ggatcttcac ctagatcctt ttaaattaaa aatgaagttt 7740
   taaatcaatc taaagtatat atgagtaaac ttggtctgac agttaccaat gcttaatcag 7800
   tgaggcacct atctcagcga tctgtctatt tcgttcatcc atagttgcct gactccccgt 7860
   cgtgtagata actacgatac gggagggctt accatctggc cccagtgctg caatgatacc 7920
   gcgagaccca cgctcaccgg ctccagattt atcagcaata aaccagccag ccggaagggc 7980
   cgagcgcaga agtggtcctg caactttatc cgcctccatc cagtctatta attgttgccg 8040
   ggaagctaga gtaagtagtt cgccagttaa tagtttgcgc aacgttgttg ccattgctac 8100
   aggcatcgtg gtgtcacgct cgtcgtttgg tatggcttca ttcagctccg gttcccaacg 8160
   atcaaggcga gttacatgat cccccatgtt gtgcaaaaaa gcggttagct ccttcggtcc 8220
   tccgatcgtt gtcagaagta agttggccgc agtgttatca ctcatggtta tggcagcact 8280
   gcataattct cttactgtca tgccatccgt aagatgcttt tctgtgactg gtgagtactc 8340
   aaccaagtca ttctgagaat agtgtatgcg gcgaccgagt tgctcttgcc cggcgtcaat 8400
   acgggataat accgcgccac atagcagaac tttaaaagtg ctcatcattg gaaaacgttc 8460
   ttcggggcga aaactctcaa ggatcttacc gctgttgaga tccagttcga tgtaacccac 8520
   tcgtgcaccc aactgatctt cagcatcttt tactttcacc agcgtttctg ggtgagcaaa 8580
   aacaggaagg caaaatgccg caaaaaaggg aataagggcg acacggaaat gttgaatact 8640
   catactcttc ctttttcaat attattgaag catttatcag ggttattgtc tcatgagcgg 8700
   atacatattt gaatgtattt agaaaaataa acaaataggg gttccgcgca catttccccg 8760
   aaaagtgcca cctgacg 8777
<210> 143
   <211> 8792
   <212> DNA
   <213> Artificial
<220>
   <223> Vector pIg-C910-Clambda
<220>
   <221> misc_feature
   <222> (1330)..(3869)
   <223> Stuffer
<400> 143
   tcgacggatc gggagatctc ccgatcccct atggtgcact ctcagtacaa tctgctctga 60
   tgccgcatag ttaagccagt atctgctccc tgcttgtgtg ttggaggtcg ctgagtagtg 120
   cgcgagcaaa atttaagcta caacaaggca aggcttgacc gacaattgtt aattaacatg 180
   aagaatctgc ttagggttag gcgttttgcg ctgcttcgct aggtggtcaa tattggccat 240
   tagccatatt attcattggt tatatagcat aaatcaatat tggctattgg ccattgcata 300
   cgttgtatcc atatcataat atgtacattt atattggctc atgtccaaca ttaccgccat 360
   gttgacattg attattgact agttattaat agtaatcaat tacggggtca ttagttcata 420
   gcccatatat ggagttccgc gttacataac ttacggtaaa tggcccgcct ggctgaccgc 480
   ccaacgaccc ccgcccattg acgtcaataa tgacgtatgt tcccatagta acgccaatag 540
   ggactttcca ttgacgtcaa tgggtggagt atttacggta aactgcccac ttggcagtac 600
   atcaagtgta tcatatgcca agtacgcccc ctattgacgt caatgacggt aaatggcccg 660
   cctggcatta tgcccagtac atgaccttat gggactttcc tacttggcag tacatctacg 720
   tattagtcat cgctattacc atggtgatgc ggttttggca gtacatcaat gggcgtggat 780
   agcggtttga ctcacgggga tttccaagtc tccaccccat tgacgtcaat gggagtttgt 840
   tttggcacca aaatcaacgg gactttccaa aatgtcgtaa caactccgcc ccattgacgc 900
   aaatgggcgg taggcgtgta cggtgggagg tctatataag cagagctcgt ttagtgaacc 960
   gtcagatcgc ctggagacgc catccacgct gttttgacct ccatagaaga caccgggacc 1020
   gatccagcct ccgcggccgg gaacggtgca ttggaatcga tgactctctt aggtagcctt 1080
   gcagaagttg gtcgtgaggc actgggcagg taagtatcaa ggttacaaga caggtttaag 1140
   gagatcaata gaaactgggc ttgtcgagac agagaagact cttgcgtttc tgataggcac 1200
   ctattggtct tactgacatc cactttgcct ttctctccac aggtgtccac tcccagttca 1260
   attacagctc gccaccatgc ggttctccgc tcagctgctg ggccttctgg tgctgtggat 1320
   tcccggcgtc tcgagatcta tcgatgcatg ccatggtacc aagcttgcca ccatgagcag 1380
   cagctcttgg ctgctgctga gcctggtggc cgtgacagcc gcccagagca ccatcgagga 1440
   gcaggccaag accttcctgg acaagttcaa ccacgaggcc gaggacctgt tctaccagag 1500
   cagcctggcc agctggaact acaacaccaa catcaccgag gagaacgtgc agaacatgaa 1560
   caacgccggc gacaagtgga gcgccttcct gaaggagcag agcacactgg cccagatgta 1620
   ccccctgcag gagatccaga acctgaccgt gaagctgcag ctgcaggccc tgcagcagaa 1680
   cggcagcagc gtgctgagcg aggacaagag caagcggctg aacaccatcc tgaacaccat 1740
   gtccaccatc tacagcaccg gcaaagtgtg caaccccgac aacccccagg agtgcctgct 1800
   gctggagccc ggcctgaacg agatcatggc caacagcctg gactacaacg agcggctgtg 1860
   ggcctgggag agctggcgga gcgaagtggg caagcagctg cggcccctgt acgaggagta 1920
   cgtggtgctg aagaacgaga tggccagggc caaccactac gaggactacg gcgactactg 1980
   gagaggcgac tacgaagtga acggcgtgga cggctacgac tacagcagag gccagctgat 2040
   cgaggacgtg gagcacacct tcgaggagat caagcctctg tacgagcacc tgcacgccta 2100
   cgtgcgggcc aagctgatga acgcctaccc cagctacatc agccccatcg gctgcctgcc 2160
   cgcccacctg ctgggcgaca tgtggggccg gttctggacc aacctgtaca gcctgaccgt 2220
   gcccttcggc cagaagccca acatcgacgt gaccgacgcc atggtggacc aggcctggga 2280
   cgcccagcgg atcttcaagg aggccgagaa gttcttcgtg agcgtgggcc tgcccaacat 2340
   gacccagggc ttttgggaga acagcatgct gaccgacccc ggcaatgtgc agaaggccgt 2400
   gtgccacccc accgcctggg acctgggcaa gggcgacttc cggatcctga tgtgcaccaa 2460
   agtgaccatg gacgacttcc tgaccgccca ccacgagatg ggccacatcc agtacgacat 2520
   ggcctacgcc gcccagccct tcctgctgcg gaacggcgcc aacgagggct ttcacgaggc 2580
   cgtgggcgag atcatgagcc tgagcgccgc cacccccaag cacctgaaga gcatcggcct 2640
   gctgagcccc gacttccagg aggacaacga gaccgagatc aacttcctgc tgaagcaggc 2700
   cctgaccatc gtgggcaccc tgcccttcac ctacatgctg gagaagtggc ggtggatggt 2760
   gtttaagggc gagatcccca aggaccagtg gatgaagaag tggtgggaga tgaagcggga 2820
   gatcgtgggc gtggtggagc ccgtgcccca cgacgagacc tactgcgacc ccgccagcct 2880
   gttccacgtg agcaacgact actccttcat ccggtactac acccggaccc tgtaccagtt 2940
   ccagttccag gaggccctgt gccaggccgc caagcacgag ggccccctgc acaagtgcga 3000
   catcagcaac agcaccgagg ccggacagaa actgttcaac atgctgcggc tgggcaagag 3060
   cgagccctgg accctggccc tggagaatgt ggtgggcgcc aagaacatga atgtgcgccc 3120
   cctgctgaac tacttcgagc ccctgttcac ctggctgaag gaccagaaca agaacagctt 3180
   cgtgggctgg agcaccgact ggagccccta cgccgaccag agcatcaaag tgcggatcag 3240
   cctgaagagc gccctgggcg acaaggccta cgagtggaac gacaacgaga tgtacctgtt 3300
   ccggagcagc gtggcctatg ccatgcggca gtacttcctg aaagtgaaga accagatgat 3360
   cctgttcggc gaggaggacg tgagagtggc caacctgaag ccccggatca gcttcaactt 3420
   cttcgtgacc gcccccaaga acgtgagcga catcatcccc cggaccgaag tggagaaggc 3480
   catccggatg agccggagcc ggatcaacga cgccttccgg ctgaacgaca actccctgga 3540
   gttcctgggc atccagccca ccctgggccc tcccaaccag ccccccgtga gcatctggct 3600
   gatcgtgttt ggcgtggtga tgggcgtgat cgtggtggga atcgtgatcc tgatcttcac 3660
   cggcatccgg gaccggaaga agaagaacaa ggcccggagc ggcgagaacc cctacgccag 3720
   catcgatatc agcaagggcg agaacaaccc cggcttccag aacaccgacg acgtgcagac 3780
   cagcttctga taatctagaa cgagctcgaa ttcgaagctt ctgcagacgc gtcgacgtca 3840
   tatggatccg atatcgccgt ggcggccgca ggccagccca aggccgctcc cagcgtgacc 3900
   ctgttccccc cctcctccga ggagctgcag gccaacaagg ccaccctggt gtgcctcatc 3960
   agcgacttct accctggcgc cgtgaccgtg gcctggaagg ccgacagcag ccccgtgaag 4020
   gccggcgtgg agaccaccac ccccagcaag cagagcaaca acaagtacgc cgccagcagc 4080
   tacctgagcc tcacccccga gcagtggaag agccaccgga gctacagctg ccaggtgacc 4140
   cacgagggca gcaccgtgga gaagaccgtg gcccccaccg agtgcagcta atagacttaa 4200
   gtttaaaccg ctgatcagcc tcgactgtgc cttctagttg ccagccatct gttgtttgcc 4260
   cctcccccgt gccttccttg accctggaag gtgccactcc cactgtcctt tcctaataaa 4320
   atgaggaaat tgcatcgcat tgtctgagta ggtgtcattc tattctgggg ggtggggtgg 4380
   ggcaggacag caagggggag gattgggaag acaatagcag gcatgctggg gatgcggtgg 4440
   gctctatggc ttctgaggcg gaaagaacca gctggggctc tagggggtat ccccacgcgc 4500
   cctgtagcgg cgcattaagc gcggcgggtg tggtggttac gcgcagcgtg accgctacac 4560
   ttgccagcgc cctagcgccc gctcctttcg ctttcttccc ttcctttctc gccacgttcg 4620
   ccggctttcc ccgtcaagct ctaaatcggg ggctcccttt agggttccga tttagtgctt 4680
   tacggcacct cgaccccaaa aaacttgatt agggtgatgg ttcacgtagt gggccatcgc 4740
   cctgatagac ggtttttcgc cctttgacgt tggagtccac gttctttaat agtggactct 4800
   tgttccaaac tggaacaaca ctcaacccta tctcggtcta ttcttttgat ttataaggga 4860
   ttttggccat ttcggcctat tggttaaaaa atgagctgat ttaacaaaaa tttaacgcga 4920
   attaattctg tggaatgtgt gtcagttagg gtgtggaaag tccccaggct ccccagcagg 4980
   cagaagtatg caaagcatgc atctcaatta gtcagcaacc aggtgtggaa agtccccagg 5040
   ctccccagca ggcagaagta tgcaaagcat gcatctcaat tagtcagcaa ccatagtccc 5100
   gcccctaact ccgcccatcc cgcccctaac tccgcccagt tccgcccatt ctccgcccca 5160
   tggctgacta atttttttta tttatgcaga ggccgaggcc gcctctgcct ctgagctatt 5220
   ccagaagtag tgaggaggct tttttggagg cctaggcttt tgcaaaaagc tcccgggagc 5280
   ttgtatatcc attttcggat ctgatcagca cgtgatgaaa aagcctgaac tcaccgcgac 5340
   gtctgtcgag aagtttctga tcgaaaagtt cgacagcgtc tccgacctga tgcagctctc 5400
   ggagggcgaa gaatctcgtg ctttcagctt cgatgtagga gggcgtggat atgtcctgcg 5460
   ggtaaatagc tgcgccgatg gtttctacaa agatcgttat gtttatcggc actttgcatc 5520
   ggccgcgctc ccgattccgg aagtgcttga cattggggaa ttcagcgaga gcctgaccta 5580
   ttgcatctcc cgccgtgcac agggtgtcac gttgcaagac ctgcctgaaa ccgaactgcc 5640
   cgctgttctg cagccggtcg cggaggccat ggatgcgatc gctgcggccg atcttagcca 5700
   gacgagcggg ttcggcccat tcggaccgca aggaatcggt caatacacta catggcgtga 5760
   tttcatatgc gcgattgctg atccccatgt gtatcactgg caaactgtga tggacgacac 5820
   cgtcagtgcg tccgtcgcgc aggctctcga tgagctgatg ctttgggccg aggactgccc 5880
   cgaagtccgg cacctcgtgc acgcggattt cggctccaac aatgtcctga cggacaatgg 5940
   ccgcataaca gcggtcattg actggagcga ggcgatgttc ggggattccc aatacgaggt 6000
   cgccaacatc ttcttctgga ggccgtggtt ggcttgtatg gagcagcaga cgcgctactt 6060
   cgagcggagg catccggagc ttgcaggatc gccgcggctc cgggcgtata tgctccgcat 6120
   tggtcttgac caactctatc agagcttggt tgacggcaat ttcgatgatg cagcttgggc 6180
   gcagggtcga tgcgacgcaa tcgtccgatc cggagccggg actgtcgggc gtacacaaat 6240
   cgcccgcaga agcgcggccg tctggaccga tggctgtgta gaagtactcg ccgatagtgg 6300
   aaaccgacgc cccagcactc gtccgagggc aaaggaatag cacgtgctac gagatttcga 6360
   ttccaccgcc gccttctatg aaaggttggg cttcggaatc gttttccggg acgccggctg 6420
   gatgatcctc cagcgcgggg atctcatgct ggagttcttc gcccacccca acttgtttat 6480
   tgcagcttat aatggttaca aataaagcaa tagcatcaca aatttcacaa ataaagcatt 6540
   tttttcactg cattctagtt gtggtttgtc caaactcatc aatgtatctt atcatgtctg 6600
   tataccgtcg acctctagct agagcttggc gtaatcatgg tcatagctgt ttcctgtgtg 6660
   aaattgttat ccgctcacaa ttccacacaa catacgagcc ggaagcataa agtgtaaagc 6720
   ctggggtgcc taatgagtga gctaactcac attaattgcg ttgcgctcac tgcccgcttt 6780
   ccagtcggga aacctgtcgt gccagctgca ttaatgaatc ggccaacgcg cggggagagg 6840
   cggtttgcgt attgggcgct cttccgcttc ctcgctcact gactcgctgc gctcggtcgt 6900
   tcggctgcgg cgagcggtat cagctcactc aaaggcggta atacggttat ccacagaatc 6960
   aggggataac gcaggaaaga acatgtgagc aaaaggccag caaaaggcca ggaaccgtaa 7020
   aaaggccgcg ttgctggcgt ttttccatag gctccgcccc cctgacgagc atcacaaaaa 7080
   tcgacgctca agtcagaggt ggcgaaaccc gacaggacta taaagatacc aggcgtttcc 7140
   ccctggaagc tccctcgtgc gctctcctgt tccgaccctg ccgcttaccg gatacctgtc 7200
   cgcctttctc ccttcgggaa gcgtggcgct ttctcatagc tcacgctgta ggtatctcag 7260
   ttcggtgtag gtcgttcgct ccaagctggg ctgtgtgcac gaaccccccg ttcagcccga 7320
   ccgctgcgcc ttatccggta actatcgtct tgagtccaac ccggtaagac acgacttatc 7380
   gccactggca gcagccactg gtaacaggat tagcagagcg aggtatgtag gcggtgctac 7440
   agagttcttg aagtggtggc ctaactacgg ctacactaga agaacagtat ttggtatctg 7500
   cgctctgctg aagccagtta ccttcggaaa aagagttggt agctcttgat ccggcaaaca 7560
   aaccaccgct ggtagcggtt tttttgtttg caagcagcag attacgcgca gaaaaaaagg 7620
   atctcaagaa gatcctttga tcttttctac ggggtctgac gctcagtgga acgaaaactc 7680
   acgttaaggg attttggtca tgagattatc aaaaaggatc ttcacctaga tccttttaaa 7740
   ttaaaaatga agttttaaat caatctaaag tatatatgag taaacttggt ctgacagtta 7800
   ccaatgctta atcagtgagg cacctatctc agcgatctgt ctatttcgtt catccatagt 7860
   tgcctgactc cccgtcgtgt agataactac gatacgggag ggcttaccat ctggccccag   7920
   tgctgcaatg ataccgcgag acccacgctc accggctcca gatttatcag caataaacca   7980
   gccagccgga agggccgagc gcagaagtgg tcctgcaact ttatccgcct ccatccagtc   8040
   tattaattgt tgccgggaag ctagagtaag tagttcgcca gttaatagtt tgcgcaacgt   8100
   tgttgccatt gctacaggca tcgtggtgtc acgctcgtcg tttggtatgg cttcattcag   8160
   ctccggttcc caacgatcaa ggcgagttac atgatccccc atgttgtgca aaaaagcggt   8220
   tagctccttc ggtcctccga tcgttgtcag aagtaagttg gccgcagtgt tatcactcat   8280
   ggttatggca gcactgcata attctcttac tgtcatgcca tccgtaagat gcttttctgt   8340
   gactggtgag tactcaacca agtcattctg agaatagtgt atgcggcgac cgagttgctc   8400
   ttgcccggcg tcaatacggg ataataccgc gccacatagc agaactttaa aagtgctcat   8460
   cattggaaaa cgttcttcgg ggcgaaaact ctcaaggatc ttaccgctgt tgagatccag   8520
   ttcgatgtaa cccactcgtg cacccaactg atcttcagca tcttttactt tcaccagcgt   8580
   ttctgggtga gcaaaaacag gaaggcaaaa tgccgcaaaa aagggaataa gggcgacacg   8640
   gaaatgttga atactcatac tcttcctttt tcaatattat tgaagcattt atcagggtta   8700
   ttgtctcatg agcggataca tatttgaatg tatttagaaa aataaacaaa taggggttcc   8760
   gcgcacattt ccccgaaaag tgccacctga cg                                8792
<210> 144
   <211> 23
   <212> DNA
   <213> Artificial
<220>
   <223> Primer OK1 (HuVK1B)
<400> 144
   gacatccagw tgacccagtc tcc 23
<210> 145
   <211> 23
   <212> DNA
   <213> Artificial
<220>
   <223> Primer OK2 (HuVK2)
<400> 145
   gatgttgtga tgactcagtc tcc 23
<210> 146
   <211> 23
   <212> DNA
   <213> Artificial
<220>
   <223> Primer OK3 (HuVK2B2)
<400> 146
   gatattgtga tgacccagac tcc 23
<210> 147
   <211> 23
   <212> DNA
   <213> Artificial
<220>
   <223> Primer OK4 (HuVK3B)
<400> 147
   gaaattgtgw tgacrcagtc tcc 23
<210> 148
   <211> 23
   <212> DNA
   <213> Artificial
<220>
   <223> Primer OK5 (HuVK5)
<400> 148
   gaaacgacac tcacgcagtc tcc 23
<210> 149
   <211> 23
   <212> DNA
   <213> Artificial
<220>
   <223> Primer OK6 (HuVK6)
<400> 149
   gaaattgtgc tgactcagtc tcc 23
<210> 150
   <211> 24
   <212> DNA
   <213> Artificial
<220>
   <223> Primer OCK (HuCK)
<400> 150
   acactctccc ctgttgaagc tctt 24
<210> 151
   <211> 23
   <212> DNA
   <213> Artificial
<220>
   <223> Primer OL1 (HuVL1A)
<400> 151
   cagtctgtgc tgactcagcc acc 23
<210> 152
   <211> 23
   <212> DNA
   <213> Artificial
<220>
   <223> Primer OL1 (HuVL1B)
<400> 152
   cagtctgtgy tgacgcagcc gcc 23
<210> 153
   <211> 23
   <212> DNA
   <213> Artificial
<220>
   <223> Primer OL1 (HuVL1C)
<400> 153
   cagtctgtcg tgacgcagcc gcc 23
<210> 154
   <211> 20
   <212> DNA
   <213> Artificial
<220>
   <223> OL2 (HuVL2B)
<400> 154
   cagtctgccc tgactcagcc 20
<210> 155
   <211> 23
   <212> DNA
   <213> Artificial
<220>
   <223> OL3 (HuVL3A)
<400> 155
   tcctatgwgc tgactcagcc acc 23
<210> 156
   <211> 23
   <212> DNA
   <213> Artificial
<220>
   <223> OL4 (HuVL3B)
<400> 156
   tcttctgagc tgactcagga ccc 23
<210> 157
   <211> 20
   <212> DNA
   <213> Artificial
<220>
   <223> OL5 (HuVL4B)
<400> 157
   cagcytgtgc tgactcaatc 20
<210> 158
   <211> 23
   <212> DNA
   <213> Artificial
<220>
   <223> OL6 (HuVL5)
<400> 158
   caggctgtgc tgactcagcc gtc 23
<210> 159
   <211> 23
   <212> DNA
   <213> Artificial
<220>
   <223> OL7 (HuVL6)
<400> 159
   aattttatgc tgactcagcc cca 23
<210> 160
   <211> 23
   <212> DNA
   <213> Artificial
<220>
   <223> OL8 (HuVL7/8)
<400> 160
   cagrctgtgg tgacycagga gcc 23
<210> 161
   <211> 23
   <212> DNA
   <213> Artificial
<220>
   <223> OL9 (HuVL9)
<400> 161
   cwgcctgtgc tgactcagcc mcc 23
<210> 162
   <211> 18
   <212> DNA
   <213> Artificial
<220>
   <223> OL9 (HuVL10)
<400> 162
   caggcagggc tgactcag 18
<210> 163
   <211> 23
   <212> DNA
   <213> Artificial
<220>
   <223> OCL (HuCL2)
<400> 163
   tgaacattct gtaggggcca ctg 23
<210> 164
   <211> 23
   <212> DNA
   <213> Artificial
<220>
   <223> OCL (HuCL7)
<400> 164
   agagcattct gcaggggcca ctg 23
<210> 165
   <211> 23
   <212> DNA
   <213> Artificial
<220>
   <223> OH1(HuVH1B7A)
<400> 165
   cagrtgcagc tggtgcartc tgg 23
<210> 166
   <211> 23
   <212> DNA
   <213> Artificial
<220>
   <223> OH1 (HuVH1C)
<400> 166
   saggtccagc tggtrcagtc tgg 23
<210> 167
   <211> 23
   <212> DNA
   <213> Artificial
<220>
   <223> OH2 (HuVH2B)
<400> 167
   cagrtcacct tgaaggagtc tgg 23
<210> 168
   <211> 18
   <212> DNA
   <213> Artificial
<220>
   <223> OH3 (HuVH3A)
<400> 168
   gaggtgcagc tggtggag 18
<210> 169
   <211> 23
   <212> DNA
   <213> Artificial
<220>
   <223> OH4 (HuVH3C)
<400> 169
   gaggtgcagc tggtggagwc ygg 23
<210> 170
   <211> 23
   <212> DNA
   <213> Artificial
<220>
   <223> OH5 (HuVH4B)
<400> 170
   caggtgcagc tacagcagtg ggg 23
<210> 171
   <211> 23
   <212> DNA
   <213> Artificial
<220>
   <223> OH6 (HuVH4C)
<400> 171
   cagstgcagc tgcaggagtc sgg 23
<210> 172
   <211> 23
   <212> DNA
   <213> Artificial
<220>
   <223> OH7 (HuVH6A)
<400> 172
   caggtacagc tgcagcagtc agg 23
<210> 173
   <211> 24
   <212> DNA
   <213> Artificial
<220>
   <223> OCM (HuCIgM)
<400> 173
   tggaagaggc acgttctttt cttt 24
<210> 174
   <211> 41
   <212> DNA
   <213> Artificial
<220>
   <223> OK1S (HuVK1B-SAL)
<400> 174
   tgagcacaca ggtcgacgga catccagwtg acccagtctc c 41
<210> 175
   <211> 41
   <212> DNA
   <213> Artificial
<220>
   <223> OK2S (HuVK2-SAL)
<400> 175
   tgagcacaca ggtcgacgga tgttgtgatg actcagtctc c 41
<210> 176
   <211> 41
   <212> DNA
   <213> Artificial
<220>
   <223> OK3S (HuVK2B2-SAL)
<400> 176
   tgagcacaca ggtcgacgga tattgtgatg acccagactc c 41
<210> 177
   <211> 41
   <212> DNA
   <213> Artificial
<220>
   <223> OK4S (HuVK3B-SAL)
<400> 177
   tgagcacaca ggtcgacgga aattgtgwtg acrcagtctc c 41
<210> 178
   <211> 41
   <212> DNA
   <213> Artificial
<220>
   <223> OK5S (HuVK5-SAL)
<400> 178
   tgagcacaca ggtcgacgga aacgacactc acgcagtctc c 41
<210> 179
   <211> 41
   <212> DNA
   <213> Artificial
<220>
   <223> OK6S (HuVK6-SAL)
<400> 179
   tgagcacaca ggtcgacgga aattgtgctg actcagtctc c 41
<210> 180
   <211> 48
   <212> DNA
   <213> Artificial
<220>
   <223> OJK1 (HuJK1-NOT)
<400> 180
   gagtcattct cgacttgcgg ccgcacgttt gatttccacc ttggtccc 48
<210> 181
   <211> 48
   <212> DNA
   <213> Artificial
<220>
   <223> OJK2 (HuJK2-NOT)
<400> 181
   gagtcattct cgacttgcgg ccgcacgttt gatctccagc ttggtccc 48
<210> 182
   <211> 48
   <212> DNA
   <213> Artificial
<220>
   <223> OJK3 (HuJK3-NOT)
<400> 182
   gagtcattct cgacttgcgg ccgcacgttt gatatccact ttggtccc 48
<210> 183
   <211> 48
   <212> DNA
   <213> Artificial
<220>
   <223> OJK4 (HuJK4-NOT)
<400> 183
   gagtcattct cgacttgcgg ccgcacgttt gatctccacc ttggtccc 48
<210> 184
   <211> 48
   <212> DNA
   <213> Artificial
<220>
   <223> OJK5 (HuJK5-NOT)
<400> 184
   gagtcattct cgacttgcgg ccgcacgttt aatctccagt cgtgtccc 48
<210> 185
   <211> 41
   <212> DNA
   <213> Artificial
<220>
   <223> OL1S (HuVL1A-SAL)
<400> 185
   tgagcacaca ggtcgacgca gtctgtgctg actcagccac c 41
<210> 186
   <211> 41
   <212> DNA
   <213> Artificial
<220>
   <223> OL1S (HuVL1B-SAL)
<400> 186
   tgagcacaca ggtcgacgca gtctgtgytg acgcagccgc c 41
<210> 187
   <211> 41
   <212> DNA
   <213> Artificial
<220>
   <223> OL1S (HuVL1C-SAL)
<400> 187
   tgagcacaca ggtcgacgca gtctgtcgtg acgcagccgc c 41
<210> 188
   <211> 38
   <212> DNA
   <213> Artificial
<220>
   <223> OL2S (HuVL2B-SAL)
<400> 188
   tgagcacaca ggtcgacgca gtctgccctg actcagcc 38
<210> 189
   <211> 41
   <212> DNA
   <213> Artificial
<220>
   <223> OL3S (HuVL3A-SAL)
<400> 189
   tgagcacaca ggtcgacgtc ctatgwgctg actcagccac c 41
<210> 190
   <211> 41
   <212> DNA
   <213> Artificial
<220>
   <223> OL4S (HuVL3B-SAL)
<400> 190
   tgagcacaca ggtcgacgtc ttctgagctg actcaggacc c 41
<210> 191
   <211> 38
   <212> DNA
   <213> Artificial
<220>
   <223> OL5S (HuVL4B-SAL)
<400> 191
   tgagcacaca ggtcgacgca gcytgtgctg actcaatc 38
<210> 192
   <211> 41
   <212> DNA
   <213> Artificial
<220>
   <223> OL6S (HuVL5-SAL)
<400> 192
   tgagcacaca ggtcgacgca ggctgtgctg actcagccgt c 41
<210> 193
   <211> 41
   <212> DNA
   <213> Artificial
<220>
   <223> OL7S (HuVL6-SAL)
<400> 193
   tgagcacaca ggtcgacgaa ttttatgctg actcagcccc a 41
<210> 194
   <211> 41
   <212> DNA
   <213> Artificial
<220>
   <223> OL8S (HuVL7/8-SAL)
<400> 194
   tgagcacaca ggtcgacgca grctgtggtg acycaggagc c 41
<210> 195
   <211> 41
   <212> DNA
   <213> Artificial
<220>
   <223> OL9S (HuVL9-SAL)
<400> 195
   tgagcacaca ggtcgacgcw gcctgtgctg actcagccmc c 41
<210> 196
   <211> 36
   <212> DNA
   <213> Artificial
<220>
   <223> OL9S (HuVL10-SAL)
<400> 196
   tgagcacaca ggtcgacgca ggcagggctg actcag 36
<210> 197
   <211> 48
   <212> DNA
   <213> Artificial
<220>
   <223> OJL1 (HuJL1-NOT)
<400> 197
   gagtcattct cgacttgcgg ccgcacctag gacggtgacc ttggtccc 48
<210> 198
   <211> 48
   <212> DNA
   <213> Artificial
<220>
   <223> OJL2 (HuJL2/3-NOT)
<400> 198
   gagtcattct cgacttgcgg ccgcacctag gacggtcagc ttggtccc 48
<210> 199
   <211> 48
   <212> DNA
   <213> Artificial
<220>
   <223> OJL3 (HuJL7-NOT)
<400> 199
   gagtcattct cgacttgcgg ccgcaccgag gacggtcagc tgggtgcc 48
<210> 200
   <211> 56
   <212> DNA
   <213> Artificial
<220>
   <223> OH1S (HuVH1B-SFI)
<400> 200
   gtcctcgcaa ctgcggccca gccggccatg gcccagrtgc agctggtgca rtctgg 56
<210> 201
   <211> 56
   <212> DNA
   <213> Artificial
<220>
   <223> OH1S (HuVH1C-SFI)
<400> 201
   gtcctcgcaa ctgcggccca gccggccatg gccsaggtcc agctggtrca gtctgg 56
<210> 202
   <211> 56
   <212> DNA
   <213> Artificial
<220>
   <223> OH2S (HuVH2B-SFI)
<400> 202
   gtcctcgcaa ctgcggccca gccggccatg gcccagrtca ccttgaagga gtctgg 56
<210> 203
   <211> 51
   <212> DNA
   <213> Artificial
<220>
   <223> OH3S (HuVH3A-SFI)
<400> 203
   gtcctcgcaa ctgcggccca gccggccatg gccgaggtgc agctggtgga g 51
<210> 204
   <211> 56
   <212> DNA
   <213> Artificial
<220>
   <223> OH4S (HuVH3C-SFI)
<400> 204
   gtcctcgcaa ctgcggccca gccggccatg gccgaggtgc agctggtgga gwcygg 56
<210> 205
   <211> 56
   <212> DNA
   <213> Artificial
<220>
   <223> OH5S (HuVH4B-SFI)
<400> 205
   gtcctcgcaa ctgcggccca gccggccatg gcccaggtgc agctacagca gtgggg 56
<210> 206
   <211> 56
   <212> DNA
   <213> Artificial
<220>
   <223> OH6S (HuVH4C-SFI)
<400> 206
   gtcctcgcaa ctgcggccca gccggccatg gcccagstgc agctgcagga gtcsgg 56
<210> 207
   <211> 56
   <212> DNA
   <213> Artificial
<220>
   <223> OH7S (HuVH6A-SFI)
<400> 207
   gtcctcgcaa ctgcggccca gccggccatg gcccaggtac agctgcagca gtcagg 56
<210> 208
   <211> 36
   <212> DNA
   <213> Artificial
<220>
   <223> OJH1 (HuJH1/2-XHO)
<400> 208
   gagtcattct cgactcgaga crgtgaccag ggtgcc 36
<210> 209
   <211> 36
   <212> DNA
   <213> Artificial
<220>
   <223> OJH2 (HuJH3-XHO)
<400> 209
   gagtcattct cgactcgaga cggtgaccat tgtccc 36
<210> 210
   <211> 36
   <212> DNA
   <213> Artificial
<220>
   <223> OJH3 (HuJH4/5-XHO)
<400> 210
   gagtcattct cgactcgaga cggtgaccag ggttcc 36
<210> 211
   <211> 36
   <212> DNA
   <213> Artificial
<220>
   <223> OJH4 (HuJH6-XHO)
<400> 211
   gagtcattct cgactcgaga cggtgaccgt ggtccc 36
<210> 212
   <211> 735
   <212> DNA
   <213> Artificial
<220>
   <223> SC06-141
<220>
   <221> CDS
   <222> (1)..(735)
<400> 212
   gag gtc cag ctg gtg cag tct ggg gct gag gtg aag aag cct ggg gcc       48
   Glu Val Gln Leu Val Gln Ser Gly Ala Glu Val Lys Lys Pro Gly Ala
   1               5                   10                  15
   tca gtg aag gtc tcc tgc aag gct tct ggg tac acc ttc acc ggc tac       96
   Ser Val Lys Val Ser Cys Lys Ala Ser Gly Tyr Thr Phe Thr Gly Tyr
               20                  25                  30
   tat gtg tac tgg gtg cga cag gcc cct gga caa ggg ctt gag tgg atg      144
   Tyr Val Tyr Trp Val Arg Gln Ala Pro Gly Gln Gly Leu Glu Trp Met
           35                  40                  45
   gga tgg atc agc gct tac aat ggt aac aca aac tat gca cag aag ttc      192
   Gly Trp Ile Ser Ala Tyr Asn Gly Asn Thr Asn Tyr Ala Gln Lys Phe
       50                  55                  60
   cag ggc aga gtc acg att acc gcg gac aaa tcc acg agc aca gcc tac      240
   Gln Gly Arg Val Thr Ile Thr Ala Asp Lys Ser Thr Ser Thr Ala Tyr
   65                  70                  75                  80
   atg gag ctg agc agc ctg aga tct gaa gac acg gct gtg tat tac tgt      288
   Met Glu Leu Ser Ser Leu Arg Ser Glu Asp Thr Ala Val Tyr Tyr Cys
                   85                  90                  95
   gcg aga agt aga tcc ctg gac gtc tgg ggc caa ggg acc acg gtc acc      336
   Ala Arg Ser Arg Ser Leu Asp Val Trp Gly Gln Gly Thr Thr Val Thr
               100                 105                 110
   gtc tcg agc ggt acg ggc ggt tca ggc gga acc ggc agc ggc act ggc      384
   Val Ser Ser Gly Thr Gly Gly Ser Gly Gly Thr Gly Ser Gly Thr Gly
           115                 120                 125
   ggg tcg acg gat gtt gtg atg act cag tct cca gac tcc ctg gct gtg      432
   Gly Ser Thr Asp Val Val Met Thr Gln Ser Pro Asp Ser Leu Ala Val
       130                 135                 140
   tct ctg ggc gag agg gcc acc atc aac tgc aag tcc agc cag agt gtt      480
   Ser Leu Gly Glu Arg Ala Thr Ile Asn Cys Lys Ser Ser Gln Ser Val
   145                 150                 155                 160
   tta tac agc tcc aac aat aag aac tac tta gct tgg tac cag cag aaa      528
   Leu Tyr Ser Ser Asn Asn Lys Asn Tyr Leu Ala Trp Tyr Gln Gln Lys
                   165                 170                 175
   cca gga cag cct cct aag ctg ctc att tac tgg gca tct acc cgg gaa      576
   Pro Gly Gln Pro Pro Lys Leu Leu Ile Tyr Trp Ala Ser Thr Arg Glu
                   180                 185                 190
   tcc ggg gtc cct gac cga ttc agt ggc agc ggg tct ggg aca gat ttc      624
   Ser Gly Val Pro Asp Arg Phe Ser Gly Ser Gly Ser Gly Thr Asp Phe
           195                 200                 205
   act ctc acc atc agc agc ctg cag gct gaa gat gtg gca gtt tat tac      672
   Thr Leu Thr Ile Ser Ser Leu Gln Ala Glu Asp Val Ala Val Tyr Tyr
       210                 215                 220
   tgt cag caa tat tat agt act cct ctc act ttc ggc gga ggg acc aaa      720
   Cys Gln Gln Tyr Tyr Ser Thr Pro Leu Thr Phe Gly Gly Gly Thr Lys
   225                 230                 235                 240
   gtg gat atc aaa cgt                                                  735
   Val Asp Ile Lys Arg
                   245
<210> 213
   <211> 245
   <212> PRT
   <213> Artificial
<220>
   <223> Synthetic Construct
<400> 213
   Glu Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Val Tyr Trp Val Arg Gln Ala Pro Gly Gln Gly Leu Glu Trp Met
           35                  40                  45
   Gly Trp Ile Ser Ala Tyr Asn Gly Asn Thr Asn Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Arg Ser Leu Asp Val Trp Gly Gln Gly Thr Thr Val Thr
               100                 105                 110
   Val Ser Ser Gly Thr Gly Gly Ser Gly Gly Thr Gly Ser Gly Thr Gly
           115                 120                 125
   Gly Ser Thr Asp Val Val Met Thr Gln Ser Pro Asp Ser Leu Ala Val
       130                 135                 140
   Ser Leu Gly Glu Arg Ala Thr Ile Asn Cys Lys Ser Ser Gln Ser Val
   145                 150                 155                 160
   Leu Tyr Ser Ser Asn Asn Lys Asn Tyr Leu Ala Trp Tyr Gln Gln Lys
                   165                 170                 175
   Pro Gly Gln Pro Pro Lys Leu Leu Ile Tyr Trp Ala Ser Thr Arg Glu
               180                 185                 190
   Ser Gly Val Pro Asp Arg Phe Ser Gly Ser Gly Ser Gly Thr Asp Phe
           195                 200                 205
   Thr Leu Thr Ile Ser Ser Leu Gln Ala Glu Asp Val Ala Val Tyr Tyr
       210                 215                 220
   Cys Gln Gln Tyr Tyr Ser Thr Pro Leu Thr Phe Gly Gly Gly Thr Lys
   225                 230                 235                 240
   Val Asp Ile Lys Arg
                   245
<210> 214
   <211> 741
   <212> DNA
   <213> Artificial
<220>
   <223> SC06-272
<220>
   <221> CDS
   <222> (1)..(741)
<400> 214
   cag atg cag ctg gtg cag tct ggg gct gag gtg aag aag cct ggg tcc       48
   Gln Met Gln Leu Val Gln Ser Gly Ala Glu Val Lys Lys Pro Gly Ser
   1               5                   10                  15
   tcg gtg aag gtc tcc tgc aag gct tct gga ggc acc ttc tcc agt tat       96
   Ser Val Lys Val Ser Cys Lys Ala Ser Gly Gly Thr Phe Ser Ser Tyr
               20                  25                  30
   gct atc acc tgg gtg cga cag gcc cct gga caa ggg ctt gag tgg atg      144
   Ala Ile Thr Trp Val Arg Gln Ala Pro Gly Gln Gly Leu Glu Trp Met
           35                  40                  45
   gga ggg atc atc ggt atg ttt ggt tca aca aac tac gca cag aac ttc      192
   Gly Gly Ile Ile Gly Met Phe Gly Ser Thr Asn Tyr Ala Gln Asn Phe
       50                  55                  60
   cag ggc aga gtc acg att acc gcg gac gaa tcc acg agc aca gcc tac      240
   Gln Gly Arg Val Thr Ile Thr Ala Asp Glu Ser Thr Ser Thr Ala Tyr
   65                  70                  75                  80
   atg gag ctg agc agc ctc aga tct gag gac acg gcc gtg tat tac tgt      288
   Met Glu Leu Ser Ser Leu Arg Ser Glu Asp Thr Ala Val Tyr Tyr Cys
                   85                  90                  95
   gcg aga agt act ggt tat tac cct gca tac ctc cac cac tgg ggc cag      336
   Ala Arg Ser Thr Gly Tyr Tyr Pro Ala Tyr Leu His His Trp Gly Gln
               100                 105                 110
   ggc acc ctg gtc acc gtc tcg agc ggt acg ggc ggt tca ggc gga acc      384
   Gly Thr Leu Val Thr Val Ser Ser Gly Thr Gly Gly Ser Gly Gly Thr
           115                 120                 125
   ggc agc ggc act ggc ggg tcg acg cag tct gcc ctg act cag cct cgc      432
   Gly Ser Gly Thr Gly Gly Ser Thr Gln Ser Ala Leu Thr Gln Pro Arg
       130                 135                 140
   tca gtg tcc ggg tct cct gga cag tca gtc acc atc tcc tgc act gga      480
   Ser Val Ser Gly Ser Pro Gly Gln Ser Val Thr Ile Ser Cys Thr Gly
   145                 150                 155                 160
   acc agc agt gat gtt ggt ggt tat aac tat gtc tcc tgg tac caa cag      528
   Thr Ser Ser Asp Val Gly Gly Tyr Asn Tyr Val Ser Trp Tyr Gln Gln
                   165                 170                 175
   cac cca ggc aaa gcc ccc aaa ctc atg att tat gat gtc agt aag cgg      576
   His Pro Gly Lys Ala Pro Lys Leu Met Ile Tyr Asp Val Ser Lys Arg
               180                 185                 190
   ccc tca ggg gtc cct gat cgc ttc tct ggc tcc aag tct ggc aac acg      624
   Pro Ser Gly Val Pro Asp Arg Phe Ser Gly Ser Lys Ser Gly Asn Thr
           195                 200                 205
   gcc tcc ctg acc atc tct ggg ctc cag gct gag gat gag gct gat tat      672
   Ala Ser Leu Thr Ile Ser Gly Leu Gln Ala Glu Asp Glu Ala Asp Tyr
       210                 215                 220
   tac tgc agc tca tat aca agc agc agc act cat gtc ttc gga act ggg      720
   Tyr Cys Ser Ser Tyr Thr Ser Ser Ser Thr His Val Phe Gly Thr Gly
   225                 230                 235                 240
   acc aag gtc acc gtc cta ggt                                          741
   Thr Lys Val Thr Val Leu Gly
                   245
<210> 215
   <211> 247
   <212> PRT
   <213> Artificial
<220>
   <223> Synthetic Construct
<400> 215
   Gln Met Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Thr Trp Val Arg Gln Ala Pro Gly Gln Gly Leu Glu Trp Met
           35                  40                  45
   Gly Gly Ile Ile Gly Met Phe Gly Ser Thr Asn Tyr Ala Gln Asn Phe
   50 55 60
   Gln Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser Thr Gly Tyr Tyr Pro Ala Tyr Leu His His Trp Gly Gln
   100 105 110
   Gly Thr Leu Val Thr Val Ser Ser Gly Thr Gly Gly Ser Gly Gly Thr
   115 120 125
   Gly Ser Gly Thr Gly Gly Ser Thr Gln Ser Ala Leu Thr Gln Pro Arg
   130 135 140
   Ser Val Ser Gly Ser Pro Gly Gln Ser Val Thr Ile Ser Cys Thr Gly
   145 150 155 160
   Thr Ser Ser Asp Val Gly Gly Tyr Asn Tyr Val Ser Trp Tyr Gln Gln
   165 170 175
   His Pro Gly Lys Ala Pro Lys Leu Met Ile Tyr Asp Val Ser Lys Arg
   180 185 190
   Pro Ser Gly Val Pro Asp Arg Phe Ser Gly Ser Lys Ser Gly Asn Thr
   195 200 205
   Ala Ser Leu Thr Ile Ser Gly Leu Gln Ala Glu Asp Glu Ala Asp Tyr
   210 215 220
   Tyr Cys Ser Ser Tyr Thr Ser Ser Ser Thr His Val Phe Gly Thr Gly
   225 230 235 240
   Thr Lys Val Thr Val Leu Gly
   245
<210> 216
   <211> 738
   <212> DNA
   <213> Artificial
<220>
   <223> SC06-296
<220>
   <221> CDS
   <222> (1)..(738)
<400> 216
   gag gtg cag ctg gtg gag acc ggg gct gag gtg aag aag cct ggg gcc        48
   Glu Val Gln Leu Val Glu Thr Gly Ala Glu Val Lys Lys Pro Gly Ala
   1               5                   10                  15
   tca gtg aag gtt tcc tgc aag gca tct gga tac acc ttc acc agc tac        96
   Ser Val Lys Val Ser Cys Lys Ala Ser Gly Tyr Thr Phe Thr Ser Tyr
               20                  25                  30
   tat atg cac tgg gtg cga cag gcc cct gga caa ggg ctt gag tgg atg       144
   Tyr Met His Trp Val Arg Gln Ala Pro Gly Gln Gly Leu Glu Trp Met
           35                  40                  45
   gga tgg atc aac cct aac agt ggt ggc aca aac tat gca cag aag ttt       192
   Gly Trp Ile Asn Pro Asn Ser Gly Gly Thr Asn Tyr Ala Gln Lys Phe
       50                  55                  60
   cag ggc agg gtc acc atg acc agg gac acg tcc atc agc aca gcc tac       240
   Gln Gly Arg Val Thr Met Thr Arg Asp Thr Ser Ile Ser Thr Ala Tyr
   65                  70                  75                  80
   atg gag ctg agc agg ctg aga tct gac gac acg gcc gtg tat tac tgt       288
   Met Glu Leu Ser Arg Leu Arg Ser Asp Asp Thr Ala Val Tyr Tyr Cys
                   85                  90                  95
   gcg aga gag ggg aaa tgg gga cct caa gcg gct ttt gat atc tgg ggc       336
   Ala Arg Glu Gly Lys Trp Gly Pro Gln Ala Ala Phe Asp Ile Trp Gly
               100                 105                 110
   caa ggg aca atg gtc acc gtc tcg agc ggt acg ggc ggt tca ggc gga       384
   Gln Gly Thr Met Val Thr Val Ser Ser Gly Thr Gly Gly Ser Gly Gly
           115                 120                 125
   acc ggc agc ggc act ggc ggg tcg acg gaa att gtg atg acg cag tct       432
   Thr Gly Ser Gly Thr Gly Gly Ser Thr Glu Ile Val Met Thr Gln Ser
       130                 135                 140
   cca ggc acc ctg tct ttg tct cca ggg gaa aga gcc acc ctc tcc tgc       480
   Pro Gly Thr Leu Ser Leu Ser Pro Gly Glu Arg Ala Thr Leu Ser Cys
   145                 150                 155                 160
   agg gcc agt cag agt gtt agc agc agc tac tta gcc tgg tac cag cag       528
   Arg Ala Ser Gln Ser Val Ser Ser Ser Tyr Leu Ala Trp Tyr Gln Gln
                   165                 170                 175
   aaa cct ggc cag gct ccc agg ctc ctc atc tat gat gca tcc agc agg       576
   Lys Pro Gly Gln Ala Pro Arg Leu Leu Ile Tyr Asp Ala Ser Ser Arg
               180                 185                 190
   gcc act gac atc cca gac agg ttc agt ggc agt ggg tct ggg aca gac       624
   Ala Thr Asp Ile Pro Asp Arg Phe Ser Gly Ser Gly Ser Gly Thr Asp
           195                 200                 205
   ttc act ctc acc atc agc aga ctg gag cct gaa gat ttt gca gtg tat       672
   Phe Thr Leu Thr Ile Ser Arg Leu Glu Pro Glu Asp Phe Ala Val Tyr
       210                 215                 220
   tac tgt cag cag tat ggt agc tca ctt tgg acg ttc ggc caa ggg acc       720
   Tyr Cys Gln Gln Tyr Gly Ser Ser Leu Trp Thr Phe Gly Gln Gly Thr
   225                 230                 235                 240
   aag gtg gag atc aaa cgt                                               738
   Lys Val Glu Ile Lys Arg
                   245
<210> 217
   <211> 246
   <212> PRT
   <213> Artificial
<220>
   <223> Synthetic Construct
<400> 217
   Glu Val Gln Leu Val Glu Thr Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Glu Gly Lys Trp Gly Pro Gln Ala Ala Phe Asp Ile Trp Gly
               100                 105                 110
   Gln Gly Thr Met Val Thr Val Ser Ser Gly Thr Gly Gly Ser Gly Gly
           115                 120                 125
   Thr Gly Ser Gly Thr Gly Gly Ser Thr Glu Ile Val Met Thr Gln Ser
       130                 135                 140
   Pro Gly Thr Leu Ser Leu Ser Pro Gly Glu Arg Ala Thr Leu Ser Cys
   145                 150                 155                 160
   Arg Ala Ser Gln Ser Val Ser Ser Ser Tyr Leu Ala Trp Tyr Gln Gln
                   165                 170                 175
   Lys Pro Gly Gln Ala Pro Arg Leu Leu Ile Tyr Asp Ala Ser Ser Arg
               180                 185                 190
   Ala Thr Asp Ile Pro Asp Arg Phe Ser Gly Ser Gly Ser Gly Thr Asp
           195                 200                 205
   Phe Thr Leu Thr Ile Ser Arg Leu Glu Pro Glu Asp Phe Ala Val Tyr
       210                 215                 220
   Tyr Cys Gln Gln Tyr Gly Ser Ser Leu Trp Thr Phe Gly Gln Gly Thr
   225                 230                 235                 240
   Lys Val Glu Ile Lys Arg
                   245
<210> 218
   <211> 738
   <212> DNA
   <213> Artificial
<220>
   <223> SC06-301
<220>
   <221> CDS
   <222> (1)..(738)
<400> 218
   gag gtg cag ctg gta gag tct ggg gga ggc ttg gta cag cct ggg ggg       48
   Glu Val Gln Leu Val Glu Ser Gly Gly Gly Leu Val Gln Pro Gly Gly
   1               5                   10                  15
   tcc ctg aga ctc tcc tgt gca gcc tct gga ttc acc ttt agc atc tat       96
   Ser Leu Arg Leu Ser Cys Ala Ala Ser Gly Phe Thr Phe Ser Ile Tyr
               20                  25                  30
   gcc atg agc tgg gtc cgc cag gca cca ggg aag ggg ctg gag tgg gtc      144
   Ala Met Ser Trp Val Arg Gln Ala Pro Gly Lys Gly Leu Glu Trp Val
           35                  40                  45
   tca gct att agt agt agt ggt gat agc aca tac tac gca gac tcc gtg      192
   Ser Ala Ile Ser Ser Ser Gly Asp Ser Thr Tyr Tyr Ala Asp Ser Val
       50                  55                  60
   aag ggc cgg ttc acc atc tcc aga gac aac gcc agg aac acg ctg tat      240
   Lys Gly Arg Phe Thr Ile Ser Arg Asp Asn Ala Arg Asn Thr Leu Tyr
   65                  70                  75                  80
   ctg caa atg aac agt ctg aga gcc gag gac acg gct gtg tat tac tgt      288
   Leu Gln Met Asn Ser Leu Arg Ala Glu Asp Thr Ala Val Tyr Tyr Cys
                   85                  90                  95
   gcg aga gcg tat ggc tac acg ttc gac ccc tgg ggc cag gga acc ctg      336
   Ala Arg Ala Tyr Gly Tyr Thr Phe Asp Pro Trp Gly Gln Gly Thr Leu
               100                 105                 110
   gtc acc gtc tcg agc ggt acg ggc ggt tca ggc gga acc ggc agc ggc      384
   Val Thr Val Ser Ser Gly Thr Gly Gly Ser Gly Gly Thr Gly Ser Gly
           115                 120                 125
   act ggc ggg tcg acg gaa att gtg ctg act cag tct cca ctc tcc ctg      432
   Thr Gly Gly Ser Thr Glu Ile Val Leu Thr Gln Ser Pro Leu Ser Leu
       130                 135                 140
   ccc gtc acc cct gga gag ccg gcc tcc atc tcc tgc agg tct agt cag      480
   Pro Val Thr Pro Gly Glu Pro Ala Ser Ile Ser Cys Arg Ser Ser Gln
   145                 150                 155                 160
   agc ctc ctg cat agt aat gga tac aac tat ttg gat tgg tac ctg cag      528
   Ser Leu Leu His Ser Asn Gly Tyr Asn Tyr Leu Asp Trp Tyr Leu Gln
                   165                 170                 175
   aag cca ggg cag tct cca cag ctc ctg atc tat ttg ggt tct aat cgg      576
   Lys Pro Gly Gln Ser Pro Gln Leu Leu Ile Tyr Leu Gly Ser Asn Arg
               180                 185                 190
   gcc tcc ggg gtc cct gac agg ttc agt ggc agt gga tca ggc aca gat      624
   Ala Ser Gly Val Pro Asp Arg Phe Ser Gly Ser Gly Ser Gly Thr Asp
           195                 200                 205
   ttt aca ctg aaa atc agc aga gtg gag gct gag gat gtt ggg gtt tat      672
   Phe Thr Leu Lys Ile Ser Arg Val Glu Ala Glu Asp Val Gly Val Tyr
       210                 215                 220
   tac tgc atg caa gct cta caa act ccc ctc act ttc ggc gga ggg acc      720
   Tyr Cys Met Gln Ala Leu Gln Thr Pro Leu Thr Phe Gly Gly Gly Thr
   225                 230                 235                 240
   aag gtg gag atc aaa cgt                                              738
   Lys Val Glu Ile Lys Arg
                   245
<210> 219
   <211> 246
   <212> PRT
   <213> Artificial
<220>
   <223> Synthetic Construct
<400> 219
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Ser Trp Val Arg Gln Ala Pro Gly Lys Gly Leu Glu Trp Val
           35                  40                  45
   Ser Ala Ile Ser Ser Ser Gly Asp Ser Thr Tyr Tyr Ala Asp Ser Val
       50                  55                  60
   Lys Gly Arg Phe Thr Ile Ser Arg Asp Asn Ala Arg Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Tyr Gly Tyr Thr Phe Asp Pro Trp Gly Gln Gly Thr Leu
               100                 105                 110
   Val Thr Val Ser Ser Gly Thr Gly Gly Ser Gly Gly Thr Gly Ser Gly
           115                 120                 125
   Thr Gly Gly Ser Thr Glu Ile Val Leu Thr Gln Ser Pro Leu Ser Leu
       130                 135                 140
   Pro Val Thr Pro Gly Glu Pro Ala Ser Ile Ser Cys Arg Ser Ser Gln
   145                 150                 155                 160
   Ser Leu Leu His Ser Asn Gly Tyr Asn Tyr Leu Asp Trp Tyr Leu Gln
                   165                 170                 175
   Lys Pro Gly Gln Ser Pro Gln Leu Leu Ile Tyr Leu Gly Ser Asn Arg
               180                 185                 190
   Ala Ser Gly Val Pro Asp Arg Phe Ser Gly Ser Gly Ser Gly Thr Asp
           195                 200                 205
   Phe Thr Leu Lys Ile Ser Arg Val Glu Ala Glu Asp Val Gly Val Tyr
       210                 215                 220
   Tyr Cys Met Gln Ala Leu Gln Thr Pro Leu Thr Phe Gly Gly Gly Thr
   225                 230                 235                 240
   Lys Val Glu Ile Lys Arg
                   245
<210> 220
   <211> 738
   <212> DNA
   <213> Artificial
<220>
   <223> SC06-327
<220>
   <221> CDS
   <222> (1)..(738)
<400> 220
   gag gtg cag ctg gtg gag acc ggg gct gag gtg aag aag cct ggg tcc        48
   Glu Val Gln Leu Val Glu Thr Gly Ala Glu Val Lys Lys Pro Gly Ser
   1               5                   10                  15
   tcg gtg aag gtc tcc tgc aag gcc tct gga ggc acc ttc agg acc cat        96
   Ser Val Lys Val Ser Cys Lys Ala Ser Gly Gly Thr Phe Arg Thr His
               20                  25                  30
   gct atc agt tgg gtg cga cag gcc cct gga caa ggg ctt gag tgg atg       144
   Ala Ile Ser Trp Val Arg Gln Ala Pro Gly Gln Gly Leu Glu Trp Met
           35                  40                  45
   gga ggg atc atc gct atc ttc gga aca gca aac tac gca cag aag ttc       192
   Gly Gly Ile Ile Ala Ile Phe Gly Thr Ala Asn Tyr Ala Gln Lys Phe
       50                  55                  60
   cag ggc aga atc acg att acc gcg gac gaa tcc acg agt aca gcc tac       240
   Gln Gly Arg Ile Thr Ile Thr Ala Asp Glu Ser Thr Ser Thr Ala Tyr
   65                  70                  75                  80
   atg gag ctg agc agc ctg aga tct gag gac acg gcc gtg tat ttc tgt        288
   Met Glu Leu Ser Ser Leu Arg Ser Glu Asp Thr Ala Val Tyr Phe Cys
                   85                  90                  95
   gcg aga ggc agt ggt tat cat ata tcg aca ccc ttt gac aac tgg ggc        336
   Ala Arg Gly Ser Gly Tyr His Ile Ser Thr Pro Phe Asp Asn Trp Gly
               100                 105                 110
   cag gga acc ctg gtc acc gtc tcg agc ggt acg ggc ggt tca ggc gga        384
   Gln Gly Thr Leu Val Thr Val Ser Ser Gly Thr Gly Gly Ser Gly Gly
           115                 120                 125
   acc ggc agc ggc act ggc ggg tcg acg tcc tat gtg ctg act cag cca        432
   Thr Gly Ser Gly Thr Gly Gly Ser Thr Ser Tyr Val Leu Thr Gln Pro
       130                 135                 140
   ccc tcg gtg tca gtg gcc cca gga cag acg gcc agg att acc tgt ggg        480
   Pro Ser Val Ser Val Ala Pro Gly Gln Thr Ala Arg Ile Thr Cys Gly
   145                 150                 155                 160
   gga aac aac att gga agt aaa ggt gtg cac tgg tac cag cag aag cct        528
   Gly Asn Asn Ile Gly Ser Lys Gly Val His Trp Tyr Gln Gln Lys Pro
                   165                 170                 175
   ggc cag gcc cct gtg ctg gtc gtc tat gat gat agc gac cgg ccc tca        576
   Gly Gln Ala Pro Val Leu Val Val Tyr Asp Asp Ser Asp Arg Pro Ser
               180                 185                 190
   ggg atc cct gag cga ttc tct ggc tcc aac tct ggg aac acg gcc acc        624
   Gly Ile Pro Glu Arg Phe Ser Gly Ser Asn Ser Gly Asn Thr Ala Thr
           195                 200                 205
   ctg acc atc agc agg gtc gaa gcc ggg gat gag gcc gac tat tac tgt        672
   Leu Thr Ile Ser Arg Val Glu Ala Gly Asp Glu Ala Asp Tyr Tyr Cys
       210                 215                 220
   cag gtg tgg gat agt agt agt gat cat gtg gta ttc ggc gga ggg acc        720
   Gln Val Trp Asp Ser Ser Ser Asp His Val Val Phe Gly Gly Gly Thr
   225                 230                 235                 240
   aag ctg acc gtc cta ggt                                                738
   Lys Leu Thr Val Leu Gly
                   245
<210> 221
   <211> 246
   <212> PRT
   <213> Artificial
<220>
   <223> Synthetic Construct
<400> 221
   Glu Val Gln Leu Val Glu Thr Gly Ala Glu Val Lys Lys Pro Gly Ser
   1               5                   10                  15
   Ser Val Lys Val Ser Cys Lys Ala Ser Gly Gly Thr Phe Arg Thr His
               20                  25                  30
   Ala Ile Ser Trp Val Arg Gln Ala Pro Gly Gln Gly Leu Glu Trp Met
   35 40 45
   Gly Gly Ile Ile Ala Ile Phe Gly Thr Ala Asn Tyr Ala Gln Lys Phe
   50 55 60
   Gln Gly Arg Ile Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Phe Cys
   85 90 95
   Ala Arg Gly Ser Gly Tyr His Ile Ser Thr Pro Phe Asp Asn Trp Gly
   100 105 110
   Gln Gly Thr Leu Val Thr Val Ser Ser Gly Thr Gly Gly Ser Gly Gly
   115 120 125
   Thr Gly Ser Gly Thr Gly Gly Ser Thr Ser Tyr Val Leu Thr Gln Pro
   130 135 140
   Pro Ser Val Ser Val Ala Pro Gly Gln Thr Ala Arg Ile Thr Cys Gly
   145 150 155 160
   Gly Asn Asn Ile Gly Ser Lys Gly Val His Trp Tyr Gln Gln Lys Pro
   165 170 175
   Gly Gln Ala Pro Val Leu Val Val Tyr Asp Asp Ser Asp Arg Pro Ser
   180 185 190
   Gly Ile Pro Glu Arg Phe Ser Gly Ser Asn Ser Gly Asn Thr Ala Thr
   195 200 205
   Leu Thr Ile Ser Arg Val Glu Ala Gly Asp Glu Ala Asp Tyr Tyr Cys
   210 215 220
   Gln Val Trp Asp Ser Ser Ser Asp His Val Val Phe Gly Gly Gly Thr
   225 230 235 240
   Lys Leu Thr Val Leu Gly
   245
<210> 222
   <211> 756
   <212> DNA
   <213> Artificial
<220>
   <223> SC06-328
<220>
   <221> CDS
   <222> (1)..(756)
<400> 222
   gag gtg cag ctg gtg gag tct ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aag gtc tcc tgc aag gct tct gga cac atc ttc agc ggc tat       96
   Ser Val Lys Val Ser Cys Lys Ala Ser Gly His Ile Phe Ser Gly Tyr
               20                  25                  30
   gca atc agt tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ggg atc atc cct atc ttt ggt aca aca aac tac gca cag aag ttc      192
   Gly Gly Ile Ile Pro Ile Phe Gly Thr Thr Asn Tyr Ala Gln Lys Phe
       50                  55                  60
   cag ggc aga gtc acg att acc gcg gac caa tcc acg agc aca gcc tac      240
   Gln Gly Arg Val Thr Ile Thr Ala Asp Gln Ser Thr Ser Thr Ala Tyr
   65                  70                  75                  80
   atg gac ctg agc aac ttg aga tct gag gac acg gcc gtc tat tac tgt      288
   Met Asp Leu Ser Asn Leu Arg Ser Glu Asp Thr Ala Val Tyr Tyr Cys
                   85                  90                  95
   gcg aga gtg aaa gat gga tat tgt act ctt acc agc tgc cct gtc ggc      336
   Ala Arg Val Lys Asp Gly Tyr Cys Thr Leu Thr Ser Cys Pro Val Gly
               100                 105                 110
   tgg tac ttc gat ctc tgg ggc cgt ggc acc ctg gtc act gtc tcg agc      384
   Trp Tyr Phe Asp Leu Trp Gly Arg Gly Thr Leu Val Thr Val Ser Ser
           115                 120                 125
   ggt acg ggc ggt tca ggc gga acc ggc agc ggc act ggc ggg tcg acg      432
   Gly Thr Gly Gly Ser Gly Gly Thr Gly Ser Gly Thr Gly Gly Ser Thr
       130                 135                 140
   gaa att gtg atg acg cag tct cca ggc acc ctg tct ttg tct cca ggg      480
   Glu Ile Val Met Thr Gln Ser Pro Gly Thr Leu Ser Leu Ser Pro Gly
   145                 150                 155                 160
   gaa aga gcc acc ctc tcg tgc agg gcc agt cag agt gtt agc agc agc      528
   Glu Arg Ala Thr Leu Ser Cys Arg Ala Ser Gln Ser Val Ser Ser Ser
                   165                 170                 175
   tac tta gcc tgg tac cag cag aaa cct ggc cag gct ccc agg ctc ctc      576
   Tyr Leu Ala Trp Tyr Gln Gln Lys Pro Gly Gln Ala Pro Arg Leu Leu
               180                 185                 190
   atc ttt ggt gcc tcc agc agg gcc act ggc atc cca gac agg ttc agt      624
   Ile Phe Gly Ala Ser Ser Arg Ala Thr Gly Ile Pro Asp Arg Phe Ser
           195                 200                 205
   ggc agt ggg tct ggg aca gac ttc act ctc acc atc agc aga ctg gag      672
   Gly Ser Gly Ser Gly Thr Asp Phe Thr Leu Thr Ile Ser Arg Leu Glu
       210                 215                 220
   cct gaa gat ttt gca gtg tat tac tgt cag cag tat ggt agc tca ctc      720
   Pro Glu Asp Phe Ala Val Tyr Tyr Cys Gln Gln Tyr Gly Ser Ser Leu
   225                 230                 235                 240
   act ttc ggc gga ggg acc aag ctg gag atc aaa cgt                      756
   Thr Phe Gly Gly Gly Thr Lys Leu Glu Ile Lys Arg
                   245                 250
<210> 223
   <211> 252
   <212> PRT
   <213> Artificial
<220>
   <223> Synthetic Construct
<400> 223
   Glu Val Gln Leu Val Glu Ser Gly Ala Glu Val Lys Lys Pro Gly Ser
   1               5                   10                  15
   Ser Val Lys Val Ser Cys Lys Ala Ser Gly His Ile Phe Ser Gly Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Thr Asn Tyr Ala Gln Lys Phe
       50                  55                  60
   Gln Gly Arg Val Thr Ile Thr Ala Asp Gln Ser Thr Ser Thr Ala Tyr
   65                  70                  75                  80
   Met Asp Leu Ser Asn Leu Arg Ser Glu Asp Thr Ala Val Tyr Tyr Cys
                   85                  90                  95
   Ala Arg Val Lys Asp Gly Tyr Cys Thr Leu Thr Ser Cys Pro Val Gly
               100                 105                 110
   Trp Tyr Phe Asp Leu Trp Gly Arg Gly Thr Leu Val Thr Val Ser Ser
           115                 120                 125
   Gly Thr Gly Gly Ser Gly Gly Thr Gly Ser Gly Thr Gly Gly Ser Thr
       130                 135                 140
   Glu Ile Val Met Thr Gln Ser Pro Gly Thr Leu Ser Leu Ser Pro Gly
   145                 150                 155                 160
   Glu Arg Ala Thr Leu Ser Cys Arg Ala Ser Gln Ser Val Ser Ser Ser
                   165                 170                 175
   Tyr Leu Ala Trp Tyr Gln Gln Lys Pro Gly Gln Ala Pro Arg Leu Leu
               180                 185                 190
   Ile Phe Gly Ala Ser Ser Arg Ala Thr Gly Ile Pro Asp Arg Phe Ser
           195                 200                 205
   Gly Ser Gly Ser Gly Thr Asp Phe Thr Leu Thr Ile Ser Arg Leu Glu
       210                 215                 220
   Pro Glu Asp Phe Ala Val Tyr Tyr Cys Gln Gln Tyr Gly Ser Ser Leu
   225                 230                 235                 240
   Thr Phe Gly Gly Gly Thr Lys Leu Glu Ile Lys Arg
                   245                 250
<210> 224
   <211> 738
   <212> DNA
   <213> Artificial
<220>
   <223> SC06-329
<220>
   <221> CDS
   <222> (1)..(738)
<400> 224
   gag gtc cag ctg gta cag tct ggg gct gag gtt aag aag cct ggg tcc       48
   Glu Val Gln Leu Val Gln Ser Gly Ala Glu Val Lys Lys Pro Gly Ser
   1               5                   10                  15
   tcg gtg aag gtc tcc tgc aag gct tct gga ggc atc ttc aga agc aat       96
   Ser Val Lys Val Ser Cys Lys Ala Ser Gly Gly Ile Phe Arg Ser Asn
               20                  25                  30
   tct atc agt tgg gtg cga cag gcc cct ggg caa ggg ctt gag tgg atg      144
   Ser Ile Ser Trp Val Arg Gln Ala Pro Gly Gln Gly Leu Glu Trp Met
           35                  40                  45
   gga ggg atc ttc gct ctt ttc gga aca aca gac tac gcg cag aag ttc      192
   Gly Gly Ile Phe Ala Leu Phe Gly Thr Thr Asp Tyr Ala Gln Lys Phe
       50                  55                  60
   cag ggc aga gtc acg att acc gcg gac gaa tct tcg acc aca gtc tac      240
   Gln Gly Arg Val Thr Ile Thr Ala Asp Glu Ser Ser Thr Thr Val Tyr
   65                  70                  75                  80
   ctg gag ctg agt agc ctg aca tct gag gac acg gcc gtt tat tac tgt      288
   Leu Glu Leu Ser Ser Leu Thr Ser Glu Asp Thr Ala Val Tyr Tyr Cys
                   85                  90                  95
   gcg aga ggc agt ggc tac acc aca cgc aac tac ttt gac tac tgg ggc      336
   Ala Arg Gly Ser Gly Tyr Thr Thr Arg Asn Tyr Phe Asp Tyr Trp Gly
               100                 105                 110
   cag ggc acc ctg gtc acc gtc tcg agc ggt acg ggc ggt tca ggc gga      384
   Gln Gly Thr Leu Val Thr Val Ser Ser Gly Thr Gly Gly Ser Gly Gly
           115                 120                 125
   acc ggc agc ggc act ggc ggg tcg acg gaa att gtg ctg act cag tct      432
   Thr Gly Ser Gly Thr Gly Gly Ser Thr Glu Ile Val Leu Thr Gln Ser
       130                 135                 140
   cca ggc acc ctg tct ttg tct cca ggg gaa aga gcc aca ctc tcc tgc      480
   Pro Gly Thr Leu Ser Leu Ser Pro Gly Glu Arg Ala Thr Leu Ser Cys
   145                 150                 155                 160
   agg gcc agt cag agt gtt agc agc aac tac tta ggc tgg tac cag cag       528
   Arg Ala Ser Gln Ser Val Ser Ser Asn Tyr Leu Gly Trp Tyr Gln Gln
                   165                 170                 175
   aaa cct ggc cag gct ccc agg ctc ctg atc tat ggt gca tcc agc agg       576
   Lys Pro Gly Gln Ala Pro Arg Leu Leu Ile Tyr Gly Ala Ser Ser Arg
               180                 185                 190
   gcc agt ggc atc cca gac agg ttc agt ggc ggt ggg tct ggg aca gac       624
   Ala Ser Gly Ile Pro Asp Arg Phe Ser Gly Gly Gly Ser Gly Thr Asp
           195                 200                 205
   ttc act ctc acc atc agc aga ctg gag cct gaa gat ttt gca gtg tat       672
   Phe Thr Leu Thr Ile Ser Arg Leu Glu Pro Glu Asp Phe Ala Val Tyr
       210                 215                 220
   tac tgt cag cag tat ggt agc tca ccc ctc act ttc ggc gga ggg acc       720
   Tyr Cys Gln Gln Tyr Gly Ser Ser Pro Leu Thr Phe Gly Gly Gly Thr
   225                 230                 235                 240
   aag gtg gag atc aaa cgt                                               738
   Lys Val Glu Ile Lys Arg
                   245
<210> 225
   <211> 246
   <212> PRT
   <213> Artificial
<220>
   <223> Synthetic Construct
<400> 225
   Glu Val Gln Leu Val Gln Ser Gly Ala Glu Val Lys Lys Pro Gly Ser
   1               5                   10                  15
   Ser Val Lys Val Ser Cys Lys Ala Ser Gly Gly Ile Phe Arg Ser Asn
               20                  25                  30
   Ser Ile Ser Trp Val Arg Gln Ala Pro Gly Gln Gly Leu Glu Trp Met
           35                  40                  45
   Gly Gly Ile Phe Ala Leu Phe Gly Thr Thr Asp Tyr Ala Gln Lys Phe
       50                  55                  60
   Gln Gly Arg Val Thr Ile Thr Ala Asp Glu Ser Ser Thr Thr Val Tyr
   65                  70                  75                  80
   Leu Glu Leu Ser Ser Leu Thr Ser Glu Asp Thr Ala Val Tyr Tyr Cys
                   85                  90                  95
   Ala Arg Gly Ser Gly Tyr Thr Thr Arg Asn Tyr Phe Asp Tyr Trp Gly
               100                 105                 110
   Gln Gly Thr Leu Val Thr Val Ser Ser Gly Thr Gly Gly Ser Gly Gly
           115                 120                 125
   Thr Gly Ser Gly Thr Gly Gly Ser Thr Glu Ile Val Leu Thr Gln Ser
       130                 135                 140
   Pro Gly Thr Leu Ser Leu Ser Pro Gly Glu Arg Ala Thr Leu Ser Cys
   145                 150                 155                 160
   Arg Ala Ser Gln Ser Val Ser Ser Asn Tyr Leu Gly Trp Tyr Gln Gln
                   165                 170                 175
   Lys Pro Gly Gln Ala Pro Arg Leu Leu Ile Tyr Gly Ala Ser Ser Arg
               180                 185                 190
   Ala Ser Gly Ile Pro Asp Arg Phe Ser Gly Gly Gly Ser Gly Thr Asp
           195                 200                 205
   Phe Thr Leu Thr Ile Ser Arg Leu Glu Pro Glu Asp Phe Ala Val Tyr
       210                 215                 220
   Tyr Cys Gln Gln Tyr Gly Ser Ser Pro Leu Thr Phe Gly Gly Gly Thr
   225                 230                 235                 240
   Lys Val Glu Ile Lys Arg
                   245
<210> 226
   <211> 729
   <212> DNA
   <213> Artificial
<220>
   <223> SC06-332
<220>
   <221> CDS
   <222> (1)..(729)
<400> 226
   cag gtg cag ctg gtg cag tct ggg gct gag gtg aag aag cct ggg tcc       48
   Gln Val Gln Leu Val Gln Ser Gly Ala Glu Val Lys Lys Pro Gly Ser
   1               5                   10                  15
   tcg gta aag gtc tcc tgc aag gct tct gga ggc ccc ttc cgc aat ttt       96
   Ser Val Lys Val Ser Cys Lys Ala Ser Gly Gly Pro Phe Arg Asn Phe
               20                  25                  30
   gct atc aac tgg gtg cga cag gcc cct gga caa ggg ctt gag tgg atg      144
   Ala Ile Asn Trp Val Arg Gln Ala Pro Gly Gln Gly Leu Glu Trp Met
           35                  40                  45
   gga ggg atc atc gct gtc ttt ggg acg aca aag tac gca cat aag ttc      192
   Gly Gly Ile Ile Ala Val Phe Gly Thr Thr Lys Tyr Ala His Lys Phe
       50                  55                  60
   cag ggc aga gtc acc atc acc gcg gac gac tcc aca aat aca gct tac      240
   Gln Gly Arg Val Thr Ile Thr Ala Asp Asp Ser Thr Asn Thr Ala Tyr
   65                  70                  75                  80
   atg gag ctg ggc agc ctg aaa tct gag gac acg gcc gtg tat tac tgt      288
   Met Glu Leu Gly Ser Leu Lys Ser Glu Asp Thr Ala Val Tyr Tyr Cys
                   85                  90                  95
   gcg aga ggt ccc cac tac tac tcc tcc tac atg gac gtc tgg ggc gaa      336
   Ala Arg Gly Pro His Tyr Tyr Ser Ser Tyr Met Asp Val Trp Gly Glu
               100                 105                 110
   ggg acc acg gtc acc gtc tcg agc ggt acg ggc ggt tca ggc gga acc      384
   Gly Thr Thr Val Thr Val Ser Ser Gly Thr Gly Gly Ser Gly Gly Thr
           115                 120                 125
   ggc agc ggc act ggc ggg tcg acg gac atc cag ttg acc cag tct cca      432
   Gly Ser Gly Thr Gly Gly Ser Thr Asp Ile Gln Leu Thr Gln Ser Pro
       130                 135                 140
   tcc tcc ctg tct gca tct gta gga gac aga gtc acc atc act tgc cgg      480
   Ser Ser Leu Ser Ala Ser Val Gly Asp Arg Val Thr Ile Thr Cys Arg
   145                 150                 155                 160
   gcg agt cag ggc att agc act tat tta gcc tgg tat cag cag aaa ccc      528
   Ala Ser Gln Gly Ile Ser Thr Tyr Leu Ala Trp Tyr Gln Gln Lys Pro
                   165                 170                 175
   ggg aaa gtt cct aaa ctc ctg atc tat gct gca tcc act ttg caa tca      576
   Gly Lys Val Pro Lys Leu Leu Ile Tyr Ala Ala Ser Thr Leu Gln Ser
               180                 185                 190
   ggg gtc cca tct cgg ttc agt ggc agt gga tct ggg aca gat ttc act      624
   Gly Val Pro Ser Arg Phe Ser Gly Ser Gly Ser Gly Thr Asp Phe Thr
           195                 200                 205
   ctc acc atc agc agc ctg cag cct gaa gat gtt gca act tat tac tgt      672
   Leu Thr Ile Ser Ser Leu Gln Pro Glu Asp Val Ala Thr Tyr Tyr Cys
       210                 215                 220
   caa aag tat aac agt gcc cct tct ttc ggc cct ggg acc aaa gtg gat      720
   Gln Lys Tyr Asn Ser Ala Pro Ser Phe Gly Pro Gly Thr Lys Val Asp
   225                 230                 235                 240
   atc aaa cgt                                                          729
   Ile Lys Arg
<210> 227
   <211> 243
   <212> PRT
   <213> Artificial
<220>
   <223> Synthetic Construct
<400> 227
   Gln Val Gln Leu Val Gln Ser Gly Ala Glu Val Lys Lys Pro Gly Ser
   1 5 10 15
   Ser Val Lys Val Ser Cys Lys Ala Ser Gly Gly Pro Phe Arg Asn Phe
                   20                  25                  30
   Ala Ile Asn Trp Val Arg Gln Ala Pro Gly Gln Gly Leu Glu Trp Met
           35                  40                  45
   Gly Gly Ile Ile Ala Val Phe Gly Thr Thr Lys Tyr Ala His Lys Phe
       50                  55                  60
   Gln Gly Arg Val Thr Ile Thr Ala Asp Asp Ser Thr Asn Thr Ala Tyr
   65                  70                  75                  80
   Met Glu Leu Gly Ser Leu Lys Ser Glu Asp Thr Ala Val Tyr Tyr Cys
                   85                  90                  95
   Ala Arg Gly Pro His Tyr Tyr Ser Ser Tyr Met Asp Val Trp Gly Glu
               100                 105                 110
   Gly Thr Thr Val Thr Val Ser Ser Gly Thr Gly Gly Ser Gly Gly Thr
           115                 120                 125
   Gly Ser Gly Thr Gly Gly Ser Thr Asp Ile Gln Leu Thr Gln Ser Pro
       130                 135                 140
   Ser Ser Leu Ser Ala Ser Val Gly Asp Arg Val Thr Ile Thr Cys Arg
   145                 150                 155                 160
   Ala Ser Gln Gly Ile Ser Thr Tyr Leu Ala Trp Tyr Gln Gln Lys Pro
                   165                 170                 175
   Gly Lys Val Pro Lys Leu Leu Ile Tyr Ala Ala Ser Thr Leu Gln Ser
               180                 185                 190
   Gly Val Pro Ser Arg Phe Ser Gly Ser Gly Ser Gly Thr Asp Phe Thr
           195                 200                 205
   Leu Thr Ile Ser Ser Leu Gln Pro Glu Asp Val Ala Thr Tyr Tyr Cys
       210                 215                 220
   Gln Lys Tyr Asn Ser Ala Pro Ser Phe Gly Pro Gly Thr Lys Val Asp
   225                 230                 235                 240
   Ile Lys Arg
<210> 228
   <211> 735
   <212> DNA
   <213> Artificial
<220>
   <223> SC06-334
<220>
   <221> CDS
   <222> (1)..(735)
<400> 228
   gag gtg cag ctg gtg gag act ggg gct gag gtg aag aag cct ggg tcc      48
   Glu Val Gln Leu Val Glu Thr Gly Ala Glu Val Lys Lys Pro Gly Ser
   1               5                   10                  15
   tcg gtg aag gtc ccc tgc aaa tct tct gga agc ccc ttc agg agt aat      96
   Ser Val Lys Val Pro Cys Lys Ser Ser Gly Ser Pro Phe Arg Ser Asn
               20                  25                  30
   gct gtc agc tgg gtg cga cag gcc ccc gga caa ggg ctt gag tgg gtg     144
   Ala Val Ser Trp Val Arg Gln Ala Pro Gly Gln Gly Leu Glu Trp Val
           35                  40                  45
   gga gga atc ctc ggt gtc ttt ggt tca cca agc tac gca cag aag ttc     192
   Gly Gly Ile Leu Gly Val Phe Gly Ser Pro Ser Tyr Ala Gln Lys Phe
       50                  55                  60
   cag ggc aga gtc acg att acc gcg gac gaa tcc acc aac aca gtc cac     240
   Gln Gly Arg Val Thr Ile Thr Ala Asp Glu Ser Thr Asn Thr Val His
   65                  70                  75                  80
   atg gag ctg aga ggt ttg aga tct gag gac acg gcc gtg tat tat tgt     288
   Met Glu Leu Arg Gly Leu Arg Ser Glu Asp Thr Ala Val Tyr Tyr Cys
                   85                  90                  95
   gcg aga ggt cct acc tac tac tac tcc tac atg gac gtc tgg ggc aaa     336
   Ala Arg Gly Pro Thr Tyr Tyr Tyr Ser Tyr Met Asp Val Trp Gly Lys
               100                 105                 110
   ggg acc acg gtc acc gtc tcg agc ggt acg ggc ggt tca ggc gga acc     384
   Gly Thr Thr Val Thr Val Ser Ser Gly Thr Gly Gly Ser Gly Gly Thr
           115                 120                 125
   ggc agc ggc act ggc ggg tcg acg tcc tat gtg ctg act cag cca ccc     432
   Gly Ser Gly Thr Gly Gly Ser Thr Ser Tyr Val Leu Thr Gln Pro Pro
       130                 135                 140
   tcg gag tca gtg gcc cca gga cag acg gcc agg att acc tgt ggg gga     480
   Ser Glu Ser Val Ala Pro Gly Gln Thr Ala Arg Ile Thr Cys Gly Gly
   145                 150                 155                 160
   aat aac att gga aga aat agt gtg cac tgg tat cag cag aag cca ggc     528
   Asn Asn Ile Gly Arg Asn Ser Val His Trp Tyr Gln Gln Lys Pro Gly
                   165                 170                 175
   cag gcc cct gtg ctg gtc gtg tat gat gat agc gac cgg ccc tca ggg     576
   Gln Ala Pro Val Leu Val Val Tyr Asp Asp Ser Asp Arg Pro Ser Gly
               180                 185                 190
   atc cct gag cga ttt tct ggc tcc aag tct ggg aac acg gcc acc ctg     624
   Ile Pro Glu Arg Phe Ser Gly Ser Lys Ser Gly Asn Thr Ala Thr Leu
           195                 200                 205
   att atc agc agg gtc gaa gtc ggg gat gag gcc gac tac tac tgt cag     672
   Ile Ile Ser Arg Val Glu Val Gly Asp Glu Ala Asp Tyr Tyr Cys Gln
       210                 215                 220
   gtg tgg cat agt agt agt gat cat tat gtc ttc gga act ggg acc aag     720
   Val Trp His Ser Ser Ser Asp His Tyr Val Phe Gly Thr Gly Thr Lys
   225                 230                 235                 240
   gtc acc gtc cta ggt                                                  735
   Val Thr Val Leu Gly
                   245
<210> 229
   <211> 245
   <212> PRT
   <213> Artificial
<220>
   <223> Synthetic Construct
<400> 229
   Glu Val Gln Leu Val Glu Thr Gly Ala Glu Val Lys Lys Pro Gly Ser
   1 5 10 15
   Ser Val Lys Val Pro Cys Lys Ser Ser Gly Ser Pro Phe Arg Ser Asn
   20 25 30
   Ala Val Ser Trp Val Arg Gln Ala Pro Gly Gln Gly Leu Glu Trp Val
   35 40 45
   Gly Gly Ile Leu Gly Val Phe Gly Ser Pro Ser Tyr Ala Gln Lys Phe
   50 55 60
   Gln Gly Arg Val Thr Ile Thr Ala Asp Glu Ser Thr Asn Thr Val His
   65 70 75 80
   Met Glu Leu Arg Gly Leu Arg Ser Glu Asp Thr Ala Val Tyr Tyr Cys
   85 90 95
   Ala Arg Gly Pro Thr Tyr Tyr Tyr Ser Tyr Met Asp Val Trp Gly Lys
   100 105 110
   Gly Thr Thr Val Thr Val Ser Ser Gly Thr Gly Gly Ser Gly Gly Thr
   115 120 125
   Gly Ser Gly Thr Gly Gly Ser Thr Ser Tyr Val Leu Thr Gln Pro Pro
   130 135 140
   Ser Glu Ser Val Ala Pro Gly Gln Thr Ala Arg Ile Thr Cys Gly Gly
   145 150 155 160
   Asn Asn Ile Gly Arg Asn Ser Val His Trp Tyr Gln Gln Lys Pro Gly
   165 170 175
   Gln Ala Pro Val Leu Val Val Tyr Asp Asp Ser Asp Arg Pro Ser Gly
   180 185 190
   Ile Pro Glu Arg Phe Ser Gly Ser Lys Ser Gly Asn Thr Ala Thr Leu
   195 200 205
   Ile Ile Ser Arg Val Glu Val Gly Asp Glu Ala Asp Tyr Tyr Cys Gln
   210 215 220
   Val Trp His Ser Ser Ser Asp His Tyr Val Phe Gly Thr Gly Thr Lys
   225 230 235 240
   Val Thr Val Leu Gly
   245
<210> 230
   <211> 735
   <212> DNA
   <213> Artificial
<220>
   <223> SC06-336
<220>
   <221> CDS
   <222> (1)..(735)
<400> 230
   cag atg cag ctg gta caa tct gga gct gag gtg aag aag cct ggg tcc       48
   Gln Met Gln Leu Val Gln Ser Gly Ala Glu Val Lys Lys Pro Gly Ser
   1               5                   10                  15
   tcg gtg aag gtc tcc tgc aag gct tct gga ggc acc ttc agc agc tat       96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ggg atc ttc ggt atg ttt ggg aca gca aac tac gcg cag aag ttc      192
   Gly Gly Ile Phe Gly Met Phe Gly Thr Ala Asn Tyr Ala Gln Lys Phe
       50                  55                  60
   cag ggc aga gtc acg att acc gcg gac gaa ttc acg agc gcg gcc tac      240
   Gln Gly Arg Val Thr Ile Thr Ala Asp Glu Phe Thr Ser Ala Ala Tyr
   65                  70                  75                  80
   atg gag ctg agc agc ctg gga tct gag gac acg gcc atg tat tac tgt      288
   Met Glu Leu Ser Ser Leu Gly Ser Glu Asp Thr Ala Met Tyr Tyr Cys
                   85                  90                  95
   gcg agg tct agt ggt tat tac ccc caa tac ttc cag gac tgg ggc cag      336
   Ala Arg Ser Ser Gly Tyr Tyr Pro Gln Tyr Phe Gln Asp Trp Gly Gln
               100                 105                 110
   ggc acc ctg gtc acc gtc tcg agc ggt acg ggc ggt tca ggc gga acc      384
   Gly Thr Leu Val Thr Val Ser Ser Gly Thr Gly Gly Ser Gly Gly Thr
           115                 120                 125
   ggc agc ggc act ggc ggg tcg acg gaa att gtg atg aca cag tct cca      432
   Gly Ser Gly Thr Gly Gly Ser Thr Glu Ile Val Met Thr Gln Ser Pro
       130                 135                 140
   ggc acc ctg tct ttg tct cca ggg caa aga gcc acc ctc tcc tgc agg      480
   Gly Thr Leu Ser Leu Ser Pro Gly Gln Arg Ala Thr Leu Ser Cys Arg
   145                 150                 155                 160
   gcc agt cag agt gtt agc agc agc tac tta gcc tgg tac cag cag aaa      528
   Ala Ser Gln Ser Val Ser Ser Ser Tyr Leu Ala Trp Tyr Gln Gln Lys
                   165                 170                 175
   cct ggc cag gct ccc aga ctc ctc atg tat ggt gca tcc agc agg gcc      576
   Pro Gly Gln Ala Pro Arg Leu Leu Met Tyr Gly Ala Ser Ser Arg Ala
               180                 185                 190
   act ggc atc cca gac agg ttc agt ggc agt ggg tct ggg aca gac ttc      624
   Thr Gly Ile Pro Asp Arg Phe Ser Gly Ser Gly Ser Gly Thr Asp Phe
           195                 200                 205
   act ctc acc atc agc aga ctg gag cct gaa gat ttt gca gtg tat tac      672
   Thr Leu Thr Ile Ser Arg Leu Glu Pro Glu Asp Phe Ala Val Tyr Tyr
       210                 215                 220
   tgt cag cag tat ggt agc tca tcg ctc act ttc ggc gga ggg acc aag      720
   Cys Gln Gln Tyr Gly Ser Ser Ser Leu Thr Phe Gly Gly Gly Thr Lys
   225                 230                 235                 240
   ctg gag atc aaa cgt                                                  735
   Leu Glu Ile Lys Arg
                   245
<210> 231
   <211> 245
   <212> PRT
   <213> Artificial
<220>
   <223> Synthetic Construct
<400> 231
   Gln Met Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Phe Gly Met Phe Gly Thr Ala Asn Tyr Ala Gln Lys Phe
       50                  55                  60
   Gln Gly Arg Val Thr Ile Thr Ala Asp Glu Phe Thr Ser Ala Ala Tyr
   65                  70                  75                  80
   Met Glu Leu Ser Ser Leu Gly Ser Glu Asp Thr Ala Met Tyr Tyr Cys
                   85                  90                  95
   Ala Arg Ser Ser Gly Tyr Tyr Pro Gln Tyr Phe Gln Asp Trp Gly Gln
               100                 105                 110
   Gly Thr Leu Val Thr Val Ser Ser Gly Thr Gly Gly Ser Gly Gly Thr
           115                 120                 125
   Gly Ser Gly Thr Gly Gly Ser Thr Glu Ile Val Met Thr Gln Ser Pro
       130                 135                 140
   Gly Thr Leu Ser Leu Ser Pro Gly Gln Arg Ala Thr Leu Ser Cys Arg
   145                 150                 155                 160
   Ala Ser Gln Ser Val Ser Ser Ser Tyr Leu Ala Trp Tyr Gln Gln Lys
                   165                 170                 175
   Pro Gly Gln Ala Pro Arg Leu Leu Met Tyr Gly Ala Ser Ser Arg Ala
               180                 185                 190
   Thr Gly Ile Pro Asp Arg Phe Ser Gly Ser Gly Ser Gly Thr Asp Phe
           195                 200                 205
   Thr Leu Thr Ile Ser Arg Leu Glu Pro Glu Asp Phe Ala Val Tyr Tyr
       210                 215                 220
   Cys Gln Gln Tyr Gly Ser Ser Ser Leu Thr Phe Gly Gly Gly Thr Lys
   225                 230                 235                 240
   Leu Glu Ile Lys Arg
                   245
<210> 232
   <211> 738
   <212> DNA
   <213> Artificial
<220>
   <223> SC06-339
<220>
   <221> CDS
   <222> (1)..(738)
<400> 232
   gag gtg cag ctg gtg gag tcc ggg gct gag gtg aag aag cct ggg tcc       48
   Glu Val Gln Leu Val Glu Ser Gly Ala Glu Val Lys Lys Pro Gly Ser
   1               5                   10                  15
   tcg gtg aag gtc tcc tgc aag gct tct gga ggc atc ttc aac agt tat       96
   Ser Val Lys Val Ser Cys Lys Ala Ser Gly Gly Ile Phe Asn Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ggc atc atc gct atc ttt cat aca cca aag tac gca cag aag ttc      192
   Gly Gly Ile Ile Ala Ile Phe His Thr Pro Lys Tyr Ala Gln Lys Phe
       50                  55                  60
   cag ggc aga gtc acg att acc gcg gac gaa tcc acg aac aca gcc tac      240
   Gln Gly Arg Val Thr Ile Thr Ala Asp Glu Ser Thr Asn Thr Ala Tyr
   65                  70                  75                  80
   atg gaa ctg aga agc ctg aaa tct gag gac acg gcc ctg tat tac tgt      288
   Met Glu Leu Arg Ser Leu Lys Ser Glu Asp Thr Ala Leu Tyr Tyr Cys
                   85                  90                  95
   gcg aga ggg tcc act tac gat ttt tcg agt ggc ctt gac tac tgg ggc      336
   Ala Arg Gly Ser Thr Tyr Asp Phe Ser Ser Gly Leu Asp Tyr Trp Gly
               100                 105                 110
   cag gga acc ctg gtc acc gtc tcg agc ggt acg ggc ggt tca ggc gga      384
   Gln Gly Thr Leu Val Thr Val Ser Ser Gly Thr Gly Gly Ser Gly Gly
           115                 120                 125
   acc ggc agc ggc act ggc ggg tcg acg cag gca ggg ctg act cag cca      432
   Thr Gly Ser Gly Thr Gly Gly Ser Thr Gln Ala Gly Leu Thr Gln Pro
       130                 135                 140
   ccc tcg gtg tca gtg gcc cca gga cag acg gcc agg att acc tgt ggg      480
   Pro Ser Val Ser Val Ala Pro Gly Gln Thr Ala Arg Ile Thr Cys Gly
   145                 150                 155                 160
   gga aac aac att gga agt aaa agt gtg cac tgg tac cag cag aag cca      528
   Gly Asn Asn Ile Gly Ser Lys Ser Val His Trp Tyr Gln Gln Lys Pro
                   165                 170                 175
   ggc cag gcc cct gtc cta gtc gtc tat gat gat agc gac cgg ccc tca      576
   Gly Gln Ala Pro Val Leu Val Val Tyr Asp Asp Ser Asp Arg Pro Ser
               180                 185                 190
   ggg atc cct gag cga ttc tct ggc tcc aac tct ggg aac acg gcc acc      624
   Gly Ile Pro Glu Arg Phe Ser Gly Ser Asn Ser Gly Asn Thr Ala Thr
           195                 200                 205
   ctg acc atc agc agg gtc gaa gcc ggg gat gag gcc gac tat tac tgt      672
   Leu Thr Ile Ser Arg Val Glu Ala Gly Asp Glu Ala Asp Tyr Tyr Cys
       210                 215                 220
   cag gtg tgg gat agt agt agt gat cat gtg gta ttc ggc gga ggg acc      720
   Gln Val Trp Asp Ser Ser Ser Asp His Val Val Phe Gly Gly Gly Thr
   225                 230                 235                 240
   aag ctg acc gtc cta ggt                                              738
   Lys Leu Thr Val Leu Gly
                   245
<210> 233
   <211> 246
   <212> PRT
   <213> Artificial
<220>
   <223> Synthetic Construct
<400> 233
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Ile Phe Asn Ser Tyr
               20                  25                  30
   Ala Ile Ser Trp Val Arg Gln Ala Pro Gly Gln Gly Leu Glu Trp Met
           35                  40                  45
   Gly Gly Ile Ile Ala Ile Phe His Thr Pro Lys Tyr Ala Gln Lys Phe
       50                  55                  60
   Gln Gly Arg Val Thr Ile Thr Ala Asp Glu Ser Thr Asn Thr Ala Tyr
   65                  70                  75                  80
   Met Glu Leu Arg Ser Leu Lys Ser Glu Asp Thr Ala Leu Tyr Tyr Cys
                   85                  90                  95
   Ala Arg Gly Ser Thr Tyr Asp Phe Ser Ser Gly Leu Asp Tyr Trp Gly
               100                 105                 110
   Gln Gly Thr Leu Val Thr Val Ser Ser Gly Thr Gly Gly Ser Gly Gly
           115                 120                 125
   Thr Gly Ser Gly Thr Gly Gly Ser Thr Gln Ala Gly Leu Thr Gln Pro
       130                 135                 140
   Pro Ser Val Ser Val Ala Pro Gly Gln Thr Ala Arg Ile Thr Cys Gly
   145                 150                 155                 160
   Gly Asn Asn Ile Gly Ser Lys Ser Val His Trp Tyr Gln Gln Lys Pro
                   165                 170                 175
   Gly Gln Ala Pro Val Leu Val Val Tyr Asp Asp Ser Asp Arg Pro Ser
               180                 185                 190
   Gly Ile Pro Glu Arg Phe Ser Gly Ser Asn Ser Gly Asn Thr Ala Thr
           195                 200                 205
   Leu Thr Ile Ser Arg Val Glu Ala Gly Asp Glu Ala Asp Tyr Tyr Cys
       210                 215                 220
   Gln Val Trp Asp Ser Ser Ser Asp His Val Val Phe Gly Gly Gly Thr
   225                 230                 235                 240
   Lys Leu Thr Val Leu Gly
                   245
<210> 234
   <211> 768
   <212> DNA
   <213> Artificial
<220>
   <223> SC06-342
<220>
   <221> CDS
   <222> (1)..(768)
<400> 234
   cag gtc cag ctg gtg cag tct ggg gct gag gtg aag aag cct ggg tcc        48
   Gln Val Gln Leu Val Gln Ser Gly Ala Glu Val Lys Lys Pro Gly Ser
   1               5                   10                  15
   tcg gtg aag gtc tcc tgc aag gct tct gga ggc ttc ttc agc agc tat        96
   Ser Val Lys Val Ser Cys Lys Ala Ser Gly Gly Phe Phe Ser Ser Tyr
               20                  25                  30
   gct atc agc tgg gtg cgc cag gcc cct gga caa gga ctt gag tgg atg       144
   Ala Ile Ser Trp Val Arg Gln Ala Pro Gly Gln Gly Leu Glu Trp Met
           35                  40                  45
   ggg ggg gtc atc cct atc ttt cgt aca gca aac tac gca cag aac ttc       192
   Gly Gly Val Ile Pro Ile Phe Arg Thr Ala Asn Tyr Ala Gln Asn Phe
       50                  55                  60
   cag ggc aga gtc acc att acc gcg gac gaa ttc aca tcg tat atg gag       240
   Gln Gly Arg Val Thr Ile Thr Ala Asp Glu Phe Thr Ser Tyr Met Glu
   65                  70                  75                  80
   ctg agc agc ctg aga tct gac gac acg gcc gtg tat tac tgt gcg agg       288
   Leu Ser Ser Leu Arg Ser Asp Asp Thr Ala Val Tyr Tyr Cys Ala Arg
                   85                  90                  95
   ttg aat tac cat gat tcg ggg act tat tat aac gcc ccc cgg ggc tgg       336
   Leu Asn Tyr His Asp Ser Gly Thr Tyr Tyr Asn Ala Pro Arg Gly Trp
               100                 105                 110
   ttc gac ccc tgg ggc cag gga acc ctg gtc acc gtc tcg agc ggt acg       384
   Phe Asp Pro Trp Gly Gln Gly Thr Leu Val Thr Val Ser Ser Gly Thr
           115                 120                 125
   ggc ggt tca ggc gga acc ggc agc ggc act ggc ggg tcg acg gac atc       432
   Gly Gly Ser Gly Gly Thr Gly Ser Gly Thr Gly Gly Ser Thr Asp Ile
       130                 135                 140
   cag atg acc cag tct cca gac tcc ctg gct gtg tct ctg ggc gag aag       480
   Gln Met Thr Gln Ser Pro Asp Ser Leu Ala Val Ser Leu Gly Glu Lys
   145                 150                 155                 160
   gcc acc atc aac tgc aag tcc agc cag agt att tta aac agc tcc aac       528
   Ala Thr Ile Asn Cys Lys Ser Ser Gln Ser Ile Leu Asn Ser Ser Asn
                   165                 170                 175
   aat aag aac tac tta gct tgg tac cag cag aaa cca gga cag cct cct       576
   Asn Lys Asn Tyr Leu Ala Trp Tyr Gln Gln Lys Pro Gly Gln Pro Pro
               180                 185                 190
   aag ctg ctc att tac tgg gca tct acc cgg gaa tcc ggg gtc cct gac       624
   Lys Leu Leu Ile Tyr Trp Ala Ser Thr Arg Glu Ser Gly Val Pro Asp
           195                 200                 205
   cga ttc agt ggc agc ggg tct ggg aca gat ttc act ctc acc atc agc       672
   Arg Phe Ser Gly Ser Gly Ser Gly Thr Asp Phe Thr Leu Thr Ile Ser
       210                 215                 220
   agc ctg cag gct gaa gat gtg gca gtt tat tac tgt cag caa tat tat      720
   Ser Leu Gln Ala Glu Asp Val Ala Val Tyr Tyr Cys Gln Gln Tyr Tyr
   225                 230                 235                 240
   agt agt ccg ccg acg ttc ggc caa ggg acc aag gtg gaa atc aaa cgt      768
   Ser Ser Pro Pro Thr Phe Gly Gln Gly Thr Lys Val Glu Ile Lys Arg
                   245                 250                 255
<210> 235
   <211> 256
   <212> PRT
   <213> Artificial
<220>
   <223> Synthetic Construct
<400> 235
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Phe Phe Ser Ser Tyr
               20                  25                  30
   Ala Ile Ser Trp Val Arg Gln Ala Pro Gly Gln Gly Leu Glu Trp Met
           35                  40                  45
   Gly Gly Val Ile Pro Ile Phe Arg Thr Ala Asn Tyr Ala Gln Asn Phe
       50                  55                  60
   Gln Gly Arg Val Thr Ile Thr Ala Asp Glu Phe Thr Ser Tyr Met Glu
   65                  70                  75                  80
   Leu Ser Ser Leu Arg Ser Asp Asp Thr Ala Val Tyr Tyr Cys Ala Arg
                   85                  90                  95
   Leu Asn Tyr His Asp Ser Gly Thr Tyr Tyr Asn Ala Pro Arg Gly Trp
               100                 105                 110
   Phe Asp Pro Trp Gly Gln Gly Thr Leu Val Thr Val Ser Ser Gly Thr
           115                 120                 125
   Gly Gly Ser Gly Gly Thr Gly Ser Gly Thr Gly Gly Ser Thr Asp Ile
       130                 135                 140
   Gln Met Thr Gln Ser Pro Asp Ser Leu Ala Val Ser Leu Gly Glu Lys
   145                 150                 155                 160
   Ala Thr Ile Asn Cys Lys Ser Ser Gln Ser Ile Leu Asn Ser Ser Asn
                   165                 170                 175
   Asn Lys Asn Tyr Leu Ala Trp Tyr Gln Gln Lys Pro Gly Gln Pro Pro
               180                 185                 190
   Lys Leu Leu Ile Tyr Trp Ala Ser Thr Arg Glu Ser Gly Val Pro Asp
           195                 200                 205
   Arg Phe Ser Gly Ser Gly Ser Gly Thr Asp Phe Thr Leu Thr Ile Ser
       210                 215                 220
   Ser Leu Gln Ala Glu Asp Val Ala Val Tyr Tyr Cys Gln Gln Tyr Tyr
   225                 230                 235                 240
   Ser Ser Pro Pro Thr Phe Gly Gln Gly Thr Lys Val Glu Ile Lys Arg
                   245                 250                 255
<210> 236
   <211> 735
   <212> DNA
   <213> Artificial
<220>
   <223> SC06-343
<220>
   <221> CDS
   <222> (1)..(735)
<400> 236
   cag gtc cag ctg gtg cag tct gga gct gag gtg aag aag cct ggg tcc        48
   Gln Val Gln Leu Val Gln Ser Gly Ala Glu Val Lys Lys Pro Gly Ser
   1               5                   10                  15
   tcg gtg aag gtc tcc tgc aag gct tct gga gtc acc ttc agt tac tat        96
   Ser Val Lys Val Ser Cys Lys Ala Ser Gly Val Thr Phe Ser Tyr Tyr
               20                  25                  30
   gct atg agc tgg gtg cga cag gcc cct gga caa ggg ctt gag tgg atg       144
   Ala Met Ser Trp Val Arg Gln Ala Pro Gly Gln Gly Leu Glu Trp Met
           35                  40                  45
   gga gga atc agc cct atg ttt ggg aca aca acc tac gca cag aag ttc       192
   Gly Gly Ile Ser Pro Met Phe Gly Thr Thr Thr Tyr Ala Gln Lys Phe
       50                  55                  60
   cag ggc aga gtc acg att act gcg gac gac tcc acg agt aca gcc tac       240
   Gln Gly Arg Val Thr Ile Thr Ala Asp Asp Ser Thr Ser Thr Ala Tyr
   65                  70                  75                  80
   atg gag gtg agg agc ctg aga tct gag gac acg gcc gtg tat tac tgt       288
   Met Glu Val Arg Ser Leu Arg Ser Glu Asp Thr Ala Val Tyr Tyr Cys
                   85                  90                  95
   gcg aga tct tcg aat tac tat gat agt gta tat gac tac tgg ggc cag       336
   Ala Arg Ser Ser Asn Tyr Tyr Asp Ser Val Tyr Asp Tyr Trp Gly Gln
               100                 105                 110
   gga acc ctg gtc acc gtc tcg agc ggt acg ggc ggt tca ggc gga acc       384
   Gly Thr Leu Val Thr Val Ser Ser Gly Thr Gly Gly Ser Gly Gly Thr
           115                 120                 125
   ggc agc ggc act ggc ggg tcg acg cag tct gtc gtg acg cag ccg ccc      432
   Gly Ser Gly Thr Gly Gly Ser Thr Gln Ser Val Val Thr Gln Pro Pro
       130                 135                 140
   tcg gag tca gtg gcc cca gga cag acg gcc agg att acc tgt ggg gga      480
   Ser Glu Ser Val Ala Pro Gly Gln Thr Ala Arg Ile Thr Cys Gly Gly
   145                 150                 155                 160
   cat aac att gga agt aat agt gtg cac tgg tac cag cag aag cca ggc      528
   His Asn Ile Gly Ser Asn Ser Val His Trp Tyr Gln Gln Lys Pro Gly
                   165                 170                 175
   cag gcc cct gtg ctg gtc gtg tat gat aat agc gac cgg ccc tca ggg      576
   Gln Ala Pro Val Leu Val Val Tyr Asp Asn Ser Asp Arg Pro Ser Gly
               180                 185                 190
   atc cct gag cga ttc tct ggc tcc aac tct ggg aac acg gcc acc ctg      624
   Ile Pro Glu Arg Phe Ser Gly Ser Asn Ser Gly Asn Thr Ala Thr Leu
           195                 200                 205
   acc atc agc agg gtc gaa gcc ggg gat gag gcc gac tat tac tgt cag      672
   Thr Ile Ser Arg Val Glu Ala Gly Asp Glu Ala Asp Tyr Tyr Cys Gln
       210                 215                 220
   gtg tgg ggt agt agt agt gac cat tat gtc ttc gga act ggg acc aag      720
   Val Trp Gly Ser Ser Ser Asp His Tyr Val Phe Gly Thr Gly Thr Lys
   225                 230                 235                 240
   gtc acc gtc cta ggt                                                  735
   Val Thr Val Leu Gly
                   245
<210> 237
   <211> 245
   <212> PRT
   <213> Artificial
<220>
   <223> Synthetic Construct
<400> 237
   Gln Val Gln Leu Val Gln Ser Gly Ala Glu Val Lys Lys Pro Gly Ser
   1               5                   10                  15
   Ser Val Lys Val Ser Cys Lys Ala Ser Gly Val Thr Phe Ser Tyr Tyr
               20                  25                  30
   Ala Met Ser Trp Val Arg Gln Ala Pro Gly Gln Gly Leu Glu Trp Met
           35                  40                  45
   Gly Gly Ile Ser Pro Met Phe Gly Thr Thr Thr Tyr Ala Gln Lys Phe
       50                  55                  60
   Gln Gly Arg Val Thr Ile Thr Ala Asp Asp Ser Thr Ser Thr Ala Tyr
   65                  70                  75                  80
   Met Glu Val Arg Ser Leu Arg Ser Glu Asp Thr Ala Val Tyr Tyr Cys
                   85                  90                  95
   Ala Arg Ser Ser Asn Tyr Tyr Asp Ser Val Tyr Asp Tyr Trp Gly Gln
               100                 105                 110
   Gly Thr Leu Val Thr Val Ser Ser Gly Thr Gly Gly Ser Gly Gly Thr
           115                 120                 125
   Gly Ser Gly Thr Gly Gly Ser Thr Gln Ser Val Val Thr Gln Pro Pro
       130                 135                 140
   Ser Glu Ser Val Ala Pro Gly Gln Thr Ala Arg Ile Thr Cys Gly Gly
   145                 150                 155                 160
   His Asn Ile Gly Ser Asn Ser Val His Trp Tyr Gln Gln Lys Pro Gly
                   165                 170                 175
   Gln Ala Pro Val Leu Val Val Tyr Asp Asn Ser Asp Arg Pro Ser Gly
               180                 185                 190
   Ile Pro Glu Arg Phe Ser Gly Ser Asn Ser Gly Asn Thr Ala Thr Leu
           195                 200                 205
   Thr Ile Ser Arg Val Glu Ala Gly Asp Glu Ala Asp Tyr Tyr Cys Gln
       210                 215                 220
   Val Trp Gly Ser Ser Ser Asp His Tyr Val Phe Gly Thr Gly Thr Lys
   225                 230                 235                 240
   Val Thr Val Leu Gly
                   245
<210> 238
   <211> 5
   <212> PRT
   <213> Homo sapiens
<400> 238
   Gly Tyr Tyr Val Tyr
   1 5
<210> 239
   <211> 17
   <212> PRT
   <213> Homo sapiens
<400> 239
   Trp Ile Ser Ala Tyr Asn Gly Asn Thr Asn Tyr Ala Gln Lys Phe Gln
   1 5 10 15
   Gly
<210> 240
   <211> 6
   <212> PRT
   <213> Homo sapiens
<400> 240
   Ser Arg Ser Leu Asp Val
   1 5
<210> 241
   <211> 17
   <212> PRT
   <213> Homo sapiens
<400> 241
   Lys Ser Ser Gln Ser Val Leu Tyr Ser Ser Asn Asn Lys Asn Tyr Leu
   1 5 10 15
   Ala
<210> 242
   <211> 7
   <212> PRT
   <213> Homo sapiens
<400> 242
   Trp Ala Ser Thr Arg Glu Ser
   1 5
<210> 243
   <211> 9
   <212> PRT
   <213> Homo sapiens
<400> 243
   Gln Gln Tyr Tyr Ser Thr Pro Leu Thr
   1 5
<210> 244
   <211> 5
   <212> PRT
   <213> Homo sapiens
<400> 244
   Ser Tyr Ala Ile Thr
   1 5
<210> 245
   <211> 17
   <212> PRT
   <213> Homo sapiens
<400> 245
   Gly Ile Ile Gly Met Phe Gly Ser Thr Asn Tyr Ala Gln Asn Phe Gln
   1 5 10 15
   Gly
<210> 246
   <211> 11
   <212> PRT
   <213> Homo sapiens
<400> 246
   Ser Thr Gly Tyr Tyr Pro Ala Tyr Leu His His
   1 5 10
<210> 247
   <211> 14
   <212> PRT
   <213> Homo sapiens
<400> 247
   Thr Gly Thr Ser Ser Asp Val Gly Gly Tyr Asn Tyr Val Ser
   1 5 10
<210> 248
   <211> 7
   <212> PRT
   <213> Homo sapiens
<400> 248
   Asp Val Ser Lys Arg Pro Ser
   1 5
<210> 249
   <211> 10
   <212> PRT
   <213> Homo sapiens
<400> 249
   Ser Ser Tyr Thr Ser Ser Ser Thr His Val
   1 5 10
<210> 250
   <211> 5
   <212> PRT
   <213> Homo sapiens
<400> 250
   Ser Tyr Tyr Met His
   1 5
<210> 251
   <211> 17
   <212> PRT
   <213> Homo sapiens
<400> 251
   Trp Ile Asn Pro Asn Ser Gly Gly Thr Asn Tyr Ala Gln Lys Phe Gln
   1 5 10 15
   Gly
<210> 252
   <211> 12
   <212> PRT
   <213> Homo sapiens
<400> 252
   Glu Gly Lys Trp Gly Pro Gln Ala Ala Phe Asp Ile
   1 5 10
<210> 253
   <211> 12
   <212> PRT
   <213> Homo sapiens
<400> 253
   Arg Ala Ser Gln Ser Val Ser Ser Ser Tyr Leu Ala
   1 5 10
<210> 254
   <211> 7
   <212> PRT
   <213> Homo sapiens
<400> 254
   Asp Ala Ser Ser Arg Ala Thr
   1 5
<210> 255
   <211> 8
   <212> PRT
   <213> Homo sapiens
<400> 255
   Gln Gln Tyr Gly Ser Ser Leu Trp
   1 5
<210> 256
   <211> 5
   <212> PRT
   <213> Homo sapiens
<400> 256
   Ile Tyr Ala Met Ser
   1 5
<210> 257
   <211> 17
   <212> PRT
   <213> Homo sapiens
<400> 257
   Ala Ile Ser Ser Ser Gly Asp Ser Thr Tyr Tyr Ala Asp Ser Val Lys
   1 5 10 15
   Gly
<210> 258
   <211> 8
   <212> PRT
   <213> Homo sapiens
<400> 258
   Ala Tyr Gly Tyr Thr Phe Asp Pro
   1 5
<210> 259
   <211> 16
   <212> PRT
   <213> Homo sapiens
<400> 259
   Arg Ser Ser Gln Ser Leu Leu His Ser Asn Gly Tyr Asn Tyr Leu Asp
   1 5 10 15
<210> 260
   <211> 7
   <212> PRT
   <213> Homo sapiens
<400> 260
   Leu Gly Ser Asn Arg Ala Ser
   1 5
<210> 261
   <211> 8
   <212> PRT
   <213> Homo sapiens
<400> 261
   Met Gln Ala Leu Gln Thr Pro Leu
   1 5
<210> 262
   <211> 5
   <212> PRT
   <213> Homo sapiens
<400> 262
   Thr His Ala Ile Ser
   1 5
<210> 263
   <211> 17
   <212> PRT
   <213> Homo sapiens
<400> 263
   Gly Ile Ile Ala Ile Phe Gly Thr Ala Asn Tyr Ala Gln Lys Phe Gln
   1 5 10 15
   Gly
<210> 264
   <211> 12
   <212> PRT
   <213> Homo sapiens
<400> 264
   Gly Ser Gly Tyr His Ile Ser Thr Pro Phe Asp Asn
   1 5 10
<210> 265
   <211> 11
   <212> PRT
   <213> Homo sapiens
<400> 265
   Gly Gly Asn Asn Ile Gly Ser Lys Gly Val His
   1 5 10
<210> 266
   <211> 7
   <212> PRT
   <213> Homo sapiens
<400> 266
   Asp Asp Ser Asp Arg Pro Ser
   1 5
<210> 267
   <211> 11
   <212> PRT
   <213> Homo sapiens
<400> 267
   Gln Val Trp Asp Ser Ser Ser Asp His Val Val
   1 5 10
<210> 268
   <211> 5
   <212> PRT
   <213> Homo sapiens
<400> 268
   Gly Tyr Ala Ile Ser
   1 5
<210> 269
   <211> 17
   <212> PRT
   <213> Homo sapiens
<400> 269
   Gly Ile Ile Pro Ile Phe Gly Thr Thr Asn Tyr Ala Gln Lys Phe Gln
   1 5 10 15
   Gly
<210> 270
   <211> 19
   <212> PRT
   <213> Homo sapiens
<400> 270
   Val Lys Asp Gly Tyr Cys Thr Leu Thr Ser Cys Pro Val Gly Trp Tyr
   1 5 10 15
   Phe Asp Leu
<210> 271
   <211> 12
   <212> PRT
   <213> Homo sapiens
<400> 271
   Arg Ala Ser Gln Ser Val Ser Ser Ser Tyr Leu Ala
   1 5 10
<210> 272
   <211> 7
   <212> PRT
   <213> Homo sapiens
<400> 272
   Gly Ala Ser Ser Arg Ala Thr
   1 5
<210> 273
   <211> 8
   <212> PRT
   <213> Homo sapiens
<400> 273
   Gln Gln Tyr Gly Ser Ser Leu Thr
   1 5
<210> 274
   <211> 5
   <212> PRT
   <213> Homo sapiens
<400> 274
   Ser Asn Ser Ile Ser
   1 5
<210> 275
   <211> 17
   <212> PRT
   <213> Homo sapiens
<400> 275
   Gly Ile Phe Ala Leu Phe Gly Thr Thr Asp Tyr Ala Gln Lys Phe Gln
   1 5 10 15
   Gly
<210> 276
   <211> 12
   <212> PRT
   <213> Homo sapiens
<400> 276
   Gly Ser Gly Tyr Thr Thr Arg Asn Tyr Phe Asp Tyr
   1 5 10
<210> 277
   <211> 12
   <212> PRT
   <213> Homo sapiens
<400> 277
   Arg Ala Ser Gln Ser Val Ser Ser Asn Tyr Leu Gly
   1 5 10
<210> 278
   <211> 7
   <212> PRT
   <213> Homo sapiens
<400> 278
   Gly Ala Ser Ser Arg Ala Ser
   1 5
<210> 279
   <211> 9
   <212> PRT
   <213> Homo sapiens
<400> 279
   Gln Gln Tyr Gly Ser Ser Pro Leu Thr
   1 5
<210> 280
   <211> 5
   <212> PRT
   <213> Homo sapiens
<400> 280
   Asn Phe Ala Ile Asn
   1 5
<210> 281
   <211> 17
   <212> PRT
   <213> Homo sapiens
<400> 281
   Gly Ile Ile Ala Val Phe Gly Thr Thr Lys Tyr Ala His Lys Phe Gln
   1 5 10 15
   Gly
<210> 282
   <211> 11
   <212> PRT
   <213> Homo sapiens
<400> 282
   Gly Pro His Tyr Tyr Ser Ser Tyr Met Asp Val
   1 5 10
<210> 283
   <211> 11
   <212> PRT
   <213> Homo sapiens
<400> 283
   Arg Ala Ser Gln Gly Ile Ser Thr Tyr Leu Ala
   1 5 10
<210> 284
   <211> 7
   <212> PRT
   <213> Homo sapiens
<400> 284
   Ala Ala Ser Thr Leu Gln Ser
   1 5
<210> 285
   <211> 8
   <212> PRT
   <213> Homo sapiens
<400> 285
   Gln Lys Tyr Asn Ser Ala Pro Ser
   1 5
<210> 286
   <211> 5
   <212> PRT
   <213> Homo sapiens
<400> 286
   Ser Asn Ala Val Ser
   1 5
<210> 287
   <211> 17
   <212> PRT
   <213> Homo sapiens
<400> 287
   Gly Ile Leu Gly Val Phe Gly Ser Pro Ser Tyr Ala Gln Lys Phe Gln
   1 5 10 15
   Gly
<210> 288
   <211> 11
   <212> PRT
   <213> Homo sapiens
<400> 288
   Gly Pro Thr Tyr Tyr Tyr Ser Tyr Met Asp Val
   1 5 10
<210> 289
   <211> 11
   <212> PRT
   <213> Homo sapiens
<400> 289
   Gly Gly Asn Asn Ile Gly Arg Asn Ser Val His
   1 5 10
<210> 290
   <211> 7
   <212> PRT
   <213> Homo sapiens
<400> 290
   Asp Asp Ser Asp Arg Pro Ser
   1 5
<210> 291
   <211> 11
   <212> PRT
   <213> Homo sapiens
<400> 291
   Gln Val Trp His Ser Ser Ser Asp His Tyr Val
   1 5 10
<210> 292
   <211> 5
   <212> PRT
   <213> Homo sapiens
<400> 292
   Ser Tyr Ala Ile Ser
   1 5
<210> 293
   <211> 17
   <212> PRT
   <213> Homo sapiens
<400> 293
   Gly Ile Phe Gly Met Phe Gly Thr Ala Asn Tyr Ala Gln Lys Phe Gln
   1 5 10 15
   Gly
<210> 294
   <211> 11
   <212> PRT
   <213> Homo sapiens
<400> 294
   Ser Ser Gly Tyr Tyr Pro Gln Tyr Phe Gln Asp
   1 5 10
<210> 295
   <211> 12
   <212> PRT
   <213> Homo sapiens
<400> 295
   Arg Ala Ser Gln Ser Val Ser Ser Ser Tyr Leu Ala
   1 5 10
<210> 296
   <211> 7
   <212> PRT
   <213> Homo sapiens
<400> 296
   Gly Ala Ser Ser Arg Ala Thr
   1 5
<210> 297
   <211> 9
   <212> PRT
   <213> Homo sapiens
<400> 297
   Gln Gln Tyr Gly Ser Ser Ser Leu Thr
   1               5
<210> 298
   <211> 5
   <212> PRT
   <213> Homo sapiens
<400> 298
   Ser Tyr Ala Ile Ser
   1               5
<210> 299
   <211> 17
   <212> PRT
   <213> Homo sapiens
<400> 299
   Gly Ile Ile Ala Ile Phe His Thr Pro Lys Tyr Ala Gln Lys Phe Gln
   1               5                   10                  15
   Gly
<210> 300
   <211> 12
   <212> PRT
   <213> Homo sapiens
<400> 300
   Gly Ser Thr Tyr Asp Phe Ser Ser Gly Leu Asp Tyr
   1               5                   10
<210> 301
   <211> 11
   <212> PRT
   <213> Homo sapiens
<400> 301
   Gly Gly Asn Asn Ile Gly Ser Lys Ser Val His
   1               5                   10
<210> 302
   <211> 7
   <212> PRT
   <213> Homo sapiens
<400> 302
   Asp Asp Ser Asp Arg Pro Ser
   1               5
<210> 303
   <211> 11
   <212> PRT
   <213> Homo sapiens
<400> 303
   Gln Val Trp Asp Ser Ser Ser Asp His Val Val
   1 5 10
<210> 304
   <211> 5
   <212> PRT
   <213> Homo sapiens
<400> 304
   Ser Tyr Ala Ile Ser
   1 5
<210> 305
   <211> 17
   <212> PRT
   <213> Homo sapiens
<400> 305
   Gly Val Ile Pro Ile Phe Arg Thr Ala Asn Tyr Ala Gln Asn Phe Gln
   1 5 10 15
   Gly
<210> 306
   <211> 19
   <212> PRT
   <213> Homo sapiens
<400> 306
   Leu Asn Tyr His Asp Ser Gly Thr Tyr Tyr Asn Ala Pro Arg Gly Trp
   1 5 10 15
   Phe Asp Pro
<210> 307
   <211> 17
   <212> PRT
   <213> Homo sapiens
<400> 307
   Lys Ser Ser Gln Ser Ile Leu Asn Ser Ser Asn Asn Lys Asn Tyr Leu
   1 5 10 15
   Ala
<210> 308
   <211> 7
   <212> PRT
   <213> Homo sapiens
<400> 308
   Trp Ala Ser Thr Arg Glu Ser
   1 5
<210> 309
   <211> 9
   <212> PRT
   <213> Homo sapiens
<400> 309
   Gln Gln Tyr Tyr Ser Ser Pro Pro Thr
   1 5
<210> 310
   <211> 5
   <212> PRT
   <213> Homo sapiens
<400> 310
   Tyr Tyr Ala Met Ser
   1 5
<210> 311
   <211> 17
   <212> PRT
   <213> Homo sapiens
<400> 311
   Gly Ile Ser Pro Met Phe Gly Thr Thr Thr Tyr Ala Gln Lys Phe Gln
   1 5 10 15
   Gly
<210> 312
   <211> 11
   <212> PRT
   <213> Homo sapiens
<400> 312
   Ser Ser Asn Tyr Tyr Asp Ser Val Tyr Asp Tyr
   1 5 10
<210> 313
   <211> 11
   <212> PRT
   <213> Homo sapiens
<400> 313
   Gly Gly His Asn Ile Gly Ser Asn Ser Val His
   1 5 10
<210> 314
   <211> 7
   <212> PRT
   <213> Homo sapiens
<400> 314
   Asp Asn Ser Asp Arg Pro Ser
   1 5
<210> 315
   <211> 11
   <212> PRT
   <213> Homo sapiens
<400> 315
   Gln Val Trp Gly Ser Ser Ser Asp His Tyr Val
   1 5 10
<210> 316
   <211> 1335
   <212> DNA
   <213> Artificial
<220>
   <223> CR6141 Heavy chain
<220>
   <221> CDS
   <222> (1)..(1335)
<400> 316
   gag gtc cag ctg gtg cag tct ggg gct gag gtg aag aag cct ggg gcc       48
   Glu Val Gln Leu Val Gln Ser Gly Ala Glu Val Lys Lys Pro Gly Ala
   1               5                   10                  15
   tca gtg aag gtc tcc tgc aag gct tct ggg tac acc ttc acc ggc tac       96
   Ser Val Lys Val Ser Cys Lys Ala Ser Gly Tyr Thr Phe Thr Gly Tyr
               20                  25                  30
   tat gtg tac tgg gtg cga cag gcc cct gga caa ggg ctt gag tgg atg      144
   Tyr Val Tyr Trp Val Arg Gln Ala Pro Gly Gln Gly Leu Glu Trp Met
           35                  40                  45
   gga tgg atc agc gct tac aat ggt aac aca aac tat gca cag aag ttc      192
   Gly Trp Ile Ser Ala Tyr Asn Gly Asn Thr Asn Tyr Ala Gln Lys Phe
       50                  55                  60
   cag ggc aga gtc acg att acc gcg gac aaa tcc acg agc aca gcc tac      240
   Gln Gly Arg Val Thr Ile Thr Ala Asp Lys Ser Thr Ser Thr Ala Tyr
   65                  70                  75                  80
   atg gag ctg agc agc ctg aga tct gaa gac acg gct gtg tat tac tgt      288
   Met Glu Leu Ser Ser Leu Arg Ser Glu Asp Thr Ala Val Tyr Tyr Cys
                   85                  90                  95
   gcg aga agt aga tcc ctg gac gtc tgg ggc caa ggg acc acg gtc acc      336
   Ala Arg Ser Arg Ser Leu Asp Val Trp Gly Gln Gly Thr Thr Val Thr
               100                 105                 110
   gtc tcg agt gct agc acc aag ggc ccc agc gtg ttc ccc ctg gcc ccc      384
   Val Ser Ser Ala Ser Thr Lys Gly Pro Ser Val Phe Pro Leu Ala Pro
           115                 120                 125
   agc agc aag agc acc agc ggc ggc aca gcc gcc ctg ggc tgc ctg gtg      432
   Ser Ser Lys Ser Thr Ser Gly Gly Thr Ala Ala Leu Gly Cys Leu Val
       130                 135                 140
   aag gac tac ttc ccc gag ccc gtg acc gtg agc tgg aac agc ggc gcc      480
   Lys Asp Tyr Phe Pro Glu Pro Val Thr Val Ser Trp Asn Ser Gly Ala
   145                 150                 155                 160
   ttg acc agc ggc gtg cac acc ttc ccc gcc gtg ctg cag agc agc ggc      528
   Leu Thr Ser Gly Val His Thr Phe Pro Ala Val Leu Gln Ser Ser Gly
                   165                 170                 175
   ctg tac agc ctg agc agc gtg gtg acc gtg ccc agc agc agc ctg ggc      576
   Leu Tyr Ser Leu Ser Ser Val Val Thr Val Pro Ser Ser Ser Leu Gly
               180                 185                 190
   acc cag acc tac atc tgc aac gtg aac cac aag ccc agc aac acc aag      624
   Thr Gln Thr Tyr Ile Cys Asn Val Asn His Lys Pro Ser Asn Thr Lys
           195                 200                 205
   gtg gac aaa cgc gtg gag ccc aag agc tgc gac aag acc cac acc tgc      672
   Val Asp Lys Arg Val Glu Pro Lys Ser Cys Asp Lys Thr His Thr Cys
       210                 215                 220
   ccc ccc tgc cct gcc ccc gag ctg ctg ggc gga ccc tcc gtg ttc ctg       720
   Pro Pro Cys Pro Ala Pro Glu Leu Leu Gly Gly Pro Ser Val Phe Leu
   225                 230                 235                 240
   ttc ccc ccc aag ccc aag gac acc ctc atg atc agc cgg acc ccc gag       768
   Phe Pro Pro Lys Pro Lys Asp Thr Leu Met Ile Ser Arg Thr Pro Glu
                   245                 250                 255
   gtg acc tgc gtg gtg gtg gac gtg agc cac gag gac ccc gag gtg aag       816
   Val Thr Cys Val Val Val Asp Val Ser His Glu Asp Pro Glu Val Lys
               260                 265                 270
   ttc aac tgg tac gtg gac ggc gtg gag gtg cac aac gcc aag acc aag       864
   Phe Asn Trp Tyr Val Asp Gly Val Glu Val His Asn Ala Lys Thr Lys
           275                 280                 285
   ccc cgg gag gag cag tac aac agc acc tac cgg gtg gtg agc gtg ctc       912
   Pro Arg Glu Glu Gln Tyr Asn Ser Thr Tyr Arg Val Val Ser Val Leu
       290                 295                 300
   acc gtg ctg cac cag gac tgg ctg aac ggc aag gag tac aag tgc aag       960
   Thr Val Leu His Gln Asp Trp Leu Asn Gly Lys Glu Tyr Lys Cys Lys
   305                 310                 315                 320
   gtg agc aac aag gcc ctg cct gcc ccc atc gag aag acc atc agc aag      1008
   Val Ser Asn Lys Ala Leu Pro Ala Pro Ile Glu Lys Thr Ile Ser Lys
                   325                 330                 335
   gcc aag ggc cag ccc cgg gag ccc cag gtg tac acc ctg ccc ccc agc      1056
   Ala Lys Gly Gln Pro Arg Glu Pro Gln Val Tyr Thr Leu Pro Pro Ser
               340                 345                 350
   cgg gag gag atg acc aag aac cag gtg tcc ctc acc tgt ctg gtg aag      1104
   Arg Glu Glu Met Thr Lys Asn Gln Val Ser Leu Thr Cys Leu Val Lys
           355                 360                 365
   ggc ttc tac ccc agc gac atc gcc gtg gag tgg gag agc aac ggc cag      1152
   Gly Phe Tyr Pro Ser Asp Ile Ala Val Glu Trp Glu Ser Asn Gly Gln
       370                 375                 380
   ccc gag aac aac tac aag acc acc ccc cct gtg ctg gac agc gac ggc      1200
   Pro Glu Asn Asn Tyr Lys Thr Thr Pro Pro Val Leu Asp Ser Asp Gly
   385                 390                 395                 400
   agc ttc ttc ctg tac agc aag ctc acc gtg gac aag agc cgg tgg cag      1248
   Ser Phe Phe Leu Tyr Ser Lys Leu Thr Val Asp Lys Ser Arg Trp Gln
                   405                 410                 415
   cag ggc aac gtg ttc agc tgc agc gtg atg cac gag gcc ctg cac aac      1296
   Gln Gly Asn Val Phe Ser Cys Ser Val Met His Glu Ala Leu His Asn
               420                 425                 430
   cac tac acc cag aag agc ctg agc ctg agc ccc ggc aag                  1335
   His Tyr Thr Gln Lys Ser Leu Ser Leu Ser Pro Gly Lys
           435                 440                 445
<210> 317
   <211> 445
   <212> PRT
   <213> Artificial
<220>
   <223> Synthetic Construct
<400> 317
   Glu Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Val Tyr Trp Val Arg Gln Ala Pro Gly Gln Gly Leu Glu Trp Met
   35 40 45
   Gly Trp Ile Ser Ala Tyr Asn Gly Asn Thr Asn Tyr Ala Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser Arg Ser Leu Asp Val Trp Gly Gln Gly Thr Thr Val Thr
   100 105 110
   Val Ser Ser Ala Ser Thr Lys Gly Pro Ser Val Phe Pro Leu Ala Pro
   115 120 125
   Ser Ser Lys Ser Thr Ser Gly Gly Thr Ala Ala Leu Gly Cys Leu Val
   130 135 140
   Lys Asp Tyr Phe Pro Glu Pro Val Thr Val Ser Trp Asn Ser Gly Ala
   145 150 155 160
   Leu Thr Ser Gly Val His Thr Phe Pro Ala Val Leu Gln Ser Ser Gly
   165 170 175
   Leu Tyr Ser Leu Ser Ser Val Val Thr Val Pro Ser Ser Ser Leu Gly
   180 185 190
   Thr Gln Thr Tyr Ile Cys Asn Val Asn His Lys Pro Ser Asn Thr Lys
   195 200 205
   Val Asp Lys Arg Val Glu Pro Lys Ser Cys Asp Lys Thr His Thr Cys
   210 215 220
   Pro Pro Cys Pro Ala Pro Glu Leu Leu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
           275                 280                 285
   Pro Arg Glu Glu Gln Tyr Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Ala Leu Pro Ala Pro Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Arg Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly Lys
           435                 440                 445
<210> 318
   <211> 657
   <212> DNA
   <213> Artificial
<220>
   <223> CR6141 Light chain
<220>
   <221> CDS
   <222> (1)..(657)
<400> 318
   gat gtt gtg atg act cag tct cca gac tcc ctg gct gtg tct ctg ggc      48
   Asp Val Val Met Thr Gln Ser Pro Asp Ser Leu Ala Val Ser Leu Gly
   1               5                   10                  15
   gag agg gcc acc atc aac tgc aag tcc agc cag agt gtt tta tac agc      96
   Glu Arg Ala Thr Ile Asn Cys Lys Ser Ser Gln Ser Val Leu Tyr Ser
               20                  25                  30
   tcc aac aat aag aac tac tta gct tgg tac cag cag aaa cca gga cag     144
   Ser Asn Asn Lys Asn Tyr Leu Ala Trp Tyr Gln Gln Lys Pro Gly Gln
           35                  40                  45
   cct cct aag ctg ctc att tac tgg gca tct acc cgg gaa tcc ggg gtc     192
   Pro Pro Lys Leu Leu Ile Tyr Trp Ala Ser Thr Arg Glu Ser Gly Val
       50                  55                  60
   cct gac cga ttc agt ggc agc ggg tct ggg aca gat ttc act ctc acc     240
   Pro Asp Arg Phe Ser Gly Ser Gly Ser Gly Thr Asp Phe Thr Leu Thr
   65                  70                  75                  80
   atc agc agc ctg cag gct gaa gat gtg gca gtt tat tac tgt cag caa     288
   Ile Ser Ser Leu Gln Ala Glu Asp Val Ala Val Tyr Tyr Cys Gln Gln
                   85                  90                  95
   tat tat agt act cct ctc act ttc ggc gga ggg acc aaa gtg gat atc     336
   Tyr Tyr Ser Thr Pro Leu Thr Phe Gly Gly Gly Thr Lys Val Asp Ile
               100                 105                 110
   aaa cgt gcg gcc gca ccc agc gtg ttc atc ttc ccc ccc tcc gac gag     384
   Lys Arg Ala Ala Ala Pro Ser Val Phe Ile Phe Pro Pro Ser Asp Glu
           115                 120                 125
   cag ctg aag agc ggc acc gcc agc gtg gtg tgc ctg ctg aac aac ttc     432
   Gln Leu Lys Ser Gly Thr Ala Ser Val Val Cys Leu Leu Asn Asn Phe
       130                 135                 140
   tac ccc cgg gag gcc aag gtg cag tgg aag gtg gac aac gcc ctg cag     480
   Tyr Pro Arg Glu Ala Lys Val Gln Trp Lys Val Asp Asn Ala Leu Gln
   145                 150                 155                 160
   agc ggc aac agc cag gag agc gtg acc gag cag gac agc aag gac tcc     528
   Ser Gly Asn Ser Gln Glu Ser Val Thr Glu Gln Asp Ser Lys Asp Ser
                   165                 170                 175
   acc tac agc ctg agc agc acc ctc acc ctg agc aag gcc gac tac gag     576
   Thr Tyr Ser Leu Ser Ser Thr Leu Thr Leu Ser Lys Ala Asp Tyr Glu
               180                 185                 190
   aag cac aag gtg tac gcc tgc gag gtg acc cac cag ggc ctg agc agc     624
   Lys His Lys Val Tyr Ala Cys Glu Val Thr His Gln Gly Leu Ser Ser
           195                 200                 205
   ccc gtg acc aag agc ttc aac cgg ggc gag tgt                         657
   Pro Val Thr Lys Ser Phe Asn Arg Gly Glu Cys
       210                 215
<210> 319
   <211> 219
   <212> PRT
   <213> Artificial
<220>
   <223> Synthetic Construct
<400> 319
   Asp Val Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Leu Thr Phe Gly Gly Gly Thr Lys Val Asp Ile
               100                 105                 110
   Lys Arg Ala Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 320
   <211> 1350
   <212> DNA
   <213> Artificial
<220>
   <223> CR6272 Heavy chain
<220>
   <221> CDS
   <222> (1)..(1350)
<400> 320
   cag atg cag ctg gtg cag tct ggg gct gag gtg aag aag cct ggg tcc        48
   Gln Met Gln Leu Val Gln Ser Gly Ala Glu Val Lys Lys Pro Gly Ser
   1               5                   10                  15
   tcg gtg aag gtc tcc tgc aag gct tct gga ggc acc ttc tcc agt tat        96
   Ser Val Lys Val Ser Cys Lys Ala Ser Gly Gly Thr Phe Ser Ser Tyr
               20                  25                  30
   gct atc acc tgg gtg cga cag gcc cct gga caa ggg ctt gag tgg atg       144
   Ala Ile Thr Trp Val Arg Gln Ala Pro Gly Gln Gly Leu Glu Trp Met
           35                  40                  45
   gga ggg atc atc ggt atg ttt ggt tca aca aac tac gca cag aac ttc       192
   Gly Gly Ile Ile Gly Met Phe Gly Ser Thr Asn Tyr Ala Gln Asn Phe
       50                  55                  60
   cag ggc aga gtc acg att acc gcg gac gaa tcc acg agc aca gcc tac       240
   Gln Gly Arg Val Thr Ile Thr Ala Asp Glu Ser Thr Ser Thr Ala Tyr
   65                  70                  75                  80
   atg gag ctg agc agc ctc aga tct gag gac acg gcc gtg tat tac tgt       288
   Met Glu Leu Ser Ser Leu Arg Ser Glu Asp Thr Ala Val Tyr Tyr Cys
                   85                  90                  95
   gcg aga agt act ggt tat tac cct gca tac ctc cac cac tgg ggc cag       336
   Ala Arg Ser Thr Gly Tyr Tyr Pro Ala Tyr Leu His His Trp Gly Gln
               100                 105                 110
   ggc acc ctg gtc acc gtc tcg agt gct agc acc aag ggc ccc agc gtg       384
   Gly Thr Leu Val Thr Val Ser Ser Ala Ser Thr Lys Gly Pro Ser Val
           115                 120                 125
   ttc ccc ctg gcc ccc agc agc aag agc acc agc ggc ggc aca gcc gcc       432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc       480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg       528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc       576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag       624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac       672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga       720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc       768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag       816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac       864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg       912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag       960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag      1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac      1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc      1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg      1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg      1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac      1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac      1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc      1344
   Glu Ala Leu His Asn His Tyr Thr Gln Lys Ser Leu Ser Leu Ser Pro
           435                 440                 445
   ggc aag                                                              1350
   Gly Lys
       450
<210> 321
   <211> 450
   <212> PRT
   <213> Artificial
<220>
   <223> Synthetic Construct
<400> 321
   Gln Met Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Thr Trp Val Arg Gln Ala Pro Gly Gln Gly Leu Glu Trp Met
           35                  40                  45
   Gly Gly Ile Ile Gly Met Phe Gly Ser Thr Asn Tyr Ala Gln Asn Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Thr Gly Tyr Tyr Pro Ala Tyr Leu His His Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 322
   <211> 660
   <212> DNA
   <213> Artificial
<220>
   <223> CR6272 Light chain
<220>
   <221> CDS
   <222> (1)..(660)
<400> 322
   cag tct gcc ctg act cag cct cgc tca gtg tcc ggg tct cct gga cag        48
   Gln Ser Ala Leu Thr Gln Pro Arg Ser Val Ser Gly Ser Pro Gly Gln
   1               5                   10                  15
   tca gtc acc atc tcc tgc act gga acc agc agt gat gtt ggt ggt tat        96
   Ser Val Thr Ile Ser Cys Thr Gly Thr Ser Ser Asp Val Gly Gly Tyr
               20                  25                  30
   aac tat gtc tcc tgg tac caa cag cac cca ggc aaa gcc ccc aaa ctc       144
   Asn Tyr Val Ser Trp Tyr Gln Gln His Pro Gly Lys Ala Pro Lys Leu
           35                  40                  45
   atg att tat gat gtc agt aag cgg ccc tca ggg gtc cct gat cgc ttc       192
   Met Ile Tyr Asp Val Ser Lys Arg Pro Ser Gly Val Pro Asp Arg Phe
       50                  55                  60
   tct ggc tcc aag tct ggc aac acg gcc tcc ctg acc atc tct ggg ctc       240
   Ser Gly Ser Lys Ser Gly Asn Thr Ala Ser Leu Thr Ile Ser Gly Leu
   65                  70                  75                  80
   cag gct gag gat gag gct gat tat tac tgc agc tca tat aca agc agc       288
   Gln Ala Glu Asp Glu Ala Asp Tyr Tyr Cys Ser Ser Tyr Thr Ser Ser
                   85                  90                  95
   agc act cat gtc ttc gga act ggg acc aag gtc acc gtc cta ggt gcg       336
   Ser Thr His Val Phe Gly Thr Gly Thr Lys Val Thr Val Leu Gly Ala
               100                 105                 110
   gcc gca ggc cag ccc aag gcc gct ccc agc gtg acc ctg ttc ccc ccc       384
   Ala Ala Gly Gln Pro Lys Ala Ala Pro Ser Val Thr Leu Phe Pro Pro
           115                 120                 125
   tcc tcc gag gag ctg cag gcc aac aag gcc acc ctg gtg tgc ctc atc       432
   Ser Ser Glu Glu Leu Gln Ala Asn Lys Ala Thr Leu Val Cys Leu Ile
       130                 135                 140
   agc gac ttc tac cct ggc gcc gtg acc gtg gcc tgg aag gcc gac agc       480
   Ser Asp Phe Tyr Pro Gly Ala Val Thr Val Ala Trp Lys Ala Asp Ser
   145                 150                 155                 160
   agc ccc gtg aag gcc ggc gtg gag acc acc acc ccc agc aag cag agc       528
   Ser Pro Val Lys Ala Gly Val Glu Thr Thr Thr Pro Ser Lys Gln Ser
                   165                 170                 175
   aac aac aag tac gcc gcc agc agc tac ctg agc ctc acc ccc gag cag       576
   Asn Asn Lys Tyr Ala Ala Ser Ser Tyr Leu Ser Leu Thr Pro Glu Gln
               180                 185                 190
   tgg aag agc cac cgg agc tac agc tgc cag gtg acc cac gag ggc agc       624
   Trp Lys Ser His Arg Ser Tyr Ser Cys Gln Val Thr His Glu Gly Ser
           195                 200                 205
   acc gtg gag aag acc gtg gcc ccc acc gag tgc agc                       660
   Thr Val Glu Lys Thr Val Ala Pro Thr Glu Cys Ser
       210                 215                 220
<210> 323
   <211> 220
   <212> PRT
   <213> Artificial
<220>
   <223> Synthetic Construct
<400> 323
   Gln Ser Ala Leu Thr Gln Pro Arg Ser Val Ser Gly Ser Pro Gly Gln
   1               5                   10                  15
   Ser Val Thr Ile Ser Cys Thr Gly Thr Ser Ser Asp Val Gly Gly Tyr
               20                  25                  30
   Asn Tyr Val Ser Trp Tyr Gln Gln His Pro Gly Lys Ala Pro Lys Leu
           35                  40                  45
   Met Ile Tyr Asp Val Ser Lys Arg Pro Ser Gly Val Pro Asp Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Thr Ser Ser
                   85                  90                  95
   Ser Thr His Val Phe Gly Thr Gly Thr Lys Val Thr Val Leu Gly Ala
               100                 105                 110
   Ala Ala Gly Gln Pro Lys Ala Ala Pro Ser Val Thr Leu Phe Pro Pro
           115                 120                 125
   Ser Ser Glu Glu Leu Gln Ala Asn Lys Ala Thr Leu Val Cys Leu Ile
       130                 135                 140
   Ser Asp Phe Tyr Pro Gly Ala Val Thr Val Ala Trp Lys Ala Asp Ser
   145                 150                 155                 160
   Ser Pro Val Lys Ala Gly Val Glu Thr Thr Thr Pro Ser Lys Gln Ser
                   165                 170                 175
   Asn Asn Lys Tyr Ala Ala Ser Ser Tyr Leu Ser Leu Thr Pro Glu Gln
               180                 185                 190
   Trp Lys Ser His Arg Ser Tyr Ser Cys Gln Val Thr His Glu Gly Ser
           195                 200                 205
   Thr Val Glu Lys Thr Val Ala Pro Thr Glu Cys Ser
       210                 215                 220
<210> 324
   <211> 1353
   <212> DNA
   <213> Artificial
<220>
   <223> CR6296 Heavy chain
<220>
   <221> CDS
   <222> (1)..(1353)
<400> 324
   gag gtg cag ctg gtg gag acc ggg gct gag gtg aag aag cct ggg gcc        48
   Glu Val Gln Leu Val Glu Thr Gly Ala Glu Val Lys Lys Pro Gly Ala
   1               5                   10                  15
   tca gtg aag gtt tcc tgc aag gca tct gga tac acc ttc acc agc tac        96
   Ser Val Lys Val Ser Cys Lys Ala Ser Gly Tyr Thr Phe Thr Ser Tyr
               20                  25                  30
   tat atg cac tgg gtg cga cag gcc cct gga caa ggg ctt gag tgg atg       144
   Tyr Met His Trp Val Arg Gln Ala Pro Gly Gln Gly Leu Glu Trp Met
           35                  40                  45
   gga tgg atc aac cct aac agt ggt ggc aca aac tat gca cag aag ttt       192
   Gly Trp Ile Asn Pro Asn Ser Gly Gly Thr Asn Tyr Ala Gln Lys Phe
       50                  55                  60
   cag ggc agg gtc acc atg acc agg gac acg tcc atc agc aca gcc tac       240
   Gln Gly Arg Val Thr Met Thr Arg Asp Thr Ser Ile Ser Thr Ala Tyr
   65                  70                  75                  80
   atg gag ctg agc agg ctg aga tct gac gac acg gcc gtg tat tac tgt       288
   Met Glu Leu Ser Arg Leu Arg Ser Asp Asp Thr Ala Val Tyr Tyr Cys
                   85                  90                  95
   gcg aga gag ggg aaa tgg gga cct caa gcg gct ttt gat atc tgg ggc       336
   Ala Arg Glu Gly Lys Trp Gly Pro Gln Ala Ala Phe Asp Ile Trp Gly
               100                 105                 110
   caa ggg aca atg gtc acc gtc tcg agt gct agc acc aag ggc ccc agc       384
   Gln Gly Thr Met Val Thr Val Ser Ser Ala Ser Thr Lys Gly Pro Ser
           115                 120                 125
   gtg ttc ccc ctg gcc ccc agc agc aag agc acc agc ggc ggc aca gcc       432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg       480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc       528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg       576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac       624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc       672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg     1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc     1344
   His Glu Ala Leu His Asn His Tyr Thr Gln Lys Ser Leu Ser Leu Ser
           435                 440                 445
   ccc ggc aag                                                         1353
   Pro Gly Lys
       450
<210> 325
   <211> 451
   <212> PRT
   <213> Artificial
<220>
   <223> Synthetic Construct
<400> 325
   Glu Val Gln Leu Val Glu Thr Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Ala Arg Glu Gly Lys Trp Gly Pro Gln Ala Ala Phe Asp Ile Trp Gly
               100                 105                 110
   Gln Gly Thr Met Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 326
   <211> 642
   <212> DNA
   <213> Artificial
<220>
   <223> CR6296 Light chain
<220>
   <221> CDS
   <222> (1)..(642)
<400> 326
   gaa att gtg atg acg cag tct cca ggc acc ctg tct ttg tct cca ggg       48
   Glu Ile Val Met Thr Gln Ser Pro Gly Thr Leu Ser Leu Ser Pro Gly
   1               5                   10                  15
   gaa aga gcc acc ctc tcc tgc agg gcc agt cag agt gtt agc agc agc       96
   Glu Arg Ala Thr Leu Ser Cys Arg Ala Ser Gln Ser Val Ser Ser Ser
               20                  25                  30
   tac tta gcc tgg tac cag cag aaa cct ggc cag gct ccc agg ctc ctc      144
   Tyr Leu Ala Trp Tyr Gln Gln Lys Pro Gly Gln Ala Pro Arg Leu Leu
           35                  40                  45
   atc tat gat gca tcc agc agg gcc act gac atc cca gac agg ttc agt      192
   Ile Tyr Asp Ala Ser Ser Arg Ala Thr Asp Ile Pro Asp Arg Phe Ser
       50                  55                  60
   ggc agt ggg tct ggg aca gac ttc act ctc acc atc agc aga ctg gag      240
   Gly Ser Gly Ser Gly Thr Asp Phe Thr Leu Thr Ile Ser Arg Leu Glu
   65                  70                  75                  80
   cct gaa gat ttt gca gtg tat tac tgt cag cag tat ggt agc tca ctt      288
   Pro Glu Asp Phe Ala Val Tyr Tyr Cys Gln Gln Tyr Gly Ser Ser Leu
                   85                  90                  95
   tgg acg ttc ggc caa ggg acc aag gtg gag atc aaa cgt gcg gcc gca      336
   Trp Thr Phe Gly Gln Gly Thr Lys Val Glu Ile Lys Arg Ala Ala Ala
               100                 105                 110
   ccc agc gtg ttc atc ttc ccc ccc tcc gac gag cag ctg aag agc ggc      384
   Pro Ser Val Phe Ile Phe Pro Pro Ser Asp Glu Gln Leu Lys Ser Gly
           115                 120                 125
   acc gcc agc gtg gtg tgc ctg ctg aac aac ttc tac ccc cgg gag gcc      432
   Thr Ala Ser Val Val Cys Leu Leu Asn Asn Phe Tyr Pro Arg Glu Ala
       130                 135                 140
   aag gtg cag tgg aag gtg gac aac gcc ctg cag agc ggc aac agc cag      480
   Lys Val Gln Trp Lys Val Asp Asn Ala Leu Gln Ser Gly Asn Ser Gln
   145                 150                 155                 160
   gag agc gtg acc gag cag gac agc aag gac tcc acc tac agc ctg agc      528
   Glu Ser Val Thr Glu Gln Asp Ser Lys Asp Ser Thr Tyr Ser Leu Ser
                   165                 170                 175
   agc acc ctc acc ctg agc aag gcc gac tac gag aag cac aag gtg tac      576
   Ser Thr Leu Thr Leu Ser Lys Ala Asp Tyr Glu Lys His Lys Val Tyr
               180                 185                 190
   gcc tgc gag gtg acc cac cag ggc ctg agc agc ccc gtg acc aag agc      624
   Ala Cys Glu Val Thr His Gln Gly Leu Ser Ser Pro Val Thr Lys Ser
           195                 200                 205
   ttc aac cgg ggc gag tgt                                              642
   Phe Asn Arg Gly Glu Cys
       210
<210> 327
   <211> 214
   <212> PRT
   <213> Artificial
<220>
   <223> Synthetic Construct
<400> 327
   Glu Ile Val Met Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Asp Ala Ser Ser Arg Ala Thr Asp Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Leu
                   85                  90                  95
   Trp Thr Phe Gly Gln Gly Thr Lys Val Glu Ile Lys Arg Ala Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 328
   <211> 1341
   <212> DNA
   <213> Artificial
<220>
   <223> CR6301 Heavy chain
<220>
   <221> CDS
   <222> (1)..(1341)
<400> 328
   gag gtg cag ctg gta gag tct ggg gga ggc ttg gta cag cct ggg ggg       48
   Glu Val Gln Leu Val Glu Ser Gly Gly Gly Leu Val Gln Pro Gly Gly
   1               5                   10                  15
   tcc ctg aga ctc tcc tgt gca gcc tct gga ttc acc ttt agc atc tat       96
   Ser Leu Arg Leu Ser Cys Ala Ala Ser Gly Phe Thr Phe Ser Ile Tyr
               20                  25                  30
   gcc atg agc tgg gtc cgc cag gca cca ggg aag ggg ctg gag tgg gtc      144
   Ala Met Ser Trp Val Arg Gln Ala Pro Gly Lys Gly Leu Glu Trp Val
           35                  40                  45
   tca gct att agt agt agt ggt gat agc aca tac tac gca gac tcc gtg      192
   Ser Ala Ile Ser Ser Ser Gly Asp Ser Thr Tyr Tyr Ala Asp Ser Val
       50                  55                  60
   aag ggc cgg ttc acc atc tcc aga gac aac gcc agg aac acg ctg tat      240
   Lys Gly Arg Phe Thr Ile Ser Arg Asp Asn Ala Arg Asn Thr Leu Tyr
   65                  70                  75                  80
   ctg caa atg aac agt ctg aga gcc gag gac acg gct gtg tat tac tgt      288
   Leu Gln Met Asn Ser Leu Arg Ala Glu Asp Thr Ala Val Tyr Tyr Cys
                   85                  90                  95
   gcg aga gcg tat ggc tac acg ttc gac ccc tgg ggc cag gga acc ctg      336
   Ala Arg Ala Tyr Gly Tyr Thr Phe Asp Pro Trp Gly Gln Gly Thr Leu
               100                 105                 110
   gtc acc gtc tcg agt gct agc acc aag ggc ccc agc gtg ttc ccc ctg      384
   Val Thr Val Ser Ser Ala Ser Thr Lys Gly Pro Ser Val Phe Pro Leu
           115                 120                 125
   gcc ccc agc agc aag agc acc agc ggc ggc aca gcc gcc ctg ggc tgc      432
   Ala Pro Ser Ser Lys Ser Thr Ser Gly Gly Thr Ala Ala Leu Gly Cys
       130                 135                 140
   ctg gtg aag gac tac ttc ccc gag ccc gtg acc gtg agc tgg aac agc      480
   Leu Val Lys Asp Tyr Phe Pro Glu Pro Val Thr Val Ser Trp Asn Ser
   145                 150                 155                 160
   ggc gcc ttg acc agc ggc gtg cac acc ttc ccc gcc gtg ctg cag agc      528
   Gly Ala Leu Thr Ser Gly Val His Thr Phe Pro Ala Val Leu Gln Ser
                   165                 170                 175
   agc ggc ctg tac agc ctg agc agc gtg gtg acc gtg ccc agc agc agc      576
   Ser Gly Leu Tyr Ser Leu Ser Ser Val Val Thr Val Pro Ser Ser Ser
               180                 185                 190
   ctg ggc acc cag acc tac atc tgc aac gtg aac cac aag ccc agc aac      624
   Leu Gly Thr Gln Thr Tyr Ile Cys Asn Val Asn His Lys Pro Ser Asn
           195                 200                 205
   acc aag gtg gac aaa cgc gtg gag ccc aag agc tgc gac aag acc cac      672
   Thr Lys Val Asp Lys Arg Val Glu Pro Lys Ser Cys Asp Lys Thr His
       210                 215                 220
   acc tgc ccc ccc tgc cct gcc ccc gag ctg ctg ggc gga ccc tcc gtg      720
   Thr Cys Pro Pro Cys Pro Ala Pro Glu Leu Leu Gly Gly Pro Ser Val
   225                 230                 235                 240
   ttc ctg ttc ccc ccc aag ccc aag gac acc ctc atg atc agc cgg acc      768
   Phe Leu Phe Pro Pro Lys Pro Lys Asp Thr Leu Met Ile Ser Arg Thr
                   245                 250                 255
   ccc gag gtg acc tgc gtg gtg gtg gac gtg agc cac gag gac ccc gag      816
   Pro Glu Val Thr Cys Val Val Val Asp Val Ser His Glu Asp Pro Glu
               260                 265                 270
   gtg aag ttc aac tgg tac gtg gac ggc gtg gag gtg cac aac gcc aag      864
   Val Lys Phe Asn Trp Tyr Val Asp Gly Val Glu Val His Asn Ala Lys
           275                 280                 285
   acc aag ccc cgg gag gag cag tac aac agc acc tac cgg gtg gtg agc      912
   Thr Lys Pro Arg Glu Glu Gln Tyr Asn Ser Thr Tyr Arg Val Val Ser
       290                 295                 300
   gtg ctc acc gtg ctg cac cag gac tgg ctg aac ggc aag gag tac aag      960
   Val Leu Thr Val Leu His Gln Asp Trp Leu Asn Gly Lys Glu Tyr Lys
   305                 310                 315                 320
   tgc aag gtg agc aac aag gcc ctg cct gcc ccc atc gag aag acc atc     1008
   Cys Lys Val Ser Asn Lys Ala Leu Pro Ala Pro Ile Glu Lys Thr Ile
                   325                 330                 335
   agc aag gcc aag ggc cag ccc cgg gag ccc cag gtg tac acc ctg ccc     1056
   Ser Lys Ala Lys Gly Gln Pro Arg Glu Pro Gln Val Tyr Thr Leu Pro
               340                 345                 350
   ccc agc cgg gag gag atg acc aag aac cag gtg tcc ctc acc tgt ctg     1104
   Pro Ser Arg Glu Glu Met Thr Lys Asn Gln Val Ser Leu Thr Cys Leu
           355                 360                 365
   gtg aag ggc ttc tac ccc agc gac atc gcc gtg gag tgg gag agc aac     1152
   Val Lys Gly Phe Tyr Pro Ser Asp Ile Ala Val Glu Trp Glu Ser Asn
       370                 375                 380
   ggc cag ccc gag aac aac tac aag acc acc ccc cct gtg ctg gac agc     1200
   Gly Gln Pro Glu Asn Asn Tyr Lys Thr Thr Pro Pro Val Leu Asp Ser
   385                 390                 395                 400
   gac ggc agc ttc ttc ctg tac agc aag ctc acc gtg gac aag agc cgg     1248
   Asp Gly Ser Phe Phe Leu Tyr Ser Lys Leu Thr Val Asp Lys Ser Arg
                   405                 410                 415
   tgg cag cag ggc aac gtg ttc agc tgc agc gtg atg cac gag gcc ctg     1296
   Trp Gln Gln Gly Asn Val Phe Ser Cys Ser Val Met His Glu Ala Leu
               420                 425                 430
   cac aac cac tac acc cag aag agc ctg agc ctg agc ccc ggc aag         1341
   His Asn His Tyr Thr Gln Lys Ser Leu Ser Leu Ser Pro Gly Lys
           435                 440                 445
<210> 329
   <211> 447
   <212> PRT
   <213> Artificial
<220>
   <223> Synthetic Construct
<400> 329
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Ser Trp Val Arg Gln Ala Pro Gly Lys Gly Leu Glu Trp Val
           35                  40                  45
   Ser Ala Ile Ser Ser Ser Gly Asp Ser Thr Tyr Tyr Ala Asp Ser Val
       50                  55                  60
   Lys Gly Arg Phe Thr Ile Ser Arg Asp Asn Ala Arg Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Tyr Gly Tyr Thr Phe Asp Pro Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Pro Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Ser Arg Glu Glu Met Thr Lys Asn Gln Val Ser Leu Thr Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 330
   <211> 654
   <212> DNA
   <213> Artificial
<220>
   <223> CR6301 Light chain
<220>
   <221> CDS
   <222> (1)..(654)
<400> 330
   gaa att gtg ctg act cag tct cca ctc tcc ctg ccc gtc acc cct gga       48
   Glu Ile Val Leu Thr Gln Ser Pro Leu Ser Leu Pro Val Thr Pro Gly
   1               5                   10                  15
   gag ccg gcc tcc atc tcc tgc agg tct agt cag agc ctc ctg cat agt       96
   Glu Pro Ala Ser Ile Ser Cys Arg Ser Ser Gln Ser Leu Leu His Ser
               20                  25                  30
   aat gga tac aac tat ttg gat tgg tac ctg cag aag cca ggg cag tct      144
   Asn Gly Tyr Asn Tyr Leu Asp Trp Tyr Leu Gln Lys Pro Gly Gln Ser
           35                  40                  45
   cca cag ctc ctg atc tat ttg ggt tct aat cgg gcc tcc ggg gtc cct      192
   Pro Gln Leu Leu Ile Tyr Leu Gly Ser Asn Arg Ala Ser Gly Val Pro
       50                  55                  60
   gac agg ttc agt ggc agt gga tca ggc aca gat ttt aca ctg aaa atc      240
   Asp Arg Phe Ser Gly Ser Gly Ser Gly Thr Asp Phe Thr Leu Lys Ile
   65                  70                  75                  80
   agc aga gtg gag gct gag gat gtt ggg gtt tat tac tgc atg caa gct      288
   Ser Arg Val Glu Ala Glu Asp Val Gly Val Tyr Tyr Cys Met Gln Ala
                   85                  90                  95
   cta caa act ccc ctc act ttc ggc gga ggg acc aag gtg gag atc aaa      336
   Leu Gln Thr Pro Leu Thr Phe Gly Gly Gly Thr Lys Val Glu Ile Lys
               100                 105                 110
   cgt gcg gcc gca ccc agc gtg ttc atc ttc ccc ccc tcc gac gag cag      384
   Arg Ala Ala Ala Pro Ser Val Phe Ile Phe Pro Pro Ser Asp Glu Gln
           115                 120                 125
   ctg aag agc ggc acc gcc agc gtg gtg tgc ctg ctg aac aac ttc tac      432
   Leu Lys Ser Gly Thr Ala Ser Val Val Cys Leu Leu Asn Asn Phe Tyr
       130                 135                 140
   ccc cgg gag gcc aag gtg cag tgg aag gtg gac aac gcc ctg cag agc      480
   Pro Arg Glu Ala Lys Val Gln Trp Lys Val Asp Asn Ala Leu Gln Ser
   145                 150                 155                 160
   ggc aac agc cag gag agc gtg acc gag cag gac agc aag gac tcc acc      528
   Gly Asn Ser Gln Glu Ser Val Thr Glu Gln Asp Ser Lys Asp Ser Thr
                   165                 170                 175
   tac agc ctg agc agc acc ctc acc ctg agc aag gcc gac tac gag aag      576
   Tyr Ser Leu Ser Ser Thr Leu Thr Leu Ser Lys Ala Asp Tyr Glu Lys
               180                 185                 190
   cac aag gtg tac gcc tgc gag gtg acc cac cag ggc ctg agc agc ccc      624
   His Lys Val Tyr Ala Cys Glu Val Thr His Gln Gly Leu Ser Ser Pro
           195                 200                 205
   gtg acc aag agc ttc aac cgg ggc gag tgt                              654
   Val Thr Lys Ser Phe Asn Arg Gly Glu Cys
       210                 215
<210> 331
   <211> 218
   <212> PRT
   <213> Artificial
<220>
   <223> Synthetic Construct
<400> 331
   Glu Ile Val Leu Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Thr Pro Leu Thr Phe Gly Gly Gly Thr Lys Val Glu Ile Lys
               100                 105                 110
   Arg Ala Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 332
   <211> 1353
   <212> DNA
   <213> Artificial
<220>
   <223> CR6327 Heavy chain
<220>
   <221> CDS
   <222> (1)..(1353)
<400> 332
   gag gtg cag ctg gtg gag acc ggg gct gag gtg aag aag cct ggg tcc      48
   Glu Val Gln Leu Val Glu Thr Gly Ala Glu Val Lys Lys Pro Gly Ser
   1               5                   10                  15
   tcg gtg aag gtc tcc tgc aag gcc tct gga ggc acc ttc agg acc cat      96
   Ser Val Lys Val Ser Cys Lys Ala Ser Gly Gly Thr Phe Arg Thr His
               20                  25                  30
   gct atc agt tgg gtg cga cag gcc cct gga caa ggg ctt gag tgg atg     144
   Ala Ile Ser Trp Val Arg Gln Ala Pro Gly Gln Gly Leu Glu Trp Met
           35                  40                  45
   gga ggg atc atc gct atc ttc gga aca gca aac tac gca cag aag ttc     192
   Gly Gly Ile Ile Ala Ile Phe Gly Thr Ala Asn Tyr Ala Gln Lys Phe
       50                  55                  60
   cag ggc aga atc acg att acc gcg gac gaa tcc acg agt aca gcc tac     240
   Gln Gly Arg Ile Thr Ile Thr Ala Asp Glu Ser Thr Ser Thr Ala Tyr
   65                  70                  75                  80
   atg gag ctg agc agc ctg aga tct gag gac acg gcc gtg tat ttc tgt     288
   Met Glu Leu Ser Ser Leu Arg Ser Glu Asp Thr Ala Val Tyr Phe Cys
                   85                  90                  95
   gcg aga ggc agt ggt tat cat ata tcg aca ccc ttt gac aac tgg ggc     336
   Ala Arg Gly Ser Gly Tyr His Ile Ser Thr Pro Phe Asp Asn Trp Gly
               100                 105                 110
   cag gga acc ctg gtc acc gtc tcg agt gct agc acc aag ggc ccc agc     384
   Gln Gly Thr Leu Val Thr Val Ser Ser Ala Ser Thr Lys Gly Pro Ser
           115                 120                 125
   gtg ttc ccc ctg gcc ccc agc agc aag agc acc agc ggc ggc aca gcc     432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg     480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc     528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg     576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac     624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc     672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc       720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg       768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac       816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg       864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac       912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc       960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc      1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg      1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc      1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag      1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct      1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg      1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 333
   <211> 451
   <212> PRT
   <213> Artificial
<220>
   <223> Synthetic Construct
<400> 333
   Glu Val Gln Leu Val Glu Thr Gly Ala Glu Val Lys Lys Pro Gly Ser
   1               5                   10                  15
   Ser Val Lys Val Ser Cys Lys Ala Ser Gly Gly Thr Phe Arg Thr His
               20                  25                  30
   Ala Ile Ser Trp Val Arg Gln Ala Pro Gly Gln Gly Leu Glu Trp Met
           35                  40                  45
   Gly Gly Ile Ile Ala Ile Phe Gly Thr Ala Asn Tyr Ala Gln Lys Phe
       50                  55                  60
   Gln Gly Arg Ile Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Arg Gly Ser Gly Tyr His Ile Ser Thr Pro Phe Asp Asn Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 334
   <211> 654
   <212> DNA
   <213> Artificial
<220>
   <223> CR6327 Light chain
<220>
   <221> CDS
   <222> (1)..(654)
<400> 334
   tcc tat gtg ctg act cag cca ccc tcg gtg tca gtg gcc cca gga cag        48
   Ser Tyr Val Leu Thr Gln Pro Pro Ser Val Ser Val Ala Pro Gly Gln
   1               5                   10                  15
   acg gcc agg att acc tgt ggg gga aac aac att gga agt aaa ggt gtg        96
   Thr Ala Arg Ile Thr Cys Gly Gly Asn Asn Ile Gly Ser Lys Gly Val
               20                  25                  30
   cac tgg tac cag cag aag cct ggc cag gcc cct gtg ctg gtc gtc tat       144
   His Trp Tyr Gln Gln Lys Pro Gly Gln Ala Pro Val Leu Val Val Tyr
           35                  40                  45
   gat gat agc gac cgg ccc tca ggg atc cct gag cga ttc tct ggc tcc       192
   Asp Asp Ser Asp Arg Pro Ser Gly Ile Pro Glu Arg Phe Ser Gly Ser
       50                  55                  60
   aac tct ggg aac acg gcc acc ctg acc atc agc agg gtc gaa gcc ggg       240
   Asn Ser Gly Asn Thr Ala Thr Leu Thr Ile Ser Arg Val Glu Ala Gly
   65                  70                  75                  80
   gat gag gcc gac tat tac tgt cag gtg tgg gat agt agt agt gat cat       288
   Asp Glu Ala Asp Tyr Tyr Cys Gln Val Trp Asp Ser Ser Ser Asp His
                   85                  90                  95
   gtg gta ttc ggc gga ggg acc aag ctg acc gtc cta ggt gcg gcc gca       336
   Val Val Phe Gly Gly Gly Thr Lys Leu Thr Val Leu Gly Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 335
   <211> 218
   <212> PRT
   <213> Artificial
<220>
   <223> Synthetic Construct
<400> 335
   Ser Tyr Val Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Gly Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Val Val Phe Gly Gly Gly Thr Lys Leu Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 336
   <211> 1374
   <212> DNA
   <213> Artificial
<220>
   <223> CR6328 Heavy chain
<220>
   <221> CDS
   <222> (1)..(1374)
<400> 336
   gag gtg cag ctg gtg gag tct ggg gct gag gtg aag aag cct ggg tcc 48
   Glu Val Gln Leu Val Glu Ser Gly Ala Glu Val Lys Lys Pro Gly Ser
   1 5 10 15
   tcg gtg aag gtc tcc tgc aag gct tct gga cac atc ttc agc ggc tat 96
   Ser Val Lys Val Ser Cys Lys Ala Ser Gly His Ile Phe Ser Gly Tyr
   20 25 30
   gca atc agt tgg gtg cga cag gcc cct gga caa ggg ctt gag tgg atg 144
   Ala Ile Ser Trp Val Arg Gln Ala Pro Gly Gln Gly Leu Glu Trp Met
   35 40 45
   gga ggg atc atc cct atc ttt ggt aca aca aac tac gca cag aag ttc 192
   Gly Gly Ile Ile Pro Ile Phe Gly Thr Thr Asn Tyr Ala Gln Lys Phe
   50 55 60
   cag ggc aga gtc acg att acc gcg gac caa tcc acg agc aca gcc tac 240
   Gln Gly Arg Val Thr Ile Thr Ala Asp Gln Ser Thr Ser Thr Ala Tyr
   65 70 75 80
   atg gac ctg agc aac ttg aga tct gag gac acg gcc gtc tat tac tgt 288
   Met Asp Leu Ser Asn Leu Arg Ser Glu Asp Thr Ala Val Tyr Tyr Cys
   85 90 95
   gcg aga gtg aaa gat gga tat tgt act ctt acc agc tgc cct gtc ggc 336
   Ala Arg Val Lys Asp Gly Tyr Cys Thr Leu Thr Ser Cys Pro Val Gly
   100 105 110
   tgg tac ttc gat ctc tgg ggc cgt ggc acc ctg gtc act gtc tcg agt 384
   Trp Tyr Phe Asp Leu Trp Gly Arg Gly Thr Leu Val Thr Val Ser Ser
   115 120 125
   gct agc acc aag ggc ccc agc gtg ttc ccc ctg gcc ccc agc agc aag 432
   Ala Ser Thr Lys Gly Pro Ser Val Phe Pro Leu Ala Pro Ser Ser Lys
   130 135 140
   agc acc agc ggc ggc aca gcc gcc ctg ggc tgc ctg gtg aag gac tac 480
   Ser Thr Ser Gly Gly Thr Ala Ala Leu Gly Cys Leu Val Lys Asp Tyr
   145 150 155 160
   ttc ccc gag ccc gtg acc gtg agc tgg aac agc ggc gcc ttg acc agc 528
   Phe Pro Glu Pro Val Thr Val Ser Trp Asn Ser Gly Ala Leu Thr Ser
   165 170 175
   ggc gtg cac acc ttc ccc gcc gtg ctg cag agc agc ggc ctg tac agc 576
   Gly Val His Thr Phe Pro Ala Val Leu Gln Ser Ser Gly Leu Tyr Ser
   180 185 190
   ctg agc agc gtg gtg acc gtg ccc agc agc agc ctg ggc acc cag acc 624
   Leu Ser Ser Val Val Thr Val Pro Ser Ser Ser Leu Gly Thr Gln Thr
   195 200 205
   tac atc tgc aac gtg aac cac aag ccc agc aac acc aag gtg gac aaa      672
   Tyr Ile Cys Asn Val Asn His Lys Pro Ser Asn Thr Lys Val Asp Lys
       210                 215                 220
   cgc gtg gag ccc aag agc tgc gac aag acc cac acc tgc ccc ccc tgc      720
   Arg Val Glu Pro Lys Ser Cys Asp Lys Thr His Thr Cys Pro Pro Cys
   225                 230                 235                 240
   cct gcc ccc gag ctg ctg ggc gga ccc tcc gtg ttc ctg ttc ccc ccc      768
   Pro Ala Pro Glu Leu Leu Gly Gly Pro Ser Val Phe Leu Phe Pro Pro
                   245                 250                 255
   aag ccc aag gac acc ctc atg atc agc cgg acc ccc gag gtg acc tgc      816
   Lys Pro Lys Asp Thr Leu Met Ile Ser Arg Thr Pro Glu Val Thr Cys
               260                 265                 270
   gtg gtg gtg gac gtg agc cac gag gac ccc gag gtg aag ttc aac tgg      864
   Val Val Val Asp Val Ser His Glu Asp Pro Glu Val Lys Phe Asn Trp
           275                 280                 285
   tac gtg gac ggc gtg gag gtg cac aac gcc aag acc aag ccc cgg gag      912
   Tyr Val Asp Gly Val Glu Val His Asn Ala Lys Thr Lys Pro Arg Glu
       290                 295                 300
   gag cag tac aac agc acc tac cgg gtg gtg agc gtg ctc acc gtg ctg      960
   Glu Gln Tyr Asn Ser Thr Tyr Arg Val Val Ser Val Leu Thr Val Leu
   305                 310                 315                 320
   cac cag gac tgg ctg aac ggc aag gag tac aag tgc aag gtg agc aac     1008
   His Gln Asp Trp Leu Asn Gly Lys Glu Tyr Lys Cys Lys Val Ser Asn
                   325                 330                 335
   aag gcc ctg cct gcc ccc atc gag aag acc atc agc aag gcc aag ggc     1056
   Lys Ala Leu Pro Ala Pro Ile Glu Lys Thr Ile Ser Lys Ala Lys Gly
               340                 345                 350
   cag ccc cgg gag ccc cag gtg tac acc ctg ccc ccc agc cgg gag gag     1104
   Gln Pro Arg Glu Pro Gln Val Tyr Thr Leu Pro Pro Ser Arg Glu Glu
           355                 360                 365
   atg acc aag aac cag gtg tcc ctc acc tgt ctg gtg aag ggc ttc tac     1152
   Met Thr Lys Asn Gln Val Ser Leu Thr Cys Leu Val Lys Gly Phe Tyr
       370                 375                 380
   ccc agc gac atc gcc gtg gag tgg gag agc aac ggc cag ccc gag aac     1200
   Pro Ser Asp Ile Ala Val Glu Trp Glu Ser Asn Gly Gln Pro Glu Asn
   385                 390                 395                 400
   aac tac aag acc acc ccc cct gtg ctg gac agc gac ggc agc ttc ttc     1248
   Asn Tyr Lys Thr Thr Pro Pro Val Leu Asp Ser Asp Gly Ser Phe Phe
                   405                 410                 415
   ctg tac agc aag ctc acc gtg gac aag agc cgg tgg cag cag ggc aac     1296
   Leu Tyr Ser Lys Leu Thr Val Asp Lys Ser Arg Trp Gln Gln Gly Asn
               420                 425                 430
   gtg ttc agc tgc agc gtg atg cac gag gcc ctg cac aac cac tac acc     1344
   Val Phe Ser Cys Ser Val Met His Glu Ala Leu His Asn His Tyr Thr
           435                 440                 445
   cag aag agc ctg agc ctg agc ccc ggc aag                             1374
   Gln Lys Ser Leu Ser Leu Ser Pro Gly Lys
       450                 455
<210> 337
   <211> 458
   <212> PRT
   <213> Artificial
<220>
   <223> Synthetic Construct
<400> 337
   Glu Val Gln Leu Val Glu Ser Gly Ala Glu Val Lys Lys Pro Gly Ser
   1               5                   10                  15
   Ser Val Lys Val Ser Cys Lys Ala Ser Gly His Ile Phe Ser Gly Tyr
               20                  25                  30
   Ala Ile Ser Trp Val Arg Gln Ala Pro Gly Gln Gly Leu Glu Trp Met
           35                  40                  45
   Gly Gly Ile Ile Pro Ile Phe Gly Thr Thr Asn Tyr Ala Gln Lys Phe
       50                  55                  60
   Gln Gly Arg Val Thr Ile Thr Ala Asp Gln Ser Thr Ser Thr Ala Tyr
   65                  70                  75                  80
   Met Asp Leu Ser Asn Leu Arg Ser Glu Asp Thr Ala Val Tyr Tyr Cys
                   85                  90                  95
   Ala Arg Val Lys Asp Gly Tyr Cys Thr Leu Thr Ser Cys Pro Val Gly
               100                 105                 110
   Trp Tyr Phe Asp Leu Trp Gly Arg Gly Thr Leu Val Thr Val Ser Ser
           115                 120                 125
   Ala Ser Thr Lys Gly Pro Ser Val Phe Pro Leu Ala Pro Ser Ser Lys
       130                 135                 140
   Ser Thr Ser Gly Gly Thr Ala Ala Leu Gly Cys Leu Val Lys Asp Tyr
   145                 150                 155                 160
   Phe Pro Glu Pro Val Thr Val Ser Trp Asn Ser Gly Ala Leu Thr Ser
                   165                 170                 175
   Gly Val His Thr Phe Pro Ala Val Leu Gln Ser Ser Gly Leu Tyr Ser
               180                 185                 190
   Leu Ser Ser Val Val Thr Val Pro Ser Ser Ser Leu Gly Thr Gln Thr
           195                 200                 205
   Tyr Ile Cys Asn Val Asn His Lys Pro Ser Asn Thr Lys Val Asp Lys
       210                 215                 220
   Arg Val Glu Pro Lys Ser Cys Asp Lys Thr His Thr Cys Pro Pro Cys
   225 230 235 240
   Pro Ala Pro Glu Leu Leu Gly Gly Pro Ser Val Phe Leu Phe Pro Pro
   245 250 255
   Lys Pro Lys Asp Thr Leu Met Ile Ser Arg Thr Pro Glu Val Thr Cys
   260 265 270
   Val Val Val Asp Val Ser His Glu Asp Pro Glu Val Lys Phe Asn Trp
   275 280 285
   Tyr Val Asp Gly Val Glu Val His Asn Ala Lys Thr Lys Pro Arg Glu
   290 295 300
   Glu Gln Tyr Asn Ser Thr Tyr Arg Val Val Ser Val Leu Thr Val Leu
   305 310 315 320
   His Gln Asp Trp Leu Asn Gly Lys Glu Tyr Lys Cys Lys Val Ser Asn
   325 330 335
   Lys Ala Leu Pro Ala Pro Ile Glu Lys Thr Ile Ser Lys Ala Lys Gly
   340 345 350
   Gln Pro Arg Glu Pro Gln Val Tyr Thr Leu Pro Pro Ser Arg Glu Glu
   355 360 365
   Met Thr Lys Asn Gln Val Ser Leu Thr Cys Leu Val Lys Gly Phe Tyr
   370 375 380
   Pro Ser Asp Ile Ala Val Glu Trp Glu Ser Asn Gly Gln Pro Glu Asn
   385 390 395 400
   Asn Tyr Lys Thr Thr Pro Pro Val Leu Asp Ser Asp Gly Ser Phe Phe
   405 410 415
   Leu Tyr Ser Lys Leu Thr Val Asp Lys Ser Arg Trp Gln Gln Gly Asn
   420 425 430
   Val Phe Ser Cys Ser Val Met His Glu Ala Leu His Asn His Tyr Thr
   435 440 445
   Gln Lys Ser Leu Ser Leu Ser Pro Gly Lys
   450 455
<210> 338
   <211> 639
   <212> DNA
   <213> Artificial
<220>
   <223> CR6328 Light chain
<220>
   <221> CDS
   <222> (1)..(639)
<400> 338
   gaa att gtg atg acg cag tct cca ggc acc ctg tct ttg tct cca ggg        48
   Glu Ile Val Met Thr Gln Ser Pro Gly Thr Leu Ser Leu Ser Pro Gly
   1               5                   10                  15
   gaa aga gcc acc ctc tcg tgc agg gcc agt cag agt gtt agc agc agc        96
   Glu Arg Ala Thr Leu Ser Cys Arg Ala Ser Gln Ser Val Ser Ser Ser
               20                  25                  30
   tac tta gcc tgg tac cag cag aaa cct ggc cag gct ccc agg ctc ctc       144
   Tyr Leu Ala Trp Tyr Gln Gln Lys Pro Gly Gln Ala Pro Arg Leu Leu
           35                  40                  45
   atc ttt ggt gcc tcc agc agg gcc act ggc atc cca gac agg ttc agt       192
   Ile Phe Gly Ala Ser Ser Arg Ala Thr Gly Ile Pro Asp Arg Phe Ser
       50                  55                  60
   ggc agt ggg tct ggg aca gac ttc act ctc acc atc agc aga ctg gag       240
   Gly Ser Gly Ser Gly Thr Asp Phe Thr Leu Thr Ile Ser Arg Leu Glu
   65                  70                  75                  80
   cct gaa gat ttt gca gtg tat tac tgt cag cag tat ggt agc tca ctc       288
   Pro Glu Asp Phe Ala Val Tyr Tyr Cys Gln Gln Tyr Gly Ser Ser Leu
                   85                  90                  95
   act ttc ggc gga ggg acc aag ctg gag atc aaa cgt gcg gcc gca ccc       336
   Thr Phe Gly Gly Gly Thr Lys Leu Glu Ile Lys Arg Ala Ala Ala Pro
               100                 105                 110
   agc gtg ttc atc ttc ccc ccc tcc gac gag cag ctg aag agc ggc acc       384
   Ser Val Phe Ile Phe Pro Pro Ser Asp Glu Gln Leu Lys Ser Gly Thr
           115                 120                 125
   gcc agc gtg gtg tgc ctg ctg aac aac ttc tac ccc cgg gag gcc aag       432
   Ala Ser Val Val Cys Leu Leu Asn Asn Phe Tyr Pro Arg Glu Ala Lys
       130                 135                 140
   gtg cag tgg aag gtg gac aac gcc ctg cag agc ggc aac agc cag gag       480
   Val Gln Trp Lys Val Asp Asn Ala Leu Gln Ser Gly Asn Ser Gln Glu
   145                 150                 155                 160
   agc gtg acc gag cag gac agc aag gac tcc acc tac agc ctg agc agc       528
   Ser Val Thr Glu Gln Asp Ser Lys Asp Ser Thr Tyr Ser Leu Ser Ser
                   165                 170                 175
   acc ctc acc ctg agc aag gcc gac tac gag aag cac aag gtg tac gcc       576
   Thr Leu Thr Leu Ser Lys Ala Asp Tyr Glu Lys His Lys Val Tyr Ala
               180                 185                 190
   tgc gag gtg acc cac cag ggc ctg agc agc ccc gtg acc aag agc ttc       624
   Cys Glu Val Thr His Gln Gly Leu Ser Ser Pro Val Thr Lys Ser Phe
           195                 200                 205
   aac cgg ggc gag tgt                                                   639
   Asn Arg Gly Glu Cys
       210
<210> 339
   <211> 213
   <212> PRT
   <213> Artificial
<220>
   <223> Synthetic Construct
<400> 339
   Glu Ile Val Met Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Phe Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Leu
                   85                  90                  95
   Thr Phe Gly Gly Gly Thr Lys Leu Glu Ile Lys Arg Ala Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Cys
   210
<210> 340
   <211> 1353
   <212> DNA
   <213> Artificial
<220>
   <223> CR6329 Heavy chain
<220>
   <221> CDS
   <222> (1)..(1353)
<400> 340
   gag gtc cag ctg gta cag tct ggg gct gag gtt aag aag cct ggg tcc 48
   Glu Val Gln Leu Val Gln Ser Gly Ala Glu Val Lys Lys Pro Gly Ser
   1 5 10 15
   tcg gtg aag gtc tcc tgc aag gct tct gga ggc atc ttc aga agc aat 96
   Ser Val Lys Val Ser Cys Lys Ala Ser Gly Gly Ile Phe Arg Ser Asn
   20 25 30
   tct atc agt tgg gtg cga cag gcc cct ggg caa ggg ctt gag tgg atg 144
   Ser Ile Ser Trp Val Arg Gln Ala Pro Gly Gln Gly Leu Glu Trp Met
   35 40 45
   gga ggg atc ttc gct ctt ttc gga aca aca gac tac gcg cag aag ttc 192
   Gly Gly Ile Phe Ala Leu Phe Gly Thr Thr Asp Tyr Ala Gln Lys Phe
   50 55 60
   cag ggc aga gtc acg att acc gcg gac gaa tct tcg acc aca gtc tac 240
   Gln Gly Arg Val Thr Ile Thr Ala Asp Glu Ser Ser Thr Thr Val Tyr
   65 70 75 80
   ctg gag ctg agt agc ctg aca tct gag gac acg gcc gtt tat tac tgt 288
   Leu Glu Leu Ser Ser Leu Thr Ser Glu Asp Thr Ala Val Tyr Tyr Cys
   85 90 95
   gcg aga ggc agt ggc tac acc aca cgc aac tac ttt gac tac tgg ggc 336
   Ala Arg Gly Ser Gly Tyr Thr Thr Arg Asn Tyr Phe Asp Tyr Trp Gly
   100 105 110
   cag ggc acc ctg gtc acc gtc tcg agt gct agc acc aag ggc ccc agc 384
   Gln Gly Thr Leu Val Thr Val Ser Ser Ala Ser Thr Lys Gly Pro Ser
   115 120 125
   gtg ttc ccc ctg gcc ccc agc agc aag agc acc agc ggc ggc aca gcc 432
   Val Phe Pro Leu Ala Pro Ser Ser Lys Ser Thr Ser Gly Gly Thr Ala
   130 135 140
   gcc ctg ggc tgc ctg gtg aag gac tac ttc ccc gag ccc gtg acc gtg 480
   Ala Leu Gly Cys Leu Val Lys Asp Tyr Phe Pro Glu Pro Val Thr Val
   145 150 155 160
   agc tgg aac agc ggc gcc ttg acc agc ggc gtg cac acc ttc ccc gcc 528
   Ser Trp Asn Ser Gly Ala Leu Thr Ser Gly Val His Thr Phe Pro Ala
   165 170 175
   gtg ctg cag agc agc ggc ctg tac agc ctg agc agc gtg gtg acc gtg 576
   Val Leu Gln Ser Ser Gly Leu Tyr Ser Leu Ser Ser Val Val Thr Val
   180 185 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg     1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg     1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc     1344
   His Glu Ala Leu His Asn His Tyr Thr Gln Lys Ser Leu Ser Leu Ser
           435                 440                 445
   ccc ggc aag                                                         1353
   Pro Gly Lys
   450
<210> 341
   <211> 451
   <212> PRT
   <213> Artificial
<220>
   <223> Synthetic Construct
<400> 341
   Glu Val Gln Leu Val Gln Ser Gly Ala Glu Val Lys Lys Pro Gly Ser
   1               5                   10                  15
   Ser Val Lys Val Ser Cys Lys Ala Ser Gly Gly Ile Phe Arg Ser Asn
               20                  25                  30
   Ser Ile Ser Trp Val Arg Gln Ala Pro Gly Gln Gly Leu Glu Trp Met
           35                  40                  45
   Gly Gly Ile Phe Ala Leu Phe Gly Thr Thr Asp Tyr Ala Gln Lys Phe
       50                  55                  60
   Gln Gly Arg Val Thr Ile Thr Ala Asp Glu Ser Ser Thr Thr Val Tyr
   65                  70                  75                  80
   Leu Glu Leu Ser Ser Leu Thr Ser Glu Asp Thr Ala Val Tyr Tyr Cys
                   85                  90                  95
   Ala Arg Gly Ser Gly Tyr Thr Thr Arg Asn Tyr Phe Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 342
   <211> 654
   <212> DNA
   <213> Artificial
<220>
   <223> CR6329 Light chain
<220>
   <221> CDS
   <222> (1)..(654)
<400> 342
   gaa att gtg ctg act cag tct cca ggc acc ctg tct ttg tct cca ggg        48
   Glu Ile Val Leu Thr Gln Ser Pro Gly Thr Leu Ser Leu Ser Pro Gly
   1               5                   10                  15
   gaa aga gcc aca ctc tcc tgc agg gcc agt cag agt gtt agc agc aac        96
   Glu Arg Ala Thr Leu Ser Cys Arg Ala Ser Gln Ser Val Ser Ser Asn
               20                  25                  30
   tac tta ggc tgg tac cag cag aaa cct ggc cag gct ccc agg ctc ctg       144
   Tyr Leu Gly Trp Tyr Gln Gln Lys Pro Gly Gln Ala Pro Arg Leu Leu
           35                  40                  45
   atc tat ggt gca tcc agc agg gcc agt ggc atc cca gac agg ttc agt       192
   Ile Tyr Gly Ala Ser Ser Arg Ala Ser Gly Ile Pro Asp Arg Phe Ser
       50                  55                  60
   ggc ggt ggg tct ggg aca gac ttc act ctc acc atc agc aga ctg gag       240
   Gly Gly Gly Ser Gly Thr Asp Phe Thr Leu Thr Ile Ser Arg Leu Glu
   65                  70                  75                  80
   cct gaa gat ttt gca gtg tat tac tgt cag cag tat ggt agc tca ccc       288
   Pro Glu Asp Phe Ala Val Tyr Tyr Cys Gln Gln Tyr Gly Ser Ser Pro
                   85                  90                  95
   ctc act ttc ggc gga ggg acc aag gtg gag atc aaa cgt gcg gcc gca       336
   Leu Thr Phe Gly Gly Gly Thr Lys Val Glu Ile Lys Arg Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 343
   <211> 218
   <212> PRT
   <213> Artificial
<220>
   <223> Synthetic Construct
<400> 343
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Asn
               20                  25                  30
   Tyr Leu Gly Trp Tyr Gln Gln Lys Pro Gly Gln Ala Pro Arg Leu Leu
           35                  40                  45
   Ile Tyr Gly Ala Ser Ser Arg Ala Ser Gly Ile Pro Asp Arg Phe Ser
       50                  55                  60
   Gly Gly Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Leu Thr Phe Gly Gly Gly Thr Lys Val Glu Ile Lys Arg Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
                 195                 200                 205
   Glu Lys Thr Val Ala Pro Thr Glu Cys Ser
       210                 215
<210> 344
   <211> 1350
   <212> DNA
   <213> Artificial
<220>
   <223> CR6332 Heavy chain
<220>
   <221> CDS
   <222> (1)..(1350)
<400> 344
   cag gtg cag ctg gtg cag tct ggg gct gag gtg aag aag cct ggg tcc       48
   Gln Val Gln Leu Val Gln Ser Gly Ala Glu Val Lys Lys Pro Gly Ser
   1               5                   10                  15
   tcg gta aag gtc tcc tgc aag gct tct gga ggc ccc ttc cgc aat ttt       96
   Ser Val Lys Val Ser Cys Lys Ala Ser Gly Gly Pro Phe Arg Asn Phe
               20                  25                  30
   gct atc aac tgg gtg cga cag gcc cct gga caa ggg ctt gag tgg atg      144
   Ala Ile Asn Trp Val Arg Gln Ala Pro Gly Gln Gly Leu Glu Trp Met
           35                  40                  45
   gga ggg atc atc gct gtc ttt ggg acg aca aag tac gca cat aag ttc      192
   Gly Gly Ile Ile Ala Val Phe Gly Thr Thr Lys Tyr Ala His Lys Phe
       50                  55                  60
   cag ggc aga gtc acc atc acc gcg gac gac tcc aca aat aca gct tac      240
   Gln Gly Arg Val Thr Ile Thr Ala Asp Asp Ser Thr Asn Thr Ala Tyr
   65                  70                  75                  80
   atg gag ctg ggc agc ctg aaa tct gag gac acg gcc gtg tat tac tgt      288
   Met Glu Leu Gly Ser Leu Lys Ser Glu Asp Thr Ala Val Tyr Tyr Cys
                   85                  90                  95
   gcg aga ggt ccc cac tac tac tcc tcc tac atg gac gtc tgg ggc gaa      336
   Ala Arg Gly Pro His Tyr Tyr Ser Ser Tyr Met Asp Val Trp Gly Glu
               100                 105                 110
   ggg acc acg gtc acc gtc tcg agt gct agc acc aag ggc ccc agc gtg      384
   Gly Thr Thr Val Thr Val Ser Ser Ala Ser Thr Lys Gly Pro Ser Val
           115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg     1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg     1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac     1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac     1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc     1344
   Glu Ala Leu His Asn His Tyr Thr Gln Lys Ser Leu Ser Leu Ser Pro
           435                 440                 445
   ggc aag                                                             1350
   Gly Lys
       450
<210> 345
   <211> 450
   <212> PRT
   <213> Artificial
<220>
   <223> Synthetic Construct
<400> 345
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Pro Phe Arg Asn Phe
               20                  25                  30
   Ala Ile Asn Trp Val Arg Gln Ala Pro Gly Gln Gly Leu Glu Trp Met
           35                  40                  45
   Gly Gly Ile Ile Ala Val Phe Gly Thr Thr Lys Tyr Ala His Lys Phe
       50                  55                  60
   Gln Gly Arg Val Thr Ile Thr Ala Asp Asp Ser Thr Asn Thr Ala Tyr
   65                  70                  75                  80
   Met Glu Leu Gly Ser Leu Lys Ser Glu Asp Thr Ala Val Tyr Tyr Cys
                   85                  90                  95
   Ala Arg Gly Pro His Tyr Tyr Ser Ser Tyr Met Asp Val Trp Gly Glu
               100                 105                 110
   Gly Thr Thr Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
   450
<210> 346
   <211> 636
   <212> DNA
   <213> Artificial
<220>
   <223> CR6332 Light chain
<220>
   <221> CDS
   <222> (1)..(636)
<400> 346
   gac atc cag ttg acc cag tct cca tcc tcc ctg tct gca tct gta gga        48
   Asp Ile Gln Leu Thr Gln Ser Pro Ser Ser Leu Ser Ala Ser Val Gly
   1               5                   10                  15
   gac aga gtc acc atc act tgc cgg gcg agt cag ggc att agc act tat        96
   Asp Arg Val Thr Ile Thr Cys Arg Ala Ser Gln Gly Ile Ser Thr Tyr
               20                  25                  30
   tta gcc tgg tat cag cag aaa ccc ggg aaa gtt cct aaa ctc ctg atc       144
   Leu Ala Trp Tyr Gln Gln Lys Pro Gly Lys Val Pro Lys Leu Leu Ile
           35                  40                  45
   tat gct gca tcc act ttg caa tca ggg gtc cca tct cgg ttc agt ggc       192
   Tyr Ala Ala Ser Thr Leu Gln Ser Gly Val Pro Ser Arg Phe Ser Gly
       50                  55                  60
   agt gga tct ggg aca gat ttc act ctc acc atc agc agc ctg cag cct       240
   Ser Gly Ser Gly Thr Asp Phe Thr Leu Thr Ile Ser Ser Leu Gln Pro
   65                  70                  75                  80
   gaa gat gtt gca act tat tac tgt caa aag tat aac agt gcc cct tct       288
   Glu Asp Val Ala Thr Tyr Tyr Cys Gln Lys Tyr Asn Ser Ala Pro Ser
                   85                  90                  95
   ttc ggc cct ggg acc aaa gtg gat atc aaa cgt gcg gcc gca ccc agc       336
   Phe Gly Pro Gly Thr Lys Val Asp Ile Lys Arg Ala Ala Ala Pro Ser
               100                 105                 110
   gtg ttc atc ttc ccc ccc tcc gac gag cag ctg aag agc ggc acc gcc       384
   Val Phe Ile Phe Pro Pro Ser Asp Glu Gln Leu Lys Ser Gly Thr Ala
           115                 120                 125
   agc gtg gtg tgc ctg ctg aac aac ttc tac ccc cgg gag gcc aag gtg       432
   Ser Val Val Cys Leu Leu Asn Asn Phe Tyr Pro Arg Glu Ala Lys Val
       130                 135                 140
   cag tgg aag gtg gac aac gcc ctg cag agc ggc aac agc cag gag agc       480
   Gln Trp Lys Val Asp Asn Ala Leu Gln Ser Gly Asn Ser Gln Glu Ser
   145                 150                 155                 160
   gtg acc gag cag gac agc aag gac tcc acc tac agc ctg agc agc acc       528
   Val Thr Glu Gln Asp Ser Lys Asp Ser Thr Tyr Ser Leu Ser Ser Thr
                   165                 170                 175
   ctc acc ctg agc aag gcc gac tac gag aag cac aag gtg tac gcc tgc       576
   Leu Thr Leu Ser Lys Ala Asp Tyr Glu Lys His Lys Val Tyr Ala Cys
               180                 185                 190
   gag gtg acc cac cag ggc ctg agc agc ccc gtg acc aag agc ttc aac       624
   Glu Val Thr His Gln Gly Leu Ser Ser Pro Val Thr Lys Ser Phe Asn
           195                 200                 205
   cgg ggc gag tgt                                                       636
   Arg Gly Glu Cys
       210
<210> 347
   <211> 212
   <212> PRT
   <213> Artificial
<220>
   <223> Synthetic Construct
<400> 347
   Asp Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Thr Tyr
               20                  25                  30
   Leu Ala Trp Tyr Gln Gln Lys Pro Gly Lys Val Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Lys Tyr Asn Ser Ala Pro Ser
                   85                  90                  95
   Phe Gly Pro Gly Thr Lys Val Asp Ile Lys Arg Ala Ala Ala Pro Ser
               100                 105                 110
   Val Phe Ile Phe Pro Pro Ser Asp Glu Gln Leu Lys Ser Gly Thr Ala
           115                 120                 125
   Ser Val Val Cys Leu Leu Asn Asn Phe Tyr Pro Arg Glu Ala Lys Val
       130                 135                 140
   Gln Trp Lys Val Asp Asn Ala Leu Gln Ser Gly Asn Ser Gln Glu Ser
   145                 150                 155                 160
   Val Thr Glu Gln Asp Ser Lys Asp Ser Thr Tyr Ser Leu Ser Ser Thr
                   165                 170                 175
   Leu Thr Leu Ser Lys Ala Asp Tyr Glu Lys His Lys Val Tyr Ala Cys
               180                 185                 190
   Glu Val Thr His Gln Gly Leu Ser Ser Pro Val Thr Lys Ser Phe Asn
           195                 200                 205
   Arg Gly Glu Cys
       210
<210> 348
   <211> 1350
   <212> DNA
   <213> Artificial
<220>
   <223> CR6334 Heavy chain
<220>
   <221> CDS
   <222> (1)..(1350)
<400> 348
   gag gtg cag ctg gtg gag act ggg gct gag gtg aag aag cct ggg tcc       48
   tcg gtg aag gtc ccc tgc aaa tct tct gga agc ccc ttc agg agt aat       96
   gct gtc agc tgg gtg cga cag gcc ccc gga caa ggg ctt gag tgg gtg      144
   gga gga atc ctc ggt gtc ttt ggt tca cca agc tac gca cag aag ttc      192
   cag ggc aga gtc acg att acc gcg gac gaa tcc acc aac aca gtc cac      240
   atg gag ctg aga ggt ttg aga tct gag gac acg gcc gtg tat tat tgt      288
   gcg aga ggt cct acc tac tac tac tcc tac atg gac gtc tgg ggc aaa      336
   ggg acc acg gtc acc gtc tcg agt gct agc acc aag ggc ccc agc gtg      384
   ttc ccc ctg gcc ccc agc agc aag agc acc agc ggc ggc aca gcc gcc      432
   ctg ggc tgc ctg gtg aag gac tac ttc ccc gag ccc gtg acc gtg agc      480
   tgg aac agc ggc gcc ttg acc agc ggc gtg cac acc ttc ccc gcc gtg      528
   ctg cag agc agc ggc ctg tac agc ctg agc agc gtg gtg acc gtg ccc      576
   agc agc agc ctg ggc acc cag acc tac atc tgc aac gtg aac cac aag      624
   ccc agc aac acc aag gtg gac aaa cgc gtg gag ccc aag agc tgc gac      672
   aag acc cac acc tgc ccc ccc tgc cct gcc ccc gag ctg ctg ggc gga      720
   ccc tcc gtg ttc ctg ttc ccc ccc aag ccc aag gac acc ctc atg atc      768
   agc cgg acc ccc gag gtg acc tgc gtg gtg gtg gac gtg agc cac gag      816
   gac ccc gag gtg aag ttc aac tgg tac gtg gac ggc gtg gag gtg cac      864
   aac gcc aag acc aag ccc cgg gag gag cag tac aac agc acc tac cgg      912
   gtg gtg agc gtg ctc acc gtg ctg cac cag gac tgg ctg aac ggc aag      960
   gag tac aag tgc aag gtg agc aac aag gcc ctg cct gcc ccc atc gag     1008
   aag acc atc agc aag gcc aag ggc cag ccc cgg gag ccc cag gtg tac     1056
   acc ctg ccc ccc agc cgg gag gag atg acc aag aac cag gtg tcc ctc     1104
   acc tgt ctg gtg aag ggc ttc tac ccc agc gac atc gcc gtg gag tgg     1152
   gag agc aac ggc cag ccc gag aac aac tac aag acc acc ccc cct gtg     1200
   ctg gac agc gac ggc agc ttc ttc ctg tac agc aag ctc acc gtg gac     1248
   aag agc cgg tgg cag cag ggc aac gtg ttc agc tgc agc gtg atg cac     1296
   gag gcc ctg cac aac cac tac acc cag aag agc ctg agc ctg agc ccc      1344
   ggc aag                                                              1350
<210> 349
   <211> 450
   <212> PRT
   <213> Artificial
<220>
   <223> Synthetic Construct
<400> 349
<210> 350
   <211> 654
   <212> DNA
   <213> Artificial
<220>
   <223> CR6334 Light chain
<220>
   <221> CDS
   <222> (1)..(654)
<400> 350
   tcc tat gtg ctg act cag cca ccc tcg gag tca gtg gcc cca gga cag      48
   Ser Tyr Val Leu Thr Gln Pro Pro Ser Glu Ser Val Ala Pro Gly Gln
   1               5                   10                  15
   acg gcc agg att acc tgt ggg gga aat aac att gga aga aat agt gtg      96
   Thr Ala Arg Ile Thr Cys Gly Gly Asn Asn Ile Gly Arg Asn Ser Val
               20                  25                  30
   cac tgg tat cag cag aag cca ggc cag gcc cct gtg ctg gtc gtg tat     144
   His Trp Tyr Gln Gln Lys Pro Gly Gln Ala Pro Val Leu Val Val Tyr
           35                  40                  45
   gat gat agc gac cgg ccc tca ggg atc cct gag cga ttt tct ggc tcc     192
   Asp Asp Ser Asp Arg Pro Ser Gly Ile Pro Glu Arg Phe Ser Gly Ser
       50                  55                  60
   aag tct ggg aac acg gcc acc ctg att atc agc agg gtc gaa gtc ggg     240
   Lys Ser Gly Asn Thr Ala Thr Leu Ile Ile Ser Arg Val Glu Val Gly
   65                  70                  75                  80
   gat gag gcc gac tac tac tgt cag gtg tgg cat agt agt agt gat cat     288
   Asp Glu Ala Asp Tyr Tyr Cys Gln Val Trp His Ser Ser Ser Asp His
                   85                  90                  95
   tat gtc ttc gga act ggg acc aag gtc acc gtc cta ggt gcg gcc gca     336
   Tyr Val Phe Gly Thr Gly Thr Lys Val Thr Val Leu Gly Ala Ala Ala
               100                 105                 110
   ggc cag ccc aag gcc gct ccc agc gtg acc ctg ttc ccc ccc tcc tcc     384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac     432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc     480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac     528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag     576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg     624
   Ser His Arg Ser Tyr Ser Cys Gln Val Thr His Glu Gly Ser Thr Val
           195                 200                 205
   gag aag acc gtg gcc ccc acc gag tgc agc                             654
   Glu Lys Thr Val Ala Pro Thr Glu Cys Ser
       210                 215
<210> 351
   <211> 218
   <212> PRT
   <213> Artificial
<220>
   <223> Synthetic Construct
<400> 351
   Ser Tyr Val Leu Thr Gln Pro Pro Ser Glu Ser Val Ala Pro Gly Gln
   1               5                   10                  15
   Thr Ala Arg Ile Thr Cys Gly Gly Asn Asn Ile Gly Arg Asn Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Lys Ser Gly Asn Thr Ala Thr Leu Ile Ile Ser Arg Val Glu Val Gly
   65                  70                  75                  80
   Asp Glu Ala Asp Tyr Tyr Cys Gln Val Trp His Ser Ser Ser Asp His
                   85                  90                  95
   Tyr Val Phe Gly Thr Gly Thr Lys Val Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 352
   <211> 1350
   <212> DNA
   <213> Artificial
<220>
   <223> CR6336 Heavy chain
<220>
   <221> CDS
   <222> (1)..(1350)
<400> 352
   cag atg cag ctg gta caa tct gga gct gag gtg aag aag cct ggg tcc       48
   Gln Met Gln Leu Val Gln Ser Gly Ala Glu Val Lys Lys Pro Gly Ser
   1               5                   10                  15
   tcg gtg aag gtc tcc tgc aag gct tct gga ggc acc ttc agc agc tat       96
   Ser Val Lys Val Ser Cys Lys Ala Ser Gly Gly Thr Phe Ser Ser Tyr
               20                  25                  30
   gct atc agc tgg gtg cga cag gcc cct gga caa ggg ctt gag tgg atg      144
   Ala Ile Ser Trp Val Arg Gln Ala Pro Gly Gln Gly Leu Glu Trp Met
           35                  40                  45
   gga ggg atc ttc ggt atg ttt ggg aca gca aac tac gcg cag aag ttc      192
   Gly Gly Ile Phe Gly Met Phe Gly Thr Ala Asn Tyr Ala Gln Lys Phe
       50                  55                  60
   cag ggc aga gtc acg att acc gcg gac gaa ttc acg agc gcg gcc tac      240
   Gln Gly Arg Val Thr Ile Thr Ala Asp Glu Phe Thr Ser Ala Ala Tyr
   65                  70                  75                  80
   atg gag ctg agc agc ctg gga tct gag gac acg gcc atg tat tac tgt      288
   Met Glu Leu Ser Ser Leu Gly Ser Glu Asp Thr Ala Met Tyr Tyr Cys
                   85                  90                  95
   gcg agg tct agt ggt tat tac ccc caa tac ttc cag gac tgg ggc cag      336
   Ala Arg Ser Ser Gly Tyr Tyr Pro Gln Tyr Phe Gln Asp Trp Gly Gln
               100                 105                 110
   ggc acc ctg gtc acc gtc tcg agt gct agc acc aag ggc ccc agc gtg      384
   Gly Thr Leu Val Thr Val Ser Ser Ala Ser Thr Lys Gly Pro Ser Val
           115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg     1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg     1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac     1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac      1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc      1344
   Glu Ala Leu His Asn His Tyr Thr Gln Lys Ser Leu Ser Leu Ser Pro
           435                 440                 445
   ggc aag                                                              1350
   Gly Lys
       450
<210> 353
   <211> 450
   <212> PRT
   <213> Artificial
<220>
   <223> Synthetic Construct
<400> 353
   Gln Met Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Ser Tyr
               20                  25                  30
   Ala Ile Ser Trp Val Arg Gln Ala Pro Gly Gln Gly Leu Glu Trp Met
           35                  40                  45
   Gly Gly Ile Phe Gly Met Phe Gly Thr Ala Asn Tyr Ala Gln Lys Phe
       50                  55                  60
   Gln Gly Arg Val Thr Ile Thr Ala Asp Glu Phe Thr Ser Ala Ala Tyr
   65                  70                  75                  80
   Met Glu Leu Ser Ser Leu Gly Ser Glu Asp Thr Ala Met Tyr Tyr Cys
                   85                  90                  95
   Ala Arg Ser Ser Gly Tyr Tyr Pro Gln Tyr Phe Gln Asp Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Glu Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 354
   <211> 642
   <212> DNA
   <213> Artificial
<220>
   <223> CR6336 Light chain
<220>
   <221> CDS
   <222> (1)..(642)
<400> 354
   gaa att gtg atg aca cag tct cca ggc acc ctg tct ttg tct cca ggg        48
   Glu Ile Val Met Thr Gln Ser Pro Gly Thr Leu Ser Leu Ser Pro Gly
   1               5                   10                  15
   caa aga gcc acc ctc tcc tgc agg gcc agt cag agt gtt agc agc agc        96
   Gln Arg Ala Thr Leu Ser Cys Arg Ala Ser Gln Ser Val Ser Ser Ser
               20                  25                  30
   tac tta gcc tgg tac cag cag aaa cct ggc cag gct ccc aga ctc ctc       144
   Tyr Leu Ala Trp Tyr Gln Gln Lys Pro Gly Gln Ala Pro Arg Leu Leu
           35                  40                  45
   atg tat ggt gca tcc agc agg gcc act ggc atc cca gac agg ttc agt       192
   Met Tyr Gly Ala Ser Ser Arg Ala Thr Gly Ile Pro Asp Arg Phe Ser
       50                  55                  60
   ggc agt ggg tct ggg aca gac ttc act ctc acc atc agc aga ctg gag       240
   Gly Ser Gly Ser Gly Thr Asp Phe Thr Leu Thr Ile Ser Arg Leu Glu
   65                  70                  75                  80
   cct gaa gat ttt gca gtg tat tac tgt cag cag tat ggt agc tca tcg       288
   Pro Glu Asp Phe Ala Val Tyr Tyr Cys Gln Gln Tyr Gly Ser Ser Ser
                   85                  90                  95
   ctc act ttc ggc gga ggg acc aag ctg gag atc aaa cgt gcg gcc gca       336
   Leu Thr Phe Gly Gly Gly Thr Lys Leu Glu Ile Lys Arg Ala Ala Ala
               100                 105                 110
   ccc agc gtg ttc atc ttc ccc ccc tcc gac gag cag ctg aag agc ggc       384
   Pro Ser Val Phe Ile Phe Pro Pro Ser Asp Glu Gln Leu Lys Ser Gly
           115                 120                 125
   acc gcc agc gtg gtg tgc ctg ctg aac aac ttc tac ccc cgg gag gcc       432
   Thr Ala Ser Val Val Cys Leu Leu Asn Asn Phe Tyr Pro Arg Glu Ala
       130                 135                 140
   aag gtg cag tgg aag gtg gac aac gcc ctg cag agc ggc aac agc cag       480
   Lys Val Gln Trp Lys Val Asp Asn Ala Leu Gln Ser Gly Asn Ser Gln
   145                 150                 155                 160
   gag agc gtg acc gag cag gac agc aag gac tcc acc tac agc ctg agc       528
   Glu Ser Val Thr Glu Gln Asp Ser Lys Asp Ser Thr Tyr Ser Leu Ser
                   165                 170                 175
   agc acc ctc acc ctg agc aag gcc gac tac gag aag cac aag gtg tac       576
   Ser Thr Leu Thr Leu Ser Lys Ala Asp Tyr Glu Lys His Lys Val Tyr
               180                 185                 190
   gcc tgc gag gtg acc cac cag ggc ctg agc agc ccc gtg acc aag agc       624
   Ala Cys Glu Val Thr His Gln Gly Leu Ser Ser Pro Val Thr Lys Ser
           195                 200                 205
   ttc aac cgg ggc gag tgt                                               642
   Phe Asn Arg Gly Glu Cys
       210
<210> 355
   <211> 214
   <212> PRT
   <213> Artificial
<220>
   <223> Synthetic Construct
<400> 355
   Glu Ile Val Met Thr Gln Ser Pro Gly Thr Leu Ser Leu Ser Pro Gly
   1               5                   10                  15
   Gln Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Met Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Ser
                   85                  90                  95
   Leu Thr Phe Gly Gly Gly Thr Lys Leu Glu Ile Lys Arg Ala Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 356
   <211> 1353
   <212> DNA
   <213> Artificial
<220>
   <223> CR6339 Heavy chain
<220>
   <221> CDS
   <222> (1)..(1353)
<400> 356
   gag gtg cag ctg gtg gag tcc ggg gct gag gtg aag aag cct ggg tcc      48
   Glu Val Gln Leu Val Glu Ser Gly Ala Glu Val Lys Lys Pro Gly Ser
   1               5                   10                  15
   tcg gtg aag gtc tcc tgc aag gct tct gga ggc atc ttc aac agt tat      96
   Ser Val Lys Val Ser Cys Lys Ala Ser Gly Gly Ile Phe Asn Ser Tyr
               20                  25                  30
   gct atc agc tgg gtg cga cag gcc cct gga caa ggg ctt gag tgg atg     144
   Ala Ile Ser Trp Val Arg Gln Ala Pro Gly Gln Gly Leu Glu Trp Met
           35                  40                  45
   gga ggc atc atc gct atc ttt cat aca cca aag tac gca cag aag ttc     192
   Gly Gly Ile Ile Ala Ile Phe His Thr Pro Lys Tyr Ala Gln Lys Phe
       50                  55                  60
   cag ggc aga gtc acg att acc gcg gac gaa tcc acg aac aca gcc tac     240
   Gln Gly Arg Val Thr Ile Thr Ala Asp Glu Ser Thr Asn Thr Ala Tyr
   65                  70                  75                  80
   atg gaa ctg aga agc ctg aaa tct gag gac acg gcc ctg tat tac tgt     288
   Met Glu Leu Arg Ser Leu Lys Ser Glu Asp Thr Ala Leu Tyr Tyr Cys
                   85                  90                  95
   gcg aga ggg tcc act tac gat ttt tcg agt ggc ctt gac tac tgg ggc     336
   Ala Arg Gly Ser Thr Tyr Asp Phe Ser Ser Gly Leu Asp Tyr Trp Gly
               100                 105                 110
   cag gga acc ctg gtc acc gtc tcg agt gct agc acc aag ggc ccc agc     384
   Gln Gly Thr Leu Val Thr Val Ser Ser Ala Ser Thr Lys Gly Pro Ser
           115                 120                 125
   gtg ttc ccc ctg gcc ccc agc agc aag agc acc agc ggc ggc aca gcc      432
   Val Phe Pro Leu Ala Pro Ser Ser Lys Ser Thr Ser Gly Gly Thr Ala
       130                 135                 140
   gcc ctg ggc tgc ctg gtg aag gac tac ttc ccc gag ccc gtg acc gtg      480
   Ala Leu Gly Cys Leu Val Lys Asp Tyr Phe Pro Glu Pro Val Thr Val
   145                 150                 155                 160
   agc tgg aac agc ggc gcc ttg acc agc ggc gtg cac acc ttc ccc gcc      528
   Ser Trp Asn Ser Gly Ala Leu Thr Ser Gly Val His Thr Phe Pro Ala
                   165                 170                 175
   gtg ctg cag agc agc ggc ctg tac agc ctg agc agc gtg gtg acc gtg      576
   Val Leu Gln Ser Ser Gly Leu Tyr Ser Leu Ser Ser Val Val Thr Val
               180                 185                 190
   ccc agc agc agc ctg ggc acc cag acc tac atc tgc aac gtg aac cac      624
   Pro Ser Ser Ser Leu Gly Thr Gln Thr Tyr Ile Cys Asn Val Asn His
           195                 200                 205
   aag ccc agc aac acc aag gtg gac aaa cgc gtg gag ccc aag agc tgc      672
   Lys Pro Ser Asn Thr Lys Val Asp Lys Arg Val Glu Pro Lys Ser Cys
       210                 215                 220
   gac aag acc cac acc tgc ccc ccc tgc cct gcc ccc gag ctg ctg ggc      720
   Asp Lys Thr His Thr Cys Pro Pro Cys Pro Ala Pro Glu Leu Leu Gly
   225                 230                 235                 240
   gga ccc tcc gtg ttc ctg ttc ccc ccc aag ccc aag gac acc ctc atg      768
   Gly Pro Ser Val Phe Leu Phe Pro Pro Lys Pro Lys Asp Thr Leu Met
                   245                 250                 255
   atc agc cgg acc ccc gag gtg acc tgc gtg gtg gtg gac gtg agc cac      816
   Ile Ser Arg Thr Pro Glu Val Thr Cys Val Val Val Asp Val Ser His
               260                 265                 270
   gag gac ccc gag gtg aag ttc aac tgg tac gtg gac ggc gtg gag gtg      864
   Glu Asp Pro Glu Val Lys Phe Asn Trp Tyr Val Asp Gly Val Glu Val
           275                 280                 285
   cac aac gcc aag acc aag ccc cgg gag gag cag tac aac agc acc tac      912
   His Asn Ala Lys Thr Lys Pro Arg Glu Glu Gln Tyr Asn Ser Thr Tyr
       290                 295                 300
   cgg gtg gtg agc gtg ctc acc gtg ctg cac cag gac tgg ctg aac ggc      960
   Arg Val Val Ser Val Leu Thr Val Leu His Gln Asp Trp Leu Asn Gly
   305                 310                 315                 320
   aag gag tac aag tgc aag gtg agc aac aag gcc ctg cct gcc ccc atc     1008
   Lys Glu Tyr Lys Cys Lys Val Ser Asn Lys Ala Leu Pro Ala Pro Ile
                   325                 330                 335
   gag aag acc atc agc aag gcc aag ggc cag ccc cgg gag ccc cag gtg     1056
   Glu Lys Thr Ile Ser Lys Ala Lys Gly Gln Pro Arg Glu Pro Gln Val
               340                 345                 350
   tac acc ctg ccc ccc agc cgg gag gag atg acc aag aac cag gtg tcc     1104
   Tyr Thr Leu Pro Pro Ser Arg Glu Glu Met Thr Lys Asn Gln Val Ser
           355                 360                 365
   ctc acc tgt ctg gtg aag ggc ttc tac ccc agc gac atc gcc gtg gag     1152
   Leu Thr Cys Leu Val Lys Gly Phe Tyr Pro Ser Asp Ile Ala Val Glu
       370                 375                 380
   tgg gag agc aac ggc cag ccc gag aac aac tac aag acc acc ccc cct     1200
   Trp Glu Ser Asn Gly Gln Pro Glu Asn Asn Tyr Lys Thr Thr Pro Pro
   385                 390                 395                 400
   gtg ctg gac agc gac ggc agc ttc ttc ctg tac agc aag ctc acc gtg      1248
   Val Leu Asp Ser Asp Gly Ser Phe Phe Leu Tyr Ser Lys Leu Thr Val
                   405                 410                 415
   gac aag agc cgg tgg cag cag ggc aac gtg ttc agc tgc agc gtg atg      1296
   Asp Lys Ser Arg Trp Gln Gln Gly Asn Val Phe Ser Cys Ser Val Met
               420                 425                 430
   cac gag gcc ctg cac aac cac tac acc cag aag agc ctg agc ctg agc      1344
   His Glu Ala Leu His Asn His Tyr Thr Gln Lys Ser Leu Ser Leu Ser
           435                 440                 445
   ccc ggc aag                                                          1353
   Pro Gly Lys
       450
<210> 357
   <211> 451
   <212> PRT
   <213> Artificial
<220>
   <223> Synthetic Construct
<400> 357
   Glu Val Gln Leu Val Glu Ser Gly Ala Glu Val Lys Lys Pro Gly Ser
   1               5                   10                  15
   Ser Val Lys Val Ser Cys Lys Ala Ser Gly Gly Ile Phe Asn Ser Tyr
               20                  25                  30
   Ala Ile Ser Trp Val Arg Gln Ala Pro Gly Gln Gly Leu Glu Trp Met
           35                  40                  45
   Gly Gly Ile Ile Ala Ile Phe His Thr Pro Lys Tyr Ala Gln Lys Phe
       50                  55                  60
   Gln Gly Arg Val Thr Ile Thr Ala Asp Glu Ser Thr Asn Thr Ala Tyr
   65                  70                  75                  80
   Met Glu Leu Arg Ser Leu Lys Ser Glu Asp Thr Ala Leu Tyr Tyr Cys
                   85                  90                  95
   Ala Arg Gly Ser Thr Tyr Asp Phe Ser Ser Gly Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Glu Glu Met Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 358
   <211> 654
   <212> DNA
   <213> Artificial
<220>
   <223> CR6339 Light chain
<220>
   <221> CDS
   <222> (1)..(654)
<400> 358
   cag gca ggg ctg act cag cca ccc tcg gtg tca gtg gcc cca gga cag        48
   Gln Ala Gly Leu Thr Gln Pro Pro Ser Val Ser Val Ala Pro Gly Gln
   1               5                   10                  15
   acg gcc agg att acc tgt ggg gga aac aac att gga agt aaa agt gtg        96
   Thr Ala Arg Ile Thr Cys Gly Gly Asn Asn Ile Gly Ser Lys Ser Val
               20                  25                  30
   cac tgg tac cag cag aag cca ggc cag gcc cct gtc cta gtc gtc tat       144
   His Trp Tyr Gln Gln Lys Pro Gly Gln Ala Pro Val Leu Val Val Tyr
           35                  40                  45
   gat gat agc gac cgg ccc tca ggg atc cct gag cga ttc tct ggc tcc       192
   Asp Asp Ser Asp Arg Pro Ser Gly Ile Pro Glu Arg Phe Ser Gly Ser
       50                  55                  60
   aac tct ggg aac acg gcc acc ctg acc atc agc agg gtc gaa gcc ggg       240
   Asn Ser Gly Asn Thr Ala Thr Leu Thr Ile Ser Arg Val Glu Ala Gly
   65                  70                  75                  80
   gat gag gcc gac tat tac tgt cag gtg tgg gat agt agt agt gat cat       288
   Asp Glu Ala Asp Tyr Tyr Cys Gln Val Trp Asp Ser Ser Ser Asp His
                   85                  90                  95
   gtg gta ttc ggc gga ggg acc aag ctg acc gtc cta ggt gcg gcc gca       336
   Val Val Phe Gly Gly Gly Thr Lys Leu Thr Val Leu Gly Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 359
   <211> 218
   <212> PRT
   <213> Artificial
<220>
   <223> Synthetic Construct
<400> 359
   Gln Ala Gly Leu Thr Gln Pro Pro Ser Val Ser Val Ala Pro Gly Gln
   1               5                   10                  15
   Thr Ala Arg Ile Thr Cys Gly Gly Asn Asn Ile Gly Ser Lys Ser Val
               20                  25                  30
   His Trp Tyr Gln Gln Lys Pro Gly Gln Ala Pro Val Leu Val Val Tyr
           35                  40                  45
   Asp Asp Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Asp Ser Ser Ser Asp His
                   85                  90                  95
   Val Val Phe Gly Gly Gly Thr Lys Leu Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 360
   <211> 1368
   <212> DNA
   <213> Artificial
<220>
   <223> CR6342 Heavy chain
<220>
   <221> CDS
   <222> (1)..(1368)
<400> 360
   cag gtc cag ctg gtg cag tct ggg gct gag gtg aag aag cct ggg tcc        48
   Gln Val Gln Leu Val Gln Ser Gly Ala Glu Val Lys Lys Pro Gly Ser
   1               5                   10                  15
   tcg gtg aag gtc tcc tgc aag gct tct gga ggc ttc ttc agc agc tat        96
   Ser Val Lys Val Ser Cys Lys Ala Ser Gly Gly Phe Phe Ser Ser Tyr
               20                  25                  30
   gct atc agc tgg gtg cgc cag gcc cct gga caa gga ctt gag tgg atg       144
   Ala Ile Ser Trp Val Arg Gln Ala Pro Gly Gln Gly Leu Glu Trp Met
           35                  40                  45
   ggg ggg gtc atc cct atc ttt cgt aca gca aac tac gca cag aac ttc       192
   Gly Gly Val Ile Pro Ile Phe Arg Thr Ala Asn Tyr Ala Gln Asn Phe
       50                  55                  60
   cag ggc aga gtc acc att acc gcg gac gaa ttc aca tcg tat atg gag       240
   Gln Gly Arg Val Thr Ile Thr Ala Asp Glu Phe Thr Ser Tyr Met Glu
   65                  70                  75                  80
   ctg agc agc ctg aga tct gac gac acg gcc gtg tat tac tgt gcg agg       288
   Leu Ser Ser Leu Arg Ser Asp Asp Thr Ala Val Tyr Tyr Cys Ala Arg
                   85                  90                  95
   ttg aat tac cat gat tcg ggg act tat tat aac gcc ccc cgg ggc tgg       336
   Leu Asn Tyr His Asp Ser Gly Thr Tyr Tyr Asn Ala Pro Arg Gly Trp
               100                 105                 110
   ttc gac ccc tgg ggc cag gga acc ctg gtc acc gtc tcg agt gct agc      384
   Phe Asp Pro Trp Gly Gln Gly Thr Leu Val Thr Val Ser Ser Ala Ser
           115                 120                 125
   acc aag ggc ccc agc gtg ttc ccc ctg gcc ccc agc agc aag agc acc      432
   Thr Lys Gly Pro Ser Val Phe Pro Leu Ala Pro Ser Ser Lys Ser Thr
       130                 135                 140
   agc ggc ggc aca gcc gcc ctg ggc tgc ctg gtg aag gac tac ttc ccc      480
   Ser Gly Gly Thr Ala Ala Leu Gly Cys Leu Val Lys Asp Tyr Phe Pro
   145                 150                 155                 160
   gag ccc gtg acc gtg agc tgg aac agc ggc gcc ttg acc agc ggc gtg      528
   Glu Pro Val Thr Val Ser Trp Asn Ser Gly Ala Leu Thr Ser Gly Val
                   165                 170                 175
   cac acc ttc ccc gcc gtg ctg cag agc agc ggc ctg tac agc ctg agc      576
   His Thr Phe Pro Ala Val Leu Gln Ser Ser Gly Leu Tyr Ser Leu Ser
               180                 185                 190
   agc gtg gtg acc gtg ccc agc agc agc ctg ggc acc cag acc tac atc      624
   Ser Val Val Thr Val Pro Ser Ser Ser Leu Gly Thr Gln Thr Tyr Ile
           195                 200                 205
   tgc aac gtg aac cac aag ccc agc aac acc aag gtg gac aaa cgc gtg      672
   Cys Asn Val Asn His Lys Pro Ser Asn Thr Lys Val Asp Lys Arg Val
       210                 215                 220
   gag ccc aag agc tgc gac aag acc cac acc tgc ccc ccc tgc cct gcc      720
   Glu Pro Lys Ser Cys Asp Lys Thr His Thr Cys Pro Pro Cys Pro Ala
   225                 230                 235                 240
   ccc gag ctg ctg ggc gga ccc tcc gtg ttc ctg ttc ccc ccc aag ccc      768
   Pro Glu Leu Leu Gly Gly Pro Ser Val Phe Leu Phe Pro Pro Lys Pro
                   245                 250                 255
   aag gac acc ctc atg atc agc cgg acc ccc gag gtg acc tgc gtg gtg      816
   Lys Asp Thr Leu Met Ile Ser Arg Thr Pro Glu Val Thr Cys Val Val
               260                 265                 270
   gtg gac gtg agc cac gag gac ccc gag gtg aag ttc aac tgg tac gtg      864
   Val Asp Val Ser His Glu Asp Pro Glu Val Lys Phe Asn Trp Tyr Val
           275                 280                 285
   gac ggc gtg gag gtg cac aac gcc aag acc aag ccc cgg gag gag cag      912
   Asp Gly Val Glu Val His Asn Ala Lys Thr Lys Pro Arg Glu Glu Gln
       290                 295                 300
   tac aac agc acc tac cgg gtg gtg agc gtg ctc acc gtg ctg cac cag      960
   Tyr Asn Ser Thr Tyr Arg Val Val Ser Val Leu Thr Val Leu His Gln
   305                 310                 315                 320
   gac tgg ctg aac ggc aag gag tac aag tgc aag gtg agc aac aag gcc     1008
   Asp Trp Leu Asn Gly Lys Glu Tyr Lys Cys Lys Val Ser Asn Lys Ala
                   325                 330                 335
   ctg cct gcc ccc atc gag aag acc atc agc aag gcc aag ggc cag ccc     1056
   Leu Pro Ala Pro Ile Glu Lys Thr Ile Ser Lys Ala Lys Gly Gln Pro
               340                 345                 350
   cgg gag ccc cag gtg tac acc ctg ccc ccc agc cgg gag gag atg acc     1104
   Arg Glu Pro Gln Val Tyr Thr Leu Pro Pro Ser Arg Glu Glu Met Thr
           355                 360                 365
   aag aac cag gtg tcc ctc acc tgt ctg gtg aag ggc ttc tac ccc agc     1152
   Lys Asn Gln Val Ser Leu Thr Cys Leu Val Lys Gly Phe Tyr Pro Ser
       370                 375                 380
   gac atc gcc gtg gag tgg gag agc aac ggc cag ccc gag aac aac tac      1200
   Asp Ile Ala Val Glu Trp Glu Ser Asn Gly Gln Pro Glu Asn Asn Tyr
   385                 390                 395                 400
   aag acc acc ccc cct gtg ctg gac agc gac ggc agc ttc ttc ctg tac      1248
   Lys Thr Thr Pro Pro Val Leu Asp Ser Asp Gly Ser Phe Phe Leu Tyr
                   405                 410                 415
   agc aag ctc acc gtg gac aag agc cgg tgg cag cag ggc aac gtg ttc      1296
   Ser Lys Leu Thr Val Asp Lys Ser Arg Trp Gln Gln Gly Asn Val Phe
               420                 425                 430
   agc tgc agc gtg atg cac gag gcc ctg cac aac cac tac acc cag aag      1344
   Ser Cys Ser Val Met His Glu Ala Leu His Asn His Tyr Thr Gln Lys
           435                 440                 445
   agc ctg agc ctg agc ccc ggc aag                                      1368
   Ser Leu Ser Leu Ser Pro Gly Lys
       450                 455
<210> 361
   <211> 456
   <212> PRT
   <213> Artificial
<220>
   <223> Synthetic Construct
<400> 361
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Phe Phe Ser Ser Tyr
               20                  25                  30
   Ala Ile Ser Trp Val Arg Gln Ala Pro Gly Gln Gly Leu Glu Trp Met
           35                  40                  45
   Gly Gly Val Ile Pro Ile Phe Arg Thr Ala Asn Tyr Ala Gln Asn Phe
       50                  55                  60
   Gln Gly Arg Val Thr Ile Thr Ala Asp Glu Phe Thr Ser Tyr Met Glu
   65                  70                  75                  80
   Leu Ser Ser Leu Arg Ser Asp Asp Thr Ala Val Tyr Tyr Cys Ala Arg
                   85                  90                  95
   Leu Asn Tyr His Asp Ser Gly Thr Tyr Tyr Asn Ala Pro Arg Gly Trp
               100                 105                 110
   Phe Asp Pro Trp Gly Gln Gly Thr Leu Val Thr Val Ser Ser Ala Ser
           115                 120                 125
   Thr Lys Gly Pro Ser Val Phe Pro Leu Ala Pro Ser Ser Lys Ser Thr
       130                 135                 140
   Ser Gly Gly Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Ser Leu Gly Thr Gln Thr Tyr Ile
           195                 200                 205
   Cys Asn Val Asn His Lys Pro Ser Asn Thr Lys Val Asp Lys Arg Val
       210                 215                 220
   Glu Pro Lys Ser Cys Asp Lys Thr His Thr Cys Pro Pro Cys Pro Ala
   225                 230                 235                 240
   Pro Glu Leu Leu Gly Gly Pro Ser Val Phe Leu Phe Pro Pro Lys Pro
                   245                 250                 255
   Lys Asp Thr Leu Met Ile Ser Arg Thr Pro Glu Val Thr Cys Val Val
               260                 265                 270
   Val Asp Val Ser His Glu Asp Pro Glu Val Lys Phe Asn Trp Tyr Val
           275                 280                 285
   Asp Gly Val Glu Val His Asn Ala Lys Thr Lys Pro Arg Glu Glu Gln
       290                 295                 300
   Tyr Asn Ser Thr Tyr Arg Val Val Ser Val Leu Thr Val Leu His Gln
   305                 310                 315                 320
   Asp Trp Leu Asn Gly Lys Glu Tyr Lys Cys Lys Val Ser Asn Lys Ala
                   325                 330                 335
   Leu Pro Ala Pro Ile Glu Lys Thr Ile Ser Lys Ala Lys Gly Gln Pro
               340                 345                 350
   Arg Glu Pro Gln Val Tyr Thr Leu Pro Pro Ser Arg Glu Glu Met Thr
           355                 360                 365
   Lys Asn Gln Val Ser Leu Thr Cys Leu Val Lys Gly Phe Tyr Pro Ser
       370                 375                 380
   Asp Ile Ala Val Glu Trp Glu Ser Asn Gly Gln Pro Glu Asn Asn Tyr
   385 390 395 400
   Lys Thr Thr Pro Pro Val Leu Asp Ser Asp Gly Ser Phe Phe Leu Tyr
   405 410 415
   Ser Lys Leu Thr Val Asp Lys Ser Arg Trp Gln Gln Gly Asn Val Phe
   420 425 430
   Ser Cys Ser Val Met His Glu Ala Leu His Asn His Tyr Thr Gln Lys
   435 440 445
   Ser Leu Ser Leu Ser Pro Gly Lys
   450 455
<210> 362
   <211> 657
   <212> DNA
   <213> Artificial
<220>
   <223> CR6342 Light chain
<220>
   <221> CDS
   <222> (1)..(657)
<400> 362
   gac atc cag atg acc cag tct cca gac tcc ctg gct gtg tct ctg ggc      48
   Asp Ile Gln Met Thr Gln Ser Pro Asp Ser Leu Ala Val Ser Leu Gly
   1               5                   10                  15
   gag aag gcc acc atc aac tgc aag tcc agc cag agt att tta aac agc      96
   Glu Lys Ala Thr Ile Asn Cys Lys Ser Ser Gln Ser Ile Leu Asn Ser
               20                  25                  30
   tcc aac aat aag aac tac tta gct tgg tac cag cag aaa cca gga cag     144
   Ser Asn Asn Lys Asn Tyr Leu Ala Trp Tyr Gln Gln Lys Pro Gly Gln
           35                  40                  45
   cct cct aag ctg ctc att tac tgg gca tct acc cgg gaa tcc ggg gtc     192
   Pro Pro Lys Leu Leu Ile Tyr Trp Ala Ser Thr Arg Glu Ser Gly Val
       50                  55                  60
   cct gac cga ttc agt ggc agc ggg tct ggg aca gat ttc act ctc acc     240
   Pro Asp Arg Phe Ser Gly Ser Gly Ser Gly Thr Asp Phe Thr Leu Thr
   65                  70                  75                  80
   atc agc agc ctg cag gct gaa gat gtg gca gtt tat tac tgt cag caa     288
   Ile Ser Ser Leu Gln Ala Glu Asp Val Ala Val Tyr Tyr Cys Gln Gln
                   85                  90                  95
   tat tat agt agt ccg ccg acg ttc ggc caa ggg acc aag gtg gaa atc     336
   Tyr Tyr Ser Ser Pro Pro Thr Phe Gly Gln Gly Thr Lys Val Glu Ile
               100                 105                 110
   aaa cgt gcg gcc gca ccc agc gtg ttc atc ttc ccc ccc tcc gac gag     384
   Lys Arg Ala Ala Ala Pro Ser Val Phe Ile Phe Pro Pro Ser Asp Glu
           115                 120                 125
   cag ctg aag agc ggc acc gcc agc gtg gtg tgc ctg ctg aac aac ttc     432
   Gln Leu Lys Ser Gly Thr Ala Ser Val Val Cys Leu Leu Asn Asn Phe
       130                 135                 140
   tac ccc cgg gag gcc aag gtg cag tgg aag gtg gac aac gcc ctg cag     480
   Tyr Pro Arg Glu Ala Lys Val Gln Trp Lys Val Asp Asn Ala Leu Gln
   145                 150                 155                 160
   agc ggc aac agc cag gag agc gtg acc gag cag gac agc aag gac tcc     528
   Ser Gly Asn Ser Gln Glu Ser Val Thr Glu Gln Asp Ser Lys Asp Ser
                   165                 170                 175
   acc tac agc ctg agc agc acc ctc acc ctg agc aag gcc gac tac gag     576
   Thr Tyr Ser Leu Ser Ser Thr Leu Thr Leu Ser Lys Ala Asp Tyr Glu
               180                 185                 190
   aag cac aag gtg tac gcc tgc gag gtg acc cac cag ggc ctg agc agc     624
   Lys His Lys Val Tyr Ala Cys Glu Val Thr His Gln Gly Leu Ser Ser
           195                 200                 205
   ccc gtg acc aag agc ttc aac cgg ggc gag tgt                         657
   Pro Val Thr Lys Ser Phe Asn Arg Gly Glu Cys
       210                 215
<210> 363
   <211> 219
   <212> PRT
   <213> Artificial
<220>
   <223> Synthetic Construct
<400> 363
   Asp Ile Gln Met Thr Gln Ser Pro Asp Ser Leu Ala Val Ser Leu Gly
   1               5                   10                  15
   Glu Lys Ala Thr Ile Asn Cys Lys Ser Ser Gln Ser Ile Leu Asn Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Ser Pro Pro Thr Phe Gly Gln Gly Thr Lys Val Glu Ile
               100                 105                 110
   Lys Arg Ala Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 364
   <211> 1350
   <212> DNA
   <213> Artificial
<220>
   <223> CR6343 Heavy chain
<220>
   <221> CDS
   <222> (1)..(1350)
<400> 364
   cag gtc cag ctg gtg cag tct gga gct gag gtg aag aag cct ggg tcc       48
   Gln Val Gln Leu Val Gln Ser Gly Ala Glu Val Lys Lys Pro Gly Ser
   1               5                   10                  15
   tcg gtg aag gtc tcc tgc aag gct tct gga gtc acc ttc agt tac tat       96
   Ser Val Lys Val Ser Cys Lys Ala Ser Gly Val Thr Phe Ser Tyr Tyr
               20                  25                  30
   gct atg agc tgg gtg cga cag gcc cct gga caa ggg ctt gag tgg atg      144
   Ala Met Ser Trp Val Arg Gln Ala Pro Gly Gln Gly Leu Glu Trp Met
           35                  40                  45
   gga gga atc agc cct atg ttt ggg aca aca acc tac gca cag aag ttc      192
   Gly Gly Ile Ser Pro Met Phe Gly Thr Thr Thr Tyr Ala Gln Lys Phe
       50                  55                  60
   cag ggc aga gtc acg att act gcg gac gac tcc acg agt aca gcc tac      240
   Gln Gly Arg Val Thr Ile Thr Ala Asp Asp Ser Thr Ser Thr Ala Tyr
   65                  70                  75                  80
   atg gag gtg agg agc ctg aga tct gag gac acg gcc gtg tat tac tgt      288
   Met Glu Val Arg Ser Leu Arg Ser Glu Asp Thr Ala Val Tyr Tyr Cys
                   85                  90                  95
   gcg aga tct tcg aat tac tat gat agt gta tat gac tac tgg ggc cag      336
   Ala Arg Ser Ser Asn Tyr Tyr Asp Ser Val Tyr Asp Tyr Trp Gly Gln
                   100                 105                 110
   gga acc ctg gtc acc gtc tcg agt gct agc acc aag ggc ccc agc gtg      384
   Gly Thr Leu Val Thr Val Ser Ser Ala Ser Thr Lys Gly Pro Ser Val
               115                 120                 125
   ttc ccc ctg gcc ccc agc agc aag agc acc agc ggc ggc aca gcc gcc      432
   Phe Pro Leu Ala Pro Ser Ser Lys Ser Thr Ser Gly Gly Thr Ala Ala
       130                 135                 140
   ctg ggc tgc ctg gtg aag gac tac ttc ccc gag ccc gtg acc gtg agc      480
   Leu Gly Cys Leu Val Lys Asp Tyr Phe Pro Glu Pro Val Thr Val Ser
   145                 150                 155                 160
   tgg aac agc ggc gcc ttg acc agc ggc gtg cac acc ttc ccc gcc gtg      528
   Trp Asn Ser Gly Ala Leu Thr Ser Gly Val His Thr Phe Pro Ala Val
                   165                 170                 175
   ctg cag agc agc ggc ctg tac agc ctg agc agc gtg gtg acc gtg ccc      576
   Leu Gln Ser Ser Gly Leu Tyr Ser Leu Ser Ser Val Val Thr Val Pro
               180                 185                 190
   agc agc agc ctg ggc acc cag acc tac atc tgc aac gtg aac cac aag      624
   Ser Ser Ser Leu Gly Thr Gln Thr Tyr Ile Cys Asn Val Asn His Lys
           195                 200                 205
   ccc agc aac acc aag gtg gac aaa cgc gtg gag ccc aag agc tgc gac      672
   Pro Ser Asn Thr Lys Val Asp Lys Arg Val Glu Pro Lys Ser Cys Asp
       210                 215                 220
   aag acc cac acc tgc ccc ccc tgc cct gcc ccc gag ctg ctg ggc gga      720
   Lys Thr His Thr Cys Pro Pro Cys Pro Ala Pro Glu Leu Leu Gly Gly
   225                 230                 235                 240
   ccc tcc gtg ttc ctg ttc ccc ccc aag ccc aag gac acc ctc atg atc      768
   Pro Ser Val Phe Leu Phe Pro Pro Lys Pro Lys Asp Thr Leu Met Ile
                   245                 250                 255
   agc cgg acc ccc gag gtg acc tgc gtg gtg gtg gac gtg agc cac gag      816
   Ser Arg Thr Pro Glu Val Thr Cys Val Val Val Asp Val Ser His Glu
               260                 265                 270
   gac ccc gag gtg aag ttc aac tgg tac gtg gac ggc gtg gag gtg cac      864
   Asp Pro Glu Val Lys Phe Asn Trp Tyr Val Asp Gly Val Glu Val His
           275                 280                 285
   aac gcc aag acc aag ccc cgg gag gag cag tac aac agc acc tac cgg      912
   Asn Ala Lys Thr Lys Pro Arg Glu Glu Gln Tyr Asn Ser Thr Tyr Arg
       290                 295                 300
   gtg gtg agc gtg ctc acc gtg ctg cac cag gac tgg ctg aac ggc aag      960
   Val Val Ser Val Leu Thr Val Leu His Gln Asp Trp Leu Asn Gly Lys
   305                 310                 315                 320
   gag tac aag tgc aag gtg agc aac aag gcc ctg cct gcc ccc atc gag     1008
   Glu Tyr Lys Cys Lys Val Ser Asn Lys Ala Leu Pro Ala Pro Ile Glu
                   325                 330                 335
   aag acc atc agc aag gcc aag ggc cag ccc cgg gag ccc cag gtg tac     1056
   Lys Thr Ile Ser Lys Ala Lys Gly Gln Pro Arg Glu Pro Gln Val Tyr
               340                 345                 350
   acc ctg ccc ccc agc cgg gag gag atg acc aag aac cag gtg tcc ctc     1104
   Thr Leu Pro Pro Ser Arg Glu Glu Met Thr Lys Asn Gln Val Ser Leu
           355                 360                 365
   acc tgt ctg gtg aag ggc ttc tac ccc agc gac atc gcc gtg gag tgg      1152
   Thr Cys Leu Val Lys Gly Phe Tyr Pro Ser Asp Ile Ala Val Glu Trp
       370                 375                 380
   gag agc aac ggc cag ccc gag aac aac tac aag acc acc ccc cct gtg      1200
   Glu Ser Asn Gly Gln Pro Glu Asn Asn Tyr Lys Thr Thr Pro Pro Val
   385                 390                 395                 400
   ctg gac agc gac ggc agc ttc ttc ctg tac agc aag ctc acc gtg gac      1248
   Leu Asp Ser Asp Gly Ser Phe Phe Leu Tyr Ser Lys Leu Thr Val Asp
                   405                 410                 415
   aag agc cgg tgg cag cag ggc aac gtg ttc agc tgc agc gtg atg cac      1296
   Lys Ser Arg Trp Gln Gln Gly Asn Val Phe Ser Cys Ser Val Met His
               420                 425                 430
   gag gcc ctg cac aac cac tac acc cag aag agc ctg agc ctg agc ccc      1344
   Glu Ala Leu His Asn His Tyr Thr Gln Lys Ser Leu Ser Leu Ser Pro
           435                 440                 445
   ggc aag                                                              1350
   Gly Lys
       450
<210> 365
   <211> 450
   <212> PRT
   <213> Artificial
<220>
   <223> Synthetic Construct
<400> 365
   Gln Val Gln Leu Val Gln Ser Gly Ala Glu Val Lys Lys Pro Gly Ser
   1               5                   10                  15
   Ser Val Lys Val Ser Cys Lys Ala Ser Gly Val Thr Phe Ser Tyr Tyr
               20                  25                  30
   Ala Met Ser Trp Val Arg Gln Ala Pro Gly Gln Gly Leu Glu Trp Met
           35                  40                  45
   Gly Gly Ile Ser Pro Met Phe Gly Thr Thr Thr Tyr Ala Gln Lys Phe
       50                  55                  60
   Gln Gly Arg Val Thr Ile Thr Ala Asp Asp Ser Thr Ser Thr Ala Tyr
   65                  70                  75                  80
   Met Glu Val Arg Ser Leu Arg Ser Glu Asp Thr Ala Val Tyr Tyr Cys
                   85                  90                  95
   Ala Arg Ser Ser Asn Tyr Tyr Asp Ser Val Tyr Asp Tyr Trp Gly Gln
               100                 105                 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Glu Glu Met Thr Lys Asn Gln Val Ser Leu
   355 360 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 366
   <211> 654
   <212> DNA
   <213> Artificial
<220>
   <223> CR6343 Light chain
<220>
   <221> CDS
   <222> (1)..(654)
<400> 366
   cag tct gtc gtg acg cag ccg ccc tcg gag tca gtg gcc cca gga cag        48
   Gln Ser Val Val Thr Gln Pro Pro Ser Glu Ser Val Ala Pro Gly Gln
   1               5                   10                  15
   acg gcc agg att acc tgt ggg gga cat aac att gga agt aat agt gtg        96
   Thr Ala Arg Ile Thr Cys Gly Gly His Asn Ile Gly Ser Asn Ser Val
               20                  25                  30
   cac tgg tac cag cag aag cca ggc cag gcc cct gtg ctg gtc gtg tat       144
   His Trp Tyr Gln Gln Lys Pro Gly Gln Ala Pro Val Leu Val Val Tyr
           35                  40                  45
   gat aat agc gac cgg ccc tca ggg atc cct gag cga ttc tct ggc tcc       192
   Asp Asn Ser Asp Arg Pro Ser Gly Ile Pro Glu Arg Phe Ser Gly Ser
       50                  55                  60
   aac tct ggg aac acg gcc acc ctg acc atc agc agg gtc gaa gcc ggg       240
   Asn Ser Gly Asn Thr Ala Thr Leu Thr Ile Ser Arg Val Glu Ala Gly
   65                  70                  75                  80
   gat gag gcc gac tat tac tgt cag gtg tgg ggt agt agt agt gac cat       288
   Asp Glu Ala Asp Tyr Tyr Cys Gln Val Trp Gly Ser Ser Ser Asp His
                   85                  90                  95
   tat gtc ttc gga act ggg acc aag gtc acc gtc cta ggt gcg gcc gca       336
   Tyr Val Phe Gly Thr Gly Thr Lys Val Thr Val Leu Gly Ala Ala Ala
               100                 105                 110
   ggc cag ccc aag gcc gct ccc agc gtg acc ctg ttc ccc ccc tcc tcc       384
   Gly Gln Pro Lys Ala Ala Pro Ser Val Thr Leu Phe Pro Pro Ser Ser
           115                 120                 125
   gag gag ctg cag gcc aac aag gcc acc ctg gtg tgc ctc atc agc gac       432
   Glu Glu Leu Gln Ala Asn Lys Ala Thr Leu Val Cys Leu Ile Ser Asp
       130                 135                 140
   ttc tac cct ggc gcc gtg acc gtg gcc tgg aag gcc gac agc agc ccc       480
   Phe Tyr Pro Gly Ala Val Thr Val Ala Trp Lys Ala Asp Ser Ser Pro
   145                 150                 155                 160
   gtg aag gcc ggc gtg gag acc acc acc ccc agc aag cag agc aac aac       528
   Val Lys Ala Gly Val Glu Thr Thr Thr Pro Ser Lys Gln Ser Asn Asn
                   165                 170                 175
   aag tac gcc gcc agc agc tac ctg agc ctc acc ccc gag cag tgg aag       576
   Lys Tyr Ala Ala Ser Ser Tyr Leu Ser Leu Thr Pro Glu Gln Trp Lys
               180                 185                 190
   agc cac cgg agc tac agc tgc cag gtg acc cac gag ggc agc acc gtg       624
   Ser His Arg Ser Tyr Ser Cys Gln Val Thr His Glu Gly Ser Thr Val
           195                 200                 205
   gag aag acc gtg gcc ccc acc gag tgc agc                               654
   Glu Lys Thr Val Ala Pro Thr Glu Cys Ser
       210                 215
<210> 367
   <211> 218
   <212> PRT
   <213> Artificial
<220>
   <223> Synthetic Construct
<400> 367
   Gln Ser Val Val Thr Gln Pro Pro Ser Glu Ser Val Ala Pro Gly Gln
   1               5                   10                  15
   Thr Ala Arg Ile Thr Cys Gly Gly His Asn Ile Gly Ser Asn Ser Val
               20                  25                  30
   His Trp Tyr Gln Gln Lys Pro Gly Gln Ala Pro Val Leu Val Val Tyr
           35                  40                  45
   Asp Asn Ser Asp Arg Pro Ser Gly Ile Pro Glu Arg Phe Ser Gly Ser
       50                  55                  60
   Asn Ser Gly Asn Thr Ala Thr Leu Thr Ile Ser Arg Val Glu Ala Gly
   65                  70                  75                  80
   Asp Glu Ala Asp Tyr Tyr Cys Gln Val Trp Gly Ser Ser Ser Asp His
                   85                  90                  95
   Tyr Val Phe Gly Thr Gly Thr Lys Val Thr Val Leu Gly Ala Ala Ala
               100                 105                 110
   Gly Gln Pro Lys Ala Ala Pro Ser Val Thr Leu Phe Pro Pro Ser Ser
           115                 120                 125
   Glu Glu Leu Gln Ala Asn Lys Ala Thr Leu Val Cys Leu Ile Ser Asp
       130                 135                 140
   Phe Tyr Pro Gly Ala Val Thr Val Ala Trp Lys Ala Asp Ser Ser Pro
   145                 150                 155                 160
   Val Lys Ala Gly Val Glu Thr Thr Thr Pro Ser Lys Gln Ser Asn Asn
                   165                 170                 175
   Lys Tyr Ala Ala Ser Ser Tyr Leu Ser Leu Thr Pro Glu Gln Trp Lys
               180                 185                 190
   Ser His Arg Ser Tyr Ser Cys Gln Val Thr His Glu Gly Ser Thr Val
           195                 200                 205
   Glu Lys Thr Val Ala Pro Thr Glu Cys Ser
       210                 215
<210> 368
   <211> 12
   <212> PRT
   <213> Influenza A virus
<400> 368
   Gly Val Thr Asn Lys Val Asn Ser Ile Ile Asp Lys
   1               5                   10
<210> 369
   <211> 12
   <212> PRT
   <213> Influenza A virus
<400> 369
   Gly Val Thr Asn Lys Val Asn Ser Ile Ile Asn Lys
   1               5                   10
<210> 370
   <211> 12
   <212> PRT
   <213> Influenza A virus
<400> 370
   Gly Val Thr Asn Lys Glu Asn Ser Ile Ile Asp Lys
   1               5                   10
<210> 371
   <211> 12
   <212> PRT
   <213> Influenza A virus
<400> 371
   Gly Val Thr Asn Lys Val Asn Arg Ile Ile Asp Lys
   1               5                   10
<210> 372
   <211> 12
   <212> PRT
   <213> Influenza A virus
<400> 372
   Gly Ile Thr Asn Lys Val Asn Ser Val Ile Glu Lys
   1               5                   10
<210> 373
   <211> 12
   <212> PRT
   <213> Influenza A virus
<400> 373
   Gly Ile Thr Asn Lys Glu Asn Ser Val Ile Glu Lys
   1               5                   10
<210> 374
   <211> 12
   <212> PRT
   <213> Influenza A virus
<400> 374
   Gly Ile Thr Asn Lys Val Asn Ser Ile Ile Asp Lys
   1               5                   10
<210> 375
   <211> 12
   <212> PRT
   <213> Influenza A virus
<400> 375
   Lys Ile Thr Ser Lys Val Asn Asn Ile Val Asp Lys
   1               5                   10
<210> 376
   <211> 12
   <212> PRT
   <213> Influenza A virus
<400> 376
   Gln Ile Asn Gly Lys Leu Asn Arg Val Ile Glu Lys
   1               5                   10
<210> 377
   <211> 12
   <212> PRT
   <213> Influenza A virus
<400> 377
   Gln Ile Asn Gly Lys Leu Asn Arg Leu Ile Glu Lys
   1               5                   10